# EUROPEAN PATENT SPECIFICATION

(11) **EP 1 651 673 B1**
(45) Date of publication and mention of the grant of the patent: **16.04.2008**
(21) Application number: 04743435.2
(22) Date of filing: 16.07.2004
(51) Int. Cl.: C07K 14/82, C12N 9/12

(54) **METHODS FOR USE OF AN LKB1/STRAD/MO25 COMPLEX**
VERFAHREN ZUR ANWENDUNG EINES AN LKB1/STRAD/MO25-KOMPLEXES
PROCEDES D'UTLISATION D'UN LKB1/STRAD/M025 COMPLEX

(30) Priority: 17.07.2003 GB 0316725; 20.12.2003 GB 0330078
(43) Date of publication of application: 03.05.2006
(73) Proprietor: University of Dundee, Dundee DD1 4HN (GB)
(72) Inventor: HARDIE, David, Grahame, Newport on Tay, Fife DD6 8JG (GB); ALESSi, Dario, Dundee DD2 1LG (GB); BOUDEAU, Jerome, Dundee DD1 4NQ (GB)
(74) Representative: Miles, John Stephen
(86) International application number: PCT/GB2004/003096
(87) International publication number: WO 2005/010174

(56) References cited:
- WO-A1-20/04113562
- HAWLEY S A ET AL: "Characterization of the AMP-activated protein kinase kinase from rat liver and identification of threonine 172 as the major site at which it phosphorylates AMP-activated protein kinase." THE JOURNAL OF BIOLOGICAL CHEMISTRY. 1 NOV 1996, vol. 271, no. 44, 1 November 1996 (1996-11-01), pages 27879-27887, XP002315194 ISSN: 0021-9258
- DATABASE WPI Section Ch, Week 200222 Derwent Publications Ltd., London, GB; Class B04, AN 2002-171818 XP002315199 & WO 02/06520 A1 (CHUGAI RES INST MOLECULAR MEDICINE INC) 24 January 2002 (2002-01-24) -& WO 02/06520 A1 (CHUGAI RES INST MOLECULAR MEDICINE INC) 24 January 2002 (2002-01-24)
- BAAS A F ET AL: "Activation of the tumour suppressor kinase LKB1 by the STE20-like pseudokinase STRAD" EMBO JOURNAL, OXFORD UNIVERSITY PRESS, SURREY, GB, vol. 22, no. 12, 16 June 2003 (2003-06-16), pages 3062-3072, XP002298130 ISSN: 0261-4189
- BOUDEAU J ET AL: "LKB1, a protein kinase regulating cell proliferation and polarity" FEBS LETTERS, ELSEVIER SCIENCE PUBLISHERS, AMSTERDAM, NL, vol. 546, no. 1, 3 July 2003 (2003-07-03), pages 159-165, XP004433636 ISSN: 0014-5793
- NATH NANDITA ET AL: "Yeast Pak1 kinase associates with and activates Snf1." MOLECULAR AND CELLULAR BIOLOGY. JUN 2003, vol. 23, no. 11, June 2003 (2003-06), pages 3909-3917, XP002315195 ISSN: 0270-7306
- BEAULOYE^A^ ^B C ET AL: "Insulin antagonizes AMP-activated protein kinase activation by ischemia or anoxia in rat hearts, without affecting total adenine nucleotides" FEBS LETTERS, ELSEVIER SCIENCE PUBLISHERS, AMSTERDAM, NL, vol. 505, no. 3, 21 September 2001 (2001-09-21), pages 348-352, XP004309604 ISSN: 0014-5793
- HAWLEY SIMON A ET AL: "Complexes between the LKB1 tumor suppressor, STRAD alpha/beta and MO25 alpha/beta are upstream kinases in the AMP-activated protein kinase cascade." JOURNAL OF BIOLOGY (ONLINE) 2003, vol. 2, no. 4, 24 September 2003 (2003-09-24), page 28, XP002298131 ISSN: 1475-4924
- SHAW R J ET AL: "The tumor suppressor LKB1 kinase directly activates AMP-activated kinase and regulates apoptosis in response to energy stress" PROCEEDINGS OF THE NATIONAL ACADEMY OF SCIENCES OF USA, NATIONAL ACADEMY OF SCIENCE. WASHINGTON, US, vol. 101, no. 10, 9 March 2004 (2004-03-09), pages 3329-3335, XP002298134 ISSN: 0027-8424
- BOUDEAU J¹RÔME ET AL: "MO25alpha/beta interact with STRADalpha/beta enhancing their ability to bind, activate and localize LKB1 in the cytoplasm." THE EMBO JOURNAL. 1 OCT 2003, vol. 22, no. 19, 1 October 2003 (2003-10-01), pages 5102-5114, XP002315196 ISSN: 0261-4189
- HONG SEUNG-PYO ET AL: "Activation of yeast Snf1 and mammalian AMP-activated protein kinase by upstream kinases." PROCEEDINGS OF THE NATIONAL ACADEMY OF SCIENCES OF THE UNITED STATES OF AMERICA. 22 JUL 2003, vol. 100, no. 15, 22 July 2003 (2003-07-22), pages 8839-8843, XP002315197 ISSN: 0027-8424
- SUTHERLAND CATHERINE M ET AL: "Elm1p is one of three upstream kinases for the Saccharomyces cerevisiae SNF1 complex." CURRENT BIOLOGY : CB. 5 AUG 2003, vol. 13, no. 15, 5 August 2003 (2003-08-05), pages 1299-1305, XP002315198 ISSN: 0960-9822
- WOODS A ET AL: "LKB1 is the upstream kinase in the AMP-activated protein kinase cascade" CURRENT BIOLOGY, CURRENT SCIENCE,, GB, vol. 13, no. 22, 11 November 2003 (2003-11-11), pages 2004-2008, XP002298132 ISSN: 0960-9822
- LIZCANO J M ET AL: "LKB1 is a master kinase that activates 13 kinases of the AMPK subfamily, including MARK/PAR-1" EMBO JOURNAL, OXFORD UNIVERSITY PRESS, SURREY, GB, vol. 23, no. 4, 25 February 2004 (2004-02-25), pages 833-843, XP002298133 ISSN: 0261-4189
- HARDIE D G: "THE AMP-ACTIVATED PROTEIN KINASE PATHWAY - NEW PLAYERS UPSTEAM AND DOWNSTREAM" JOURNAL OF CELL SCIENCE, CAMBRIDGE UNIVERSITY PRESS, LONDON, GB, vol. 117, no. PART 23, 2004, pages 5479-5487, XP008040901 ISSN: 0021-9533

## Description

The present invention relates to protein kinases and their regulation.

LKB1 is a Ser/Thr protein kinase not closely related to other protein kinases (reviewed in (Yoo et al., 2002)). Mutations in LKB1 have been linked to Peutz Jeghers Cancer Syndrome (PJS), an autosomal dominant inherited disorder (Hemminki et al., 1998; Jenne et al., 1998) characterised in those affected by the development multiple gastro-intestinal hamartomatous polyps as well as a wide spectrum of benign and malignant tumours (Hemminki, 1999), with a 15-fold increased risk of developing malignant cancers in many tissues [20,21]. To date, nearly 100 different mutations in the LKB 1 gene have been identified in PJS patients, most of which would be expected to impair the activity of LKB 1 [22]. Deletion of both alleles of the LKB 1 gene in mice results in embryonic lethality at midgestation arising from multiple defects (Ylikorkala et al., 2001), indicating that LKB1 plays an essential role in development. Importantly LKB1 heterozygous mice are viable but develop a PJS-like disease characterised by gastro-intestinal hamartomas, although it is controversial whether these are caused by haploinsufficiency or loss of heterozygosity (reviewed in [22]). Later on in life (for example over 50 weeks of age) these animals also develop malignant tumours, such as hepatocellular carcinoma which were shown to be associated with loss of LKB 1 expression (Bardeesy et al., 2002; Jishage et al., 2002; Miyoshi et al., 2002; Nakau et al., 2002; Rossi et al., 2002). Overexpression of LKB1 in certain cancer cells results in growth suppression due to a G1-cell cycle arrest which is dependent on LKB1 catalytic activity (Tiainen et al., 1999) and this has been proposed to be mediated through activation of p21^{WAF1/CIP1} through a p53-dependent mechanism (Tiainen et al., 2002). Taken together, these findings strongly indicate that LKB1 is likely to function as a tumour suppressor. Recent work has indicated that the *C. elegans* (Watts et al., 2000), *Drosophila* (Martin and St Johnston, 2003) and *Xenopus* (Ossipova et al., 2003) homologues of LKB1 regulate cell polarity and, if this function is conserved in humans, loss of cell polarity in PJS patients could account for the development of hamartomas. Although LKB1 is essentially nuclear when overexpressed in cells, a low level of cytosolic localisation is also frequently observed (Karuman et al., 2001; Smith et al., 1999; Tiainen et al., 1999). Significantly, mutants of LKB 1 that are excluded from the nucleus retain full growth suppression activity, suggesting that the cytoplasmic localisation of this enzyme is important for its tumour suppressor function (Tiainen et al., 2002). Several mutant forms of LKB 1 found in PJS patients localise only in the nucleus and are not detectable in the cytoplasm (Boudeau et al., 2003b; Tiainen et al., 2002), further suggesting that cytoplasmic localisation of LKB1 is important.

Relatively little is known about how LKB1 is regulated and how it functions. LKB1 is phosphorylated at multiple residues in vivo and mutation of certain phosphorylation sites hinders the ability of LKB1 to suppress cell growth, but the mechanisms by which these phosphorylation events regulate LKB1 function have not yet been defined (Collins et al., 2000; Sapkota et al., 2002a; Sapkota et al., 2002b; Sapkota et al., 2001). There has also been considerable interest in identifying LKB1-interacting proteins, which may regulate its function. To date, a number of proteins have been shown to bind LKB1, namely the p53 tumour suppressor protein (Karuman et al., 2001), the brahma-related gene 1 protein (Brg1), which is a component of chromatin remodelling complexes (Marignani et al., 2001), a protein termed LKB 1 interacting protein-1 (LIP1) (Smith et al., 2001) and the kinase-specific chaperone complex consisting of Cdc37 and Hsp90 (Boudeau et al., 2003a). The binding of LKB 1 to p53 was proposed to be essential for mediating p53-dependent cell death (Karuman et al., 2001), whereas the binding of LKB1 to Brg1 was suggested to be required for Brg1-dependent cell growth arrest (Marignani et al., 2001). The interaction of LKB 1 with LIP 1 anchored LKB 1 in the cytoplasm (Smith et al., 2001), while the binding of LKB 1 to Cdc37 and Hsp90 stabilised the LKB 1 protein in cells (Boudeau et al., 2003a).

Recently, we have shown that LKB 1 is associated with a STE20-related pseudokinase termed STRADα (STe20-Related ADaptor protein-α), which lacks catalytic activity because key residues required for the function of nearly all protein kinases are missing (Baas et al., 2003). Moreover, LKB 1 binds STRADα through its catalytic domain and this interaction enhances LKB 1 *in vivo* activity as well as promoting LKB1 cytoplasmic localisation (Baas et al., 2003). LKB1 is no longer able to suppress cell growth in cells in which STRADα has been depleted using an siRNA approach, suggesting that the binding of LKB1 to STRADα plays an important role in mediating its tumour suppressor function. STRADα is also phosphorylated in vitro and in vivo by LKB1, making STRADα the first physiological substrate for LKB1 identified thus far (Baas et al., 2003).

AMP-activated protein kinase kinase (AMPKK) and AMP-activated protein kinase (AMPK) are the upstream and downstream components of a protein kinase cascade that acts as a sensor of cellular energy charge [1,2]. AMPK is activated by an elevation, in cellular 5'-AMP that always accompanies a fall in the ATP:ADP ratio due to the reaction catalysed by adenylate kinase (2ADP ↔ ATP + ADP). This occurs during metabolic stresses such as hypoxia, ischaemia, glucose deprivation and, in skeletal and cardiac muscle, during contraction or exercise [1-3]. This applies to both catalytic subunit isoforms of AMPK (AMPKα1 and AMPKα2). Once activated by stress, AMPK switches on glucose and fatty acid uptake and oxidative metabolism of these fuels to generate ATP, while switching off biosynthetic pathways that consume ATP. It achieves this metabolic switching both by direct phosphorylation of metabolic enzymes and by longer-term effects on gene expression [1,2]. AMPK is also activated by metformin, one of the most widely used diabetes drugs (though required at high doses ie >2g per day in adults), by an unknown mechanism that does not involve lowering of cellular ATP levels. The activation of AMPK by both ATP-depletion and phenformin requires phosphorylation of the AMPK catalytic (α) subunit at its T-loop residue (Thr 172 in AMPKα1) by an upstream kinase. While AMPK is best known for its effects on metabolism, recent work suggests that it also regulates cell growth and proliferation. Firstly, activation of the kinase inhibits protein synthesis by causing phosphorylation of elongation factor-2 [4], and by inhibiting phosphorylation of p70S6K by the mTOR pathway [5,6]. Secondly, it has both pro-apoptotic effects [7-9] and anti-apoptotic effects [10,11] in different cells. Thirdly, it reduces the level of the RNA binding protein HuR in the cytoplasm, decreasing the stability of mRNAs encoding proliferative genes such as cyclins A and B1 [12]. Fourthly, it inhibits proliferation of Hep G2 cells by stabilizing p53 [13].

In WO 02/06520 the specific inhibition of the Ras/MEK/ERK signal transduction pathway by LKB1 is described.

A method for assaying LKB 1 activity is described in WO 2004/113 562.

The regulation of LKB1 and its known activities are reviewed in Boudeau et al (FEBS Letters (2003) 546: 159-165).

We have previously partially purified from rat liver at upstream kinase, AMPKK, that activates AMPK by phosphorylation at Thr-172 within the activation loop of the kinase domain [14; Hawley et al (J. Biol. Chem. (1996) 271: 27879-87)].

However, we have been unable to purify enough material to obtain amino acid sequence and identify the activity as a defined gene product. We searched for kinases upstream of the *Saccharomyces cerevisiae* homologue of AMPK (the SNF1 complex), by assaying the ability of a collection of 119 yeast protein kinase-glutathione-S-transferase (GST) fusions to activate the mammalian and yeast kinases. This approach identified the Elml protein kinase as a potential upstream kinase [15]. By analysing protein kinases that interacted with Snf1 by two hybrid analysis, Schmidt and colleagues identified Pak1 as another potential upstream kinase in yeast [16]. Neither *elm1Δ* nor *pak1Δ* strains, in which the single kinase genes were deleted, had the same phenotype as a *snf1Δ* strain. However, Elm1 and Pak1 form a small sub-family with the Tos3 protein kinase, and a triple *elm1Δ pak1Δ tos3Δ* mutant strain had phenotype similar to *snf1Δ* that was restored to wild type by expression of any one of the three kinases [15]. This proved that these three protein kinases act as upstream kinases for the SNF1 complex in a partially redundant manner.

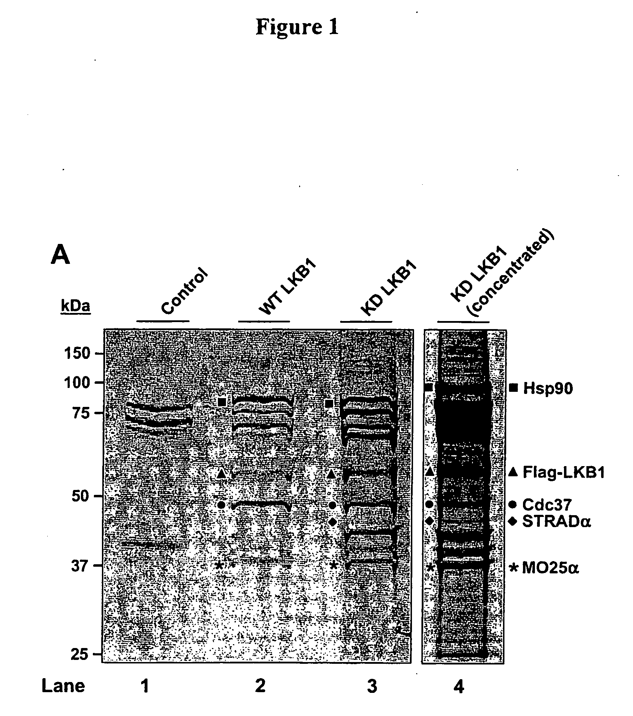

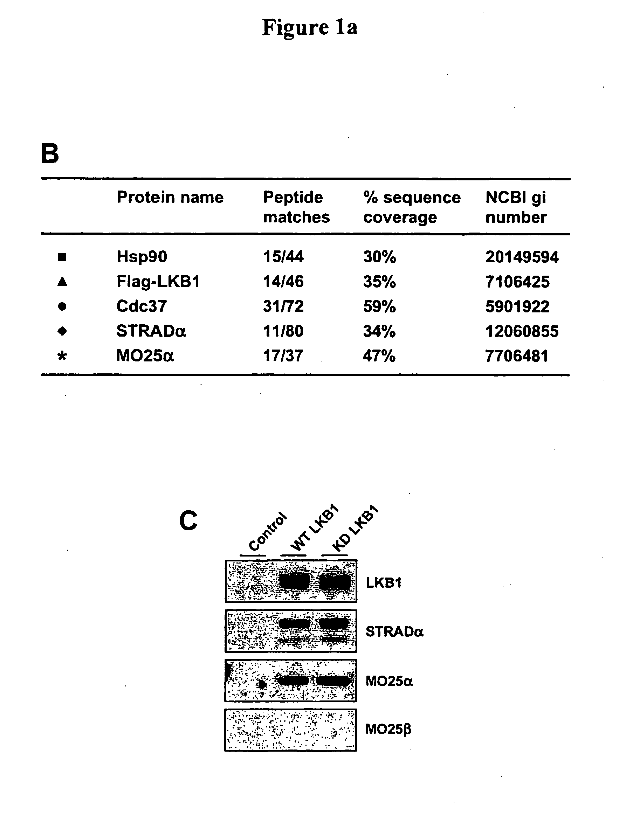

The nearest relatives of Elm1, Pak1 and Tos3 encoded by the human genome are the β isoform of calmodulin-dependent protein kinase kinase (CaMKKβ). We have previously shown that a CaMKK purified from pig brain could activate AMPK in cell-free assays, albeit rather poorly in comparison to the activation of calmodulin-dependent protein kinase I (CAMKI). However, the AMPKK previously purified from rat liver was not calmodulin-dependent [17]. Amongst other less closely related relatives to the Elml/Pakl/Tos3 family in humans is LKB 1 (see alignment in Fig. 1), a 50 kDa serine/threonine kinase that was originally discovered as the gene mutated in the inherited disorder Peutz-Jeghers syndrome (PJS) [18,19], as discussed above. Several human tumour cell lines, including HeLa and G361 (melanoma) cells, lack expression of LKB1 mRNA and protein, and expression of wild type LKB1 in these cells caused a growth arrest in G1 phase, whereas expression of catalytically inactive or several LKB1 mutations isolated from PJS mutations failed to arrest cell growth [23]. Growth arrest induced by expression of LKB1 in G361 cells was also reported to be reversed by co-expression of cyclin D 1 or E, was associated with the transcriptional induction of the cyclin-dependent kinase inhibitor p21^{WAF1/CIP1}, and was shown to be dependent on p53 [24]. Taken together, these results show that LKB1 acts as a tumour suppressor and that the catalytic activity of LKB1 is essential for this function. However, the downstream substrate(s) that LKB1 phosphorylates to mediate the suppression of cell growth remained unknown.

We identify MO25α (MOuse protein 25-α) as a novel component of the LKB1-STRADα complex and establish that MO25α plays an important role in stabilising this complex in the cell cytoplasm as well as enhancing LKB 1 catalytic activity. For each of STRADα and MO25α there is also a closely related isoform (STRADβ and MO25β) encoded in the human genome, which can also associate with LKB1. We demonstrate that MO25 may function as a scaffolding component of the LKB1-STRAD complex. Although STRADα [30] and STRADβ are related to the Ste20 protein kinases, several of the residues expected in an active protein kinase are not conserved, and they are therefore classified as "pseudokinases". MO25α (for example) binds to the C-terminus of, for example, STRADα and stabilizes the association between STRADα and LKB1. The association of LKB1 with STRAD and MO25 increases the kinase activity against an artificial substrate (myelin basic protein) and also enhances its cytoplasmic localization, which was previously implicated in the tumour suppressor function of LKB1, since mutants lacking a nuclear localisation signal were still capable of suppressing cell growth [24].

Further, we describe the resolution from rat liver of two upstream kinases (AMPKK1 and AMPKK2). We show that AMPKK1 and AMPKK2 both contain LKB1 complexed with STRADα and MO25α, and that both activities are immunoprecipitated with anti-LKB1 antibodies. Moreover, both endogenous and recombinant LKB1/STRAD/MO25 complexes isolated from HEK-293T cells activate AMPK via phosphorylation of Thr-172 on the α subunit. We demonstrate that the ability of LKB1 to phosphorylate and activate AMPK is enhanced dramatically by the presence of STRAD and M025 subunits, suggesting that both subunits are essential for maximal activity. Finally, drugs that activate AMPK in other cell types (AICA riboside and phenformin), did not activate the AMPK in HeLa cells, which do not express LKB 1. However, the stable expression of wild type LKB1 but not a catalytically-inactive mutant, restored activation of AMPK by these drugs. Accordingly, we show that the LKB1, in complex with STRAD and MO25, is the physiological activator of AMPK. It is now possible to perform screens for identifying compounds for modulating the physiological activation of AMPK.

A first aspect of the invention provides a method for identifying a compound for use in modulating, for example promoting, the activation or phosphorylation of AMPK (AMP-activated protein kinase) or AMPK subfamily member in a cell, the method comprising the steps of (1) determining whether a test compound modulates, for example promotes, the protein kinase activity of LKB1 and (2) selecting a compound which modulates, for example promotes, the protein kinase activity of LKB1, wherein the LKB1 is in a preparation with STRAD and MO25, wherein the MO25 is recombinant and is expressed from a recombinant nucleic acid.

The protein kinase activity of LKB1 that is modulated/assessed in the screening method may be phosphorylation of a non-physiological substrate such as myelin basic protein (for example as discussed in the Examples).

Alternatively, the protein kinase activity of LKB1 that is modulated/assessed in the screening method may be phosphorylation of AMPK or an AMPK subfamily member, or fragment either thereof, as discussed further. Phosphorylation of AMPK or an AMPK subfamily member may be assessed by measuring activation or phosphorylation of the AMPK or AMPK subfamily member, as discussed further below and in the Examples. For example, suitable peptide substrates for LKB1 are discussed in Example 3 and 4 and include the NUAK2 T-loop peptide (termed LKBtide). AMPK activation (for example) may be assessed using a reporter gene whose expression is modulated by AMPK. Other techniques and reagents that may be useful in measuring LKB protein kinase activity and/or phosphorylation or activation of AMPK or an AMPK subfamily member (or fragment thereof) are described in the Examples, for example in Example 4.

The protein kinase activity may be increased or reduced by an alteration in the Vₘₐₓ or the Kₘ (or both) of the LKB 1 (or AMKP or subfamily member, as appropriate) for a particular substrate. For example, activity may be increased by an increased Vₘₐₓ or decreased Kₘ. It will be appreciated that it may not be necessary to determine the value of either Vₘₐₓ or Kₘ in order to determine whether the LKB1 (or AMKP or subfamily member, as appropriate) has been activated or deactivated. It will be appreciated that dephosphorylated (deactivated) AMPK or subfamily member may retain some enzymatic activity.

Activity may be measured as the amount of a substrate phosphorylated in a given time; a change of activity may therefore be detected as a change in the amount of substrate (for example, at a single concentration) that is phosphorylated in a given time. It is preferred that the activity is increased or decreased, as appropriate, by at least 2, preferably 5, 10, 15, 20, 25, 30 or 50-fold.

It will be appreciated that it may be necessary to determine the effect of the compound on the activity of the substrate (for example AMPK), for example by measuring the activity of the substrate when exposed to the compound (1) after exposure of the substrate to LKB1, (2) before exposure of the substrate to LKB1 and/or (3) without exposure to LKB1.

Expression of a protein encoded by an RNA transcribed from a promoter regulated (directly or indirectly) by a substrate of LKB1 (or production of the RNA itself) may be measured. The protein may be one that is physiologically regulated by a substrate of LKB1, for example AMPK or subfamily member, or may be a "reporter" protein, as well known to those skilled in the art (ie a recombinant construct may be used). A reporter protein may be one whose activity may easily be assayed, for example β-galactosidase, chloramphenicol acetyltransferase or luciferase (see, for example, Tan *et al* (1996)).

AMPK activation, for example in the liver, decreases the expression of enzymes of gluconeogenesis (phosphenolpyruvate carboxylase and glucose-6-phosphatase); see Lochhead et al 5-aminoimidazole-4-carboxamide riboside mimics the effects of insulin on the expression of the 2 key gluconeogenic genes PEPCK and glucose-6-phosphatase.Diabetes. 2000 Jun;49(6):896-903; Yamauchi et al Adiponectin stimulates glucose utilization and fatty-acid oxidation by activating AMP-activated protein kinase. Nat Med. 2002 Nov;8(11):1288-95. Another class of genes that are switched off by AMPK, for example in the liver, are those encoding enzymes of fatty acid biosynthesis (fatty acid synthase and AcetylCoa carboxylase); see Woods et al Characterization of the role of AMP-activated protein kinase in the regulation of glucose-activated gene expression using constitutively active and dominant negative forms of the kinase. Mol Cell Biol. 2000 Sep;20(18):6704-11. AMPK activation also reduces the level of 4 transcription factors: Sterol response element binding protein-1C (Zhou et al Role of AMP-activated protein kinase in mechanism of metformin action. J Clin Invest. 2001 Oct;108(8):1167-74.); hepatocyte nuclear factor-4alpha (Leclerc et al Hepatocyte nuclear factor-4alpha involved in type 1 maturity-onset diabetes of the young is a novel target of AMP-activated protein kinase. Diabetes. 2001 Jul;50(7):1515-21); C/EBPalpha and PPAR-gamma (Habinowski et al The effects of AICAR on adipocyte differentiation of 3T3-L1 cells. Biochem Biophys Res Commun. 2001 Sep 7;286(5):852-6). Accordingly, such genes (or recombinant genes incorporating regulatory sequences from these genes) may be used as reporter genes.

By modulation of the protein kinase activity is included inhibition or an increase in the protein kinase activity.

It will be appreciated that in the methods of the invention wherein phosphorylation of a polypeptide may occur that the presence of a suitable phosphate donor may be required, as described for the above aspect of the invention. Suitable phosphate donors will be known to those skilled in the art and include ATP, for example as the magnesium salt (MgATP), as described in the Examples.

The LKB1 is in a preparation with STRAD and MO25. As noted above, LKB1 is considered to be present in a complex with these polypeptides in cells and the presence of these polypeptides is considered to enhance LKB1 activity. The LKB1 (and preferably also STRAD) may, for example, be purified from cells in which the LKB1 (and preferably also STRAD) are expressed naturally, but it may be more convenient for at least one of the LKB1, STRAD to be recombinant. The MO25 is recombinant and is expressed from a recombinant nucleic acid.

The term AMPK will be well known to those skilled in the art, as indicated above. The AMPK (or other substrate, for example myelin basic protein) used in the assay may be recombinant or non-recombinant. The AMPK may be a bacterially-expressed AMPKα1 catalytic domain (for example as described in [44]) or a heterotrimeric complex such as those described in [45] and in Example 2. The AMPK may have the amino acid sequence of a naturally occuring AMPK, or may be or comprise a fusion polypeptide (for example as described in [45] or Example 2), or may be a fragment or variant of a naturally occuring AMPK that retains the ability to be phosphorylated or activated by LKB1, for example as described in Example 2. Thus, it is preferred that the AMPK is an AMPK that retains a threonine (or serine) residue at the position equivalent to Threonine172 of full length wild-type human α1 catalytic domain. It is preferred that the AMPK is not a mutant in which Thr172 is replaced by a residue other than serine, for example is replaced by alanine. A fragment derivable from AMPK (or from an AMPK subfamily member) which encompasses the Thr172 residue and at least part of the T-loop sequence which includes this residue, for example at least the 2, 3, 4, 5, 6 or 7 residues C-terminal and N-terminal of this residue, is a suitable substrate for use in the screening method. An example of such a peptide substrate is described in Example 3.

Examples of Accession GI numbers for catalytic domain of human AMPK alpha 1 subunit include:
>gi|5453964|ref|NP_006242.1| LocusLink info protein kinase, AMP-activated, alpha 1 catalytic subunit; AMPK alpha 1; Protein kinase, AMP-activated, catalytic, alpha-1 [Homo sapiens]
gi|20178277|sp|Q13131|AAK1_HUMAN 5'-AMP-activated protein kinase, catalytic alpha-1 chain (AMPK alpha-1 chain)
gi|4115829|dbj|BAA36547.1| LocusLink info AMP-activated protein kinase alpha-1 [Homo sapiens]

Examples of Accession GI numbers for catalytic domain of human AMPK alpha 2 subunit include:
>gi|11493357|emb|CAC17574.1| dJ758N20.1 (protein kinase, AMP-activated, alpha 2 catalytic subunit) [Homo sapiens]
>gi|5453966|ref|NP_006243.1| LocusLink info protein kinase, AMP-activated, alpha 2 catalytic subunit; protein kinase, AMP-activated [Homo sapiens]

The term LKB 1 will similarly be well known to those skilled in the art. The LKB1 used in the assay may be recombinant or non-recombinant. The LKB 1 may be bacterially expressed, but it is preferred that it is expressed in a mammalian system and/or expressed alongside STRAD and/or MO25, preferably both, for example as described in Examples 1 and 2. The LKB 1 may have the amino acid sequence of a naturally occuring LKB1, or may be a fusion polypeptide (for example as described in the Examples), or may be a fragment or variant of a naturally occuring LKB 1 that retains the ability to phosphorylate or activate AMPK, for example on Thr172 of AMPK, for example as described in Example 2. Thus, the LKB1 is an LKB1 that retains an active kinase domain. A fragment of LKB 1 which contains the intact kinase domain but not other regions of LKB1 may be useful; this region of LKB 1 is sufficient to retain protein kinase activity and to interact with STRAD. The LKB1 used in the assay is not a kinase-dead mutant such as is described in the Examples. It is preferred that the LKB1 retains the ability to interact with STRAD and/or M025, as discussed further below.

Accession numbers for LKB 1 include the following:
AAC15742 Peutz-Jeghers syndrome protein [Homo sapiens] gi|3063585|gb|AAC15742.1|[3063585]
Q15831 Serine/threonine protein kinase 11 (Serine/threonine-protein kinase LKB1) gi|3024670|sp|Q15831|STKB_HUMAN[3024670]
NP_000446 serine/threonine protein kinase 11 [Homo sapiens] gi|4507271|ref|NP_000446.1|[4507271]

It is particularly preferred, although not essential, that the LKB1 polypeptide has at least 30% of the enzyme activity of full-length human LKB1 with respect to the phosphorylation of full-length human AMPKα1 on residue Thr172 (preferably in the presence of STRAD and MO25) or a peptide substrate encompassing this region, or phosphorylation of myelin basic protein. It is more preferred if the LKB1 polypeptide has at least 50%, preferably at least 70% and more preferably at least 90% of the enzyme activity of full-length human LKB1 with respect to the phosphorylation of full-length human AMPKα1 on residue Thr172 (preferably in the presence of STRAD and M025) or a peptide substrate encompassing this region.

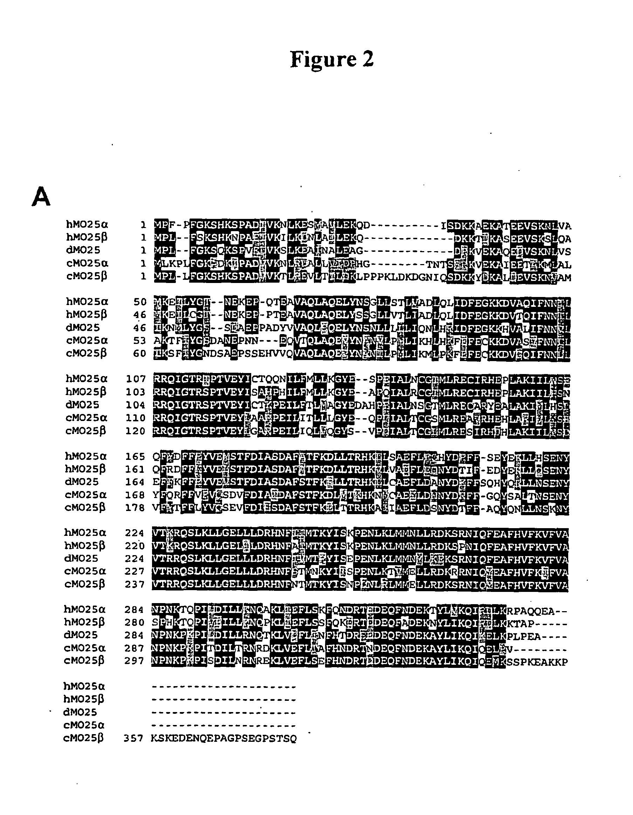

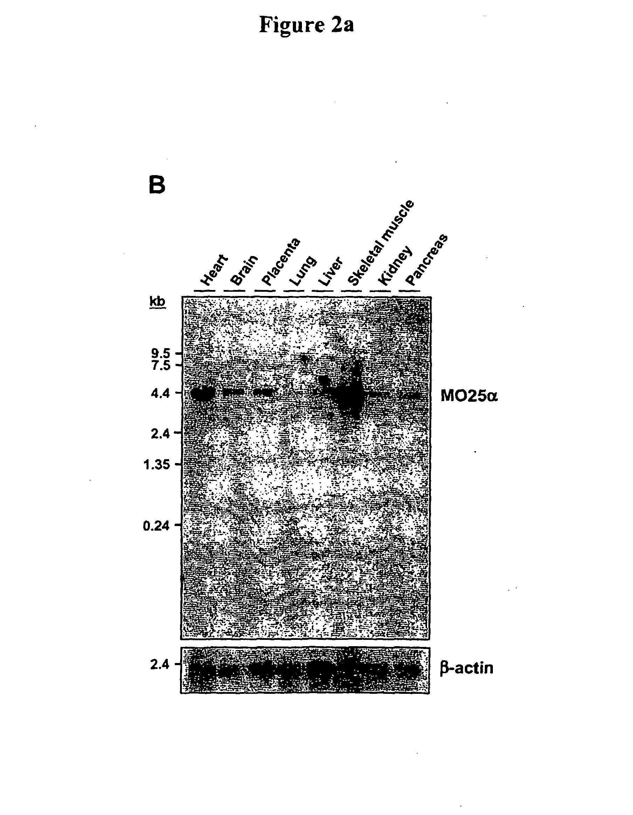

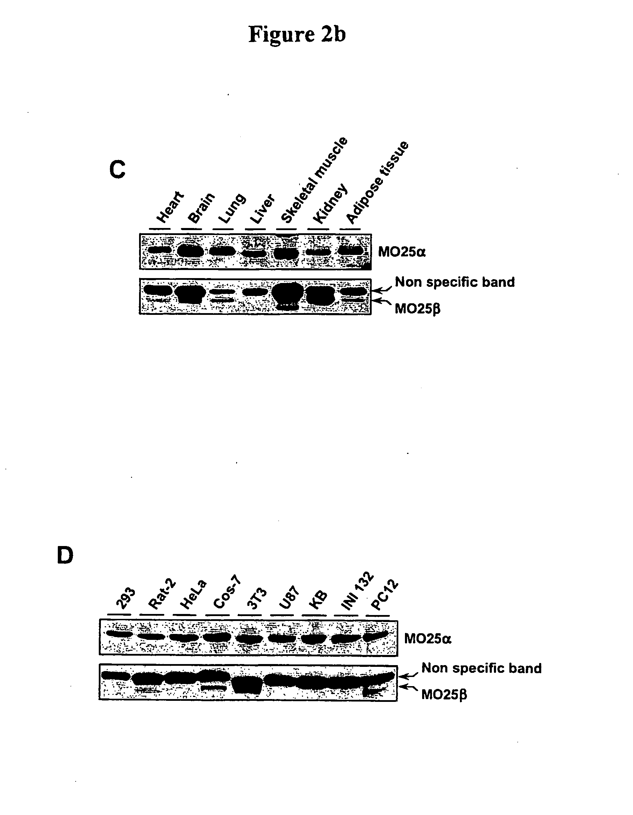

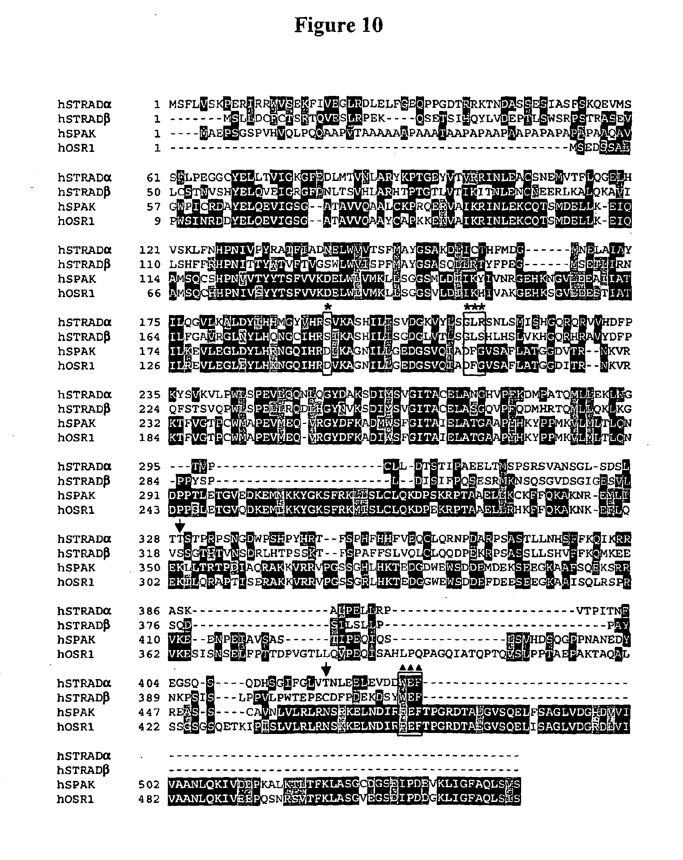

Similarly, the terms STRAD or M025 will similarly be well known to those skilled in the art. For example, a STRAD polypeptide (STRADα) is described in Baas et al (2003) EMBO J 22(12), 3062-3072 and in EMBL/GenBank Accession No AF308302. Human STRADα and STRADβ (NCBI accession number AAM19143) sequences are shown in Figure 10. M025 polypeptides are described in Miyamoto *et al* (1993); Karos & Fisher (1999) and Nozaki *et al* (1996) and, for example, in Accession No NP_057373 (human MO25α) and Q9H9S4 (human MO25β). Further examples of MO25 polypeptides are shown in Figure 2 and further Accession Nos are given in the figure legend. MO25 bears no sequence homology to other proteins in the database but recent studies indicate that it is structurally related to the Armadillo repeat domain (Milburn et al., 2003). The STRAD used in the assay may be recombinant or non-recombinant but the MO25 used in the assay is recombinant. The STRAD or MO25 may be bacterially expressed, but it is preferred that they are expressed in a mammalian system and/or expressed alongside LKB1, for example as described in Examples 1 and 2. The STRAD or MO25 may have the amino acid sequence of a naturally occuring STRAD or MO25, or may be a fusion polypeptide (for example as described in the Examples), or may be a fragment or variant of a naturally occuring STRAD or MO25 that retains the ability for the STRAD to bind to the M025 and to LKB1, and for the MO25 to bind to the STRAD or complex of LKB1 and STRAD. At least the C-terminal region of MO25 may be required. In view of the sequence conservation throughout the length of MO25 it is considered that it may be desirable to use full length MO25. It is preferred that the STRAD polypeptide has the C-terminal sequence Trp-Asp/Glu-Phe (which M025 is considered to recognise) but this is not considered to be essential, because MO25 can bind to STRAD lacking these residues when the STRAD is bound to LKB 1 (see Example 1). It is also preferred that the pseudokinase domain of STRAD is present, as this may be required for MO25 binding. For example, it is preferred that the STRAD is not a fragment lacking the residues corresponding to the C-terminal 88 amino acids of full length STRADα (which has a truncated pseudokinase domain and does not interact with human LKB 1 or MO25α). It may be desirable to use full length STRAD.

The capability of, for example, the said polypeptide with regard to interacting with or binding to, for example, a STRAD polypeptide, may be measured by any method of detecting/measuring a protein/protein interaction, as discussed further below. Suitable methods include yeast two-hybrid interactions, co-purification, ELISA, co-immunoprecipitation, cross-linking studies, structural studies, fluorescence resonance energy transfer (FRET) methods and surface plasmon resonance methods. Thus, the said MO25 (for example human MO25α) may be considered capable of binding to or interacting with a STRAD polypeptide (for example human STRADα) if an interaction may be detected between the said M025 polypeptide and the said STRAD polypeptide by ELISA, co-immunoprecipitation or surface plasmon resonance methods or by a yeast two-hybrid interaction or copurification method, for example as described in Example 1 or Example 2.

It is preferred that the interaction can be detected using a surface plasmon resonance method, as described in, for example WO 00/56864. One polypeptide may be immobilised on the test surface, for example it can be coupled through amino groups to a SensorChip CM5™, according to the manufacturer's instructions, or a biotinylated polypeptide can be bound to an avidin coated SensorChip SA. The other polypeptide (at concentrations between, for example 0 and between 10µM and 1.0µM, for example 2µM) is then injected over the surface and steady state binding determined in each case. From these measurements a K_{d} can be determined. It is preferred that the interaction has a K_{d} of less than 8µM, more preferably less than 5µM, 2µM, 1µM, 500nM, 300nM, 200nM or 100nM, for example about 150nM. Alternatively, a K_{d} can be determined for a polypeptide in competition with the immobilised polypeptide. One polypeptide (for example at a concentration of 0.5µM) is mixed with free polypeptide (for example, at concentrations between 0 and 3µM) and the mixture injected over the immobilised polypeptides. The steady state binding is determined in each case, from which the K_{d} of the interaction can be determined using the Cheng-Prescott relationship. Alternatively, the interaction may be expressed in terms of an observed response or relative observed responses, measured in terms of mass of protein bound to the surface.

By "variants" of a polypeptide we include insertions, deletions and substitutions, either conservative or non-conservative. In particular we include variants of the polypeptide where such changes do not substantially alter the protein kinase activity or ability to bind to particular binding partners, as appropriate.

By "conservative substitutions" is intended combinations such as Gly, Ala; Val, Ile, Leu; Asp, Glu; Asn, Gln; Ser, Thr; Lys, Arg; and Phe, Tyr.

The three-letter or one letter amino acid code of the IUPAC-IUB Biochemical Nomenclature Commission is used herein, with the exception of the symbol Zaa, defined above. In particular, Xaa represents any amino acid. It is preferred that at least the amino acids corresponding to the consensus sequences defined herein are L-amino acids.

It is particularly preferred if the polypeptide variant has an amino acid sequence which has at least 65% identity with the amino acid sequence of the relevant human polypeptide, more preferably at least 70%, 71%, 72%, 73% or 74%, still more preferably at least 75%, yet still more preferably at least 80%, in further preference at least 85%, in still further preference at least 90% and most preferably at least 95% or 97% identity with the amino acid sequence of the relevant human polypeptide.

It is still further preferred if a protein kinase variant has an amino acid sequence which has at least 65% identity with the amino acid sequence of the catalytic domain of the human polypeptide, more preferably at least 70%, 71%, 72%, 73% or 74%, still more preferably at least 75%, yet still more preferably at least 80%, in further preference at least 83 or 85%, in still further preference at least 90% and most preferably at least 95% or 97% identity with the relevant human amino acid sequence.

It will be appreciated that the catalytic domain of a protein kinase-related polypeptide may be readily identified by a person skilled in the art, for example using sequence comparisons as described below. Protein kinases show a conserved catalytic core, as reviewed in Johnson et al (1996) Cell, 85, 149-158 and Taylor & Radzio-Andzelm (1994) Structure 2, 345-355. This core folds into a small N-terminal lobe largely comprising anti-parallel β-sheet, and a large C-terminal lobe which is mostly α-helical.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson *et al.,* 1994). The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05.
Scoring matrix: BLOSUM.

It will be appreciated that the MO25 sequences shown in Figure 2 are examples of MO25 variants as defined above.

The residue equivalent to, for example, Thr 172 of full-length human AMPKα1 may be identified by alignment of the sequence of the polypeptide with that of full-length human AMPKα1 in such a way as to maximise the match between the sequences. The alignment may be carried out by visual inspection and/or by the use of suitable computer programs, for example the GAP program of the University of Wisconsin Genetic Computing Group, which will also allow the percent identity of the polypeptides to be calculated. The Align program (Pearson (1994) in: Methods in Molecular Biology, Computer Analysis of Sequence Data, Part II (Griffin, AM and Griffin, HG eds) pp 365-389, Humana Press, Clifton). Thus, residues identified in this manner are also "equivalent residues".

It will be appreciated that in the case of truncated forms of AMPKα1 or in forms where simple replacements of amino acids have occurred it is facile to identify the "equivalent residue".

It is preferred that the polypeptides used in the screen are mammalian, preferably human (or a species useful in agriculture or as a domesticated or companion animal, for example dog, cat, horse, cow), including naturally occurring allelic variants (including splice variants). The polypeptides used in the screen may comprise a GST portion or may be biotinylated or otherwise tagged, for example with a 6His, HA, myc or other epitope tag, as known to those skilled in the art, or as described in Examples 1 and 2. This may be useful in purifying and/or detecting the polypeptide(s).

The effect of the compound may be determined by comparing the rate or degree of phosphorylation of the substrate polypeptide by the LBK1 in the presence of different concentrations of the compound, for example in the absence and in the presence of the compound, for example at a concentration of about 100µM, 30µM, 10µM, 3µM, 1µM, 0.1µM, 0.01µM and/or 0.001µM.

The compound may mimic the effect of the interaction of STRAD and/or M025 with LKB1. A compound that mimics the effect of STRAD and/or M025 on LKB 1 may increase the rate or extent of phosphorylation of the substrate polypeptide (for example AMPK or a subfamily member or a fragment any thereof).

By "mimic the effect of the interaction of STRAD and/or MO25 with LKB1" is meant that the compound has a quantitative or qualitative effect on the LKB1, for example on its protein kinase activity or stability, that is the same as an effect of the STRAD or MO25 on the protein kinase, for example on its protein kinase activity or stability, as discussed in Examples 1 and 2. For example, STRAD and M025 are considered to increase the rate at which LKB1 phosphorylates, for example, AMPK or myelin basic protein; a mimic of STRAD or MO25 may increase the rate at which LKB 1 (in the absence of STRAD and/or M025) phosphorylates a substrate polypeptide, for example AMPK.

The AMPK subfamily is discussed in Example 2 (and see also [41]) and includes the following polypeptides:
MELK (Heyer et al., 1999 Expression of Melk, a new protein kinase, during early mouse development. Dev Dyn, 215, 344-351; Heyer et al., 1997 New member of theSnf1/AMPK kinase family, Melk, is expressed in the mouse egg and preimplantation embryo. Mol Reprod Dev, 47, 148-156.)
QIK (Xia et al., 2000 The new serine-threonine kinase, Qik, is a target of the Qin oncogene. Biochem Biophys ResCommun, 276, 564-570)
SIK (Coyle et al., 2003 Sam68 enhances the cytoplasmic utilization of intron-containing RNA and is functionally regulated by the nuclear kinase Sik/BRK. Mol Cell Biol, 23, 92-103; Derry et al., 2003 Altered localization and activity of the intracellular tyrosine kinase BRK/Sik in prostate tumor cells. Oncogene, 22, 4212-4220; Derry et al., 2000 Sik (BRK) phosphorylates Sam68 in the nucleus and negatively regulates its RNA binding ability. Mol Cell Biol, 20, 6114-6126; Feldman et al., 2000 The salt-inducible kinase, SIK, is induced by depolarization in brain. J Neurochem, 74, 2227-2238; Horike et al., 2002 Roles of several domains identified in the primary structure of salt-inducible kinase (SIK). Endocr Res, 28, 291-294.; Lin et al., 2000 SIK (Salt-inducible kinase): regulation of ACTH-mediated steroidogenic gene expression and nuclear/cytosol redistribution. Endocr Res, 26, 995-1002.; Llor et al., 1999 BRK/Sik expression in the gastrointestinal tract and in colon tumors. Clin Cancer Res, 5, 1767-1777.; Vasioukhin and Tyner, 1997 A role for the epithelial-cell-specific tyrosine kinase Sik during keratinocyte differentiation. Proc Natl Acad Sci U S A, 94, 14477-14482.; Wang et al., 1999 Cloning of a novel kinase (SIK) of the SNF1/AMPK family from high salt diet-treated rat adrenal. FEBS Lett, 453, 135-139)

Other family members include NuaK1, NuaK2, BrsK1, BrsK2 and QSK, about which little is known. We consider that LKB1 may phosphorylate and activate these other protein kinases in the AMPK sub-family.
Other family members (which lie on an immediately adjacent branch of the kinase tree to the family members indicated above) include MARK1, MARK2, MARK3 and MARK4. See, for example, Manning et al (2002) The protein kinase complement of the human genome Science 298, 1912-1934. MARK3 is also known as PARIA or C-TAK1 (Peng et al (1998) Cell Growth Differ 9, 197-208; Spicer et al (2003) Oncogene 22, 4752-4756.

Metformin or its analogues may be acting through activation of one of these kinases as well as through AMPK. Such a sub-family member or fragment thereof or fusion of such a fragment (for example a T-loop peptide, as discussed above and in Example 3) may be a suitable substrate for use in the screening method of the first aspect of the invention.

A compound identified by a method of the invention may modulate the ability of the protein kinase to phosphorylate different substrates, for example different naturally occuring members of the AMPK subfamily, to different extents. Thus, it is preferred, but not essential, that when screening for a compound for use in modulating AMPK activity that AMPK or a fragment thereof is used as the substrate. Similarly, it is preferred, but not essential, that when screening for a compound for use in modulating the activity of a particular AMPK subfamily member that that subfamily member or a fragment thereof is used as the substrate.

The method is useful in identifying compounds that, for example, promote the activation of AMPK or a sub-family member. A compound that triggers the activation of AMPK or an AMPK sub-family member may be useful in the treatment of diabetes and obesity. Such a compound may mimic the activity of metformin or its analogues and may be of higher potency.

LKB 1 is also a tumour suppressor protein kinase that inhibits cell growth and proliferation. The results presented herein indicate that LKB1's tumour suppressor functions may be mediated through LKB1 phosphorylating and triggering the activation of AMPK and/or one or more subfamily members indicated above. Thus, these polypeptides may be novel tumour suppressor protein kinases and a compound which activates one or more of these protein kinases may be useful for treating cancer.

A compound which promotes phosphorylation or activation of AMPK or an AMPK family member may act by stabilising the LKB1-STRAD-MO25 complex. It may be useful to screen for such a stabilising effect (either before or after or instead of selection on the basis of a protein kinase activity assay as described above). For example, such a screen could involve assessing the effects of a compound on complex formation or stability, for example using methods for assessing molecular interactions, as discussed above. For example, coimmunoprecipitation or copurification methods may be used, or a reporter gene/yeast two hybrid system may be used. A complex may also be detected using fluorescence resonance energy transfer (FRET) techniques (for example using fusion proteins comprising fluorescent proteins, for example green, blue or yellow fluorescent proteins (GFPs; YFPs, BFPs, as well known to those skilled in the art)), for example in material from cells in which the LKB1 and the STRAD and/or MO25 are coexpressed, as described in Examples 1 and 2.

The compound may be one which binds to or near a region of contact between two or more of LKB1, STRAD and MO25, or maybe one which binds to another region and, for example, induces a conformational or allosteric change which stabilises (or destabilises) the complex; or promotes (or inhibits) its formation. The compound may bind to LKB 1 or STRAD or MO25 (or to the complex as a whole) so as to increase the LKB1 protein kinase activity by an allosteric effect. This allosteric effect may be an allosteric effect that is involved in the natural regulation of LKB1's activity.

A further aspect of the invention provides a method for making a preparation comprising LKB1, STRAD and recombinant MO25. The preparation may comprise recombinant LKB1 and/or recombinant STRAD. The preparation may be useful in an assay of the first aspect of the invention.

By "purifed" is meant that the preparation has been at least partially separated from other components in the presence of which H has been formed, for example other components of a recombinant cell. Examples of methods of purification than may be used are described in the Examples.

The preparation may be substantially pure. By "substantially pure" we mean that the said polypeptide(s) are substantially free of other proteins. Thus, we include any composition that includes at least 30% of the protein content by weight as the said polypeptides, preferably at least 50%, more preferably at least 70%, still more preferably at least 90% and most preferably at least 95% of the protein content is the said polypeptides.

Thus, the invention also includes compositions comprising the said polypeptides and a contaminant wherein the contaminant comprises less than 70% of the composition by weight, preferably less than 50% of the composition, more preferably less than 30% of the composition, still more preferably less than 10% of the composition and most preferably less than 5% of the composition by weight.

The invention also includes the substantially pure said polypeptides when combined with other components *ex vivo,* said other components not being all of the components found in the cell in which said polypeptides are found.

A further aspect of the invention provides a cell capable of expressing LKB1, STRAD and overexpressed or recombinant M025. The cell may comprise a recombinant nucleic acid encoding M025, and/or a recombinant nucleic acid encoding LKB1, and/or a recombinant nucleic acid encoding STRAD. The cell may be capable of overexpressing MQ25 from the endogenous sequence encoding MO25, for example using techniques of sequence-specific targeting of transcription activators. The cell may be a prokaryotic or eukaryotic cell. For example it may be a eukaryotic cell, for example an insect, yeast or mammalian cell, for example a human cell. Examples of suitable cells are described, for example, in the Examples.

The recombinant nucleic acid is preferably suitable for expressing the encoded polypeptide. The recombinant nucleic acid may be in the form of an expression vector. Recombinant polynucleotides suitable for expressing a given polypeptide are well known to those skilled in the art, and examples are described in Examples 1 and 2.

A further aspect of the invention provides a cell comprising LKB1, STRAD and overexpressed or recombinant M025. The cell may comprise recombinant LKB1 and/or recombinant STRAD. The cell may be a cell according to the preceding aspect of the invention. The cell may comprise at least 1.1, 1.2, 1.5, 2, 3, 5, 10 or 20-fold more M025 (or LKB1, or STRAD, as appropriate) than an equivalent cell which has not been modified in order to overexpress M025 or to express recombinant M025.

By "suitable for expressing" is mean that the polynucleotide is a polynucleotide that may be translated to form the polypeptide, for example RNA, or that the polynucleotide (which is preferably DNA) encoding the polypeptide of the invention is inserted into an expression vector, such as a plasmid, in proper orientation and correct reading frame for expression. The polynucleotide may be linked to the appropriate transcriptional and translational regulatory control nucleotide sequences recognised by any desired host; such controls may be incorporated in the expression vector.

Characteristics of vectors suitable for replication in mammalian/eukaryotic cells are well known to those skilled in the art, and examples are given below. It will be appreciated that a vector may be suitable for replication in both prokaryotic and eukaryotic cells.

A variety of methods have been developed to operably link polynucleotides, especially DNA, to vectors for example *via* complementary cohesive termini. Suitable methods are described in Sambrook et al (1989) Molecular Cloning, A Laboratory Manual, Cold Spring Harbor Laboratory, Cold Spring Harbor, NY.

A desirable way to modify the DNA encoding a polypeptide of the invention is to use the polymerase chain reaction as disclosed by Saiki et al (1988) Science 239, 487-491. This method may be used for introducing the DNA into a suitable vector, for example by engineering in suitable restriction sites, or it may be used to modify the DNA in other useful ways as is known in the art.

In this method the DNA to be enzymatically amplified is flanked by two specific primers which themselves become incorporated into the amplified DNA. The said specific primers may contain restriction endonuclease recognition sites which can be used for cloning into expression vectors using methods known in the art.

The DNA (or in the case of retroviral vectors, RNA) is then expressed in a suitable host to produce a polypeptide comprising the compound of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al*,* 4,710,463 issued 1 December 1987 to Murray, 4,757,006 issued 12 July 1988 to Toole, Jr. et al*,* 4,766,075 issued 23 August 1988 to Goeddel et al and 4,810,648 issued 7 March 1989 to Stalker, all of which are incorporated herein by reference.

The DNA (or in the case of retroviral vectors, RNA) encoding the polypeptide may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example E. *coli* and *Bacillus subtilis),* yeasts (for example *Saccharomyces cerevisiae),* filamentous fungi (for example *Aspergillus),* plant cells, animal cells and insect cells.

The vectors include a prokaryotic replicon, such as the ColE1 *ori,* for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as E. *coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and p*Trc*99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps).

The host cell can be either prokaryotic or eukaryotic. Bacterial cells are preferred prokaryotic host cells and typically are a strain of *E. coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic cell line. Yeast host cells include YPH499, YPH500 and YPH501 which are generally available from Stratagene Cloning Systems, La Jolla, CA 92037, USA. Preferred mammalian host cells include human embryonic kidney 293 cells (see Example 1), Chinese hamster ovary (CHO) cells available from the ATCC as CCL61, NIH Swiss mouse embryo cells NIH/3T3 available from the ATCC as CRL 1658, and monkey kidney-derived COS-1 cells available from the ATCC as CRL 1650. Preferred insect cells are Sf9 cells which can be transfected with baculovirus expression vectors.

Transformation of appropriate cell hosts with a DNA construct is accomplished by well known methods that typically depend on the type of vector used. With regard to transformation of prokaryotic host cells, see, for example, Cohen et al (1972) Proc. Natl. Acad. Sci. USA 69, 2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming and/or transfecting cells and is well known in the art for transforming yeast cell, bacterial cells, insect cells and vertebrate cells.

For example, many bacterial species may be transformed by the methods described in Luchansky et al (1988) Mol. Microbiol. 2, 637-646 incorporated herein by reference. The greatest number of transformants is consistently recovered following electroporation of the DNA-cell mixture suspended in 2.5X PEB using 6250V per cm at 25:FD.

Methods for transformation of yeast by electroporation are disclosed in Becker & Guarente (1990) Methods Enzymol. 194, 182.

Successfully transformed cells, ie cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) J. Mol. Biol. 98, 503 or Berent et al (1985) Biotech. 3, 208.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium.

A further aspect of the invention method for making a preparation of the invention, comprising the step of purifying the preparation from a cell according to the invention.. Methods of cultivating host cells and isolating recombinant proteins are well known in the art. Examples of suitable purification techniques are described in the Examples. For example, one or more component of the preparation may be tagged so as to aid purification using affinity reagents, as will be well known to those skilled in the art and as described in the Examples. Chromatographic techniques may also be used, for example as described in the Examples.

The preparation of the preceding aspect of the invention may comprise, for example, tagged LKB1, STRAD or MO25. The ratios of LKB1:STRAD:MO25 may be 1:1:1, for example as measured using techniques as described in the Examples. It will be appreciated that the ratio determined may vary depending upon the details of the method used in providing the preparation. The preparation may comprise a complex comprising the LKB1, STRAD and MO25, as described in the Examples.

The method of the first aspect of the invention may be performed with LKB1 in the form of a preparation made by the method of the second aspect of the invention; or a preparation or complex obtained or obtainable by the method as indicated above; or in a cell of the invention.

The above polypeptides may be made by methods well known in the art and as described below and in Example 1 or 2, for example using molecular biology methods or automated chemical peptide synthesis methods.

It will be appreciated that peptidomimetic compounds may also be useful. Thus, by "polypeptide" or "peptide" we include not only molecules in which amino acid residues are joined by peptide (-CO-NH-) linkages but also molecules in which the peptide bond is reversed. Such retro-inverse peptidomimetics may be made using methods known in the art, for example such as those described in Mézière et al (1997) J. Immunol. 159, 3230-3237, incorporated herein by reference. This approach involves making pseudopeptides containing changes involving the backbone, and not the orientation of side chains. Mézière *et al* (1997) show that, at least for MHC class II and T helper cell responses, these pseudopeptides are useful. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the Cα atoms of the amino acid residues is used; it is particularly preferred if the linker moiety has substantially the same charge distribution and substantially the same planarity as a peptide bond.

It will be appreciated that the peptide may conveniently be blocked at its Nor C-terminus so as to help reduce susceptibility to exoproteolytic digestion.

Thus, it will be appreciated that the STRAD or M025 polypeptide may be a peptidomimetic compound.

A further aspect of the invention provides a method for identifying a compound for modulating cellular LKB1 activity, the method comprising the steps of (1) determining whether a test compound modulates (for example increases) the LKB1 protein kinase activity of a preparation or complex or cell as defined in relation to any preceding aspect of the invention and (2) selecting a compound which modulates the said LKB1 protein kinase activity. The LKB 1 protein kinase activity may b measured using, for example, myelin basic protein as a substrate, or using AMPK or AMPK subfamily member or a fragment thereof (as discussed above and in Example 3) as the substrate. A compound which modulates, for example increases LKB 1 protein kinase activity may be useful in medicine, for example for treating cancer.

The compounds identified in the methods may themselves be useful as a drug or they may represent lead compounds for the design and synthesis of more efficacious compounds.

The compound may be a drug-like compound or lead compound for the development of a drug-like compound for each of the above methods of identifying a compound. It will be appreciated that the said methods may be useful as screening assays in the development of pharmaceutical compounds or drugs, as well known to those skilled in the art.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons. A drug-like compound may additionally exhibit features of selective interaction with a particular protein or proteins and be bioavailable and/or able to penetrate cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, difficult to synthesise or has poor bioavailability) may provide a starting-point for the design of other compounds that may have more desirable characteristics.

It will be understood that it will be desirable to identify compounds that may modulate the activity of the protein kinase *in vivo.* Thus it will be understood that reagents and conditions used in the method may be chosen such that the interactions between, for example, the said LKB1 and, for example, the STRAD or MO25 polypeptides, are substantially the same as between the human LKB1 and human STRAD and/or MO25. It will be appreciated that the compound may bind to the LKB1, or may bind to the STRAD or M025.

The compounds that are tested in the screening methods of the assay or in other assays in which the ability of a compound to modulate the protein kinase activity of a protein kinase, for example LKB1, may be measured, may be compounds that have been selected and/or designed (including modified) using molecular modelling techniques, for example using computer techniques. The selected or designed compound may be synthesised (if not already synthesised) and tested for its effect on the LKLB1, for example its effect on the protein kinase activity. The compound may be tested in a screening method of the invention.

It will be appreciated that screening assays which are capable of high throughput operation will be particularly preferred. Examples may include the cell based assays described and protein-protein binding assays. A further example is an SPA-based (Scintillation Proximity Assay; Amersham International) system as well known to those skilled in the art. Other methods of detecting polypeptide/polypeptide interactions include ultrafiltration with ion spray mass spectroscopy/HPLC methods or other physical and analytical methods. Fluorescence Energy Resonance Transfer (FRET) methods, for example, well known to those skilled in the art, may be used, in which binding of two fluorescent labelled entities may be measured by measuring the interaction of the fluorescent labels when in close proximity to each other

A further aspect of the invention is a compound identified or identifiable by a screening method of the invention.

A still further aspect of the invention is a compound of the invention for use in medicine.

The compound may be administered in any suitable way, usually parenterally, for example intravenously, intraperitoneally, subcutaneous or intramuscular or intravesically, in standard sterile, non-pyrogenic formulations of diluents and carriers. The compound may also be administered topically. The compound may also be administered in a localised manner, for example by injection. The treatment may consist of a single dose or a plurality of doses over a period of time. The compound may be useful as an anticancer agent or for the treatment of, for example, diabetes or obesity.

Whilst it is possible for a compound of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

Thus, the invention also provides pharmaceutical compositions comprising the compound identified or identifiable by the screening methods of the invention and a pharmaceutically acceptable carrier.

A further aspect of the invention provides a kit of parts comprising LKB 1 or a recombinant polynucleotide encoding LKB1, STRAD or a recombinant polynucleotide encoding STRAD, or a recombinant polynucleotide encoding MO25. A further aspect of the invention provides a kit of parts comprising (1) AMPK or AMPK subfamily member, or recombinant polynucleotide encoding AMPK or AMPK subfamily member (including a fragment thereof as discussed above or in Example 3) and (2) a kit of parts as defined in relation to the preceding aspect of the invention, or cell of the invention. Such kits may be useful in forming a preparation or complex which may be useful in, for example a screening method of the first aspect of the invention. The recombinant polynucleotide(s) may be in an expression vector (for example as discussed above) or (less desirably) useful for *in vitro* expression.

A further aspect of the invention provides a method for preparing an LKB1/STRAD/MO25 complex comprising the steps of (1) selecting a cell type in which to overexpress LKB1, comprising the step of determining whether the cell type is one that expresses STRAD and MO25, and preparing an LKB1/STRAD/MO25 complex by overexpressing LKB1 in the selected cell type. Methods for determining whether the cell type expresses STRAD and MO25 will be well known to those skilled in the art, and examples of such methods are described in the Examples. Alternatively, the cell type may be one that is already known to express STRAD and MO25. Examples of such cells include 293, HeLa, G361 and Rat2 cells. STRADα and MO25α are expressed at good levels in most cells analysed.

A further aspect of the invention provides a method for preparing an LKB1/STRAD/MO25 complex comprising the steps of (1) overexpressing LKB1 in a cell using a method according to the preceding aspect of the invention and (2) preparing said complex from the cell. Suitable methods for preparing the LKB1/STRAD/MO25 complex are described in the Examples.

Overexpression of LKB1 may lead to growth arrest, for example in eukaryotic cells. It may therefore be necessary to express LKB1 using a transient expression system, for example as described in the Examples, or using a regulated (inducible) expression system, as well known to those skilled in the art.

A further aspect of the invention provides a method for identifying a genetic difference associated with PJS (Peutz-Jeghers Syndrome) comprising the steps of (1) investigating the sequence of a gene encoding a STRAD or MO25 isoform in at least one patient having PJS (2) identifying any difference between the said patient sequence and equivalent sequence from an individual without PJS. The association of LKB1 with STRAD and MO25 and the association of LKB1 deficiency with PJS suggests that defects in M025 or STRAD could also be involved in PJS. Techniques for identifying mutations in candidate genes, and for investigating whether a mutation is linked with disease or is not so linked will be well known to those skilled in the art.

Thus, the invention provides STRAD or MO25 or a recombinant polynucleotide encoding same for use in medicine. A further aspect of the invention provides the use of STRAD or M025 or a recombinant polynucleotide encoding same in the manufacture of a medicament for treating cancer, PJS, diabetes or obesity. The recombinant polynucleotide may be in the form of a gene therapy vector, for example a viral gene therapy vector, as well known to those skilled in the art.

A further aspect of the invention method for identifying a compound which activates AMPK or AMPK subfamily member by a similar mechanism to metformin or phenformin or AICA riboside in which the effect of a test compound on the activation of AMPK or AMPK subfamily member by a preparation or complex or cell of the invention is compared with the effect of metformin or phenformin or AICA riboside on the activation of AMPK or AMPK subfamily member and a compound with a similar effect is selected. Such a method may be useful in determining whether a compound is more potent than metformin or phenformin or AICA riboside.

By the term "antibody" is included synthetic antibodies and fragments and variants (for example as discussed above) of whole antibodies which retain the antigen binding site. The antibody may be a monoclonal antibody, but may also be a polyclonal antibody preparation, a part or parts thereof (for example an F_{ab} fragment or F(ab')₂) or a synthetic antibody or part thereof. Fab, Fv, ScFv and dAb antibody fragments can all be expressed in and secreted from *E. coli,* thus allowing the facile production of large amounts of the said fragments. By "ScFv molecules" is meant molecules wherein the V_{H} and V_{L} partner domains are linked via a flexible oligopeptide. IgG class antibodies are preferred.

Suitable monoclonal antibodies to selected antigens may be prepared by known techniques, for example those disclosed in "Monoclonal Antibodies: A manual of techniques", H. Zola (CRC Press, 1988) and in "Monoclonal Hybridoma Antibodies: techniques and Applications", JGR Hurrell (CRC Press, 1982), modified as indicated above. Bispecific antibodies may be prepared by cell fusion, by reassociation of monovalent fragments or by chemical cross-linking of whole antibodies. Methods for preparing bispecific antibodies are disclosed in Corvalen et al, (1987) Cancer Immunol. Immunother. 24, 127-132 and 133-137 and 138-143.

A general review of the techniques involved in the synthesis of antibody fragments which retain their specific binding sites is to be found in Winter & Milstein (1991) Nature 349, 293-299.

The invention is now described further by reference to the following, nonlimiting, Figures and Examples.

### Figure Legends

**Figure 1** **. Association of MO25α with LKB1**. (A) Cell lysates derived from control parental HeLa cells or HeLa cells stably expressing N-terminal Flag epitope tagged wild type (WT) LKB1 or kinase dead (KD) LKB1 were passed through an anti-Flag M2-affinity agarose column, LKB 1 eluted with the Flag peptide and the samples concentrated as described in Materials and Methods. The samples were electrophoresed on a polyacrylamide gel and the protein bands visualised following colloidal blue staining. Protein bands unique to the WT and KD LKB1 preparations are indicated. (B) The colloidal blue-stained bands labelled as indicated in (A) were excised from the gel, the proteins digested in gel with trypsin, and their identities were determined by tryptic peptide mass-spectral fingerprint. The identity of STRADα and M025α were confirmed by LC-MS/MS sequence analysis on a Q-TOF2 mass spectrometer. The number of tryptic peptides, percentage of sequence coverage and NCBI gi numbers for each protein identified are indicated. (C) The samples purified in (A) were immunoblotted with the indicated antibodies. Identical results were obtained following 2 independent purifications of LKB 1 from HeLa cells.
**Figure 2** **. Amino acid sequence and tissue distribution patterns of MO25α and MO25β isoforms.** (A) Amino acid sequence alignment of the human MO25α (NCBI accession number NP_057373) and MO25β (NCBI accession number Q9H9S4) isoforms as well as *C. elegans* MO25α (NCBI accession number CAB 16486) and MO25β (NCBI accession number NP_508691) and *Drosophila* MO25 (NCBI accession number P91891) putative homologues. Conserved residues are boxed in black, and homologous residues are shaded in grey. Sequence alignments were performed using the CLUSTALW and BOXSHADE programmes at httn://www.ch.embnet.org/ using standard parameters. (B) A ³²P-labelled fragment of the MO25α cDNA was used to probe a Northern blot containing polyadenylated RNA isolated from the indicated human tissues. The membrane was autoradiographed, and the MO25α probe was observed to hybridise to a 4.2-kb message, identical to the size predicted for the MO25α message from database analysis. As a loading control, the Northern Blot was hybridised with a β-actin probe. (C &D) The indicated mouse tissue (C) or cell (D) extracts containing 20 µg of total cell protein were immunoblotted with the anti-MO25α and anti-MO25β antibodies.

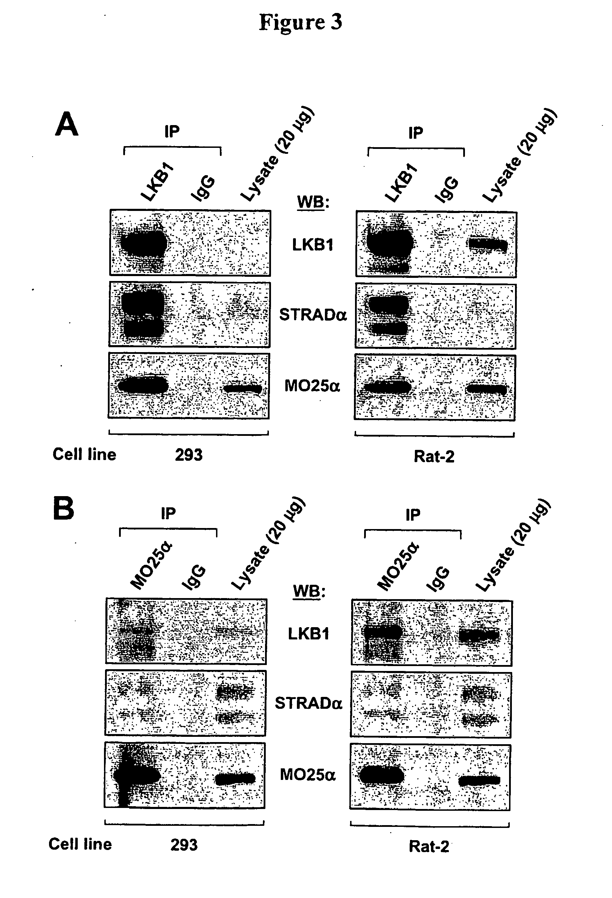

**Figure 3****. Endogenous LKB1 is associated with MO25**α**.** (A) LKB1 was immunoprecipitated from 2 mg of the indicated lysates using 10 µg of anti-LKB1 antibody covalently coupled to protein G-Sepharose, and the immunoprecipitates were immunoblotted with the indicated antibodies. As a control, immunoprecipitations were also performed in parallel experiments with preimmune IgG antibodies covalently coupled to protein G-Sepharose. In each gel, 20 µg of total cell lysate was also immunoblotted in parallel. (B) MO25α was immunoprecipitated as above except that 15 µg of the MO25α antibody was employed. The results shown are from a single experiment that was repeated 3 times with similar results.

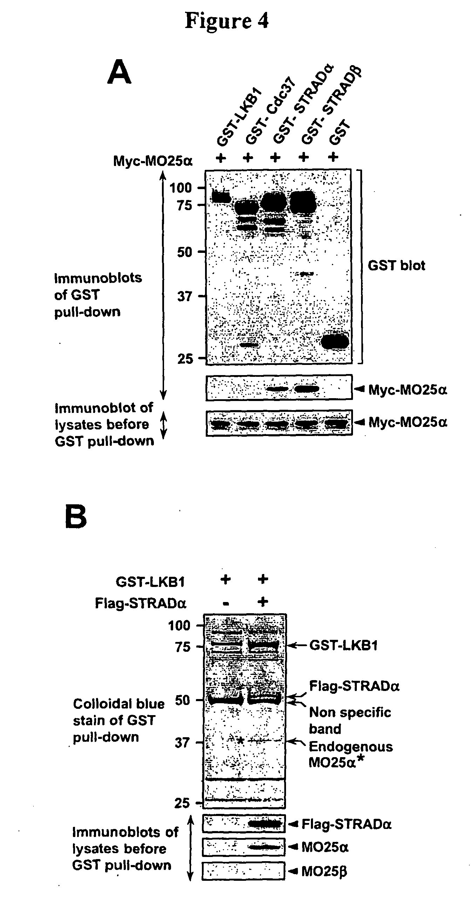

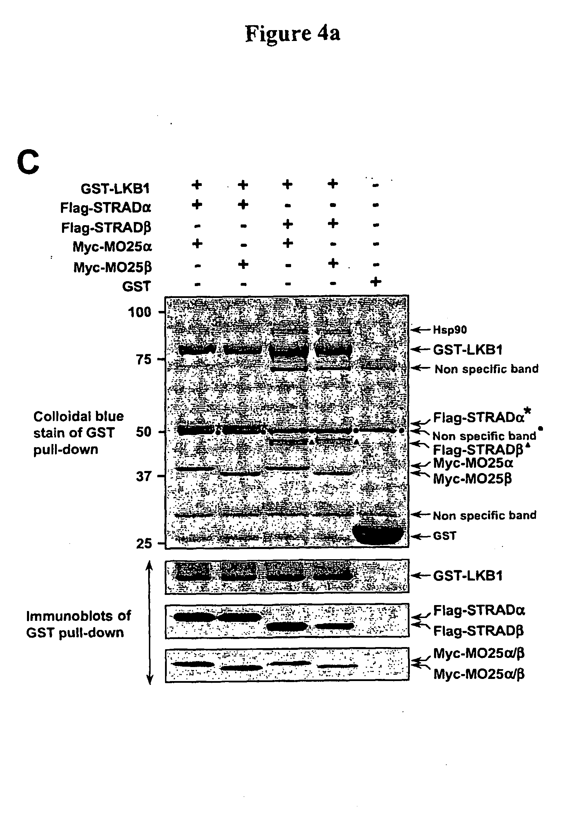

**Figure 4** **. MO25α is associated with LKB1 through STRAD** (A) 293 cells were transfected with plasmids encoding for the expression of the indicated GST fusion proteins together with Myc-MO25α. 36 h post transfection, the GST tagged proteins were affinity purified from the cell lysates using glutathione-Sepharose as described in Materials and Methods. Similar amounts of the purified GST-fusion proteins were subjected to SDS-polyacrylamide gel electrophoresis and immunoblotted with anti-Myc antibody to detect co-purified Myc-MO25α, or with anti-GST antibody to ensure that comparable amounts of the GST tagged proteins were present in each lane (upper and middle panels). 5 µg of total cell lysates prior to affinity purification were also subjected to immunoblotting with anti-Myc antibody to ensure that Myc-MO25α was expressed at similar levels in each co-transfection (lower panel). (B) N-terminal GST tagged LKB1 was expressed in 293 cells in the presence or absence of Flag-STRADα and purified as above. The purified proteins were subjected to SDS-polyacrylamide gel electrophoresis and visualised by Colloidal blue staining (upper panel). The faint protein band indicated, corresponding to an endogenous protein of 40-kDa (marked *), was identified by tryptic peptide mass-spectral fingerprint as endogenously expressed MO25α (with 38% sequence coverage). The purified proteins were also immunoblotted with the indicated antibodies (lower panels). (C) As above except that GST-LKB1 was expressed in 293 cells together with the indicated isoforms of Flag-STRAD and Myc-M025 and the proteins visualised by colloidal blue staining were also immunoblotted with the indicated antibodies. For all panels, similar results were obtained in at least 3 separate experiments.

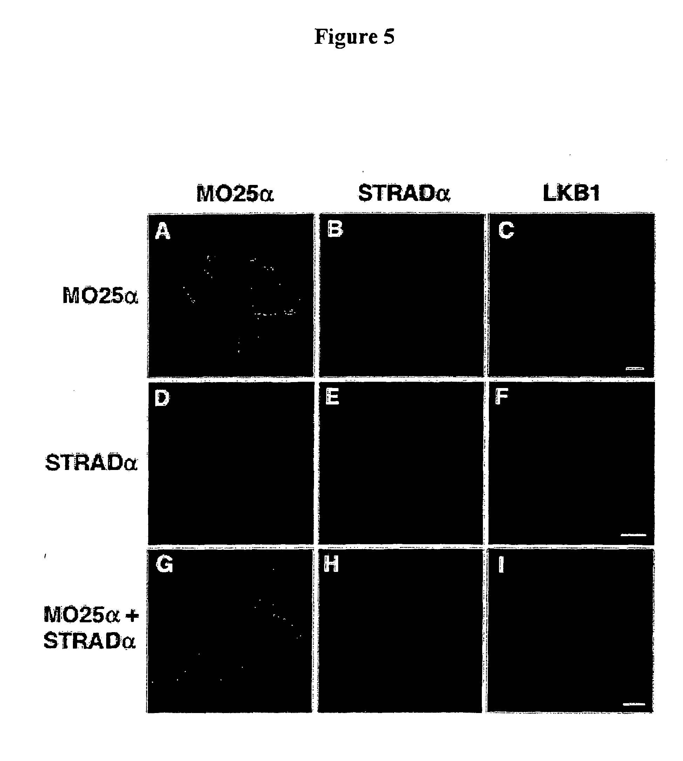

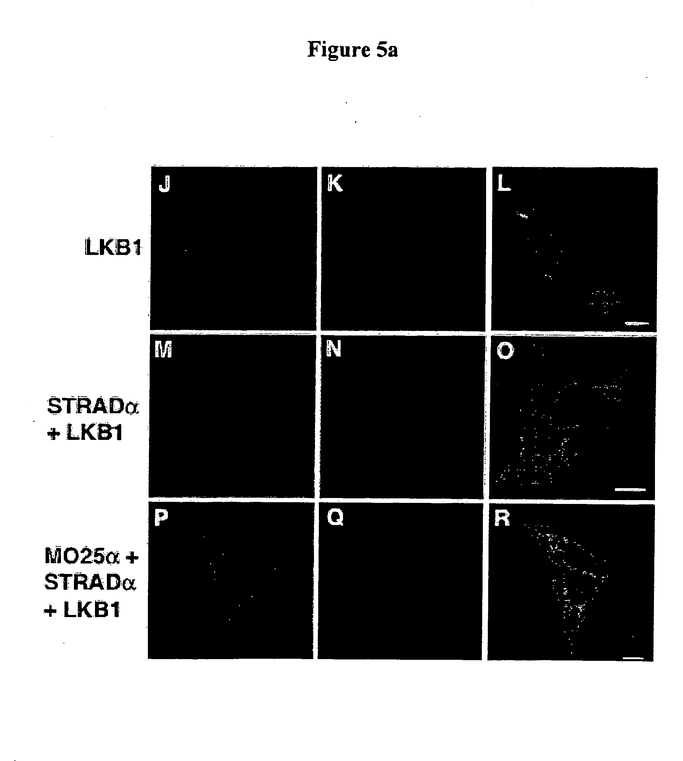

**Figure 5** **. MO25α and STRADα anchor LKB1 in the cytoplasm**. HeLa cells were transfected with the indicated constructs encoding for the expression of Myc-MO25α, Flag-STRADα and GFP-LKB1. 24 h post transfections the cells were fixed in 4% (by vol) paraformaldehyde and immunostained with anti-MO25α antibody to detect MO25α (TR anti-sheep secondary antibody, red channel) and anti-Flag to detect STRADα (Cy5 anti-mouse secondary antibody, blue channel). GFP-LKB1 localisation was visualised directly through the GFP fluorescence (green channel). The cells were imaged using a Zeiss LSM 510 META confocal microscope. The cells shown are representative images obtained in 3 separate experiments. The scale bars correspond to 10 µm.

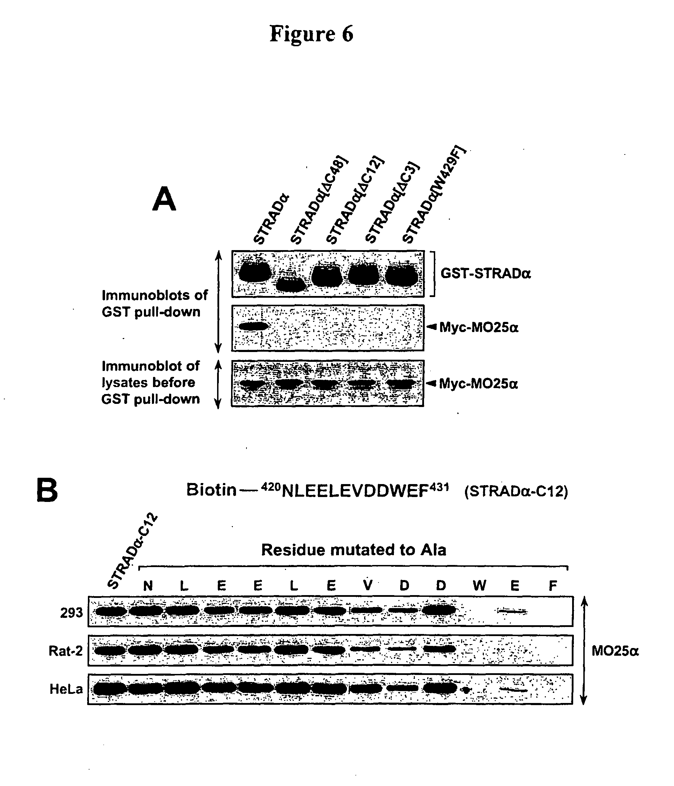

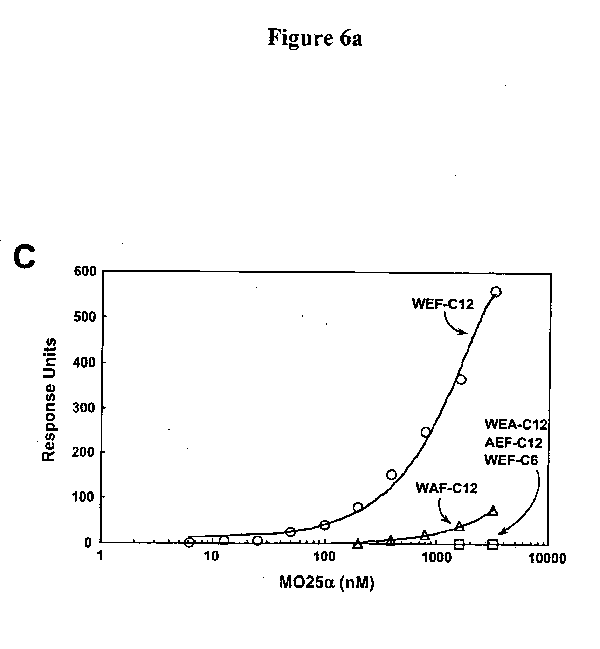

**Figure 6** **. MO25α recognises the C-terminal 3 residues of STRADα.** (A) N-terminal GST tagged wild type STRADα or the indicated mutants of STRADα were expressed in 293 cells together with Myc-MO25α, and 36 h post transfection the STRADα proteins were affinity purified from the cell lysates using glutathione-Sepharose. Similar amounts of the purified proteins were subjected to SDS-polyacrylamide gel electrophoresis and immunoblotting with anti-Myc antibody to detect co-purified Myc-MO25α, or with anti-GST antibody to ensure that comparable amounts of the GST tagged proteins were present in each lane (upper and middle panels). 5 µg of the total cell lysates prior to affinity purification were also subjected to immunoblotting with anti-Myc antibody to ensure that Myc-MO25α was expressed at similar levels in each condition (lower panel). (B) 0.5 mg of the indicated cell lysates was incubated with 5 µg of an N-terminal biotinylated peptide encompassing either the C-terminal 12 residues STRADα conjugated to streptavidin-Sepharose (NLEELEVDDWEF, termed STRADα-C12) or mutants of this peptide in which the indicated residues were individually mutated to Ala. Following isolation and washing of the beads, the samples were subjected to SDS-polyacrylamide gel electrophoresis and immunoblotted with an anti-MO25α antibody. (C) Binding of bacterially expressed MO25α to the indicated peptides was analysed by surface plasmon resonance BiaCore analysis as described in Materials and Methods. Binding was analysed over a range of MO25α concentrations (6.25-3200 nM) and the response level at the steady-state binding was plotted versus the log of the MO25α concentration. The estimated K_{d} for the STRADα-C12 peptide was obtained by fitting the data to the formula [ml X m0/(m0 + m2)] using Kaleidagraph software and the K_{d} was calculated to be 850 nM. WEF-C12 corresponds to NLEELEVDDWEF, WEA-C12 corresponds to NLEELEVDDWEA, AEF-C12 corresponds to NLEELEVDDAEF, WAF-C12 corresponds to NLEELEVDDWAF, WEF-C6 corresponds to VDDWEF.

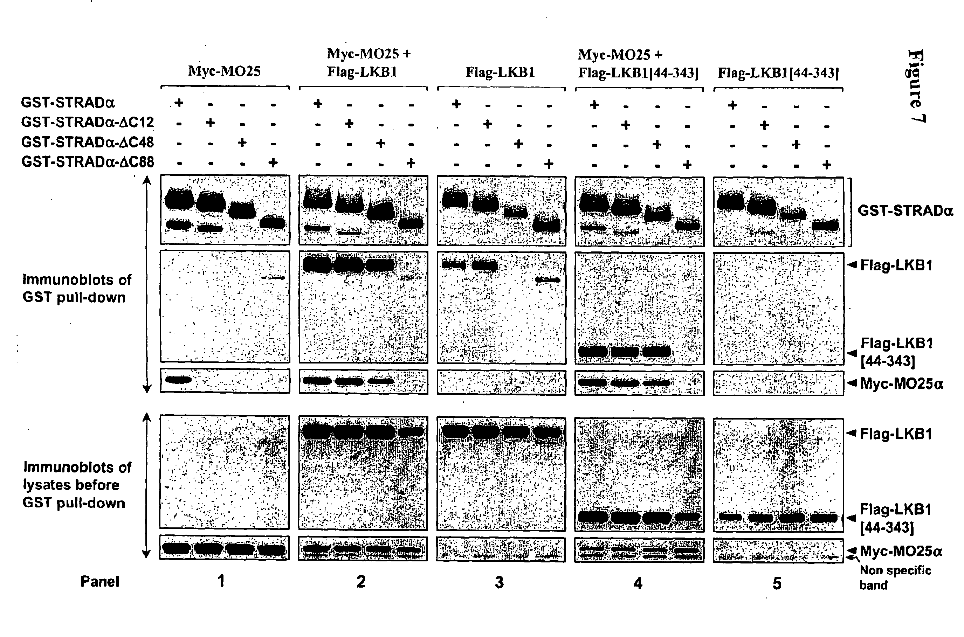

**Figure 7****. Binding of LKB1 to STRADα creates novel binding site(s) for MO25α.** The indicated forms of GST-STRADα were co-expressed in 293 cells together with Myc-MO25α and/or wild type Flag-LKB 1 or the isolated LKB 1 catalytic domain (LKB1 [44-343]. 36 h post-transfection, the GST-STRADα proteins were affinity purified, subjected to SDS-polyacrylamide gel electrophoresis and immunoblotting with anti-Myc antibody to detect Myc-MO25α, anti-Flag antibody to detect co-purified Flag-LKB 1 and Flag-LKB 1 [44-343], or with anti-GST antibody to detect STRADα forms (upper panels). 5 µg of total cell lysates prior to affinity purification were also subjected to immunoblotting with anti-Myc and anti-Flag antibodies to ensure that MO25α and LKB 1 were expressed at similar levels in each co-transfection (lower panels). Similar results were obtained in 3 separate experiments.

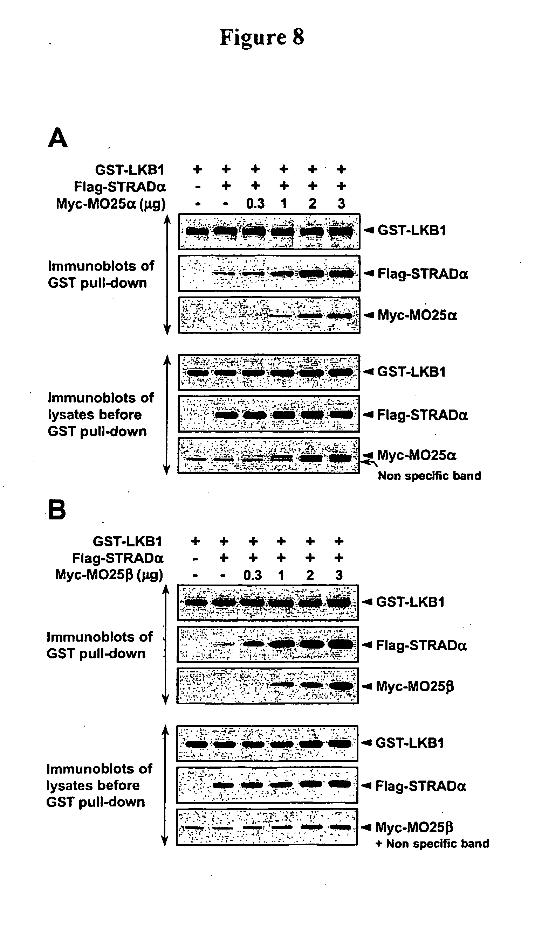

**Figure 8****. MO25 isoforms stabilise the LKB10STRADα complex.** (A) 293 cells were transfected with 3 µg of the DNA construct encoding for expression of GST-LKB1, in the presence or absence of 3 µg of Flag-STRADα construct and in the absence or presence of indicated amounts of Myc-MO25α construct. 36 h post-transfection, GST-LKB1 was affinity purified from the cell lysates and immunoblotted with appropriate epitope antibodies to detect LKB1, STRADα and MO25α. 5 µg of total cell lysates prior to affinity purification were also immunoblotted with the indicated antibodies (lower panels). (B) is performed as above except that the MO25β construct was employed instead of the MO25α construct. Similar results were obtained in 3 separate experiments.

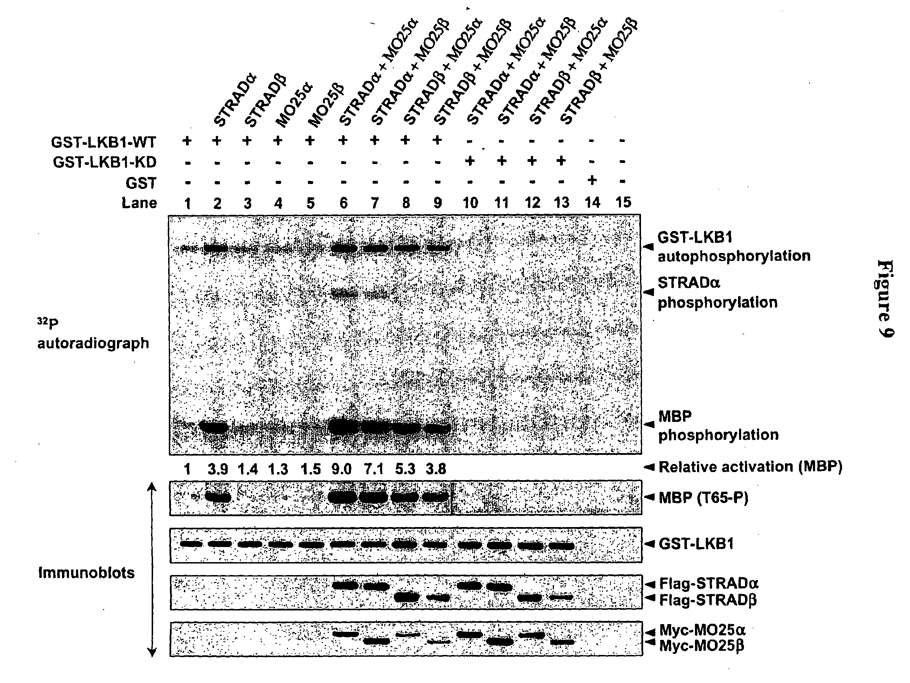

**Figure 9****. Activation of LKB1 by association with STRAD and MO25 isoforms.** 293 cells were transfected with constructs encoding GST-LKB1 in the presence or absence of constructs encoding the indicated isoforms of STRAD and MO25. 36 h post-transfection, GST-LKB1 was affinity purified and assayed for autophosphorylation and transphosphorylation of MBP as described in the materials and methods. The reactions were electrophoresed and the gel autoradiographed (top panel). Phosphorylation of MBP by LKB 1 at Thr65 was also monitored by immunoblotting with a phosphospecific antibody (T65-P), which recognises MBP phosphorylated by LKB1 at this residue. Each reaction was also immunoblotted with the appropriate epitope tag antibodies to monitor levels of LKB1, STRAD and M025 isoforms. Similar results were obtained in 3 separate experiments.
**Figure 10****. Amino acid sequence alignment of STRAD isoforms with closest STE20-kinase relatives.** (A) Amino acid sequence alignment of the human STRADα (NCBI accession number AAG48269) and STRADβ (NCBI accession number AAM19143) with human SPAK (NCBI accession number AAC72238) and human OSR1 (NCBI accession number NP_005100). Conserved residues are boxed in black, and homologous residues are shaded in grey. The catalytic Asp (subdomain VI) and Asp-Phe-Gly (subdomain VII) present in the active SPAK and OSR1 protein kinases, but lacking in the STRADα and STRADβ pseudokinases are boxed and marked with an asterisk. The C-terminal Trp-Glu-Phe motif present in STRADα and STRADβ corresponding to a Phe-Glu-Phe sequence in SPAK and OSR1 are boxed and marked with triangles and the sites of LKB1 phosphorylation in STRADα (Thr329 and Thr419) (Baas et al., 2003) are marked with arrows. Sequence alignments were performed using the CLUSTALW and BOXSHADE programmes at http://www.ch.embnet.org/ using standard parameters.

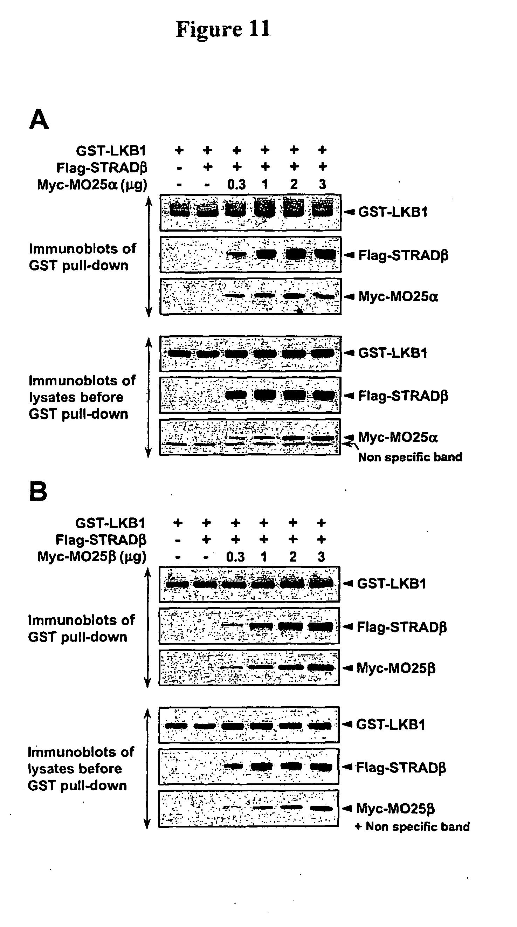

**Figure 11****. MO25 isoforms increase expression of STRADβ and formation of the LKB1-STRADβ complex.** (A) 293 cells were transfected with 3 µg of the DNA construct encoding for expression of GST-LKB1, in the presence or absence of 3 µg of Flag-STRADβ construct and in the absence or presence of indicated amounts of Myc-MO25α construct. 36 h post-transfection, GST-LKB1 was affinity purified from the cell lysates and immunoblotted with appropriate epitope antibodies to detect LKB1, STRADβ and MO25α. 5 µg of total cell lysates prior to affinity purification were also immunoblotted with the indicated antibodies (lower panels). (B) is performed as above except that the MO25β construct was employed instead of the MO25α construct. Similar results were obtained in 3 separate experiments and also when STRADβ was expressed as a GST-fusion protein in the pEBG-2T vector instead of the pCMV5 vector (data not shown).

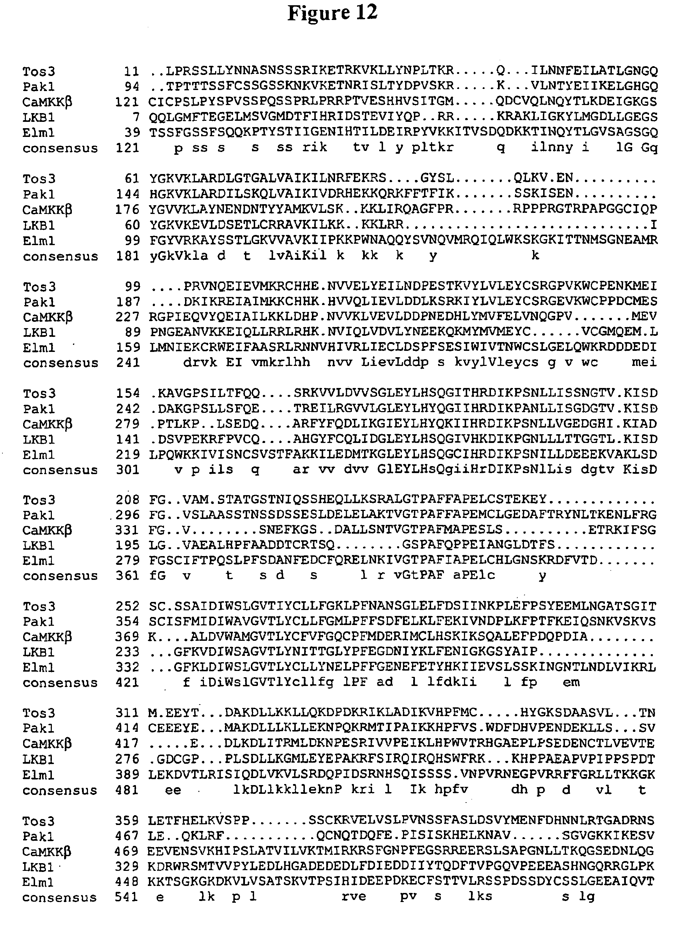

**Figure 12****:** Alignment of the amino acid sequences of Tos3, Pak1 and Elml from yeast and LKB 1 and CaMKKβ (β3 variant) from humans. The alignment was created using PILEUP from the GCH suite [49] and the consensus sequence determined and colouring added using BOXSHADE v3.2.1 [50]. Residues that are identical in at least half of the sequences are coloured grey, with conservative changes also in grey. The consensus sequence is represented by upper case letter if the residue at a particular position is identical in all sequences, and by a lower case letter if at least half are conserved. Only the conserved central regions containing the kinase domains (residues 50-310 of LKB 1 approx.) are shown.

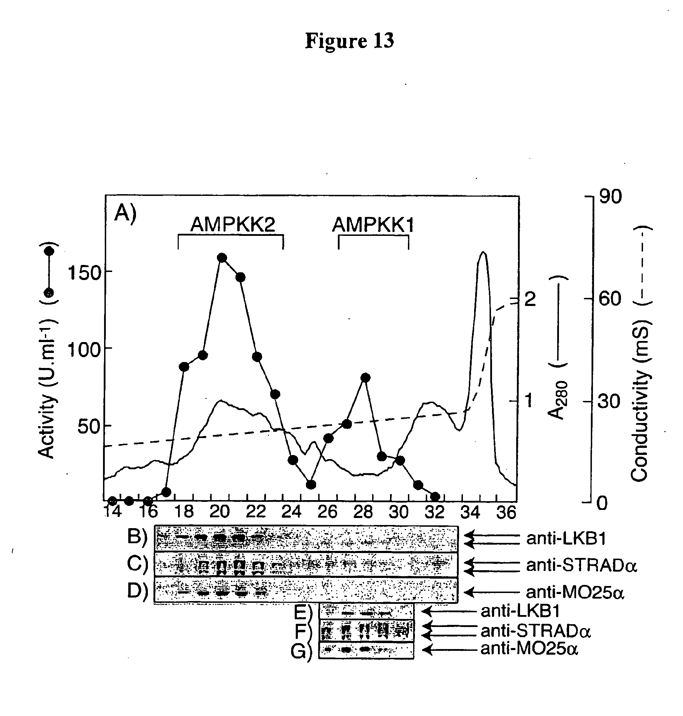

**Figure 13** : Two AMPKKs can be resolved from rat liver extracts but both are complexes between LKB1, STRADα and MO25α. (A) Separation of two activities that activate GST-AMPKα1-catalytic domain by Q-Sepharose chromatography. The graph shows AMPKK activity in fractions (open circles, absorbance at 280 nm (continuous line) and conductivity in the eluate (dashed line). The "x" axis represents the fraction number (4.5 ml fractions). B, C, D: probing of blots of column fractions after SDS-PAGE (1 µl per lane) using anti-LKB1 (B), anti-STRADα (C) and anti-MO25α (D). E, F, G: Fractions 26-30 were concentrated from 4.5 ml to 250 µl using Amicon Ultra-4 30,000 MWCO centrifugal concentrators, and 2 µl was loaded per lane. Following SDS-PAGE the gels were transferred to nitrocellulose and the membranes probed with anti-LKB1 (E), anti-STRADα (F) and anti-MO25α (G).

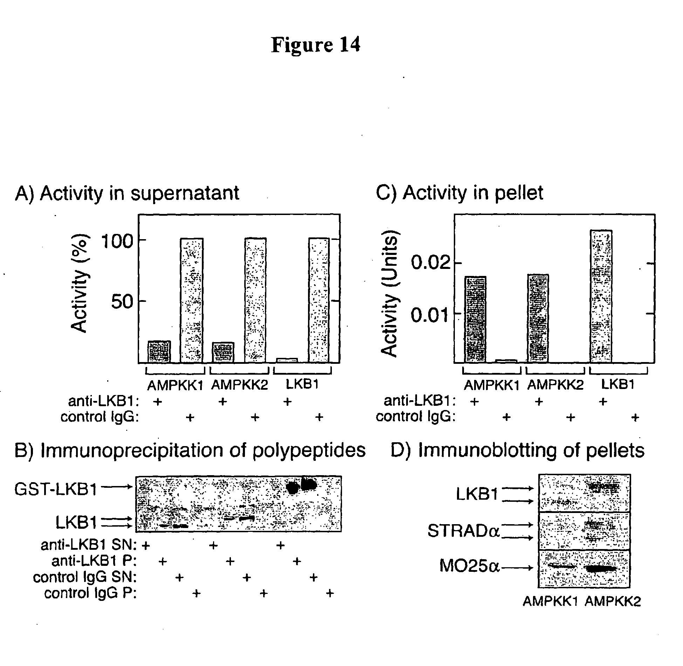

**Figure 14** **:** AMPKK activity, and the LKB1, STRADα and MO25α polypeptides, can be immunoprecipitated from rat liver AMPKK1 and AMPKK2 using anti-LKB 1 antibody. (A) Depletion of AMPKK activity from supernatant. Sheep anti-human LKB1 or pre-immune control immunoglobulin (IgG) was pre-bound to Protein G-Sepharose beads and crosslinked with DMP as described previously [51], except that a final wash of the beads with 100 mM glycine, pH2.5, was performed. Bead-bound antibodies (40 µl) were incubated with the peak fraction of AMPKK1 (0.04 Units), AMPKK2 (0.03 Units) or recombinant GST-LKB1:STRADa:MO25α complex (0.06 Units) for 20 minutes and the beads removed in a microcentrifuge (14,000xg, 2 min). AMPKK activity was determined in the supernatants and is expressed as a percentage of the value obtained using the control IgG. (B) The pellets from the experiment in (A) were resuspended in the original volume and samples of the supernatants and pellets analysed by Western blotting with anti-LKB1 antibody. The recombinant LKB1 migrates at a higher molecular mass because of the GST tag. (C) As A, except that the amount of AMPKK1, AMPKK2 and recombinant GST-LKB1-STRADα-MO25α complex were increased to 0.44, 0.70 and 1.4 Units respectively, and the activities were determined in the resuspended pellets. In this experiment the amount of antibody was limiting so only a portion of the activity was precipitated. (D) The pellets from the experiment in (C) were resuspended and samples analysed by Western blotting with anti-LKB1, anti-STRADα and anti-MO25α antibodies.

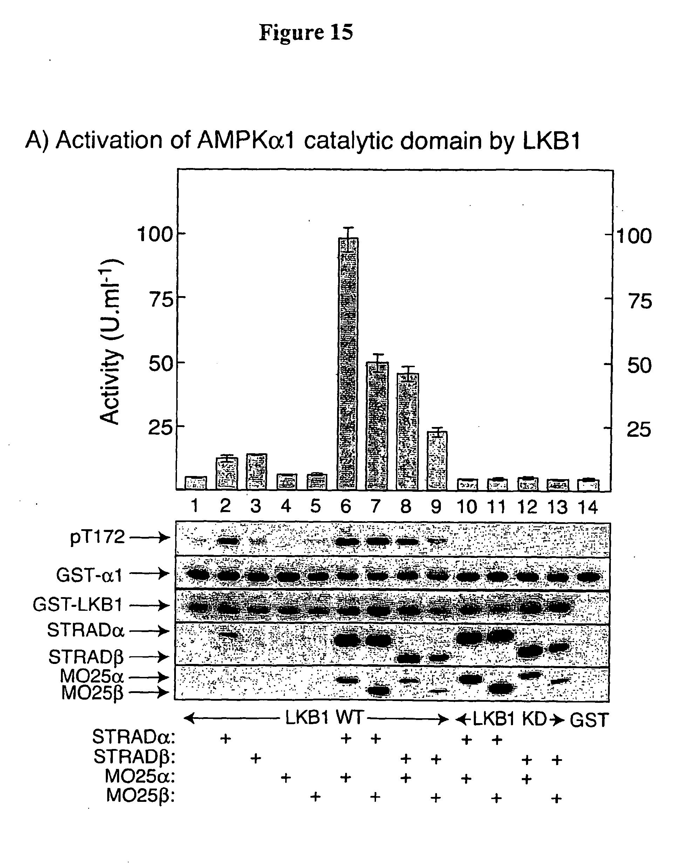

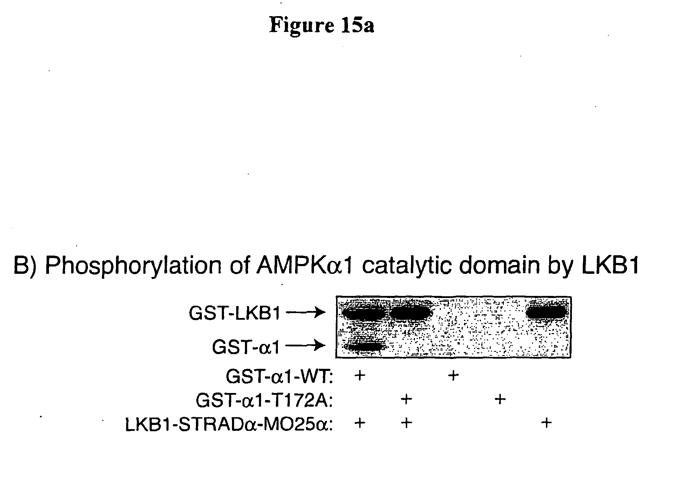

**Figure 15** : Recombinant LKB1-STRAD-MO25 complexes can efficiently activate AMPK via phosphorylatation at Thr-172. (1): the indicated combinations of GST-tagged wild type LKB 1 (WT, lanes 1-9), or kinase-dead (D194A, KD, lanes 10-13) LKB1 mutant, or GST-alone (lane 14), FLAG-tagged STRADα or STRADβ, and *myc*-tagged MO25α or MO25β were co-expressed in HEK-293T cells, purified on glutathione-Sepharose and tested for their ability to activate GST-AMKα1 catalytic domain (top panel). Samples from each incubation were also analysed by Western blotting and probed using the indicated antibodies (from top to bottom(: anti-pT172, anti-AMPKα1 catalytic domain (GST-α1), anti-GST to detect GST-LKB1, anti-FLAG to detect STRADα and STRADβ, and anti-*myc* to detect MO25α and MO25β. All proteins migrated with the expected mobility, taking into account the epitope tags. The bottom three blots were conducted on blank reactions lacking GST-AMPKα1 catalytic domain, as the latter appeared to cause some interference with detection. (B): recombinant GST-LKB1:STRADα:MO25α complex was used to phosphorylate wild type GST-α1 catalytic domain (GST-α1-WT) or a T172A mutant (GST-α1-T172A) using [γ-³²]ATP as described under Methods. The reaction was analysed by SDS-PAGE and autoradiography. Arrows show the migration of GST-LKB 1 (which autophosphorylates) and GST-α1 catalytic domain.

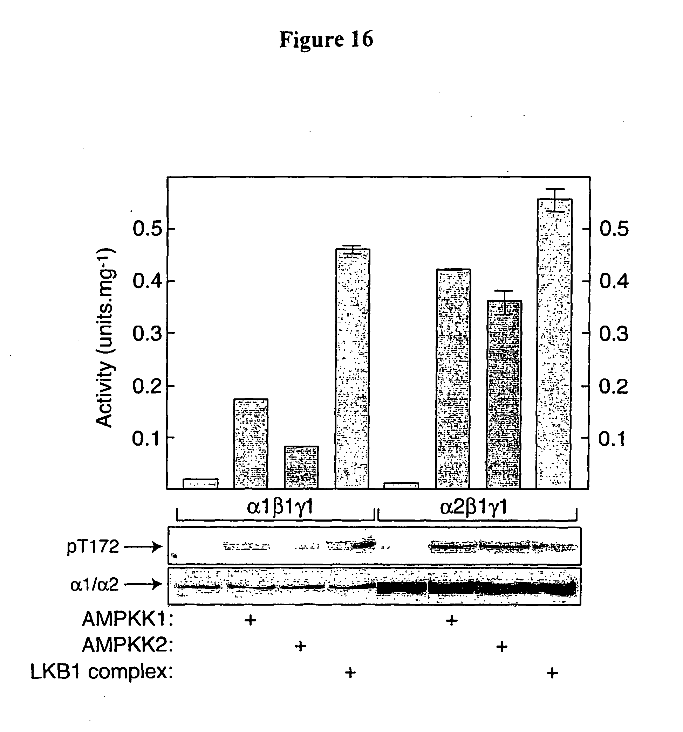

**Figure 16****:** Activation and phosphorylation of heterotrimeric AMPK complexes by AMPKK1, AMPKK2 and recombinant GST-LKB1:STRADα:MO25α complexes. combinant α1β1γ1 or α2β1γ1 complexes where incubated for 10 min with MgATP and AMPKK1 (2.8 or 1.4 units/ml), AMPKK2 (10.3 or 5.15 units/ml) or GST-LKB1:STRADα:MO25α (14.3 or 7.2 units/ml), and AMPK activity determined. Twice as much upstream kinase was added when the α1β1γ1 complex was the substrate, as pilot experiments showed that it was activated less readily. The images are of Western blots probed with phosphospecific anti-pT172 antibody (upper panel), and with a mixture of anti-α1 and -α2 antibodies to demonstrate uniform lodaing of the α1β1γ1 or α2β1γ1 complexes (note that the anti-α2 antibody always gives a stronger signal than anti-α1 even for equal protein loadings).

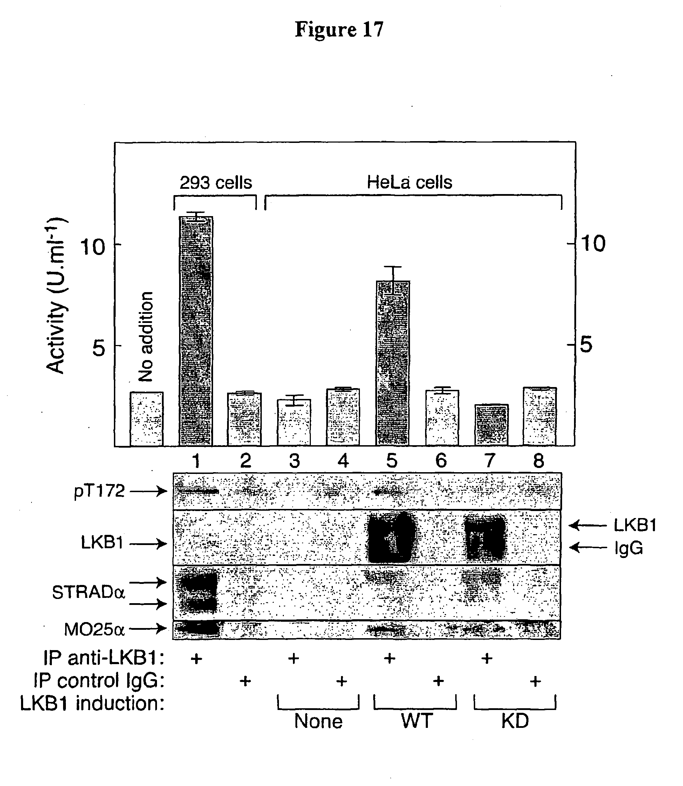

**Figure 17****:** Endogenous AMPKK activity can be immunoprecipitated from 293 cells using anti-LKB 1 antibody, but activity can only be immunoprecpitated from HeLa cells if they stably express LKB 1 (but not a catalytically-inactive mutant) from an inducible promoter. LKB1 was immunoprecipitated from 0.5 mg of cell extract derived from untransfected HEK-293T cells (lanes 1,2), untransfected HeLa cells (lanes 3,4), or HeLa cells stably expressing wild type (WT) LKB1 (lanes 5,6) or a kinase-dead (KD) LKB1 (D194A) mutant (lanes 7,8) from an inducible promoter. Immunoprecipitation was with anti-LKB1 (lanes 1,3,5,7) or a pre-immune control immuoglobulin (IgG, lanes 2, 4, 6, 8). Samples of each immunoprecipitate were used to assay activation of GST-AMPKα1-catalytic domain, to analyze phosphorylation of GST-AMPKα1-catalytic domain on Thr-172 (middle panel), and to determine recovery of LKB1 (bottom panel). Samples of each immunoprecipitate were also immunoblotted for the presence of endogenous STRADα and MO25α with appropriate antibodies. Also shown in the top panel is the basal activity obtained when the GST-AMPKα1-catalytic domain was incubated with MgATP on its own (no addition).

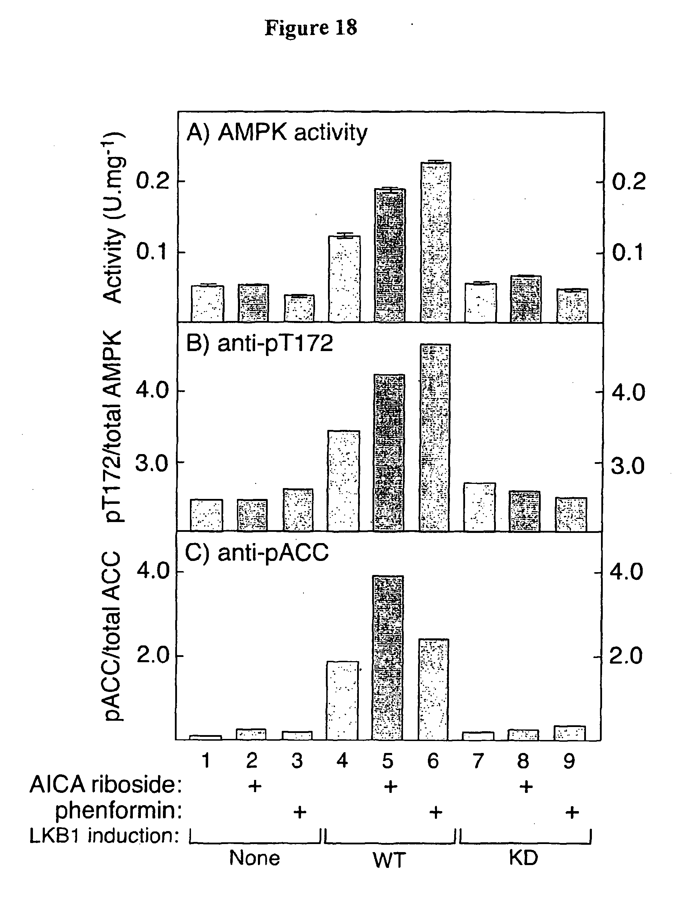

**Figure 18** **:** Restoration of the ability of AMPK to be activated by AICA riboside and phenformin in HeLa cells by expression of LKB 1. Control HeLa cells (lanes 1, 2,3), HeLa cells expressing wild type (WT) LKB1 (lanes 4,5,6) and kinase dead (D194A, KD) mutant LKB1 (lanes 7, 8, 9) were either left unstimulated or treated with 2 mM AICA riboside or 10 mM phenformin for 60 min and lysed. (A) Endogenous AMPK was immunoprecipitated from the cell extracts and assayed. (B) The cell lysates was immunoblotted with antibodies recognising AMPKα1 phosphorylated at Thr172 or total AMPKα1; the results were analysed using the LI-COR IR Odyssesy™ imager as described under Materials and Methods, and are expressed as a ratio of the two signals. (C) The cell lysates was analysed by Western blotting and the membranes probed with antibodies recognising ACC phosphorylated at Ser-79, or streptavidin to determine total AMPKα1. The results were analysed using the LI-COR IR imager as for (B).

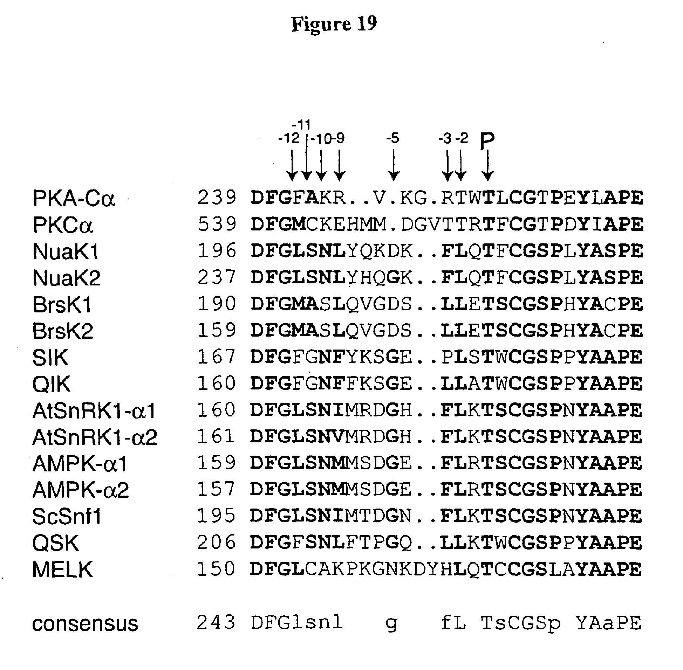

**Figure 19****:** Sequence alignment of the activation loop sequences of AGC sub-family kinases (PKAα and PKCα, which are not thought to be activated by LKB1) with the T-loop residues of the AMPK/ SNF1 subfamily of protein kinases. The methods and representation are as described in Figure 1. The threonine residues corresponding to Thr-172 of AMPK ("P") and other residues mentioned in the text are indicated with downward arrows. All sequences are from humans, except AtSnRK1-α1, AtSnRK1-α2 and ScSnf1, which are the *Arabidopsis thaliana* (AtSnRK1, SNF1-related kinase-1) and *Saccharomyces cerevisiae* (ScSnfl) homologues of AMPK. Although the human, *Arabidopsis* and *S. cerevisiae* homologues, as well as the PKAα and PKCα kinases, are known to activated by phosphorylation of the threonine residue equivalent to Thr-172, it has yet to be established whether the other AMPK subfamily members (NuaK1, NuaK2, BrsK1, BrsK2, SIK, QIK, QSK, MELK) are activated by phosphorylation of this residue, and whether LKB1-STRAD-MO25 complexes can mediate this phosphorylation.

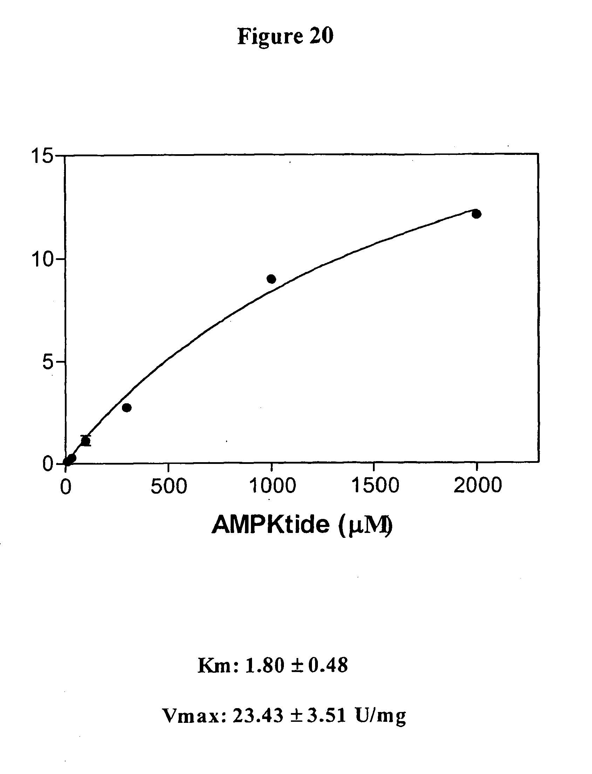

**Figure 20****: Km and Vmax for a peptide substrate (termed AMPKtide).** Assay conditions are described in Example 3.

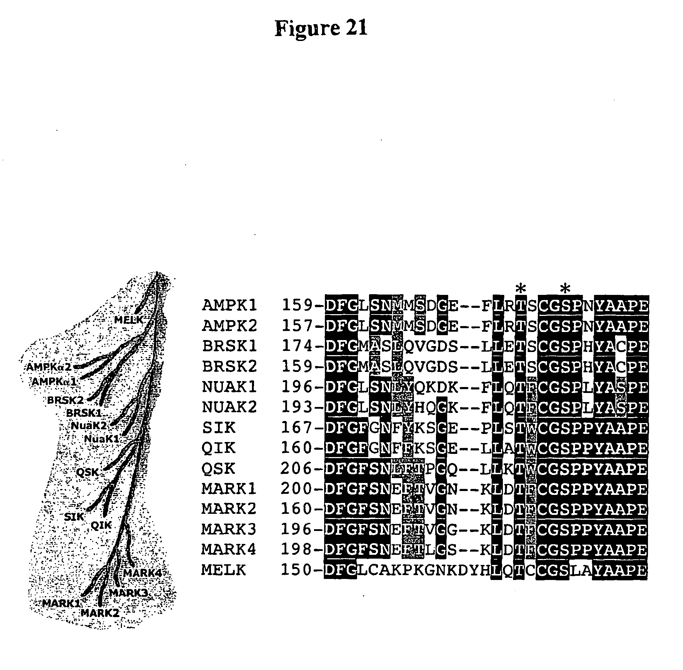

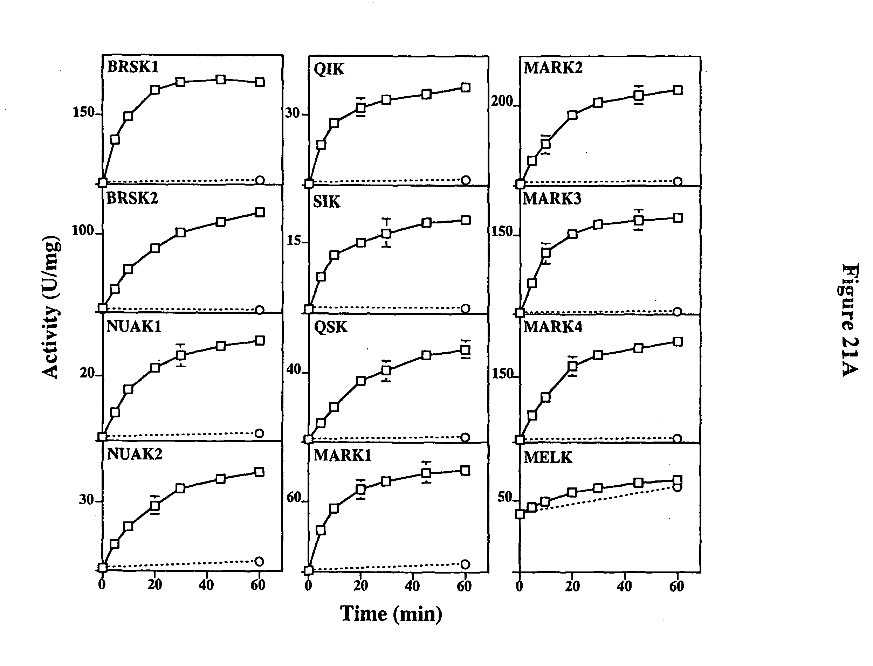

**Fig 21****. Activation of AMPK-related kinases by LKB1.** (A) Dendrogram and T-loop sequences of AMPK subfamily of protein kinases (Manning et al., 2002). The identical residues are shaded black and the conserved residues in grey. The T-loop Thr and Ser are indicated with an asterisks. (B) The indicated AMPK-related kinases were incubated with wild type LKB1:STRAD:MO25 (open squares) or catalytically inactive LKB1[D194A]:STRAD:MO25 (open circles) complexes in the p-esence of Mg²⁺ and ATP. At the indicated times, the activity of the AMPK-related kinases was assayed with the *AMARA* peptide and results are expressed as specific activity. Results shown are means ± SD of assays carried out in triplicate and representative of two independent experiments. The error bars are only shown when larger than the size of the open squares.

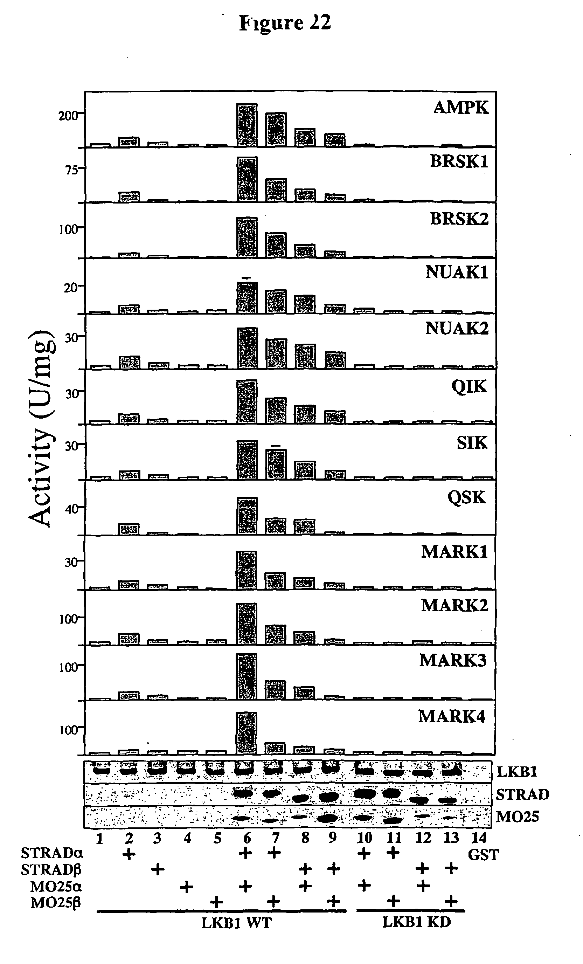

**Fig 22****. Efficient activation of AMPK-related kinases by LKB1 requires STRAD and MO25 subunits.** The indicated combinations of GST-tagged wild type LKB1 (WT, lanes 1-9), or catalytically inactive (D194A, lanes 10-13) LKB1, or GST alone (lane 14), FLAG-tagged STRADα or STRADβ, and *myc*-tagged MO25α or MO25β were co-expressed in HEK-293T cells and purified on glutathione-Sepharose. The complexes were tested for their ability to activate the catalytic domain of AMPKα1 or the indicated AMPK-related kinases. The results are expressed as specific activity employing the *AMARA* peptide as substrates. Results shown are means ± SD of assays carried out in triplicate and representative of two independent experiments. Samples from each incubation were also analysed by Western blotting and probed using the indicated antibodies (from top to bottom): anti-GST to detect LKB1; anti-FLAG to detect STRADα and STRADβ; and anti-*myc* to detect MO25α and MO25β. All proteins migrated with the expected mobility, taking into account the epitope tags.

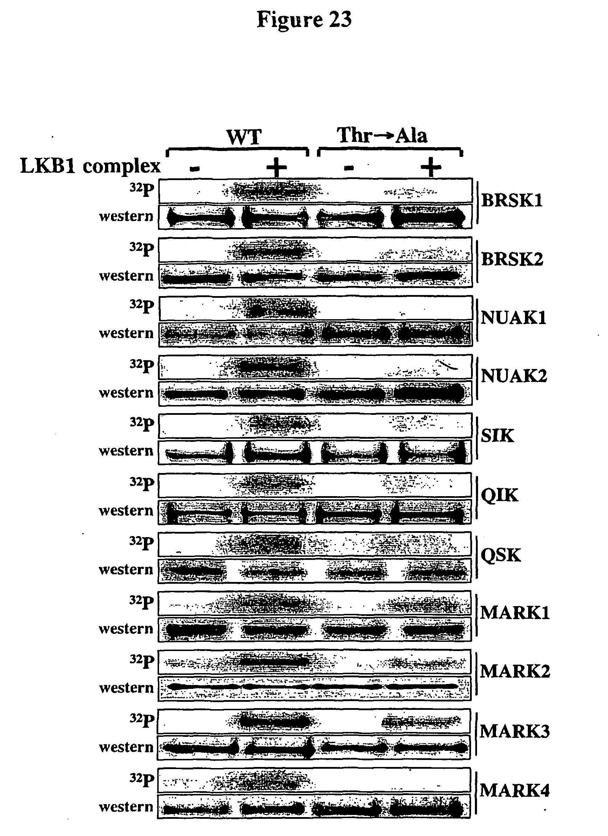

**Fig 23****. The T-loop Thr is the major site of LKB1 phosphorylation on the AMPK-related kinases**. Wild type (WT) and T-loop Thr to Ala (Thr→Ala) mutants of the indicated GST-AMPK-related kinases, were incubated with LKB1:STRAD:MO25 complex in the presence o⁻ Mg²⁺ and [γ³²P]ATP. Phosphorylation of protein substrates was determined by electrophoresis on a polyacrylamide gel and subsequent autoradiography of the Coomassie Blue-stained bands corresponding to each substrate. An aliquot of each incubation was also analysed by Western blotting probing with an anti-HA antibody to ensure equal loading of wild type and mutant AMPK-related kinases (which all possess a HA epitope tag). All proteins migrated with the expected mobility, taking into account the epitope tags. Similar results were obtained in 3 separate experiments.

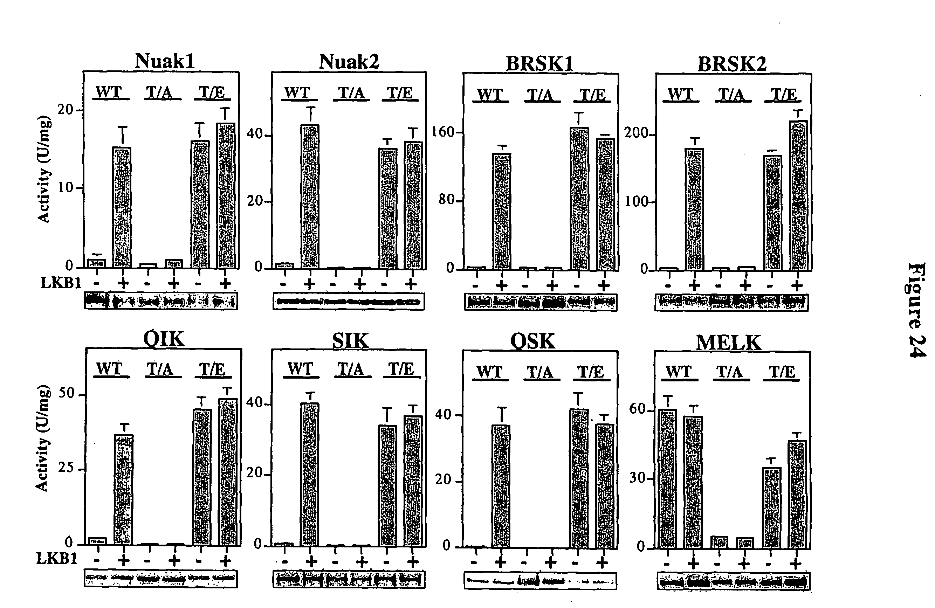

**Fig 24****. Effect of mutation of the Thr in the T-loop on activation of AMPK-related kinases by LKB1**. The indicated wild type (WT) AMPK-related kinases or mutants of these enzymes in which the T-loop Thr was changed to either Ala (T/A) or Glu (T/E), were incubated in the absence (-) or presence (+) of wild type LKB1:STRAD:MO25 in the presence of Mg²⁺ and ATP. After 30 min, the AMPK-related kinases were assayed with the *AMARA* peptide and results are expressed as specific activity. Results shown are means ± SD of assays carried out in triplicate and representative of two independent experiments. An aliquot of each incubation was also analysed by Western blotting probing with an anti-HA antibody to ensure equal loading of wild type and mutant AMPK-related kinases (which all possess a HA tag).

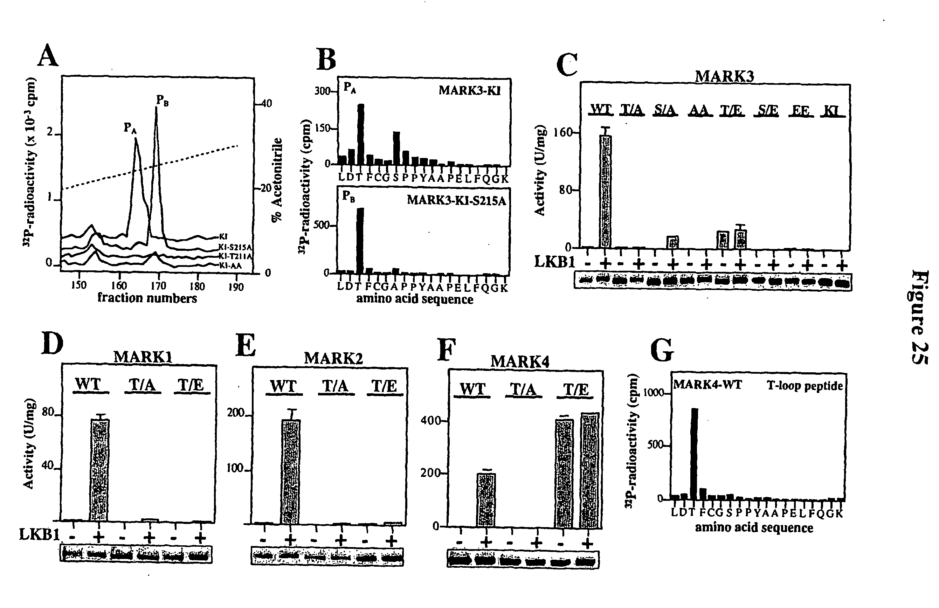

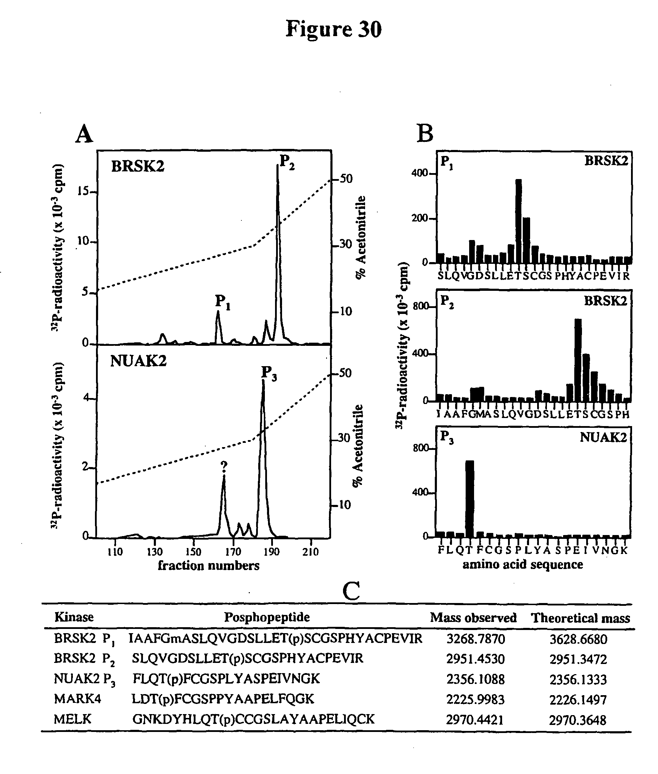

**Fig 25****. Analysis of phosphorylation and activation of MARK kinases.** (A) Catalytically inactive MARK3[D196A] (KI), that can not autophosphorylate and the indicated mutants, were incubated with LKB1 complex for 30 min with Mg²⁺ -[γ³²P]ATP and separated by electrophoresis on a polyacrylamide gel which was then autoradiographed. The ³²P-labelled MARK3 proteins were digested with trypsin and the resulting ³²P-labelled peptides were chromatographed on a C₁₈ column. Fractions containing the ³²P-labelled T-loop tryptic peptide (Peptides P_{A} and P_{B}) are shown. (B) Peptides P_{A} and P_{B} were subjected to solid phase sequencing and ³²P-radioactivity was measured after each cycle of Edman degradation. In combination with MALDI TOF-TOF mass spectrometry, this enabled the identification of the sites phosphorylated in each peptide. Peptides P_{A} comprises the MARK3 T-loop peptide phosphorylated at Thr211 and Ser215 and Peptides P_{B} the MARK3 T-loop peptide phosphorylated at Thr211. (C to F) The indicated wild type (WT) or mutant forms of MARK kinases in which the the T-loop Thr or Ser was mutated to either Ala (T/A, S/A) or Glu to (T/E, S/E), were incubated in the absence (-) or presence (+) of wild type LKB1:STRAD:MO25 in the presence of Mg²⁺ and ATP. After 30 min, the MARK kinases were assayed using the *AMARA* peptide, and results are expressed as specific activity. An aliquot of each incubation was also analysed by Western blotting probing with an anti-HA antibody to ensure equal loading of wild type and mutant MARK kinases (which all possessed an HA epitope tag). Results shown are average ± SD of a triplicate assay and results are representative of at least two independent experiments. (G) MARK4 was phosphorylated with LKB 1 complex as in A and the major ³²P-labelled peptide was analysed by solid phase Edman sequencing as in (B). In combination with MALDI TOF-TOF mass spectrometry (Fig 30C), this peptide was shown to comprise the T-loop of MARK4 phosphorylated at only the Thr residue.

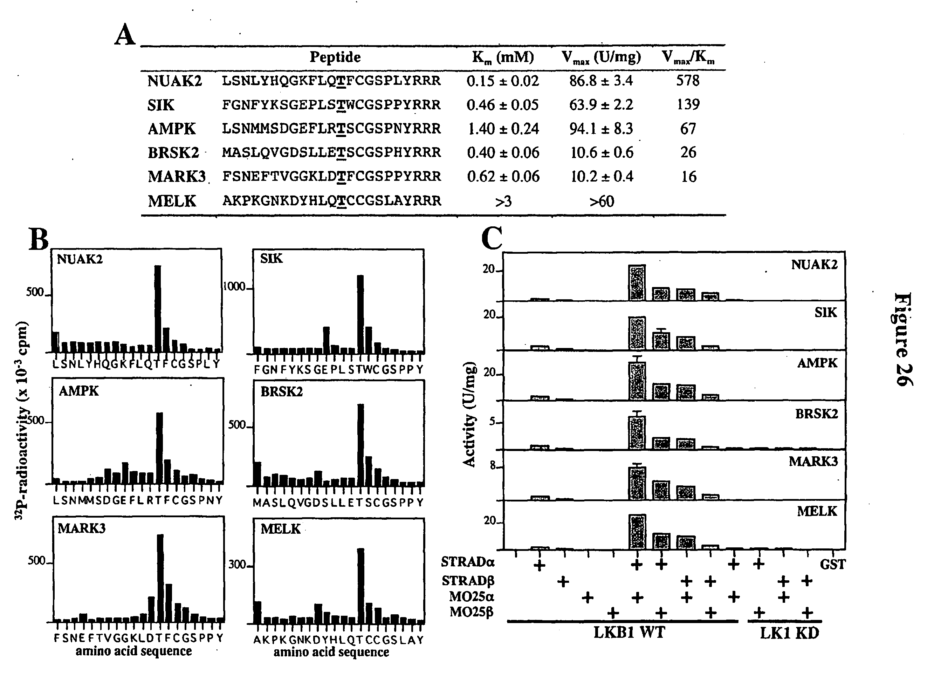

**Fig 26****. Identification of peptide substrates for LKB1.** (A) Kinetic analysis of the phosphorylation of the indicated T-loo by the LKB1:STRAD:MO25 complex was performed. The T-loop Thr residue in each peptide is underlined and in boldface type and 3 Arg residues were added to the C-terminus of each T-loop peptide to enable their capture on phosphocellulose p81 paper. Kₘ and Vₘₐₓ values were determined from nonlinear regression. (B) An aliquot of each peptide phosphorylated by LKB1 complex was subjected to solid phase Edman sequencing and ³²P-radioactivity was measured after each cycle of Edman degradation. A small proportion of each peptide can become coupled to the Sequelon arylamine membrane through acidic internal Asp and Glu residues rather than their C-terminal carboxyl group. This accounts for the apparent small releases of ³²P-radiactivity that are observed at some Asp and Glu residues. (C) The same combinations of GST-tagged wild type LKB1 (WT, lanes 1-9), or catalytically inactive (D194A, lanes 10-13) LKB1, or GST-alone (lane 14), FLAG-tagged STRADα or STRADβ, and *myc*-tagged MO25α or MO25β employed in Fig 22, were tested for their ability to phosphorylate the indicated peptides (peptide concentration 200 µM). The results are expressed as the peptide kinase activity generated per mg of LKB1:STRAD:MO25 added to the assay. Results shown are average ± SD of 3 assays and are representative of at least two independent experiments.

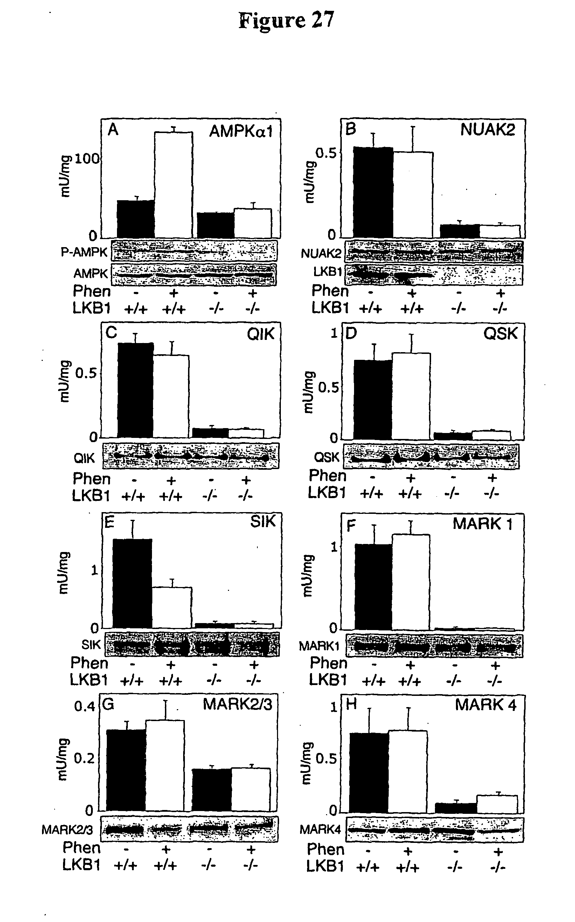

**Fig. 27** **Activity of AMPK-related kinases in LKB1^{+/+} and LKB1^{-/-} MEFs.** LKB1^{+/+} or LKB1^{-/-} MEFs were either left untreated (black bars) or stimulated with 10 mM phenformin (Phen, white bars) for 1h. AMPKα1 and AMPK-related kinases were immunoprecipitated from the cell lysates, and in vitro kinase activity towards the *AMARA* peptide was measured as described in Materials and Methods (Example 4). To confirm equal expression of the kinases in each sample, cell lysates (AMPKα1, NUAK2, MARK2/3 and MARK4) or immunoprecipitated proteins (QIK, QSK, SIK and MARK1) were subjected to SDS-PAGE and western blot analysis. All AMPK-related kinases migrated with the expected molecular mass. In the case of AMPKα1, cell lysates were immunoblotted with a phospho Thr172 antibody (P-AMPK), that recognises the phosphorylated T-loop. A control immunoblot of LKB1 levels in cell lysates is also included in panel B. Results shown are average + SD of 2-4 assays and are representative of at least two independent experiments.

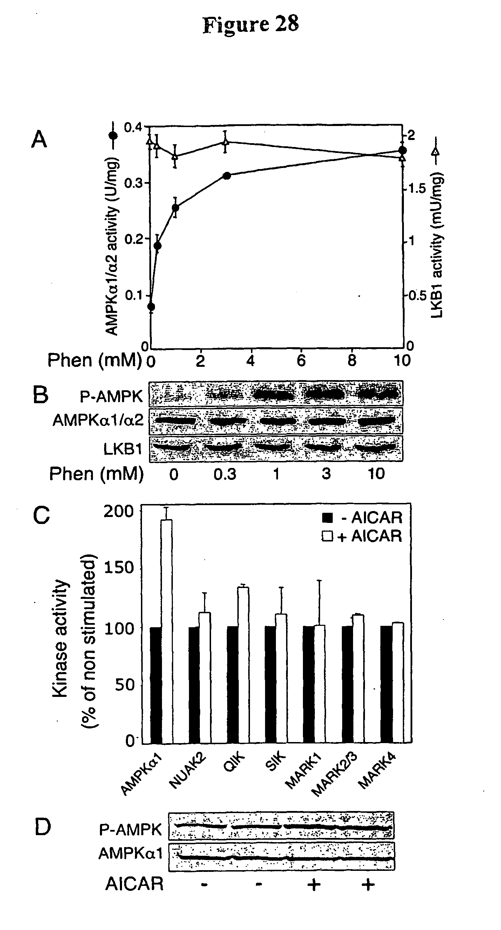

**Fig. 28****. AICA riboside does not activate AMPK-related kinases**. (A) LKB1^{+/+} MEFs were either left untreated or stimulated with the indicated concentrations of phenformin (Phen) for 1h. AMPKα1/α2 and LKB1 were immunoprecipitated from the cell lysates, and in vitro kinase activity towards the *AMARA* and LKBtide peptides respectively, were measured as described in Materials and Methods (Example 4). (B) To confirm equal expression of the kinases in each sample, cell lysates were subjected to SDS-PAGE and western blot analysis with the indicated antibodies. The phospho pT172 antibody (P-AMPK) recognises the phosphorylated T-loop of AMPKα1. Results are shown as the average ± SD for a triplicate assay and are representative of two independent experiments. (C) LKB1^{+/+} MEFs were either left untreated (black bars) or stimulated with 2 mM AICA riboside (AICAR, white bars) for 1h. AMPKα1 and the indicated AMPK-related kinases were immunoprecipitated from the cell lysates, and in vitro kinase activity towards the *AMARA* peptide measured as described in Materials and Methods. Results are presented as % relative to the activity observed in non-stimulated cells, and are averages +SEM of two independent experiments. 100% corresponds to the following absolute activities; AMPKα1; 135 mU/mg, NUAK2; 0.25 mU/mg, QIK; 0.76 mU/mg, SIK; 2.73 mU/mg; MARK1; 0.14 mU/mg, MARK2/3; 3.5 mU/mg and MARK4; 1.6 mU/mg. (D) Immunoblotting of AMPK was performed as in A.

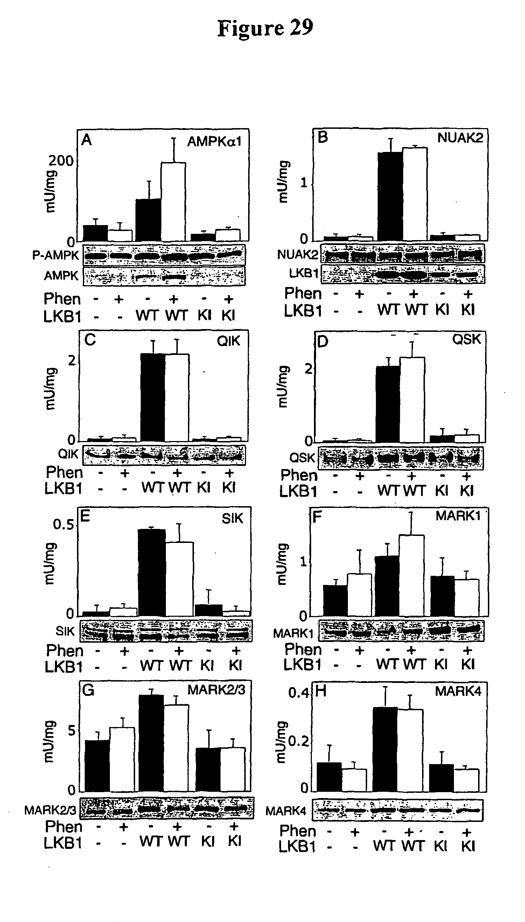

**Fig 29****. Activity of AMPK-related kinases in HeLa cells.** Control HeLa cells lacking LKB1 expression (-), or HeLa cells stably expressing wild type LKB 1 (WT) or kinase inactive LKB 1 (KI), were either left untreated (black bars) or stimulated with 10 mM phenformin (Phen, white bars) for 1h. AMPKα1 and AMPK-related kinases were immunoprecipitated from the cell lysates, and in vitro kinase activity towards the *AMARA* peptide was measured as described in Materials and Methods (Example 4). To confirm equal expression of the kinases in each sample, cell lysates (AMPKα1, MARK2/3 and MARK4) or immunoprecipitated proteins (NUAK2, QIK, QSK, SIK and MARK1) were subjected to SDS-PAGE and western immunoblot analysis. All AMPK-related kinases migrated with the expected molecular mass. In the case of AMPKα1, cell lysates were immunoblotted with a pThr172 antibody (P-AMPK), that recognises the phosphorylated T-loop. Results shown are average + SD of 2-4 assays and are representative of at least two independent experiments.
**Figure 30****. Analysis of phosphorylation of BRSK2, NUAK2 and MARK4 and MELK.** (A) Catalytically inactive mutants of BRSK2[D159A] and NUAK2[D193A], that are unable to autophosphorylate, were incubated with LKB1 complex for 30 min with Mg²⁺ -[γ³²P]ATP and separated by electrophoresis on a polyacrylamide gel which was then autoradiographed. The ³²P-labelled proteins were digested with trypsin and the resulting peptides were chromatographed on a C₁₈ column. Fractions containing the major ³²P-labelled peptides are marked. (B) Peptides P₁, P₂ and P₃ were subjected to solid phase sequencing and ³²P-radioactivity was measured after each cycle of Edman degradation. We were unable to determine the identity of the NUAK2 peptide indicated with "?". (C) The indicated peptides were analysed by MALDI TOF-TOF mass spectrometry as described above. The deduced amino acid sequence and the site of phosphorylation are indicated, together with the observed and theoretical mass. The peptide labelled "MARK4" was derived from MARK4 phosphorylated by LKB 1 as described in the legend to Fig 25G. This peptide encompasses the T-loop motif of MARK4 phosphorylated at only the Thr residue. The peptide labelled "MELK" is derived from a tryptic digest of unlabelled MELK that had been expressed and purified from E. *coli* as described above. This peptide encompasses the T-loop of MELK phosphorylated at the Thr residue. Abbreviations: m, methionine sulphone; (p) indicates preceeding Thr residue is phosphorylated.

### Example 1: MO25 isoforms interact with the STE20-related pseudokinases STRADα/β and enhance their ability to bind, activate and localise the LKB1 tumour suppressor in the cytoplasm

Mutations in the LKB 1 protein kinase result in the inherited Peutz Jeghers Cancer Syndrome. Research to date has indicated that LKB 1 functions as a tumour suppressor protein but still little is known about how LKB1 is regulated. Recent work revealed that LKB1 can be activated through its interaction with the STE20-related, catalytically inactive pseudokinase termed STRADα and that this interaction was required for LKB1 to suppress cell growth. In this Example we have found that the endogenous LKB1-STRADα complex immunoprecipitated from a number of mammalian cells is also associated with a 40-kDa protein of unknown function, containing no identifiable functional domains, termed MO25α. We show that MO25α does not bind directly to LKB1 but instead is associated with LKB1 through its interaction with STRADα. MO25α specifically recognises the last 3 residues of STRADα (Trp-Glu-Phe) as mutation or truncation of these amino acids abolishes the interaction of STRADα with MO25α. Cellular localisation studies indicate that MO25α and STRADα form a cytoplasmic complex anchoring LKB1 in the cytoplasm, excluding it from the nucleus. The amount of LKB1 associated with STRADα in cells is markedly increased by overexpression of MO25α, suggesting that MO25α enhances the formation of the LKB1-STRADα complex in vivo. We also establish that the association of LKB1 with STRADα and MO25α stimulates the catalytic activity of LKB1 nearly 10-fold. Moreover, we provide evidence that the closely related isoform of STRADα (also known as ILPIP or PAP kinase), which we named STRADβ, and MO25β isoforms are also able to stabilise LKB1 in an active complex, and we demonstrate that it is possible to isolate heterotrimeric complexes of LKB 1 bound to STRAD and M025 isoforms, in which the subunits are present in equimolar amounts. Our results indicate that the M025 may function as a scaffolding component of the LKB1-STRAD complex and play a crucial role in regulating LKB1 activity and cellular localisation mediated by its interaction with the STRAD pseudokinases.

### Results

### Identification of MO25α in an LKB1 complex.

In order to identify proteins associated with LKB1, we have utilised previously described HeLa cells stably expressing low levels or either the wild type or catalytically inactive LKB1 with an N-terminal Flag epitope tag to enable facile immuno-purification of LKB1-associated proteins employing the Flag antibody (Boudeau et al., 2003a). Flag-LKB1 was immuno-purified from one hundred 10 cm dishes of HeLa cell lysate derived from the cells expressing wild type LKB1 or kinase dead LKB1, or from the control parental HeLa cell line that does not express LKB1 (see Materials and Methods). The preparations were subjected to electrophoresis on a polyacrylamide gel, which was stained with colloidal blue (Fig 1A). A sample of the kinase dead LKB1 preparation was also concentrated further to enable better visualisation of lower abundance proteins. Several bands were observed which were present in both the wild type and kinase dead LKB1 preparations, but were absent in the control sample (Fig 1A). The identity of the colloidal blue-stained bands labelled in Figure 1A was established by tryptic peptide mass-spectral fingerprinting procedures (Fig 1B), and confirmed by immunoblotting with appropriate antibodies (Fig 1C). Proteins previously established to be associated with LKB1, including the Hsp90 and Cdc37 chaperone proteins (Boudeau et al., 2003a) and STRADα (Baas et al., 2003) were detected. In addition, a protein of ~40 kDa identified as MO25α, co-immuno-purified with both wild type and kinase dead LKB1 but was not present in the control purification (Fig 1A and 1C).

MO25α was first identified as a gene expressed at the early cleavage stage of mouse embryogenesis (Miyamoto et al., 1993) and was shown to be a highly evolutionary conserved protein (Karos and Fischer, 1999; Nozaki et al., 1996) for which no cellular function has been ascribed. From analysis of databases, we have identified a closely related isoform of M025α, which we termed MO25β, and a sequence alignment of both MO25 isoforms together with their putative homologues in *Drosophila* and *C*. *elegans* is shown in Figure 2A. Northern blot analysis indicated that M025α is widely expressed in human tissues, with highest levels of expression in skeletal muscle (Fig 2B). Immunoblot analysis using an antibody raised against the MO25α protein, which does not cross react with MO25β (Fig 2C & D), and analysis of EST databases (Table 1) confirmed that MO25α is expressed in many tissues and cell lines. Although we were unable to detect significant levels of MO25β RNA by Northern blot analysis, immunoblotting using an antibody raised against the MO25β protein, which does not cross react with MO25α, suggested that MO25β is also expressed in a variety of tissues and cell lines tested (Fig 2D). This is consistent with the observation that MO25β EST sequences have been identified from a number of human tissues (Table 1). However, expression of MO25β may be more restricted than MO25α, as it was not detected in liver and several cell lines including HeLa cells (Fig 2D), perhaps explaining why it was not co-purified with LKB1 expressed in these cells (Fig 1).

**Table 1. Tissue origin ofESTs encoding human MO25α and MO25β**

| *Protein* | | *Tissue NCBI Accession* |
|---|---|---|
| MO25α | duodenum, adenocarcinoma | NM_016289 |
| | leiomyosarcoma | BM474393, BU167673, BE884976 |
| | embryonal carcinoma | BQ433499, BM905560, BG259357 |
| | squamous cell carcinoma | BG679680 |
| | melanotic melanoma | BQ432236 |
| | mammary adenocarcinoma | BM013638, BG034567 |
| | chronic lymphotic leukemia | AI760873 |
| | ductal carcinoma | BU190932 |
| | osteosarcoma | BG108474 |
| | adenocarcinoma | BG250662, BG121835, AW188515 |
| | metastatic chondrosarcoma | CA436298 |
| | epithelioid carcinoma | BQ433038 |
| | retinoblastoma | BM480142 |
| | neuroblastoma | BE383169 |
| | uterus, tumor | BF095794 |
| | melanotic melanoma | BE891921, B0231688 |
| | astrocytoma | BM913690, BM560554 |
| | transitional cell papilloma | G260085 |
| | skin tumor | AA379224 |
| | testis | BG773506. BG719459 |
| | sympathetic trunk | BQ718408 |
| | marrow | BI019682, BI019684 |
| | normal pigmented retinal epithelium | BG750933, BM050770 |
| | cervix | BI089979 |
| | hypothalamus | AV722015, AV729226 |
| | fetal brain | BM927264, AA351866 |
| | senescent fibroblasts | W47588 |
| | infant brain | AA434013,, R21390, R46486 |
| | germinal center B cell | AA280522, AA280264, AA262054 |
| | ovarian tumor | AA442950. AA165271. AA164403 |
| | placenta | R67490, R66347, BG435220, |
| | osteosarcoma | BG025988 |
| | breast, normal | BE004664, AW373436 |
| | fetal liver spleen | T86658, T86848 |
| | cervix | BE538532 |
| | stomach | BM822360, BM833391, BM766941 |
| | fetal eyes | BM711209 |
| | purified pancreatic islet | BU079122, BU078843 |
| | prostate, normal | BE835801 |
| | lung | BM979124, BM982100, BM985320 |
| | kidney | AW235071, AA961656 |
| MO25β | prostate | BF680686 |
| | placenta, choriocarcinoma | BC010993, BE281207, BF107447 |
| | teratocarcinoma | AU125107, AU148840, AK022639 |
| | parathyroid, tumor | W37952 |
| | sciatic nerve | BQ899617 |
| | germinal center B cell | AA278473, AA279145 |
| | lung tumor | BF879769 |
| | epidermoid carcinoma | BQ669953 |
| | lung, normal | BF845952, BE042886, |
| | colon | BE928225, AW008806 |
| | metastatic chondrosarcoma | BQ021348 |
| | fibrosarcoma | CA441154 |
| | kidney | AW242839 |
| | stomach | BM820071, BM831835, BM822014 |
| | liver and spleen | AI247543 |
| | testis | AA781806 |
| | pancreas | AI956166 |
| | retina | AA001436, AA018841 |

**Endogenous MO25α is present in complex with endogenous LKB1.** We next immunoprecipitated endogenous LKB 1 from 293 cells (Fig 3A, left panel) or Rat-2 cells (Fig 3A, right panel), followed by immunoblotting for LKB1, STRADα and MO25α. The experiments showed that MO25α, as well as STRADα, were co-immunoprecipitated with LKB1, but not with preimmune IgG. We also immunoprecipitated endogenous MO25α from 293 cells (Fig 3B, left panel) or Rat-2 cells (Fig 3B, right panel), and immunoblotted for the presence of LKB1, STRADα and MO25α (Fig 3B). Endogenous LKB1 and STRADα were co-immunoprecipitated with MO25α, but not with preimmune IgG, consistent with the notion that these proteins form a complex.

### MO25 interacts with STRAD rather than with LKB1.

To verify which component of the LKB1-STRAD complex MO25α interacted with, we co-transfected 293 cells with constructs expressing MO25α and either N-terminal glutathione S-transferase (GST) tagged LKB1, STRADα or the closely related STRADβ pseudokinase, as well as Cdc37, a chaperone previously shown to bind LKB1 (Boudeau et al., 2003a). A sequence alignment of STRADα and STRADβ is shown in the Figure 10 and both lack the same key conserved residues in subdomain VI and VII in the kinase domain that are required for catalysis. The GST-tagged proteins were affinity purified and immunoblotted for M025α (Fig 4A). We found that MO25α interacts only with STRADα and STRADβ but not with LKB 1 or Cdc37.

We next expressed LKB 1 in 293 cells in the presence or absence of STRADα, and the protein composition of the affinity purified LKB 1 was analysed following electrophoresis and staining with colloidal blue. As expected, STRADα was co-purified with LKB1. However, in addition, a faintly staining band corresponding to an endogenous protein of ~40 kDa was observed in the LKB1-STRADα complex, which was not present in the uncomplexed LKB1 preparation. Tryptic peptide mass spectral fingerprinting revealed that this protein corresponded to endogenous MO25α. This was confirmed by immunoblotting with an anti-MO25α antibody (Fig 4B), further suggesting that MO25α is associated with LKB1 through STRADα. In Figure 4C, we demonstrate by performing appropriate co-transfections, that it is possible to purify a heterotrimeric LKB 1 complex in which LKB1, STRADα and MO25α are present in equivalent equimolar proportions. We also demonstrate that it is possible to form similar complexes of LKB1-STRADα-MO25β, LKB1-STRADβ-MO25α and LKB1-STRADβ-MO25β (Fig 4C), indicating that both isoforms of STRAD and MO25 are able to bind each other, as well as LKB1.

### MO25α cooperates with STRADα to localise LKB1 in the cytoplasm.

The cytoplasmic fraction of LKB1 was recently shown to conduct a G1 cell arrest (Tiainen et al., 2002). Therefore, we were interested in studying how he formation of a complex of LKB1, STRADα and MO25α affected the ocalisation of these proteins. HeLa cells were transfected with combinations of tagged MO25α, STRADα and LKB1 and these proteins were visualised in cells by confocal fluorescence microscopy in which the MO25α, STRADα and LKB1 proteins could be detected independently in the same cell. MO25α (Fig 5A) and STRADα (Fig 5E) expressed on their own were localised throughout the cytoplasm and nucleus. Strikingly however, when MO25α and STRADα were co-expressed, both proteins were re-localised to the cytoplasm and were excluded from the nucleus (Fig 5G & 5H). As reported previously in COS cells, LKB 1 expressed alone was mainly nuclear (Fig 5L) and co-expression with STRADα promoted significant cytoplasmic localisation of LKB1, however significant levels of LKB 1 remained in the nucleus under these conditions (Fig 5O) (Baas et al., 2003). In the presence of both MO25α and STRADα however, LKB 1 was essentially only localised in the cytoplasm and virtually excluded from the nucleus (Fig 5R). These observations indicate that MO25α and STRADα form a complex that anchors LKB1 in the cytoplasm more effectively than STRADα alone.

### MO25α recognises the last 3 residues of STRADα.

To define the binding site of MO25α on STRADα, a series of deletion mutations of STRADα were tested for their ability to interact with MO25α in a co-transfection binding assay. Strikingly, deletion of only the last 3 amino acids of STRADα (Trp-Glu-Phe) abolished binding of MO25α to STRADα. Moreover, mutation of the C-terminal Trp residue to Phe, also vastly reduced binding of MO25α to STRADα (Fig 6A). We then tested whether it was possible to affinity purify endogenously expressed MO25α from 3 different mammalian cell lysates employing biotinylated peptides encompassing the C-terminal residues of STRADα conjugated to streptavidin-Sepharose. We found that a peptide encompassing the C-terminal 12 residues (Fig 6B) but not the last 6 residues (data not shown and Fig 6C) of STRADα efficiently purified endogenous MO25α from all cell lysates tested (Fig 6B). To further delineate the MO25α binding site on the STRADα peptide, we performed an alanine scan, mutating each residue of the peptide individually to Ala and verifying how this affected binding to MO25α (Fig 6B). Consistent with the Trp-Glu-Phe residues being required for MO25α recognition, mutation of any of these 3 residues in the C-terminal STRADα peptide abolished or vastly reduced M025α binding in 3 different cell lysates, whereas mutation of the other residues either did not affect binding or only had a moderate effect. These findings were also confirmed in a more quantitative surface plasmon resonance Biacore binding assay (Fig 6C).

### Binding of LKB1 to STRADα creates novel binding site(s) for MO25α

We next investigated how binding of LKB1 to STRADα affected the interaction with MO25αusing a co-expression binding assay in 293 cells in which full length wild type and deletion mutants of GST-STRADα were co-expressed in the presence or absence of MO25α and LKB1. Consistent with previous findings, STRADα mutants that lack either the C-terminal 12 or 48 residues did not bind MO25α in the absence of LKB1 (Fig 7, panel 1). Strikingly though, when these STRADα mutants were co-expressed with LKB1, MO25α was recovered in the purified complexes (Fig 7, panel 2), indicating that the binding of LKB1 to STRADα generates additional binding site(s) for MO25α within the LKB1-STRADα complex. We also consistently noticed that the amount of LKB1 recovered in the GST-STRADα pull downs was significantly higher in the presence of MO25α (Fig 7, compare panels 2 and 3), suggesting that MO25α might stabilise the formation of a heterotrimeric LKB 1 complex. Moreover, a catalytic fragment of LKB1 encompassing only the kinase domain (residues 44-343) interacted much more efficiently with STRADα in the presence of MO25α (Fig 7, compare panels 4 and 5). These observations indicate that MO25α could be stabilising the LKB1-STRADα complex by generating additional MO25α binding site(s) on STRADα and/or LKB1. However, a mutant of STRADα lacking the C-terminal 88 residues that truncates a region of the pseudokinase domain, was no longer able to interact with LKB 1 or MO25α when these proteins were co-expressed (Fig 7). This suggested that either a secondary MO25α binding site is located towards the C-terminus of STRADα or that the integrity of the pseudokinase domain, which could be disrupted by truncation of the 88 C-terminal residues of STRADα, is required in enabling formation of the LKB1-STRADα-MO25α heterotrimeric complex.

### Further evidence that MO25 stabilises the LKB1-STRAD complex.

To obtain further evidence that M025α could stabilise the binding of LKB1 to STRADα, 293 cells were co-transfected with constructs expressing LKB 1 and STRADα in the absence or presence of increasing amounts of the MO25α DNA construct. LKB 1 was purified and the levels of associated STRADα and MO25α were measured by immunoblotting. As a control, the levels of expression of LKB1, STRADα and MO25α were also measured in the cell lysates prior to affinity purification of LKB1. In the absence of MO25α, a moderate amount of STRADα was associated with LKB1, which was markedly enhanced by increasing expression of MO25α in a dose dependent manner (Fig 8A, upper panel). Increased association of STRADα with LKB 1 was not due to increased expression of LKB 1 or STRADα as these were expressed in cell lysates at similar levels in the absence or presence of increasing amounts of MO25α (Fig 8A, lower panel). MO25β was similarly able to enhance the interaction between LKB 1 and STRADα (Figure 8B).
We also investigated how M025α and MO25β stabilised the binding of LKB1 to STRADβ (Fig 11). No detectable association of STRADβ was observed to LKB 1 in the absence of M025, whereas co-expression of MO25α or MO25β resulted in a dose dependent increase in STRADβ binding to LKB1. However, in contrast to STRADα, the levels of STRADβ expression in 293 cells were low in the absence of M025 isoforms and significantly increased in their presence, which could explain the increased binding of LKB1 to STRADβ in the presence of MO25 isoforms (Supplementary Fig 2). Similar results were obtained when STRADβ was expressed as a GST-fusion protein from a distinct expression vector (data not shown).

### Stabilisation of the LKB1-STRAD complex by MO25 isoforms correlates with increased activity.

To determine how the binding of MO25α and MO25β to the LKB1-STRADα or LKB1-STRADβ complex affected LKB1 activity, 293 cells were transfected with wild type GST-LKB1 in the presence or absence of STRAD and/or M025 isoforms. The activity of equal amounts of affinity purified GST-LKB1 was evaluated by measuring autophosphorylation activity as well as the transphosphorylation of myelin basic protein (MBP), a non-specific exogenous substrate of LKB1, in the presence of [γ-³²P]ATP. We also monitored MBP phosphorylation using a phospho-specific antibody that recognises the major LKB1 phosphorylation site on MBP (Thr65) (Baas et al., 2003). The different LKB1 activity assays gave comparable results (Fig 9). In the absence of STRAD isoforms, LKB 1 was poorly active (Fig 9, lane 1) and, as shown previously (Baas et al., 2003), co-expression with STRADα induced an ~4-fold increase in LKB 1 activity (Fig 9, lane 2). In the presence of STRADα and either MO25α or MO25β, LKB1 activity was increased a further ~2-fold so that LKB1 became ~9-fold more active than LKB1 expressed alone (Fig 9, lanes 6 or 7). LKB1 was only activated by STRADβ in the presence of M025 isoforms (Fig 9, compare lanes 3 with lanes 8 & 9), consistent with the observation that LKB1 is barely associated with STRADβ in the absence of MO25 (Fig 11). LKB 1 was previously shown to phosphorylate STRADα (Baas et al., 2003), and we show that the M025-mediated increase in the association of STRADα to LKB1 results in increased STRADα phosphorylation (Fig 9, compare lanes 2 with 6 & 7). In contrast to STRADα, STRADβ was not significantly phosphorylated by LKB1 in the presence of M025 (Fig 9, lanes 8 & 9). LKB1 phosphorylates STRADα at Thr329 and Thr419 (Baas et al., 2003), sites that are not conserved in STRADβ (Fig 10). This explains why STRADβ is not phosphorylated by LKB1. We also showed that catalytically inactive LKB1 is able to associate with both STRAD isoforms in the presence of either isoform of M025. However, as expected, it is unable to autophosphorylate or phosphorylate STRADα or MBP (Fig 9, lanes 10-13).

### Discussion.

One of the major findings of this study is the discovery that the highly conserved MO25α protein found in many eukaryotic species, for which no function had been ascribed to previously, is present in a complex with STRADα and LKB1 in vivo. Interestingly, in the absence of LKB1, MO25α specifically recognises the last 3 amino acids of STRADα that lie in a Trp-Glu-Phe sequence, and mutation of any of these 3 residues to Ala essentially prevented the binding of MO25α to STRADα. In this regard, MO2Sα functions similarly to a PDZ domain, which also recognises the extreme C-terminal residues of its protein binding partners (Songyang et al., 1997). However, there is no homology between a PDZ domain and MO25α and, to our knowledge, other domains characterised thus far do not possess this property. The C-terminal non-pseudokinase domain ~50 residues of STRADα and STRADβ are poorly conserved but, significantly, both terminate with the Trp-Glu-Phe sequence (Fig 10), emphasising that STRADα and STRADβ have probably evolved to interact specially with MO25 isoforms. The most closely related proteins to STRADα and STRADβ are the active STE20 member kinases termed SPAK (Johnston et al., 2000) and OSR1 (Tamari et al., 1999), which possess all residues required for protein kinase catalysis (Fig 10) and are therefore active enzymes. Although neither SPAK nor OSR1 terminate in a Trp-Glu-Phe sequence, both possess a Phe-Glu-Phe sequence at a similar region of their C-terminal non-catalytic domain (Fig 10). This observation prompted us to mutate the C-terminus Trp-Glu-Phe sequence of STRADα to Phe-Glu-Phe and we found that this prevented MO25α from binding to STRADα (Fig 6A). This further emphasizes that the presence of a Trp residue three residues from the end of the protein is essential for binding of STRADα to MO25α, suggesting that MO25α will not interact with SPAK or OSR1.

Our data indicate that one of the key roles of MO25α is to stabilise the interaction between LKB1 and STRADα. This is based on the observation that the amount of STRADα associated with LKB1 in cells is significantly enhanced by the expression of MO25α (Fig 8). The finding that STRADα lacking the C-terminal Trp-Glu-Phe residues can form a complex with MO25α in the presence of LKB1 (Fig 7) indicates that the interaction of LKB1 with STRADα generates novel binding site(s) for MO25α, which could explain how MO25α stabilises the association of LKB1 with STRADα. Our findings do not enable us to discriminate whether the additional MO25α binding site(s) are located within STRADα and/or LKB1. Detailed structural studies of this complex will be required to address this issue. Our observations are consistent with the notion that MO25α or MO25β may function as a scaffolding component of the LKB1-STRAD complex.

It has previously been shown that co-expression of STRADα with LKB1 enhanced LKB1 in vitro activity (Baas et al., 2003) and here we showed that the association of LKB1-STRAD with MO25 isoforms further enhanced LKB1 activity 2-fold (Fig 9). These results indicate that stabilisation of the LKB1-STRADα complex by MO25 is accompanied by activation of LKB1. Importantly, however, it should be noted that the complex of LKB1 and STRADα purified from mammalian 293 cells overexpressing tagged LKB1 and STRADα is also associated with significant levels of endogenous MO25α (Fig 4B). It is therefore not possible to measure the activity of the LKB1-STRADα complex in the absence of MO25α by expression of these proteins in mammalian cells. Thus we cannot quantify how association of STRADα with LKB 1 in the complete absence of MO25α affects LKB1 activity. To overcome the problem of association of overexpressed STRADα with endogenous MO25α in mammalian cells, we have attempted to combine E.coli expressed LKB1, STRADα and MO25α to see whether we could form a complex in which LKB1 could be activated. However, this approach was unsuccessful (JB data not shown), which indicate that the association of LKB1 with STRADα and MO25α is a complicated process that may require additional factors and/or that this complex may need to be assembled in vivo. We were able to isolate from mammalian cells a heterotrimeric complex in which LKB1, STRAD and M025 subunits are present at a similar stoichiometry (Fig 4C), suggesting a relatively high affinity of the individual subunits for each other.

We also demonstrated that MO25α and STRADα, when expressed alone in cells, are both localised in the cytoplasm and nucleus, but they are only localised in the cytoplasm and excluded from the nucleus when expressed together (Fig 5). The molecular mechanism underlying this observation has not been investigated. It is possible that the interaction of MO25α and STRADα leads to the masking of a nuclear localisation signal, exposure of a novel nuclear export signal or cytoplasmic anchoring motif, or that the STRADα-MO25α complex is to large to freely translocate into the nucleus. Under the conditions we have performed our localisation studies in HeLa cells, we found that MO25α significantly cooperates with STRADα to localise LKB1 in the cytoplasm of cells, excluding it from the nucleus, as the nuclear exclusion and cytoplasmic localisation of LKB1 observed following expression of LKB 1 and STRADα was markedly enhanced in the presence of MO25α (Fig 5). Previous studies in COS cells also showed that STRADα could localise LKB1 in cytoplasm of cells (Baas et al., 2003). However, as our data indicate that significant levels of endogenous MO25α will bind to the LKB1-STRADα complex (Fig 4B), it is not possible to rule out that endogenous MO25 isoforms could contribute to cytoplasmic localisation of LKB1 when it is co-transfected with STRADα. Cytoplasmic localisation of LKB 1 may be important, as mutants of LKB 1 that can not enter the nucleus still suppress cell growth (Tiainen et al., 2002). Additional mechanisms may also exist to maintain LKB1 in the cell cytoplasm. The interaction of LKB 1 with the LIP 1 protein has been shown to induce LKB I cytoplasmic localisation (Smith et al., 2001). It has yet to be tested whether LIP1 can interact with the heterotrimeric LKB1-STRAD-MO25 complex, but LIP1 has not been reported to influence LKB1 activity. LKB1 is also prenylated at its C-terminus (Collins et al., 2000; Sapkota et al., 2001), which could promote interactions with cell membranes. Recent work has indicated that prenylation of the *Drosophila* LKB1 homologue is important for association of LKB 1 with membranes in controlling cell polarity (Martin and St Johnston, 2003).

Putative M025 homologues are found not only in mammals, *Drosophila* and *C.elegans* but also found in fission yeast (NCBI accession number NP_594685, 51% identity to human MO25α), budding yeast (HYM1, NCBI accession number P32464, 33% identity to human MO25α) and plant (NCBI accession number Q9M0M4, 43% identity to human MO25α). To our knowledge only the putative budding yeast M025 homologue termed HYM1, has been investigated and apparently plays a key role in regulating polarized cell growth and cell separation (Bidlingmaier et al., 2001; Dorland et al., 2000; Jorgensen et al., 2002). To our knowledge, LKB 1 and STRAD homologues have not been reported in yeast and thus it is not clear whether there could be a link between LKB1 regulating polarity in *C. elegans* and *Drosophila* (Martin and St Johnston, 2003; Watts et al., 2000) and HYM1 regulating cell polarity in yeast.

It is also possible that M025 could have functions that are independent of its ability to regulate LKB1. M025 could interact with other proteins terminating in a similar motif to STRADα/STRADβ and there are over 20 human proteins that terminate in a Trp-Glu/Asp-Phe motif. It may be interesting to investigate whether any of these bind M025 isoforms. It is also probable that STRADα and STRADβ have functions other than regulating LKB1. In this regard, recent work has shown that overexpression of STRADβ in 293 cells protected them against apoptosis by enl ancing the ability of the JNK pathway to be activated by overexpression of XIAP, an upstream component of the JNK pathway (Sanna et al., 2002). Moreover, in another study, overexpression of STRADβ was reported to lead to activation of JNK and p38γ MAP kinases (Nishigaki et al., 2003). The mechanism by which STRADβ enhanced JNK/p38γ activation was not defined in these studies and it would be of interest to test whether LKB1 and/or M025 is involved in these processes.

A significant number of inherited forms of PJS found in certain families do not exhibit mutations in the LKB1 genes (Buchet-Poyau et al., 2002), indicating that there is a second causative locus for PJS. Genetic analysis to date has excluded LIP1 and provided no evidence that other proteins reported to interact with LKB 1 are mutated in these patients (Buchet-Poyau et al., 2002; Resta et al., 2002). The results in this study indicate that it would be worthwhile to verify whether mutations in the genes encoding STRAD or MO25 isoforms occur in any of the PJS families that do not have mutations in the LKB 1 gene.

In conclusion, we define M025 as a novel component of the LKB1 complex in vivo. Our results indicate that isoforms of M025 function to stabilise the interaction of LKB 1 with the catalytically inactive STRAD pseudokinase isoforms. This markedly enhances LKB1 activity and its cytoplasmic localisation. In future studies to further define the physiological functions of the M025 and STRAD homologues in regulating LKB1 function, it would also be important to generate mice lacking these genes. The ability to express more active forms of LKB 1 heterotrimeric complexes also opens up the opportunity to employ biochemical approaches to search for novel LKB1 substrates.

**Materials**. Protease-inhibitor cocktail tablets were obtained from Roche. Dimethyl pimelimidate and the anti-Flag M2-agarose affinity gel were purchased from Sigma. Tetracyclin-free foetal bovine sera (FBS) was from Perbio, and other tissue culture reagents were from Biowhittaker unless otherwise stated. Precast SDS/4-12% and 10% polyacrylamide Bis-Tris gels and myelin basic protein (MBP) were obtained from Invitrogen. Sensor chips SA were from BiaCore AB (Stevenage, UK). [γ-³²P]ATP, Glutathione-Sepharose, Protein G-Sepharose, and Streptavidin-Sepharose High Performance were purchased from Amersham Biosciences.
**Peptides**. The biotinylated peptides STRADa-C12 (Biotin-C6spacer-NLEELEVDDWEF), and STRADα-C6 (Biotin-C6spacer-VDDWEF) whose sequences encompass the last 12, and 6 residues of STRADα respectively, the Flag peptide (DYKDDDDK), and peptides used to raise antibodies were synthesised by Dr G. Bloomberg (University of Bristol, UK). The biotinylated peptides encompassing the last 12 residues of STRADα in which each residue was individually replaced by an Alanine were synthesized using previously described methodology (Lizcano et al., 2002).
**Antibodies.** The LKB1 antibody used for immunoblotting was raised against the mouse LKB1 protein as described previously (Sapkota et al., 2001). As this antibody does not immunoprecipitate efficiently the human LKB1 protein, we raised an antibody in sheep against the human LKB1 protein expressed as a GST-fusion protein in E. *coli,* that efficiently immunoprecipitated human and rat LKB 1. This antibody was used for all the immunoprecipitating experiments in this study. The anti-MO25α and anti-MO25β antibodies were raised in sheep against the MO25α and MO25β human proteins expressed in *E. coli* as described below. The LKB1 and M025 antibodies were affinity-purified on CH-Sepharose covalently coupled to the protein antigen used to raise the antibody. The monoclonal antibody recognising the STRADα protein has been described previously (Baas et al., 2003). The phospho-specific antibody recognizing MBP phosphorylated at Thr65 (T65-P) was raised in sheep against peptide GHHAARTTHYGSLPQ corresponding to residues 58-72 of bovine MBP in which the underlined residue is phosphothreonine. The antibody was affinity-purified on CH-Sepharose covalently coupled to the phosphorylated peptide and then passed through a column of CH-Sepharose coupled to the non-phosphorylated peptide. Antibody that did not bind to the latter column was selected. Monoclonal antibodies recognizing the GST and Flag epitope tags were obtained from Sigma, the monoclonal antibody recognizing the Myc epitope tag was purchased from Roche, and secondary antibodies coupled to horseradish peroxidase used for immunoblotting were obtained from Pierce. Preimmune IgG used in control immunoprecipitation experiments was obtained from preimmune serum employing Protein G-Sepharose.
**General methods and buffers**. Restriction enzyme digests, DNA ligations, and other recombinant DNA procedures were performed using standard protocols. DNA constructs used for transfection were purified from *E. coli* DH5α using Qiagen plasmid Mega kit according to the manufacturer's protocol. All DNA constructs were verified by DNA sequencing, which was performed by The Sequencing Service, School of Life Sciences, University of Dundee, Scotland, UK, using DYEnamic ET terminator chemistry (Amersham Biosciences) on Applied Biosystems automated DNA sequencers. Lysis Buffer contained 50 mM Tris/HCl pH 7.5, 1 mM EGTA, 1 mM EDTA, 1% (w/v) Triton-X 100, 1 mM sodium orthovanadate, 50 mM sodium fluoride, 5 mM sodium pyrophosphate, 0.27 M sucrose, 0.1% (v/v) 2-mercaptoethanol and 'complete' proteinase inhibitor cocktail (one tablet/50 ml). Buffer A contained 50 mM Tris/HCl pH 7.5, 0.1 mM EGTA, and 0.1% (v/v) 2-mercaptoethanol. SDS sample Buffer contained 50 mM Tris/HCl pH 6.8, 2% (w/v) SDS, 10% (v/v) glycerol, 0.005% (w/v) bromophenol blue, and 1% (v/v) 2-mercaptoethanol.
DNA constructs. Constructs expressing the full length wild type and kinase dead mouse LKB1 have been described previously (Sapkota et al., 2002a; Sapkota et al., 2002b; Sapkota et al., 2001). In order to generate the N-terminal Myc-tagged MO25α cDNA, a PCR was performed with primers: 5'-ggatccgccaccatggagcagaagctgatctctgaagaggacttgccgttcccgtttgggaagtctcacaaa t-3' and
5'-ggatccttaagcttcttgctgagctggtctcttc-3' using an IMAGE Consortium EST clone (IMAGE clone 4822388, NCBI Acc. BG719459) as a template. The resulting PCR product was ligated into pCR2.1-TOPO vector (Invitrogen), and subcloned as a *Bam*H1- *Bam*H1 fragment into the pEBG-2T (Sanchez et al., 1994), pCMV5 (Anderson et al., 1989) and pGEX-6T (Amersham) vectors. To generate the N-terminal Myc-tagged MO25β cDNA, a PCR was performed with primers
5'-caccggatccgccaccatggagcagaagctgatctctgaagaggacttgcctttgtttagtaaatcacacaa aaatcc-3' and 5'- ggatcctcaaggggccgttttcttcaagtctcgg-3' using an IMAGE Consortium EST clone (IMAGE 1958217, NCBI Acc. AI252223) as a template. The resulting PCR product was ligated into pENTR/D-TOPO Gateway intermediate vector (Invitrogen), and converted into Gateway modified pEBG-2T, pCMV5 and pGEX-6T expression vectors. It should be noted that the human MO25β IMAGE clone we obtained encodes for an Ala instead of a Val at position 153, compared to the submitted sequence (NCBI accession number Q9H9S4). Sequence analysis of the nucleotide sequence of several independent MO25β sequences listed Table 1, indicated that all of these possess an Ala at position 153. Expression constructs for Flag-tagged STRADα were PCR amplified with primers 5'-ggatccgccaccatggactacaaggacgacgatgacaagtcatttcttgtaagtaaaccagagcgaatc-3' and 5'- ggatcctcagaactcccaatcgtccacctccagct-3 using a human STRADα cDNA as a template (Baas et al., 2003). The PCR product was ligated into pCR2.1-TOPO vector and subcloned as a *Bam*H1*-Not*1 fragment into pEBG-2T or as a *Bam*H1*-Xba*1 insert into pCMV5.

In order to clone STRADβ, two IMAGE EST clones (5301936 and 5501540), which together encompass the entire coding sequence were used as templates to generate full length flag-tagged STRADβ cDNA by PCR using primers 5'-ggatccgccaccatggactacaaggacgacgatgacaagtctcttttggattgcttctgcacttcaag-3' and
5'-ggatccctagaattcccagtatgagtctttttcatc-3'. The PCR product was ligated into pCR2.1-TOPO vector and subcloned as a BamH1-*Bam*H1 fragment into the pEBG-2T and pCMV5 vectors. The catalytic fragment of mouse LKB1 (residues 44-343) possessing an N-terminal Flag epitope tag was PCR amplified from a mouse LKB1 cDNA (Sapkota et al., 2001) using the primers: 5'-actagtgccaccatggactacaaggacgacgatgacaagaagctcatcggcaagtacctgatgggg-3' and 5'-actagttcagtcctccaggtagggcactacagtcat-3' and subcloned into pCMV5 vector as an *Spe*I*-Spe*I fragment. The construct encoding for the expression of GFP tagged human LKB1 with no other epitope tag was described previously (Boudeau et al., 2003b). Site directed mutagenesis was performed employing standard procedures using the Quickchange mutagenesis kit (Stratagene). Deletion mutations were generated by introducing STOP codons at the indicated positions.

**Cell culture conditions and cell lysis**. The generation of HeLa cells stably expressing wild type or kinase dead LKB 1 has been described previously (Sapkota et al., 2002b). The cells were cultured in Minimum Essential Medium Eagle supplemented with 10% (v/v) tetracyclin-free FBS, in the presence of 1 X non-essential amino acid solution, 1 X Penicillin/Streptomycin solution (Invitrogen), 100 µg/ml zeocin and 5 µg/ml blasticidin (Invitrogen). Human embryonic kidney (HEK) 293, embryonic fibroblast Rat-2 cells, and HeLa cells were maintained in Dulbecco's modified Eagle's medium supplemented with 10% (v/v) FBS. For all experiments, cells were cultured on a 10-cm diameter dish and lysed in 0.5 to 1 ml of ice-cold Lysis Buffer. Lysates were clarified by centrifugation at 4° C for 10 min at 14 000 g.

**Immunoblotting**. The amounts of proteins indicated in the figure legends were subjected to SDS-polyacrylamide gel electrophoresis and transferred to nitrocellulose. The membranes were blocked for 1 hour in 50 mM Tris/HCl (pH 7.5), 0.15 M NaCl, 0.5% (v/v) Tween containing 10% (w/v) skimmed milk powder for 1 h. The membranes were then incubated in the same buffer containing 1 µg/ml antibody (LKB1, MO25α and MO25β antibodies) or 0.1 µg/ml (STRADα antibody) for 8 h at 4 °C. Immunoblotting with antibody T65-P was carried out as described above, except that the non-phosphorylated peptide corresponding to the antigen used to raise the antibody was included at a concentration of 10 µg/ml. Immunoblotting with the GST, Flag, and Myc antibodies was carried out as above with 0.5 µg/ml antibody except that skimmed milk powder was replaced with 5% (w/v) BSA. Detection was performed using the appropriate horseradish peroxidase-conjugated secondary antibodies and the enhanced chemiluminescence reagent (Amersham Pharmacia Biotech).

**Immunoprecipitation of endogenous LKB1 and MO25α.** The LKB1 and MO25α antibodies were covalently coupled to protein G-Sepharose in a ratio of 1 mg of antibody to 1 ml of resin using a dimethyl pimelimidate cross-linking procedure (Harlow and Lane, 1988). Clarified lysates of 293 and Rat-2 cells generated as described above except that 2-mercaptoethanol was omitted from the lysis buffer, containing 2 mg of total cell protein were incubated at 4°C for 1 h on a vibrating platform with 10 µl of the LKB1-protein G-Sepharose conjugate or 15 µl of the MO25α -protein G-Sepharose conjugate. The immunoprecipitates were washed twice with 1 ml of Lysis Buffer (not containing 2-mercaptoethanol) containing 150 mM NaCl and twice with 1 ml of Buffer A (not containing 2-mercaptoethanol). The beads were then resuspended in a volume of 20 µl of Buffer A to which 5 µl of SDS sample buffer lacking 2-mercaptoethanol was added. The samples were subjected to electrophoresis and then immunoblotted for LKB 1, STRADα and MO25α as described above.

**Expression of GST-fusion proteins in 293 cells and affinity purification.** 10-cm diameter dishes of 293 cells were transiently transfected with 3 to 10 µg of the pEBG-2T constructs using a modified calcium phosphate method (Alessi et al., 1996). 36 h post-transfection, the cells were lysed and the clarified lysates were incubated for 1 h on a rotating platform with glutathione-Sepharose (25 µl/dish of lysate) previously equilibrated in lysis Buffer. The beads were washed 4 times with lysis Buffer containing 150 mM NaCl and twice with Buffer A. For immunoblotting analysis, the beads were then resuspended in SDS sample Buffer after this step to give a final volume of slurry of 75 µl, and the samples immunoblotted as described above. For protein kinase assays and gel electrophoresis, the beads were washed a further twice with Buffer A containing 0.27 M sucrose and the proteins were eluted from the resin by incubation with the same buffer containing 20 mM of glutathione. The beads were then removed by filtration through a 0.44-µm filter and the eluate divided into aliquots and stored at - 80° C.

**Expression and purification of GST-M025 isoforms in E.** ***coli.*** The pGEX-6T constructs encoding N-terminally GST tagged MO25α or GST tagged MO25β were transformed into *E. coli* BL21 cells and a 0.5-liter culture was grown at 37°C in Luria-Bertani broth until the absorbance at 600 nm was ~0.7. Isopropyl-β-D-thiogalactopyranoside was then added at a concentration of 250 µM to induce protein expression, and the cells were cultured for a further 16 h at 26°C. The cells were harvested by centrifugation for 30 min at 5 000 g and resuspended in 25 ml of Lysis Buffer. Lysis was completed by one round of freeze/thawing and ten 15-sec cycles of sonication. The lysate was centrifuged at 4°C for 30 min at 20 000 g, the supernatant filtered through a 0.44 µm filter and incubated for 1 h on a rotating platform with 1 ml of glutathione-Sepharose previously equilibrated in lysis Buffer. The beads were washed 4 times with lysis Buffer containing 150 mM NaCl, twice with Buffer A and twice with Buffer A containing 0.27 M sucrose. In order to elute MO25 isoforms from the resin, 15 µg of GST tagged PreScission protease was added which cleaves the N-terminal GST tag on the GST-fusion proteins. The resin was incubated with the protease for 16 h at 4 °C on a rotating platform and the eluted M025 proteins collected by filtration through a 0.44-µm filter. The eluted M025 isoforms were further incubated with 0.25 ml of glutathione-Sepharose for 30 min at 4°C to remove any traces of uncleaved GST-fusion protein and GST-PreScission protease. 1 mg of homogenous M025 was typically isolated and after snap freezing in liquid nitrogen stored in aliquots at -80°C.
**Purification of Flag-LKB1 and mass spectrometry analysis.** Flag-LKB1 was immuno-purified from HeLa cells stably expressing wild type LKB1 or kinase dead LKB1, or from control HeLa cells using anti-Flag M2-affinity agarose gel as described previously (Boudeau et al., 2003a). For each purification, hundred 10 cm diameter dishes of each HeLa cell line was employed and yielded a 0.4 ml of eluted affinity purified LKB1 solution. 40 µl of each sample (Fig 1A lane 1-3) were electrophoresed on a precast 4-12% SDS-polyacrylamide gel, and the gel stained with colloidal blue and photographed. For the concentrated sample (Fig 1A, lane 4), 250 µl of the KD-LKB1 preparation was methanol precipitated, resuspended in 1X SDS Sample Buffer and analysed. The bands indicated in Figure 1A were excised, washed and digested with trypsin as described previously (Woods et al., 2001). Tryptic peptides were analysed by MALDI-TOF mass spectrometry, using α-cyanocinnamic acid as the matrix, on a PerSeptive Biosystems Elite STR mass spectrometer. Spectra were acquired in the reflectron mode, internally calibrated and the tryptic peptide masses were scanned against NCBInr and SwissProt databases using the MS-FIT program of Protein Prospector (UCSF, CA) run on a local server. The identity of STRADα and MO25α was also confirmed by LC-MS/MS sequence analysis on a Q-TOF2 mass spectrometer as described previously (Way et al., 2002).
**Affinity purification of MO25α with peptides**. Streptavidin-Sepharose previously equilibrated in PBS was incubated with each biotinylated peptide in a ratio of 1 µg of peptide tol µl of resin on a vibrating platform for 30 min, and the beads were then washed twice with PBS to remove any unconjugated peptide. The indicated cell lysates (0.5 mg) were incubated with Streptavidin-Sepharose peptide conjugate (5 µl) for 1 h at 4°C on a vibrating platform. The beads were washed twice with 1 ml of Lysis Buffer containing 150 mM NaCl and twice with 1 ml of Buffer A, and then resuspended in a volume of 20 µl of Buffer A to which 5 µl of SDS sample buffer was added. The samples were then subjected to electrophoresis and immunoblotted for MO25α as described above.
**Biacore analysis**. Binding was analysed in a BiaCore 3000 system (BiaCore AB, Stevenage, UK). The indicated biotinylated peptides were bound to a streptavidin-coated Sensor chip (SA) (20 response units, RU). Various concentrations of bacterially-expressed M025α previously dialysed in buffer HBS-EP (10 mM HEPES pH 7.4, 0.15 M NaCl, 3 mM EDTA, 0.005% (v/v) polysorbate-20), were injected over the immobilised peptides at a flow rate of 30 µl/min. Interactions between each peptide and the MO25α protein were analysed over a range of MO25α concentrations of 6.25-3200 nM and steady state binding was determined at each concentration. Dissociation of MO25α from each peptide was monitored over a 1 min period. Regeneration of the Sensor chip surface between each injection was performed with 3 consecutive 5-µl injections of 10 mM NaOH, 1 M NaCl.

**LKB1 autophosphorylation and phosphorylation of MBP.** 0.5 µg of wild-type or catalytically inactive GST-LKB1 of the indicated complexes of LKB1 (Figure 8) that had been affinity purified on glutathione-Sepharose from 293 cells, was incubated in a 30-µl reaction mixture containing 50 mM Tris-HCl (pH 7.5), 0.1% 2-mercaptoethanol, 0.1 mM EGTA, 10 mM manganese chloride, 0.5 µM microcystin-LR, and 100 µM [γ-³²P]ATP (1000 cpm/pmol) in the presence of 3.3 µg of bovine MBP. After incubation for 40 min at 30°C, the reactions were terminated by the addition of 8 µl of sample Buffer. 30 µl was electrophoresed on SDS-polyacrylamide gels, which were then dried and analysed by autoradiography to quantify LKB 1 autophosphorylation, MBP and STRADα phosphorylation. 5 µl of each reaction was diluted in 45 µl of 1 X SDS Sample Buffer. Of the resulting solution, 10 µl were used for immunoblotting with the anti-GST (recognises LKB1), anti-Flag (recognises STRADα and STRADβ) and anti-Myc (recognises MO25α and MO25β) antibodies. 30 µl of the sample was used for immunoblotting with the T65-P MBP antibody.
**Localisation studies**. HeLa cells were cultured to 50% confluence on glass coverslips (no. 1.5) on 10-cm diameter dishes and transfected with a total of 2 µg of a construct encoding wild-type GFP-LKB1 together with the indicated pCMV5 constructs using Effectene transfection reagent (Qiagen) according to the manufacturer's protocol. A duplicate set of dishes was used for each condition. The cells were washed with PBS 20 h post-transfection, and were fixed for 10 min in freshly prepared 4% (v/v) paraformaldehyde in PHEM Buffer (60 mM Pipes, 25 mM Hepes, 10 mM EGTA and 2 mM magnesium sulphate, pH 7.0). The cells were then washed twice with PBS and permeablised with 1% (by vol) NP40 in PBS and blocked with 10% normal goat serum. The cells were immunolabelled with both the anti-MO25α antibody and anti-Flag antibody (to detect Flag-tagged STRADα), washed in PBS and counter stained with Texas Red anti-sheep IgG and Cy5 anti-mouse IgG antibodies. The cells were imaged using a Zeiss LSM 510 META confocal microscope. Each channel was scanned independently to avoid cross talk (Multi-Tracking).
**Northern Blot Analysis.** A cDNA corresponding to human MO25α (NCBI Acc, N. NM_016289, nucleotide residues 1550 to 2500), was ³²P-labelled by random priming using a Hi Prime DNA labelling kit (Roche). This probe was used to screen a Multiple Tissue Northern blot (Clontech, PT1200-1) using Rapid-Hyb Buffer (Amersham Pharmacia) according to the protocol provided by the manufacturer. Following autoradiography, the probe was stripped and the blot was then re-hybridised as above with a β-actin probe provided by the manufacturer.

### References

Alessi, D.R., Caudwell, F.B., Andjelkovic, M., Hemmings, B.A. and Cohen, P. (1996) Molecular basis for the substrate specificity of protein kinase B; comparison with MAPKAP kinase-1 and p70 S6 kinase. FEBS Lett, 399, 333-338.
Anderson, S., Davie, D.N., Dahlbäck, H., Jörnvall, H. and Russell, D.W. (1989) Cloning, structure, and expression of the mitochondrial cytochrome-P- 450 sterol 26-hydroxylase, a bile-acid D biosynthetic enzyme. J. Biol. Chem., 264, 8222-8229.
Harlow, E. and Lane, D. (1988) Antibodies a Laboratory Manual. Cold Spring Harbor Laboratory Publications, New York.
Lizcano, J.M., Deak, M., Morrice, N. and Alessi, D.R. (2002) Identification of the residues of S6K, SGK and PKB that are phosphorylated by NEK6. Manuscript in Preparation*.*
Sanchez, I., Hughes, R.T., Mayer, B.J., Yee, K., Woodgett, J.R., Avruch, J., Kyriakis, J.M. and Zon, L.I. (1994) Role of SAPK/ERK kinase-1 in the stress-activated pathway regulating transcription factor c-Jun. Nature, 372, 794-798.
Way, G., Morrice, N., Smythe, C. and O'Sullivan, A.J. (2002) Purification and identification of secernin, a novel cytosolic protein that regulates exocytosis in mast cells. Mol Biol Cell, 13, 3344-3354.
Woods, Y.L., Rena, G., Morrice, N., Barthel, A., Becker, W., Guo, S., Unterman, T.G. and Cohen, P. (2001) The kinase DYRK1A phosphorylates the transcription factor FKHR at Ser329 in vitro, a novel in vivo phosphorylation site. Biochem J, 355, 597-607.
Baas, A.F., Boudeau, J., Sapkota, G.P., Smit, L., Medema, R., Morrice, N.A., Alessi, D.R. and Clevers, H.C. (2003) Activation of the tumour suppressor kinase LKB1 by the STE20-like Pseudokinase STRAD.EMBO J 22(12), 3062-3072
Bardeesy, N., Sinha, M., Hezel, A.F., Signoretti, S., Hathaway, N.A., Sharpless, N.E., Loda, M., Carrasco, D.R. and DePinho, R.A. (2002) Loss of the Lkb1 tumour suppressor provokes intestinal polyposis but resistance to transformation. Nature, 419, 162-167.
Bidlingmaier, S., Weiss, E.L., Seidel, C., Drubin, D.G. and Snyder, M. (2001) The Cbklp pathway is important for polarized cell growth and cell separation in Saccharomyces cerevisiae. Mol Cell Biol, 21, 2449-2462.
Boudeau, J., Deak, M., Lawlor, M.A., Morrice, N.A. and Alessi, D.R. (2003a) Heat-shock protein 90 and Cdc37 interact with LKB1 and regulate its stability. Biochem J, 370, 849-857.
Boudeau, J., Kieloch, A., Alessi, D.R., Stella, A., Guanti, G. and Resta, N. (2003b) Functional analysis of LKB1/STK11 mutants and two aberrant isoforms found in Peutz-Jeghers Syndrome patients. Hum Mutat, 21, 172.
Buchet-Poyau, K., Mehenni, H., Radhakrishna, U. and Antonarakis, S.E. (2002) Search for the second Peutz-Jeghers syndrome locus: exclusion of the STK13, PRKCG, KLK10, and PSCD2 genes on chromosome 19 and the STK11IP gene on chromosome 2. Cytogenet Genome Res, 97, 171-178.
Collins, S.P., Reoma, J.L., Gamm, D.M. and Uhler, M.D. (2000) LKB1, a novel serine/threonine protein kinase and potential tumour suppressor, is phosphorylated by cAMP-dependent protein kinase (PKA) and prenylated in vivo. Biochem J, 345 Pt 3, 673-680.
Dorland, S., Deegenaars, M.L. and Stillman, D.J. (2000) Roles for the Saccharomyces cerevisiae SDS3, CBK1 and HYM1 genes in transcriptional repression by SIN3. Genetics, 154, 573-586.
Hemminki, A. (1999) The molecular basis and clinical aspects of Peutz-Jeghers syndrome. Cell Mol Life Sci, 55, 735-750.
Hemminki, A., Markie, D., Tomlinson, I., Avizienyte, E., Roth, S., Loukola, A., Bignell, G., Warren, W., Aminoff, M., Hoglund, P., Jarvinen, H., Kristo, P., Pelin, K., Ridanpaa, M., Salovaara, R., Toro, T., Bodmer, W., Olschwang, S., Olsen, A.S., Stratton, M.R., de la Chapelle, A. and Aaltonen, L.A. (1998) A serine/threonine kinase gene defective in Peutz-Jeghers syndrome. Nature, 391, 184-187.
Jenne, D.E., Reimann, H., Nezu, J., Friedel, W., Loff, S., Jeschke, R., Muller, O., Back, W. and Zimmer, M. (1998) Peutz-Jeghers syndrome is caused by mutations in a novel serine threonine kinase. Nat Genet, 18, 38-43.
Jishage, K.I., Nezu, J.I., Kawase, Y., Iwata, T., Watanabe, M., Miyoshi, A., Ose, A., Habu, K., Kake, T., Kamada, N., Ueda, O., Kinoshita, M., Jenne, D.E., Shimane, M. and Suzuki, H. (2002) Role of Lkb1, the causative gene of Peutz-Jegher's syndrome, in embryogenesis and polyposis. Proc Natl Acad Sci USA, 99, 8903-8908.
Johnston, A.M., Naselli, G., Gonez, L.J., Martin, R.M., Harrison, L.C. and DeAizpurua, H.J. (2000) SPAK, a STE20/SPS1-related kinase that activates the p38 pathway. Oncogene, 19, 4290-4297.
Jorgensen, P., Nelson, B., Robinson, M.D., Chen, Y., Andrews, B., Tyers, M. and Boone, C. (2002) High-Resolution Genetic Mapping With Ordered Arrays of Saccharomyces cerevisiae Deletion Mutants. Genetics, 162, 1091-1099.
Karos, M. and Fischer, R. (1999) Molecular characterization of HymA, an evolutionarily highly conserved and highly expressed protein of Aspergillus nidulans. Mol Gen Genet, 260, 510-521.
Karuman, P., Gozani, O., Odze, R.D., Zhou, X.C., Zhu, H., Shaw, R., Brien, T.P., Bozzuto, C.D., Ooi, D., Cantley, L.C. and Yuan, J. (2001) The Peutz-Jegher gene product LKB 1 is a mediator of p53-dependent cell death. Mol Cell, 7, 1307-1319.
Marignani, P.A., Kanai, F. and Carpenter, C.L. (2001) Lkb1 associates with brgl and is necessary for brgl-induced growth arrest. J Biol Chem, 276, 32415-32418.
Martin, S.G. and St Johnston, D. (2003) A role for Drosophila LKB1 in anterior-posterior axis formation and epithelial polarity. Nature, 421, 379-384.
Miyamoto, H., Matsushiro, A. and Nozaki, M. (1993) Molecular cloning of a novel mRNA sequence expressed in cleavage stage mouse embryos. Mol Reprod Dev, 34,1-7.
Miyoshi, H., Nakau, M., Ishikawa, T.O., Seldin, M.F., Oshima, M. and Taketo, M.M. (2002) Gastrointestinal hamartomatous polyposis in Lkb1 heterozygous knockout mice. Cancer Res, 62, 2261-2266.
Nakau, M., Miyoshi, H., Seldin, M.F., Imamura, M., Oshima, M. and Taketo, M.M. (2002) Hepatocellular carcinoma caused by loss of heterozygosity in 1kb 1 gene knockout mice. Cancer Res, 62, 4549-4553.
Nishigaki, K., Thompson, D., Yugawa, T., Rulli, K., Hanson, C., Cmarik, J., Gutkind, J.S., Teramoto, H. and Ruscetti, S. (2003) Identification and characterization of a novel Ste-20/GCK-related kinase, PAP kinase (PAPK). J Biol Chem.
Nozaki, M., Onishi, Y., Togashi, S. and Miyamoto, H. (1996) Molecular characterization of the Drosophila Mo25 gene, which is conserved among Drosophila, mouse, and yeast. DNA Cell Biol, 15, 505-509.
Resta, N., Stella, A., Susca, F.C., Di Giacomo, M., Forleo, G., Miccolis, I., Rossini, F.P., Genuardi, M., Piepoli, A., Grammatico, P. and Guanti, G. (2002) Two novel mutations and a new STK11/LKB1 gene isoform in Peutz-Jeghers patients. Hum Mutat, 20, 78-79.
Rossi, D.J., Ylikorkala, A., Korsisaari, N., Salovaara, R., Luukko, K., Launonen, V., Henkemeyer, M., Ristimaki, A., Aaltonen, L.A. and Makela, T.P. (2002) Induction of cyclooxygenase-2 in a mouse model of Peutz-Jeghers polyposis. Proc Natl Acad Sci U S A, 99, 12327-12332.
Sanna, M.G., da Silva Correia, J., Luo, Y., Chuang, B., Paulson, L.M., Nguyen, B., Deveraux, Q.L. and Ulevitch, R.J. (2002) ILPIP, a novel anti-apoptotic protein that enhances XIAP-mediated activation of JNK1 and protection against apoptosis. J Biol Chem, 277, 30454-30462.
Sapkota, G.P., Boudeau, J., Deak, M., Kieloch, A., Morrice, N. and Alessi, D.R. (2002a) Identification and characterization of four novel phosphorylation sites (Ser31, Ser325, Thr336 and Thr366) on LKB1/STK11, the protein kinase mutated in Peutz-Jeghers cancer syndrome. Biochem J, 362, 481-490.
Sapkota, G.P., Deak, M., Kieloch, A., Morrice, N., Goodarzi, A.A., Smythe, C., Shiloh, Y., Lees-Miller, S.P. and Alessi, D.R. (2002b) Ionizing radiation induces ataxia telangiectasia mutated kinase (ATM)-mediated phosphorylation of LKB1/STK1 at Thr-366. Biochem J, 368,507-516.
Sapkota, G.P., Kieloch, A., Lizcano, J.M., Lain, S., Arthur, J.S., Williams, M.R., Morrice, N., Deak, M. and Alessi, D.R. (2001) Phosphorylation of the protein kinase mutated in Peutz-Jeghers Cancer Syndrome, LKB1/STK11, at Ser431 by p90RSK and cAMP-dependent protein kinase, but not its farnesylation at Cys433, is essential for LKB1 to suppress cell growth. J Biol Chem, 276, 19469-19482.
Smith, C.M., Radzio-Andzelm, E., Madhusudan, Akamine, P. and Taylor, S.S. (1999) The catalytic subunit of cAMP-dependent protein kinase: prototype for an extended network of communication. Prog Biophys Mol Biol, 71, 313-341.
Smith, D.P., Rayter, S.I., Niederlander, C., Spicer, J., Jones, C.M. and Ashworth, A. (2001) LIP1, a cytoplasmic protein functionally linked to the Peutz-Jeghers syndrome kinase LKB1. Hum Mol Genet, 10, 2869-2877.
Songyang, Z., Fanning, A.S., Fu, C., Xu, J., Marfatia, S.M., Chishti, A.H., Crompton, A., Chan, A.C., Anderson, J.M. and Cantley, L.C. (1997) Recognition of unique carboxyl-terminal motifs by distinct PDZ domains. Science, 275, 73-77.
Tamari, M., Daigo, Y. and Nakamura, Y. (1999) Isolation and characterization of a novel serine threonine kinase gene on chromosome 3p22-21.3. J Hum Genet, 44, 116-120.
Tiainen, M., Vaahtomeri, K., Ylikorkala, A. and Makela, T.P. (2002) Growth arrest by the LKB1 tumor suppressor: induction of p21(WAF1/CIP1). Hum Mol Genet, 11, 1497-1504.
Tiainen, M., Ylikorkala, A. and Makela, T.P. (1999) Growth suppression by Lkb1 is mediated by a G(1) cell cycle arrest. Proc Natl Acad Sci U S A, 96, 9248-9251.
Watts, J.L., Morton, D.G., Bestman, J. and Kemphues, K.J. (2000) The C. elegans par-4 gene encodes a putative serine-threonine kinase required for establishing embryonic asymmetry. Development, 127, 1467-1475.
Ylikorkala, A., Rossi, D.J., Korsisaari, N., Luukko, K., Alitalo, K., Henkemeyer, M. and Makela, T.P. (2001) Vascular abnormalities and deregulation of VEGF in Lkb1-deficient mice. Science, 293, 1323-1326.
Yoo, L.I., Chung, D.C. and Yuan, J. (2002) LKB1 A master tumour suppressor of the small intestine and beyond. Nat Rev Cancer, 2, 529-535.

### Example 2: Complexes between the LKB1 tumour suppressor, Stradα/β and Mo25α/β are upstream kinases in the AMP-activated protein kinase cascade

The AMP-activated protein kinase (AMPK) cascade is a sensor of cellular energy charge that acts as a "metabolic master switch" and inhibits cell proliferation. Activation requires phosphorylation within the activation loop (Thr-172) by upstream kinases (AMPKKs) that have not been identified. Recently we identified three related protein kinases acting upstream of the yeast homologue of AMPK, which do not have obvious mammalian homologues. However, a closely related mammalian kinase is LKB1, for which no physiological substrates have been identified. LKB1 acts as a tumour suppressor and is mutated in human Peutz-Jeghers syndrome. We show in Example 1 that it exists as a complex with two accessory subunits, STRADα/β and M025α/β.

We report that: (i) two AMPKK activities purified from rat liver contain LKB1, STRADα and MO25α, and can be immunoprecipitated using anti-LKB1 antibodies; (ii) both endogenous and recombinant complexes of LKB1, STRADα/β and MO25α/β activate AMPK via phosphorylation of Thr-172, (iii) catalytically active LKB1 and STRADα or STRADβ and MO25α or MO25β are required for full activity; (iv) the drugs AICA riboside and phenformin do not activate AMPK in HeLa cells (which lack LKB1), but this can be restored by stably expressing wild type, but not catalytically inactive, LKB 1 in these cells.

These results provide the first description of a physiological substrate for the LKB1 tumour suppressor and suggest that it functions as an upstream regulator of AMPK. Our findings indicate that the formation of tumours in Peutz-Jeghers syndrome could result from deficient activation of AMPK as a consequence of LKB 1 inactivation.

### Results

### Resolution of two AMPKKs from rat liver extracts

While experimenting with different conditions to optimise recovery at the Q-Sepharose step of our previous purification protocol for AMPKK [14], we found that we were able to resolve two peaks of activity (Fig. 13A). Because the second peak appears to correspond to the AMPKK originally purified [14], we refer to it as AMPKK1, with the first peak eluting from the column termed AMPKK2, The AMPKK assays in Fig. 13A were conducted using as a substrate a bacterially expressed glutathione-S-transferase (GST) fusion of the catalytic domain of rat AMPKα1. This is more convenient than the native rat liver AMPK heterotrimer used previously [14] because the AMPKα1 catalytic domain is already completely dephosphorylated and therefore inactive when expressed in bacteria. It is also possible to carry out the assays with the substrate bound to glutathione-Sepharose beads, allowing any endogenous AMPK in the AMPKK fraction that might otherwise interfere to be washed away. On size exclusion chromatography on Sephacryl S-200, AMPKK1 and AMPKK2 eluted as proteins of large but distinct size. By comparison with standards, the Stokes radii for AMPKK1 and AMPKK2 were estimated as 5.2 and 5.7 nm respectively. Ca2+ and calmodulin stimulated neither AMPKK1 nor AMPKK2, and neither were immunoprecipitated using an antibody against CaMKK (not shown).

### AMPKK1 and AMPKK2 both contain LKB1, STRADα and MO25α

To examine whether either of these two fractions might contain LKB1, we probed blots of fractions across the Q-Sepharose chromatography using antibodies against LKB1, STRADα and MO25α (Fig. 13B-D). This revealed that the activity of AMPKK2 correlated with the presence of the ~50 kDa polypeptide of LKB1 (≈50 kDa) as well as those of STRADα (≈45/48 kDa) and Mo25α(≈40 kDa) in fractions 18-22.^{~} STRADα was detected as a doublet as reported previously [30]; the reason for this behaviour is not known. We did not obtain any signal of the correct molecular mass in these fractions using anti-MO25β antibody (not shown), consistent with our observations that the MO25β isoform is not expressed in mouse liver (Example 1). We also obtained a faint signal for LKB1 and STRADα in fractions 27-29 that contained AMPKK1, but at this loading MO25α was below the limit of detection in these fractions. However, the presence of LKB1, STRADα and MO25α in AMPKK1 was confirmed by analysing a higher loading of fractions 26-30 (Fig. 12E-G). An interesting finding was that the LKB1 polypeptide migrated with a slightly faster mobility in AMPKK1 compared with AMPKK2: this is evident by inspection of Fig. 13B (see also Figs. 14B and 14D).

### AMPKK activity can be immunoprecipitated from AMPKK1 and AMPKK2

Using anti-LKB1 antibody but not a pre-immune control IgG, we were able to immunoprecipitate AMPKK activity from both AMPKK1 and AMPKK2. Fig. 14A shows results of an experiment where the amount of kinase kinases was limiting and the antibody was in excess, and shows that we were able to remove >80% of the activity from the peak fractions containing AMPKK1 and AMPKK2 by immunoprecipitating with anti-LKB1 antibody, while no activity was removed using a pre-immune control immunoglobulin. We could remove >95% of the AMPKK activity of a recombinant GST-tagged LKB1-STRADα-MO25α complex (see next section) under the same conditions. The small amount of activity remaining in the supernatants of the AMKK1 and AMPKK2 fractions could be accounted for by the fact that the immunoprecipitation was not quantitative in these cases, with about 20% of the LKB 1 polypeptide remaining in the supernatant (Fig. 14B).
Due to the small amount of AMPKK1 and AMPKK2 used in this experiment, it proved difficult to analyse the pellets for AMPKK activity md the presence of the other polypeptides. We therefore repeated the experiment with more kinase kinase and less antibody (the amount of mtibody was now limiting) and analysed the pellets only. This showed that we could recover almost as much AMPKK activity in the pellet from the peak fractions containing AMPKK1 and AMPKK2 as we could from the recombinant LKB1-STRADα-MO25α complex, with no activity being recovered using pre-immune control immunoglobulin (Fig. 14C). Western blotting of the AMPKK1 and AMPKK2 pellets showed that they contained LKB1, STRADα and MO25α, although the STRADα doublet was very faint in the AMPKK1 pellet (Fig. 14D).

### Recombinant LKB1-STRAD-MO25 complexes activate AMPKα1 catalytic domain in cell-free assays

To examine whether LKB1 activated AMPK on its own or whether the accessory subunits STRADα/β and Mo25α/β were required, we expressed GST-tagged LKB1, FLAG-tagged STRADα/β and *myc*-tagged Mo25α/β in various combinations in HEK-293T cells, and purified the complexes on glutathione-Sepharose. We also used a GST-tagged kinase-inactive mutant of LKB1 (D194A), and a plasmid expressing GST alone, as controls. The purified complexes were incubated with the AMPKα1 catalytic domain in the presence of MgATP, and activation of the catalytic domain as well as phosphorylation of Thr172 (using a phosphospecific anti-pT172 antibody) was measured. Fig. 15A shows that LKB1 alone did not significantly increase the activity, or phosphorylation of Thr172, of the AMPKα1 catalytic domain above the basal activity observed in the presence of GST alone (compare lanes 1 and 14). The same result was obtained with LKB1 that had been co-expressed with MO25α or MO25β (lanes 4 and 5), which was expected as these proteins do not interact with LKB1 in the absence of STRADα/β (Example 1). An LKB1-STRADα complex did give a small but significant activation, and Thr172 phosphorylation, of the AMPKα1 catalytic domain above the basal value (compare lanes 1 and 2). However to produce a large activation and phosphorylation of the AMPKα1 catalytic domain, a heterotrimeric complex containing LKB1, STRADα or STRADβ, and MO25α or MO25β was required (lanes 6 to 9). With the heterotrimeric complexes the degree of activation was in the order LKB1-STRADα-MO25α > LKB1-STRADα-MO25β ≈ LKB1-STRADβ-MO25α > LKB1-STRADβ-MO25β. Importantly, the ability of LKB1-STRAD-MO25 complexes to activate AMPKα1 was entirely dependent on LKB1 catalytic activity, because complexes of a catalytically inactive mutant of LKB1 D194A) with the various combinations of STRADα/β and MO25α/β lanes 10-13), were unable to activate or phosphorylate AMPKα1. The degree of activation obtained with these various complexes of wild type LKB1 correlated well with the phosphorylation of Thr-172. Probing with an antibody against the whole AMPKα1 catalytic domain (labelled GST-α1-KD in Fig. 15A) confirmed that the loading was uniform in these samples. The bottom three panels in Fig. 15A confirm that the relevant STRAD and M025 subunit co-precipitated with LKB1 when DNAs encoding these subunits had been co-transfected. When STRADα was co-expressed with LKB1 in the absence of an M025 subunit, the amount of STRADα subunit co-precipitated with LKB 1 was reduced (lanes 2 and 3). This is consistent with previous findings that the presence of an M025 subunit stabilizes LKB1-STRAD complexes (Example 1).

Fig. 15B provides evidence that Thr-172 was the only site on the AMPKα1 catalytic domain phosphorylated by the LKB1-STRADα-MO25α complex. When the two proteins were incubated together in the presence of [γ-³²P]ATP, the wild type AMPKα1 catalytic domain became ³²P-labelled, but a Thr172→Ala mutant of the AMPKα1 catalytic domain did not.

### AMPKK1, AMPKK2 and recombinant LKB1-STRAD-MO25 complexes also activate hterotrimeric AMPK complexes

Although most of the AMPKK assays in this study were conducted using the AMPKα1 catalytic domain as substrate, Fig 16 shows that AMPKK1, AMPKK2 and the recombinant GST-LKB1:STRADα:MO25α complex also activated heterotrimeric AMPK complexes. Plasmids encoding the α1, β1 and γ1 or α2, β1 and γ1 subunits of AMPK were co-expressed in CCL13 cells and purified by means of a *myc* tag on the α subunits. The recombinant AMPK complexes bound to protein G-Sepharose beads were incubated with MgATP and AMPKK1, AMPKK2 or GST-LKB1:STRADα:MO25α, the upstream kinase washed away and the activity of the AMPK complexes determined. Figure 16 shows that both the α1β1γ and α2β1γ 1 complexes were activated by all three upstream kinase preparations, and that this correlated with the phosphorylation of THr-172 on the α subunits, shown using the anti-pT172 antibody (which recognises phosphorylated Thr-172 on both α1 and α2). The blot using anti-α1 and - α2 antibodies confirms uniform loading (note that a stronger signal is always obtained with the anti-α2 than the anti-α1 antibody, even when the protein loading is the same). Although the activity of AMPKK2 used was 3.5 times higher than that of AMPKK1 and only 30% lower than that of GST-LKB1-STRADα-MO25α in terms of its ability to activate the AMPKα1 catalytic domain, AMPKK2 appeared to be much less effective at activating the α1β1γ1 complex. These differences were less obvious using the α2β1γ1 complex as the substrate.

### Endogenous LKB1that activates AMPK can be immunoprecipitated from 293 cells but not HeLa cells

Fig. 17 shows that an activity that activated the AMPKα1 catalytic domain and phosphorylated Thr-172 could be immunoprecipitated from untransfected HEK-293T cell extract using anti-LKB1 antibody (lane 1), but not a pre-immune control immunoglobulin (lane 2). As reported previously [30, Example 1], immunoprecipitation of endogenous LKB1 resulted in the co-precipitation of STRADα and MO25α (lane 1). As a further control, we employed normal HeLa cells as an LKB 1 null cell line, as it is known that LKB1 is not expressed in these cells [23]. Consistent with this, no LKB1, STRADα and MO25α subunits or AMPKK activity was immunoprecipitated from the same amount of HeLa cell extract protein using anti-LKB1 antibody (lane 3). However, AMPKK activity and Thr-172 phosphorylation, as well as detectable STRADα and MO25α subunits, were recovered following immunoprecipitation of LKB1 from HeLa cells [34], that stably express wild type LKB1 [32] (lane 5). In cells expressing a catalytically-inactive mutant of LKB1, the LKB1, STRADα and MO25α polypeptides were recovered by immunoprecipitation using anti-LKB1 antibody, but no AMPKK activity (lane 7). Although the LKB1 polypeptide was over-expressed to a large extent in the HeLa cells compared to the endogenous levels observed in 293 cells (Fig 14, compare lanes 1 and 5), it is clear that the amount of STRADα and MO25α in the cells is limiting in these cells. There was a lower amount of AMPKK activity and Thr-172 phosphorylation , as well of co-precipitating STRADα and MO25α, in HeLa cells expressing LKB1 than in 293 cells, even though LKB1 itself was over-expressed. The AMPKK activity in the immunoprecipitates from 293 cells and HeLa cells expressing wild type LKB1 correlated with the levels of STRADα and MO25α in the complex rather than with the levels of LKB1. This further emphasises the essential nature of the STRAD and MO25 subunits in enhancing the AMPKK activity of LKB1.

### Expression of LKB1 restores activation of AMPK in HeLa cells

We have previously found (unpublished results) that, although AMPK is expressed in HeLa cells, it is not activated by the drugs 5-aminoimidazole-4-carboxamide (AICA) riboside and metformin, which readily activate the kinase in other cell types [33 34]. As HeLa cells do not express LKB1 [23] this could provide a potential explanation for this anomaly. To examine whether expression of recombinant LKB 1 might restore the ability of HeLa cells to respond to these drugs, we used the HeLa cell that stably express wild type LKB1, or the catalytically inactive mutant [32], as well as a control HeLa cell line not expressing LKB1. In these experiments we used phenformin, which is a close relative of metformin that we have found to activate AMPK more rapidly and more potently than metformin in other cell types. Fig. 18A shows that neither AICA riboside nor phenformin activated AMPK in control HeLa cells. However, in cells expressing wild type LKB1, but not the kinase-inactive mutant, both AICA riboside and phenformin caused a robust activation of AMPK. This correlated with the phosphorylation of Thr-172 on AMPKα, shown by probing with the anti-pT172 antibody (Fig. 18B), and also with the phosphorylation of a downstream target of AMPK, acetyl-CoA carboxylase (ACC), shown by probing with a phosphospecific antibody against Ser-79, the primary AMPK site on that protein (anti-pACC, Fig. 18C). Interestingly, stable expression of wild type LKB 1 in the HeLa cells caused a small but reproducible degree of up-regulation of expression of AMPKα1 and a marked down-regulation of expression of ACC (not shown). Because the expression of these proteins was not uniform in these cells, in order to accurately quantitate their phosphorylation status we simultaneously probed single blots of lysates using either anti-pT172 and anti-α1/α2 antibodies (to detect Thr-172 phosphorylation and total AMPKα), or with anti-pACC and avidin (to detect Ser-79 phosphorylation and total ACC). The two probing reagents used in each of these dual-labelling protocols were labelled with different fluorescent dyes emitting in different regions of the infra-red (IR) spectrum, and the results were quantified in two separate channels using an IR laser scanner. In Fig. 18B and 18C the results are expressed as ratios of the signal obtained using the phosphospecific antibody to the signal obtained for the total protein. This method corrects for different levels of expression of the proteins, and reveals that there was a good correlation between activation of AMPK and phosphorylation of Thr-172. There was also a correlation with phosphorylation of ACC, although in this case AICA riboside appeared to have a larger effect than phenformin.

### Discussion

Our results provide strong evidence that LKB1-STRAD-MO25 complexes represent the major upstream kinase activities acting on AMPK. The key evidence may be summarised as follows:
1. During previous extensive efforts over 10 years to purify from rat liver extracts activities that activate dephosphorylated AMPK, ([14] and subsequent unpublished work), we have not detected any activities other than AMPKK1 and AMPKK2, at least under the assay conditions used.
2. Both AMPKK1 and AMPKK2 contained LKB1, STRADα and MO25α that were detectable by Western blotting and that correlated with the activity (Fig.13).
3. Crucially, the ability of the AMPKK1 and AMPKK2 fractions to activate the AMPK α1 catalytic domain was almost completely eliminated by immunoprecipitation with anti-LKB 1 antibody but not a control immunoglobulin. Activity was detected in the anti-LKB1 immunoprecipitates, together with the LKB1, STRADα and MO25α polypeptides, but not in the control immunoprecipitates (Fig. 14).
4. The AMPKK activity in AMPKK1 and AMPKK2 w s not a contaminant that co-precipitated with anti-LKB1 antibody, because recombinant complexes of GST-LKB1, STRAD and MO25 expressed in 293 cells and purified on glutathione-Sepharose activated the AMPK α1 catalytic domain efficiently, and phosphorylated Thr-172 (Fig. 15). This activity was dependent on the integrity of the LKB 1 catalytic domain, because complexes formed from catalytically inactive LKB 1 with a STRAD and MO25 subunit failed to activate or phosphorylate AMPK. Phosphorylation of the α1 catalytic domain appeared to occur exclusively at Thr-172, because the wild type AMPKα1 catalytic domain, but not a T172A mutant, could be phosphorylated using [γ-³²P]ATP and the GST-LKB1-STRADα-MO25α complex.
5. Although most of the experiments in this paper were conducted using the bacterially expressed AMPK α1 catalytic domain as substrate, AMPKK1, AMPKK2 and recombinant LKB1-STRADα-MO25α complexes also activated heterotrimeric AMPK complexes (α1β1γ1 and α2β1γ1) efficiently, and phosphorylated them at Thr-172 on the α subunits (Fig. 16).
6. HeLa cells do not express LKB1 (Fig. 17) and therefore represent a natural "knock-out" cell line. The drugs AICA riboside and phenformin, which activate AMPK in other cell types via distinct mechanisms [33,34], did not activate AMPK in HeLa cells. However, in cells stably transfected with DNA that expressed wild type LKB1 (but not a catalytically-inactive mutant) from an inducible promoter, the ability of AICA riboside and phenformin to activate AMPK, to phosphorylate Thr-172 on the AMPKα subunit, and to cause phosphorylation of a downstream target (ACC) was restored (Fig. 18). Although phenformin caused a larger activation of AMPK and phosphorylation of Thr-172 than AICA riboside in these cells, the reverse was true for phosphorylation of ACC. The reason for this is not known, but one possibility is that the two agents are differentially activating distinct pools of AMPK at different subcellular locations.

Although the LKB1 polypeptide was over-expressed in the HeLa cells, the degree of AMPK activation and the amount of STRADα and MO25α co-precipitating with LKB1 was actually lower than in 293 cells (Fig. 17). This strongly supports the findings in Fig. 15 showing that both accessory subunits are necessary to enhance the ability of LKB1 to activate AMPK, and also show that the active form of LKB 1 is not being over-expressed compared to 293 cells. The results in the HeLa cells suggest that LKB1 and its accessory subunits are both necessary and sufficient for activation of AMPK in intact cells. However, they do not formally prove this, because if there are other kinases acting upstream of AMPK, it is conceivable that these are also deleted in HeLa cells. Our numerous attempts at purification of AMPKKs from rat liver extracts have only ever detected AMPKK1 and AMPKK2, which now appear to both represent LKB1-STRADα-MO25α complexes. However, as in any protein purification protocol the recovery of activity is not quantitative, and we cannot rule out the possibility that other upstream kinases could have been missed. We would also not detect any upstream kinase that was not active under the assay conditions used. For example, the assays were conducted in the presence of EGTA, so we would not detect any Ca²⁺-dependent protein kinases. We have already shown that a Ca²⁺/calmodulin-dependent protein kinase kinase (CaMKK) from pig brain can activate AMPK in cell-free assays [17], although it was much more effective at activating calmodulin- dependent protein kinase I, and there is no evidence that it phosphorylates AMPK *in vivo.*

Both AMPKK1 and AMPKK2 appear to contain LKB1, STRADα and MO25α, and thus it is not clear at present why they resolve on Q-Sepharose chromatography. One interesting difference was that the LKB 1 polypeptide in AMPKK1 migrated significantly faster on SDS-PAGE than that in AMPKK2 (Figs. 13 and 14). LKB 1 is know to be phosphorylated at up to eight sites, and it is also farnesylated at Cys-433 near the C-terminus [34-37]. We therefore suspect that the difference in mobility may be due to a difference in covalent modification. Another difference between AMPKK1 and AMPKK2 was their Stokes radii estimated by size exclusion chromatography (5.7 versus 5.2 nm respectively). We previously estimated the Stokes radius for AMPKK1 to be 5.6 nm, and combining this with a sedimentation coefficient of 8.5S obtained by glycerol gradient centrifugation had obtained an estimate of 195 kDa for the molecular mass [14]. Assuming that AMPKK1 and AMPKK2 have a similar shape, this would yield a molecular mass of about 175 kDa for AMPKK2, which is reasonably close to the calculated molecular mass (not counting covalent modifications) of a 1:1:1 LKB1-STRADα-MO25α complex of 140 kDa. Although we cannot rule out the possibility that AMPKK1 contains an additional associated protein, it is possible that differences in covalent modification between AMPKK1 and AMPKK2 might affect the shape of the complex and hence the Stokes radius. Consistent with the behaviour of the AMPKK1 and AMPKK2 complexes on size exclusion chromatography, endogenous LKB1 was previously shown to migrate on gel filtration with an apparent molecular mass of 150-250 kDa, in marked contrast to LKB1 expressed in *E. coli,* which had an apparent molecular mass of 50 kDa [38]. Our present results confirm, using a probable physiological substrate, previous findings using an artificial substrate (myelin basic protein) that a STRAD subunit stimulates the kinase activity of LKB1 [30], and that the MO25 subunit stimulates the activity further, probably by stabilizing the LKB1-STRAD complex (Example 1). No AMPKK activity was obtained with recombinant LKB1 unless a STRAD subunit was also expressed, and the activity was increased substantially by the additional presence of an M025 subunit (Fig. 15). It was also noticeable that the amount of STRADα and STRADβ that co-precipitated with LKB1 was greatly enhanced by the co-expression of MO25α or MO25β (Fig. 15) consistent with our previous findings (Example 1). The exact mechanism by which the STRAD and MO25 subunits activate LKB1 remains unclear, but these accessory subunits introduce scope for additional regulation of the kinase. It is already known that LKB1 phosphorylates STRADα at 2 distinct sites [30], and that STRADα and MO25α form a complex that retains LKB1 in the cytoplasm (Example 1). It is also interesting to note that both AMPK and its upstream kinase are heterotrimers.

Does activation of AMPK explain the ability of LKB 1 to act as a tumour suppressor and to arrest cell growth and proliferation? This certainly seems plausible, because apart from the fact that AMPK is a general inhibitor of biosynthesis [1,2], there is also accumulating evidence that it can regulate progress through the cell cycle. For example, activation of AMPK inhibits proliferation of HepG2 cells by stabilizing p53 [13], and reduces the level of the RNA binding protein HuR in the cytoplasm, decreasing the stability of mRNAs encoding cyclins A and B1 [12]. Interestingly, expression of LKB1 in G361 cells that do not express the kinase causes an arrest in G1 phase that is associated with an induction of p21 and is dependent on p53 [23,24].
Another exciting possibility is that LKB1-STRAD-MO25 complexes might also act as upstream kinases for other protein kinases, in the same manner that PDK1 phosphorylates threonine residues in the activation loop of a number of kinases of the "AGC" sub-family [39]. A dendrogram showing the relationships between catalytic domain sequences of 518 human protein kinases encoded in the human genome [40] shows that the AMPKα1 and AMPKα2 subunits lie on a small sub-branch also containing eight other protein kinases (NuaK1, NuaK2, BrsK1, BrsK2, SIK, QIK, QSK and MELK; see [41] for details), most of which have either not been studied previously or about which very little is known. An alignment of the activation loop sequences of these kinases is shown in Fig. 19, together with those of the close relative "AGC" sub-family catalytic Cα subunit of cyclic AMP-dependent protein kinase (PKAα, which can be phosphorylated in the activation loop by autophosphorylation) and the α isoform of protein kinase C (PKCα), which is activated by phosphorylation in the activation loop by PDK1. Also included in Fig. 19 are the activation loop sequences of the *Arabidopsis thaliana* (AtSnRK1α1, AtSnRKα2) and *Saccharomyces cerevisiae* (ScSnf1) homologues of AMPK, both of which have been shown previously to be activated by rat liver AMPKK in cell-free assays [42,43]. All of the sequences are well conserved on the C-terminal side of the threonine residue that is the target for phosphorylation, but the AMPK/SnfI subfamily are more highly conserved on the N-terminal side between the invariant "DFG" motif and the threonine residue. Sequence features that are conserved in the AMPK/SNF1 subfamily but not in the "AGC" family include pairs of hydrophobic residues at the -3, -2 and the -9, -8 positions. NuaK1 and NuaK2 and the human, plant and yeast AMPK/SNF1 kinases also have a conserved "LSN" motif at the -12 to -10 positions. It remains to be determined whether the other human kinases in the AMPK sub-family are activated by phosphorylation of the equivalent activation loop threonine residue, and whether they might also be downstream targets for LKB1/STRAD/MO25 complexes. If this is the case, these other kinases could mediate some of the tumour suppressor functions of LKB1. Since the catalytic domains in this sub-family are closely related, but the other regions tend to be more variable, one could speculate that these kinases may be activated by different stresses or other stimuli via phosphorylation by LKB1/STRAD/MO25 complexes, and together may mediate the diverse cellular effects that are triggered by LKB 1. It is also possible that they might share some common downstream targets with AMPK.

### Conclusions

Our results provide strong evidence, both in cell-free assays and in intact cells, that complexes between LKB1, STRADα/β and MO25α/β constitute the long sought after upstream kinases that activate AMPK via phosphorylation at Thr-172 in the activation loop. Because it is already known that pharmacological activation of AMPK causes a general inhibition of biosynthesis, as well as a p53-dependent arrest in G1 phase, activation of AMPK by LKB1 might explain, at least in part, the ability of the latter to act as a tumour suppressor. LKB 1 may also act as an upstream kinase for other members of the AMPK/SNF1 sub-family of protein kinases.

### Material and methods

### Materials, proteins and antibodies

Protein G-Sepharose and prepacked Q-Sepharose columns were from Amersham Pharmacia Biotech, UK. GST-AMPKα1-catalytic domain was expressed in *E. coli* and purified as described previously [44]. Sheep antibodies against the α1 and α2 subunits of AMPK [45], human LKB1, MO25α and MO25β (Example 1), and phosphospecific antibody against the Thr-172 site on the AMPK α subunit (anti-pT172) [46] were described previously. Sheep antibody against AMPKα1-catalytic domain was raised against the peptide CDPMKRATpIKDIRE (cysteine + residues 252-264 of rat α1, Tp = phosphothreonine) using methods described for anti-pT172 [46]. Although designed as a phosphospecific antibody, it recognizes GST-AMPKα1-catalytic domain expressed in bacteria and recognition is not affected by protein phosphatase treatment. The monoclonal antibody against STRADα was described previously [30]. Sources of other materials and proteins were describe previously [14].

### Enzyme assays

AMPK [14]and AMPKK [34] were assayed, and units defined, as described previously. Rapid lysis of cells for AMPK assays was as described previously [47]. Assays were carried out in triplicate and results are expressed as mean ± standard deviation.

### Purification of AMPKK1 and AMPKK2

AMPKK was purified to the Blue-Sepharose stage as described previously [14]. The flow-through from this column was adjusted to 160 mM NaCl by dilution in buffer A (50 mM Hepes, 10% (w/v) glycerol, 0.02% (w/v) Brij-35, 1 mM EDTA, 1 mM EGTA, 1 mM DTT, 1 mM benzamidine, 0.1 mM PMSF, 1 µg/ml soybean trypsin inhibitor), and applied to a high performance Q-Sepharose HiLoad 16/10 column in buffer A plus 160 mM NaCl at 3 ml/min. The column was washed in buffer A plus 160 mM NaCl until the A₂₈₀ was <0.05, and AMPKK activity was then eluted with a linear gradient (120 ml) from 160-400 mM NaCl in buffer A.

### Expression of recombinant LKB1 complexes in HEK-293T cells

Various combinations of GST-tagged LKB1, FLAG-tagged STRADα or STRADβ, and myc-tagged MO25α or MO25β were expressed in HEK-293T cells and the complexes purified on glutathione-Sepharose as described in Example 1.

### Phosphorylation of GST-al catalytic domain using [γ-³²P]ATP

GST-α1 catalytic domain (50µg/ml), either wild type or a T172A mutant [44] was incubated for 30 min at 30°C with 5 mM MgCl₂ and [γ-³²P]ATP (200µM; ~750 cpm/pmole) in the presence or absence of the GST-LKB1:STRADα:MO25α complex (20 units/ml). The reaction was terminated by the addition of SDS sample buffer (Invitrogen), the polypeptides resolved by SDS-PAGE and the dried gel subjected to autoradiography.

### Preparation of recombinant AMPK hterotrimers

Plasmids encoding *myc*-α1, β1 and γ1 were co-expressed in CCL13 cells [45], and cells harvested by the rapid lysis method [47]. Lysates were immunoprecipitated with anti-*myc* antibody and resuspended in 50mM NaHEPES, 1 mM dithiothreitol, 0.02% (w/v) Brij-35, pH 7.5.

### Immunoprecipitation of endogenous LKB1

Immunoprecipitation of endogenous LKB 1 using anti-human antibody and protein G-Sepharose is described in Example 1. The kinase kinase assays were conducted using GST-α1-catalytic domain as substrate in shaking incubators as described previously for immunoprecipitate assays of AMPK [47].

### HeLa cells expressing LKB1

The generation of HeLa cells stably expressing inducible (Tet-ON) wild type or kinase-inactive mutant LKB1, and conditions for their culture, has been described previously [32].

### Protein analysis and electrophoresis

Protein concentration was determined using the dye-binding method of Bradford [48]. SDS-PAGE was achieved using precast Bis-Tris 4-12% gradient polyacrylamide gels, in the MOPS buffer system (Invitrogen), except for analysis of acetyl-CoA carboxylase, where pre-cast 3-8% Tris-acetate gels (Invitrogen) were used. Proteins were transferred to nitrocellulose membranes (BioRad) using the Xcell II Blot Module (Invitrogen).

### Detection of Western blots by infra-red imaging

To analyse phosphorylation of ACC, samples were analysed by SDS-PAGE, transferred to nitrocellulose (BioRad) and incubated in Odyssey™ Blocking buffer containing 0.2% Tween-20 for 1 h. Anti-pACC antibody (1.46 µg/ml in blocking buffer) was then added and left shaking for 1 h. The membranes were washed 6 x 5 min with TBS (10 mM Tris-HCl, pH 7.4, 0.5M NaCl) plus Tween-20 (0.2%). The membranes were immersed in blocking buffer containing 0.2% Tween-20 and 1 µg/ml anti-sheep IgG conjugated to IR dye 680 (Molecular Probes, Leiden, The Netherlands) and 1µg/ml streptavidin conjugated to IR Dye 800 (Rockland Inc., Philadelphia, PA) and left shaking for 1 h, protected from light. The membranes were then washed 6 x 5 min using TBS-Tween (0.2%) and 1 x 5 min in PBS. The membranes were scanned in two different channels using the Odyssey™ IR imager, the results quantified using Odyssey™ software and expressed as a ratio of the signal obtained with the pACC antibody to that obtained with streptavidin. Analysis of phosphorylation of GST-α1 catalytic domain was similar except that the 4-12% Bis-Tris gels were used, and the membranes were simultaneously probed for 1 h with the sheep anti-pT172 and anti-AMPKα1-catalytic domain antibodies, directly labelled with the IR dye 680 and IR Dye 800 respectively, according to manufacturers' instructions.

### References

1. Hardie, DG, Scott, JW, Pan, DA, Hudson, ER: Management of cellular energy by the AMP-activated protein kinase system. FEBS Lett. 2003 546, 113-120.
2. Hardie, DG, Hawley, SA: AMP-activated protein kinase: the energy charge hypothesis revisited. BioEssays 2001, 23: 1112-1119.
3. Coven, DL, Hu, X, Cong, L, Bergeron, R, Shulman, GI, Hardie, DG, Young, LH: Physiologic role of AMP-activated protein kinase (AMPK) in the heart: graded activation during exercise. Am J Physiol Endocrinol Metab 2003,
4. Horman, S et al.: Activation of AMP-activated protein kinase leads to the phosphorylation of Elongation Factor 2 and an inhibition of protein synthesis. Current Biol. 2002, 12: 1419-1423.
5. Krause, U, Bertrand, L, Hue, L: Control of p70 ribosomal protein S6 kinase and acetyl-CoA carboxylase by AMP-activated protein kinase and protein phosphatases in isolated hepatocytes. Eur. J. Biochem. 2002, 269: 3751-3759.
6. Bolster, DR, Crozier, SJ, Kimball, SR, Jefferson, LS: AMP-activated protein kinase suppresses protein synthesis in rat skeletal muscle through downregulated mTOR signaling. J. Biol. Chem. 2002, 277: 23977-23980.
7. Meisse, D, Van de Casteele, M, Beauloye, C, Hainault, I, Kefas, B, Rider, M, Foufelle, F, Hue, L: Sustained activation of AMP-activated protein kinase induces c-Jun N-terminal kinase activation and apoptosis in liver cells. FEBS Lett. 2002, 526: 38-42.
8. Kefas, BA, Cai, Y, Ling, Z, Heimberg, H, Hue, L, Pipeleers, D, Van De Casteele, M: AMP-activated protein kinase can induce apoptosis of insulin-producing MIN6 cells through stimulation of c-Jun-N-terminal kinase. J. Mol. Endocrinol. 2003, 30: 151-161.
9. Kefas, BA, Heimberg, H, Vaulont, S, Meisse, D, Hue, L, Pipeleers, D, Van De Casteele, M: AICA-riboside induces apoptosis of pancreatic beta cells through stimulation of AMP-activated protein kinase. Diabetologia 2003, 46: 250-254.
10. Stefanelli, C, Stanic, I, Bonavita, F, Flamigni, F, Pignatti, C, Guarnieri, C, Caldarera, CM: Inhibition of glucocorticoid-induced apoptosis with 5-aminoimidazole-4- carboxamide ribonucleoside, a cell-permeable activator of AMP-activated protein kinase. Biochem. Biophys. Res. Commun. 1998, 243: 821-826.
11. Blazquez, C, Geelen, MJ, Velasco, G, Guzman, M: The AMP-activated protein kinase prevents ceramide synthesis de novo and apoptosis in astrocytes. FEBS Lett. 2001, 489: 149-153.
12. Wang, W et al.: AMP-activated kinase regulates HuR subcellular localization. Mol. Cell. Biol. 2002, 27: 3425-3436.
13. Imamura, K, Ogura, T, Kishimoto, A, Kaminishi, M, Esumi, H: Cell Cycle Regulation via p53 Phosphorylation by a 5'-AMP Activated Protein Kinase Activator, 5-Aminoimidazole- 4-Carboxamide-1-beta-d- Ribofuranoside, in a Human Hepatocellular Carcinoma Cell Line. Biochem. Biophys. Res. Commun. 2001, 287: 562-567.
14. Hawley, SA, Davison, M, Woods, A, Davies, SP, Beri, RK, Carling, D, Hardie, DG: Characterization of the AMP-activated protein kinase kinase from rat liver, and identification of threonine-172 as the major site at which it phosphorylates and activates AMP-activated protein kinase. J. Biol. Chem. 1996, 271: 27879-27887.
15. Sutherland, CM, Hawley, SA, Stark, MJR, Hardie, DG: Identification of Elmlp as one of three upstream kinases for the Saccharomyces cerevisiae SNF1 complex. Curr. Biol. 2003: submitted.
16. Nath, N, McCartney, RR, Schmidt, MC: Yeast Pak1 kinase associates with and activates Snf1. Mol. Cell. Biol. 2003, 23: 3909-3917.
17. Hawley, SA, Selbert, MA, Goldstein, EG, Edelman, AM, Carling, D, Hardie, DG: 5'-AMP activates the AMP-activated protein kinase cascade, and Ca2+/calmodulin the calmodulin-dependent protein kinase I cascade, via three independent mechanisms. J. Biol. Chem. 1995, 270: 27186-27191.
18. Hemminki, A et al.: A serine/threonine kinase gene defective in Peutz-Jeghers syndrome. Nature 1998, 391: 184-187.
19. Jenne, DE, Reimann, H, Nezu, J, Friedel, W, Loff, S, Jeschke, R, Muller, O, Back, W, Zimmer, M: Peutz-Jeghers syndrome is caused by mutations in a novel serine threonine kinase. Nat. Genet. 1998, 18: 38-43.
20. Hemminki, A: The molecular basis and clinical aspects of Peutz-Jeghers syndrome. Cell. Mol. Life Sci. 1999, 55: 735-750.
21. Giardiello, FM, Brensinger, JD, Tersmette, AC, Goodman, SN, Petersen, GM, Booker, SV, Cruz-Correa, M, Offerhaus, JA: Very high risk of cancer in familial Peutz-Jeghers syndrome. Gastroenterology 2000,119: 1447-1453.
22. Boudeau, J, Sapkota, G, Alessi, DR: LKB1, a protein kinase regulating cell proliferation and polarity. FEBS Lett. 2003, 546: 159-165.
23. Tiainen, M, Ylikorkala, A, Makela, TP: Growth suppression by LKB1 is mediated by a G(1) cell cycle arrest. Proc. Natl. Acad. Sci. USA 1999, 96: 9248-9251.
24. Tiainen, M, Vaahtomeri, K, Ylikorkala, A, Makela, TP: Growth arrest by the LKB1 tumor suppressor: induction of p21(WAF1/CIP1). Hum. Mol. Genet. 2002, 11: 1497-1504.
25. Jishage, K et al.: Role of Lkb1, the causative gene of Peutz-Jegher's syndrome, in embryogenesis and polyposis. Proc. Natl. Acad. Sci. USA 2002, 99: 8903-8908.
26. Bardeesy, N, Sinha, M, Hezel, AF, Signoretti, S, Hathaway, NA, Sharpless, NE, Loda, M, Carrasco, DR, DePinho, RA: Loss of the LKB1 tumour suppressor provokes intestinal polyposis but resistance to transformation. Nature 2002, 419: 162-167.
27. Miyoshi, H, Nakau, M, Ishikawa, TO, Seldin, MF, Oshima, M, Taketo, MM: Gastrointestinal hamartomatous polyposis in LKB1 heterozygous knockout mice. Cancer Res. 2002, 62: 2261-2266.
28. Rossi, DJ et al.: Induction of cyclooxygenase-2 in a mouse model of Peutz-Jeghers polyposis. Proc. Natl. Acad. Sci. U S A 2002, 99: 12327-12332.
29. Nakau, M, Miyoshi, H, Seldin, MF, Imamura, M, Oshima, M, Taketo, MM: Hepatocellular carcinoma caused by loss of heterozygosity in LKB1 gene knockout mice. Cancer Res. 2002, 62: 4549-4553.
30. Baas, AF, Boudeau, J, Sapkota, GP, Smit, L, Medema, R, Morrice, NA, Alessi, DR, Clevers, HC: Activation of the tumour suppressor kinase LKB1 by the STE20-like pseudokinase STRAD. EMBO J. 2003, 22: 3062-3072.
31. Boudeau, J, Baas, AF, FDeak, M, Morrice, NA, Kieloch, A, Schutowski, M, Prescott, AR, Clevers, HC, Alessi, DR: MO25 isoforms interact with the STE20-related psedokinase STRADα/β and enhance their ability to bind, activate and localise the LKB1 tumour suppressor in the cytoplasm. EMBO J. 2003: submitted; Example 1.
32. Corton, JM, Gillespie, JG, Hawley, SA, Hardie, DG: 5-Aminoimidazole-4-carboxamide ribonucleoside: a specific method for activating AMP-activated protein kinase in intact cells? Eur. J. Biochem. 1995, 229: 558-565.
33. Hawley, SA, Gadalla, AE, Olsen, GS, Hardie, DG: The anti-diabetic drug metformin activates the AMP-activated protein kinase cascade via an adenine nucleotide-independent mechanism. Diabetes 2002, 51: 2420-2425.
34. Sapkota, GP, Deak, M, Kieloch, A, Morrice, N, Goodarzi, AA, Smythe, C, Shiloh, Y, Lees-Miller, SP, Alessi, DR: Ionizing radiation induces ataxia telangiectasia mutated kinase (ATM)-mediated phosphorylation of LKB1/STKll at Thr-366. Biochem. J. 2002,368:507-516.
35. Collins, SP, Reoma, JL, Gamm, DM, Uhler, MD: LKB1, a novel serine/threonine protein kinase and potential tumour suppressor, is phosphorylated by cAMP-dependent protein kinase (PKA) and prenylated in vivo. Biochem. J. 2000, 345 Pt 3: 673-680.
36. Sapkota, GP, Kieloch, A, Lizcano, JM, Lain, S, Arthur, JS, Williams, MR, Morrice, N, Deak, M, Alessi, DR: Phosphorylation of the protein kinase mutated in Peutz-Jeghers cancer syndrome, LKB1/STK11, at Ser431 by p90(RSK) and cAMP-dependent protein kinase, but not its farnesylation at Cys(433), is essential for LKB1 to suppress cell vrowth. J. Biol. Chem. 2001, 276: 19469-19482.
37. Sapkota, GP, Boudeau, J, Deak, M, Kieloch, A, Morrice, N, Alessi, DR: Identification and characterization of four novel phosphorylation sites (Ser31, Ser325, Thr336 and Thr366) on LKB1/STK11, the protein kinase mutated in Peutz-Jeghers cancer syndrome. Biochem. J. 2002, 362: 481-490.
38. Boudeau, J, Deak, M, Lawlor, MA, Morrice, NA, Alessi, DR: Heat-shock protein 90 and Cdc37 interact with LKB1 and regulate its stability. Biochem. J. 2003, 370: 849-857.
39. Alessi, DR: Discovery of PDK1, one of the missing links in insulin signal transduction. Colworth Medal Lecture. Biochem. Soc. Trans. 2001, 29: 1-14.
40. Manning, G, Whyte, DB, Martinez, R, Hunter, T, Sudarsanam, S: The protein kinase complement of the human genome. Science 2002, 298: 1912-1934.
41. **Kinase.com** [http://www.kinase.com/]
42. Sugden, C, Donaghy, PG, Halford, NG, Hardie, DG: Two SNF1-related protein kinases from spinach leaf phosphorylate and inactivate HMG-CoA reductase, nitrate reductase and sucrose phosphate synthase in vitro. Plant Physiol. 1999, 120: 257-274.
43. Wilson, WA, Hawley, SA, Hardie, DG: The mechanism of glucose repression/derepression in yeast: SNF1 protein kinase is activated by phosphorylation under derepressing conditions, and this correlates with a high AMP:ATP ratio. Current Biol. 1996, 6: 1426-1434.
44. Scott, JW, Norman, DG, Hawley, SA, Kontogiannis, L, Hardie, DG: Protein kinase substrate recognition studied using the recombinant catalytic domain of AMP-activated protein kinase and a model substrate. J. Mol. Biol. 2002, 317: 309-323.
45. Woods, A, Salt, I, Scott, J, Hardie, DG, Carling, D: The α1 and α2 isoforms of the AMP-activated protein kinase have similar activities in rat liver but exhibit differences in substrate specificity in vitro. FEBS Lett. 1996, 397: 347-351.
46. Sugden, C, Crawford, RM, Halford, NG, Hardie, DG: Regulation of spinach SNF1-related (SnRK1) kinases by protein kinases and phosphatases is associated with phosphorylation of the T loop and is regulated by 5'-AMP. Plant J. 1999, 19: 1-7.
47. Hardie, DG, Salt, IP, Davies, SP: Analysis of the role of the AMP-activated protein kinase in the response to cellular stress. Methods Mol. Biol. 2000, 99: 63-75.
48. Bradford, MM: A rapid and sensitive method for the quantitation of microgram quantities of protein utilizing the principle of protein-dye binding. Anal. Biochem. 1976, 72: 248-254.
49. Devereux, J, Haeberli, P, Smithies, O: A comprehensive set of sequence analysis programs for the VAX. Nuc. Acids Res. 1984,12: 387-395.
50. **BOXSHADE v3.21** [http://www.ch.embnet.org/software/BOX_fbrm.html]

### Example 3: Peptide substrate for LKB1

A kinase assay for LKB1 can be performed using a peptide substrate, for example using the T-loop peptide of AMPK whose sequence is
LSNMMSDGEFLRTSCGSPNRRR
(HUMAN AMPKalpha-1 Residues 163-181 with 3 additional Arg residues added to the C-terminal to enable binding to P81 paper).

An assay may be performed as follows. Other assays using such a substrate peptide may be used, for example in a high throughput format. The standard assay (50 µl) contained: 0.3 µg of purified GST-LKB1-STRADα-MO25α complex, 50 mM Tris/HCl pH 7.5, 0.1 mM EGTA, 0.1% (by vol) 2-mercaptoethanol, 10mM magnesium acetate, 0.1 mM [γ³²P]ATP (~200 cpm/pmol) and T-loop peptide (LSNMMSDGEFLRTSCGSPRRR, 10-2000 µM). The assays were carried out for 30 min at 30°C, then terminated by pipetting the reaction mixture onto a 1 cm square of p81 paper, which was washed with 6 changes of 250 ml of 50 mM phosphoric acid (2 min each wash), the paper washed once in 200 ml of acetone and dried. Radioactivity bound to the p81 paper was quantitated by Cherenkov counting. 1 unit of activity is the amount of enzyme that catalyses the phosphorylation of 1 nmol of peptide per minute.

The Km for the peptide was estimated to be 1.80± 0.48 and the Vmax 23.4 ± 3.5

### Example 4: Activation of AMPK-related kinases by LKB1.

12 other protein kinases (NUAK1, NUAK2, BRSK1, BRSK2, QIK, QSK, SIK, MARK1, MARK2, MARK3, MARK4 and MELK) are closely related to the AMP-activated protein kinase (AMPK). Here we demonstrate that LKB1 can phosphorylate the T-loop of all members of the AMPK-subfamily, (not demonstrated for MELK), increasing their activity >50-fold. LKB 1 catalytic activity and the presence of M025 and STRAD are required to activate all the AMPK-related kinases. Mutation of the T-loop. Thr phosphorylated by LKB1 to Ala prevented activation, while mutation to Glu produced constitutively active forms of many of the AMPK-related kinases. We developed assays to measure the activities of endogenous NUAK2, QIK, QSK, SIK, MARK1, MARK2/3 and MARK4 kinases in cells expressing LKB1, and exploited these to show that the activities of these enzymes were markedly reduced in LKB1^{-*l*-} fibroblasts or in HeLa cells that do not express LKB1. Interestingly, neither LKB 1 activity, nor that of any of the AMPK-related kinases is stimulated by phenformin or AICA riboside, two drugs that induce the phosphorylation and activation of AMPK. Our results show that LKB1 functions as a master upstream protein kinase, regulating the activities of the AMPK-related kinases as well as AMPK. Between them, these kinases may mediate some of the physiological effects of LKB1, including its tumour suppressor function.

Inspection of the human kinome indicates that there are 12 protein kinases (BRSK1, BRSK2, NUAK1, NUAK2, QIK, QSK, SIK, MARK1, MARK2, MARK3, MARK4 and MELK) that are closely related to AMPKα1 and AMPKα2 (Fig 21A and B). The nomenclature we will employ for the "AMPK-related kinases" is based upon that used by Manning and colleagues (Manning et al., 2002). MARK3 is also known as PAR1A or C-TAK1 (Peng et al., 1998; Spicer et al., 2003), NUAK1 as ARK5 (Suzuki et al., 2003b), NUAK2 as SNARK (Lefebvre et al., 2001) and QIK as SIK2 (Horike et al., 2003). The T-loop Thr residue of AMPKα1 and AMPKα2 that is phosphorylated by LKB1, and many of the surrounding residues, are conserved in the AMPK-related kinases (Fig 21A & B). This indicates that LKB1 might also phosphorylate the T-loop Thr residue and hence regulate the AMPK-related kinases in the same way that PDK1 regulates the activity of a group of related AGC kinases (Alessi, 2001). The MARK kinases have been proposed to play key roles in controlling cell polarity and are known to be regulated by phosphorylation of their T-loop Thr residue (Lrewes and Nurse, 2003; Spicer et al., 2003). Studies in *Drosophila* initially indicated that MARK3 might lie upstream of LKB1 (Martin and St Johnston, 2003), but recent work in mammalian cells disputed this conclusion and suggested that LKB 1 lies upstream of MARK3 and controls its activity (Spicer et al., 2003). Little is known about the function or mechanism of regulation of the remaining AMPK-related enzymes. In this Example we therefore sought to investigate the role that LKB1 plays in regulating the activity of the AMPK-related kinases.

### Results

**Activation of AMPK-related kinases by the LKB1 complex.** We cloned and expressed in E. *coli* the full-length versions of all of the twelve AMPK-related kinases and developed assays for these enzymes employing the *AMARA* AMPK peptide substrate for AMPK (Dale et al., 1995), which is not phosphorylated by the LKB1 complex (JML, data not shown). We found that all of the AMPK-related kinases purified from E. *coli,* phosphorylated this peptide and possessed low basal activities of < 1 U/mg (1 Unit = 1 nmol peptide phosphorylated per min), with the exception of MELK, which had an activity of ~40 U/mg (Fig 21). Following incubation of the AMPK-related kinases with the LKB1:STRAD:MO25 complex and MgATP, the activity of the AMPK-related kinases, was increased 50 to 200-fold, except for MELK whose activity was hardly increased at all (Fig 21). Catalytically inactive LKB1 complexed to STRADα and MO25α failed to increase the basal activity of the AMPK-related kinases, indicating that the kinase activity of LKB 1 was required (Fig 21).
**Activation of AMPK-related kinases by LKB1 requires STRAD and MO25**. To determine the importance of the STRAD and M025 subunits in enabling LKB1 to activate the AMPK-related kinases, we expressed GST-tagged LKB1, FLAG-tagged STRADα/β and *myc*-tagged MO25α/β in various combinations in 293 cells, and affinity purified the complexes on glutathione-Sepharose (Fig 22). The purified complexes were incubated in the presence of MgATP with the AMPK-related kinases and their activation was measured. LKB1 on its own did not activate any of the AMPK-related kinases significantly (Fig 22, compare lanes 1 and 14). The same result was obtained with LKB1 that had been co-expressed with MO25α or MO25β (Fig 22, lanes 4 and 5). This was as expected, since these proteins do not interact with LKB1 in the absence of STRADα/β (Example 1; Boudeau et al., 2003a). An LKB1:STRADα complex gave a small activation (Fig 22, compare lanes 1 and 2), but a heterotrimeric complex containing LKB1, STRADα or STRADβ, and MO25α or MO25β was required to obtain a large activation (Fig 22, lanes 6 to 9). As previously reported for AMPKα1 (Fig 22 and Example 2; Hawley et al., 2003), all AMPK-related kinases were activated most efficiently by the LKB1:STRADα:MO25α complex, although the relative effectiveness of different heterotrimeric complexes varied from substrate to substrate (Fig 22). Heterotrimeric complexes containing a catalytically inactive mutant of LKB 1 were unable to activate any enzyme (Fig 22, lanes 10-13). It should be noted that when STRAD isoforms were co-expressed with LKB1 in the absence of MO25, the amount of STRAD co-precipitated with LKB1 was markedly reduced (Fig 22 compare lanes 2 and 3 with lanes 6 and 7), as M025 was required to stabilise the LKB1:STRAD complex (Example 1; Boudeau et al., 2003a). **The LKB1 complex phosphorylates AMPK-related** kinases **at the activation loop.** As the LKB1 complex was previously shown to phosphorylate AMPKα1 specifically at Thr172 in its activation loop, we first mutated the equivalent T-loop residue in the AMPK-related kinases (Fig 21A & B) to Ala, and tested how this affected phosphorylation of these enzymes by the LKB 1 complex. We demonstrated that the wild type AMPK-related kinases were phosphorylated by the LKB1 complex and mutation of the T-loop Thr residue to Ala abolished or significantly reduced phosphorylation (Fig 23). We also performed detailed phosphorylation site analysis of catalytically inactive BRSK2 and NUAK2 mutants that had been phosphorylated in vitro by the LKB 1 complex (Fig 30). The ³²P-labelled BRSK2 and NUAK2 proteins were digested with trypsin and the resulting peptides separated by chromatography on a C₁₈ column. The major ³²P-labelled peptides were analysed by MALDI TOF-TOF mass spectrometry and solid phase Edman sequencing, which demonstrated that these enzymes were phosphorylated at their T-loop Thr residue (Fig 30).
**T-loop phosphorylation activates AMPK-related kinases**. To study the role that T-loop phosphorylation of BRSK1, BRSK2, NUAK1, NUAK2, QIK, QSK, SIK and MELK plays in their regulation, the T-loop Thr residues of these enzymes were mutated to either Ala to prevent phosphorylation, or Glu to mimic phosphorylation. In all cases, the basal activity of the Ala mutants was low and their activity not increased by incubation with the LKB 1 complex (Fig 24). In contrast, the basal activity of the Glu mutants was similar to that of the wild type enzyme phosphorylated by LKB1, and was not further increased following incubation with the LKB1 complex and MgATP (Fig 24). In the case of MELK, the Ala mutant possessed low activity and the Glu mutant was of similar activity to the wild type enzyme, suggesting that wild type MELK (like other AMPK-related kinases) required T-loop phosphorylation for activity. As wild type MELK expressed in E. *coli* was active MELK may be able to catalyse phosphorylation of its own T-loop Thr residue. Consistent with this, MALDI TOF-TOF analysis of wild type MELK expressed in *E*. *coli* established that the T-loop was indeed phosphorylated (Fig 30C).
**Phosphorylation and activation of the MARK kinases by LKB1.** Previous studies have revealed that the MARK3 and MARK3 kinases, in addition to being phosphorylated at the T-loop Thr residue, are also phosphorylated at a nearby Ser residue (Fig 21A and B and (Drewes et al., 1997; Johnson et al., 1996; Timm et al., 2003)). Peptide-mapping of catalytically inactive MARK3 phosphorylated by the LKB1 complex, revealed that both the Thr211 and Ser215 residues within the T-loop were phosphorylated (Fig 25A). Interestingly, catalytically inactive MARK3[T211A] mutant was not phosphorylated at Ser215 by LKB1, but the catalytically inactive MARK3[S215A] mutant was still phosphorylated at Thr211 (Fig 25A). We next investigated how mutation of the T-loop Thr or Ser residue affected activation of MARK3 by the LKB1 complex (Fig 25C). The MARK3[T211A] mutant could not be activated by the LKB1 complex but the MARK3[S215A] mutant was activated to a small extent. The MARK3[T211E] mutant possessed only ~15% of the activity of the wild type enzyme activated by the LKB1 complex and could not be activated further by LKB 1. The MARK3[S215E] mutant was inactive and could not be activated by LKB 1 (Fig 25C). As expected, we also found that mutation of the T-loop Thr to Ala in MARK1, MARK2 and MARK4 prevented their activation by LKB1 (Fig 25D to F). Mutation of the T-loop Thr residue to Glu in MARK1, MARK2 and MARK3, resulted in either no activation or only a small activation of these enzymes (Fig 25D to F). In contrast, the equivalent mutation in MARK4, vastly increased activity (Fig 25F). Peptide-mapping studies and MALDI TOF-TOF mass spectrometry demonstrated that LKB1 phosphorylated the MARK4 T-loop at only the Thr residue and not the Ser residue (Fig 25G and Fig 30C).

**Identification of peptide substrates for LKB1**. We next tested whether the LKB1 complex was able to phosphorylate peptides encompassing the activation loop of AMPKα1, BRSK2, NUAK2, SIK, MARK3 and MELK. All the peptides were phosphorylated, but with differing efficiencies (Fig 26A). Solid phase Edman sequencing confirmed that every peptide was specifically phosphorylated by LKB1 at the T-loop Thr residue (Fig 26B). The optimal peptide substrate derived from the T-loop of NUAK2 was phosphorylated by LKB1 with a Kₘ value of 150 µM and was a better substrate than the peptide derived from the T-loop of AMPKα1 (Kₘ, 1. 4 mM) (Fig 26A). The MELK peptide was the poorest substrate. We also assayed the different combinations of LKB1 STRADα/β and MO25α/β complexes used in Fig 22, with the T-loop peptides as substrates, and the pattern of LKB 1 activity mirrored that observed using the full-length proteins (Compare Fig 22 and Fig 26C). These results indicate that the T-loop peptides can be employed as bona fide substrates to measure LKB1 activity in a facile single step assay. The NUAK2 T-loop peptide was termed LKBtide.
**Role of LKB1 in regulating AMPK-related kinases in fibroblasts.** In order to investigate the role of LKB 1 in regulating AMPK-related kinases in intact cells, we generated peptide antibodies against specific sequences in the twelve AMPK-related kinases. The ability of these antibodies to immunoprecipitate the active forms of the AMPK-related kinases was assessed employing HEK-293 cell lysates in which the kinases had been over-expressed and found to be active (data not shown). Using this approach we were able to develop antibodies that selectively immunoprecipitated all the AMPK-related kinases except for MARK2 and MARK3, whose activity vas assessed using a commercial antibody that immunoprecipitated both of these kinases, but not MARK1 and MARK4 (data not shown). Using the specific antibodies, we were able to immunoprecipitate and assay endogenously expressed NUAK2, QIK, QSK, SIK MARK1, MARK2/3 and MARK4 in LKB1^{+/+} mouse embryonic fibroblasts (MEFs) (Fig 27). We then compared the activities of these kinases immunoprecipitated from LKB1^{+/+} and LKB1^{-/-} MEFs, and found that the activity of NUAK2, QIK, QSK, SIK, MARK1 and MARK4 was reduced between 7 to 35-fold in the LKB1^{-/-} MEFs (Fig 27). The combined MARK2/3 activity was reduced ~3-fold in the LKB1^{-/-} MEFs (Fig 27G). Immunoblotting demonstrated that the reduced activity of AMPK-related kinases in LKB1^{-/-} cells was not caused by a decrease in expression of the enzymes, which were either present at the same or slightly reduced levels in the knockout cells.

Interestingly, and in contrast to AMPKα1 (Fig 27A), the AMPK-related kinases assayed, were not significantly stimulated phenformin (Fig 27B to 27H). This suggested that phenformin was not triggering activation of AMPK by activating LKB1. Consistent with this, we demonstrated that increasing doses of phenformin that progressively activated AMPK (and increased the phosphorylation of Thr172), but did not stimulate LKB1 activity (Fig 28A). The drug 5-aminoimidazole-4-carboxamide (AICA) riboside activates AMPK in intact cells by being taken up and converted by adenosine kinase to AICA riboside monophosphate, which mimics the effect of AMP on the AMPK system (Corton et al., 1995). AICA riboside activated AMPK in LKB1^{+/+} MEFs, but failed to markedly activate any of the AMPK-related kinases (Fig 28B).
**Expression of LKB1 restores activation of AMPK-related kinases in HeLa cells.** To obtain further genetic evidence that LKB1 acts as an upstream regulator of the AMPK-related kinases, we compared the activity of AMPK-related kinases in HeLa cells, which do not to express LKB1, with HeLa cells stably expressing either wild type or catalytically inactive LKB1 (Example 2; Hawley et al., 2003; Sapkota et al., 2002). In control HeLa cells not expressing LKB1, or cells expressing catalytically inactive LKB1, the activity of NUAK2, QIK, QSK and SIK was 20 to 40 fold lower than that observed in HeLa cells that express wild type LKB 1 (Fig 29B to 29E). MARK1, combined MARK2/3 activity and MARK4 activity in control HeLa cells was about ~2-3 fold lower than in cells expressing LKB1 (Fig 29F to 29H). In contrast to AMPKα1 (Fig 29A), none of the AMPK-related kinases were significantly activated when HeLa cells were stimulated with phenformin (Fig 29B to 29H).

### Discussion

In this Example we provide evidence that LKB1 functions to regulate the activity of 11 of the 12 members of the AMPK-related family of protein kinases. In cell-free systems we established that all AMPK-related kinases tested, with the exception of MELK, are activated over 50-fold following the phosphorylation of their T-loop Thr residue, by the LKB1:STRAD:MO25 complex. In the case of MELK, our results indicate that this enzyme requires T-loop phosphorylation for activity, but that it is able to phosphorylate its own T-loop residue. To our knowledge this is the first demonstration that the BRSK1, BRSK2, NUAK1, NUAK2, QIK, QSK, SIK as well as MELK enzymes can be activated by phosphorylation. Mutation of the T-loop T1 residue to Ala prevented the activation of all of these enzymes by the LKB1 complex, whereas its mutation to Glu was sufficient to activate BRSK1, BRSK2, NUAK1, NUAK2, SIK, QIK, QSK and MARK4 to specific activities similar to that observed for the LKB1-phosphorylated forms of these enzymes (Fig 24). This latter finding could be exploited in future over-expression or knock-in studies to introduce constitutively active forms of these enzymes in cells to examine their cellular roles.

Previous work has indicated that MARK2 and MARK3 were phosphorylated at the T-loop Thr residue as well as at a nearby Ser residue (Drewes et al., 1997; Johnson et al., 1996). The T-loop Ser residue phosphorylated in MARK2/MARK3 is conserved in AMPKα1, AMPKα2 and all AMPK-related kinases (Fig 21A & B), but at least for AMPKα1 there is no evidence that this residue is phosphorylated in vivo (Woods et al., 2003b). Our analysis (Fig 25A and 25B), as well as that of a recent study (Timm et al., 2003), showed that phosphorylation of the T-loop Thr residue of the MARK kinases, played the most crucial role in regulating the activity of this class of kinase. The LKB1 complex phosphorylated a catalytically inactive MARK3 mutant at both the Thr and Ser T-loop residue (Fig 25A). However, the isolated MARK3 T-loop peptide was only phosphorylated by LKB1 at the Thr residue (Fig 26), signifying that the primary sequence of the peptide was not sufficient to enable LKB1 to phosphorylate the Ser residue. The finding that mutation of the T-loop Thr211 to Ala on MARK3 prevented phosphorylation of Ser215 by the LKB1 complex, suggests that phosphorylation of Thr211 is required for Ser215 phosphorylation. MARK4 may be regulated differently as peptide-mapping studies indicated that MARK4 was only phosphorylated at the T-loop Thr by LKB1 (Fig 25G), and that mutation of the T-loop Thr to Glu increased activity to a much greater level than observed for other MARK isoforms (Fig 25F). It was previously reported for MARK2 that mutation of the T-loop to Glu increased its specific activity 3-fold (Timm et al., 2003) and we have made similar findings with this mutation increasing MARK2 activity form 2.2 U/mg to 6.7 U/mg (Fig 25D). However, in contrast to TAO1 that only activated MARK2 10-fold (Timm et al., 2003), the LKB1 complex increased MARK2 activation 200-fold, establishing that mutation of the MARK2 T-loop Thr to Glu does not markedly activate the kinase.

As observed for the activation of AMPKα1 and AMPKα2 (Hawley et al., 2003), the presence of STRAD and M025 subunits was essential for LKB 1 to activate all the AMPK-related kinases in cell free-assays. There is considerable evidence that many protein kinases rely on residues, termed docking sites, lying outside of the catalytic core, to bring the kinase and substrate together. It is possible that the STRAD and M025 subunits play a role in facilitating the interaction with, and hence the phosphorylation by LKB1. The finding that the LKB1:STRAD:MO25 complex phosphorylated the short T-loop peptide of AMPKα1 and AMPK-related kinases, at a much higher rate than LKB 1 alone, indicated that the STRAD and M025 subunits activate LKB 1 directly. However, this result does not rule out the possibility that STRAD and MO25 also play roles in substrate recognition.

Employing LKB 1^{-/-} MEFs and/or HeLa cells that lack LKB 1, we were able to demonstrate that the activities of endogenously expressed NUAK1, NUAK2, QIK, QSK, and SIK were 7 to 40-fold lower than in LKB^{+/+} MEFs or HeLa cells that stably express wild type LKB1. This provides genetic evidence that LKB1 is rate-limiting in activation of these enzymes in intact cells. However, our data do not rule out the possibility that other kinases can regulate the activity of the AMPK-related kinases in vivo in addition to LKB1. Recently the TAO1 kinase was purified from pig brain as the major activity that phosphorylated the T-loop Thr residue of MARK2, resulting in its activation (Timm et al., 2003). Although TAO1 also activated MARK2 in over-expression studies (Timm et al., 2003), thus far no genetic evidence indicating how the lack of TAO1 affected the activity of MARK family kinases in vivo, has been reported. Such evidence may be hard to acquire, as mammalian cells possess 3 closely related TAO isoforms (Manning et al., 2002). Lack of specific immunoprecipitating antibodies meant that we were unable to establish how a deficiency of LKB1 affected the individual activities of MARK2 or MARK3. However, the finding that the combined activity of MARK2 and MARK3 in LKB1^{-/-} and control HeLa cells was substantial, implies that other kinases, such as TAO1, may regulate the activities of these enzymes. It should be noted that LKB1 and TAO1 share no obvious amino acid sequence homology and lie in distinct regions of the human kinase dendrogram (Manning et al., 2002). TAO1 belongs to the STE20 group of kinases and is therefore related to STRADα and STRADβ, but whether this is significant is not known.

The MARK (MAP/microtubule affinity regulating kinase) family of kinases are the most studied AMPK-related kinase family members and are thought to play key roles in establishing cell polarity. For example genetic studies indicate that MARK homologues control partitioning of the *C. elegans* zygote (Guo and Kemphues, 1995) and embryonic axis formation in *Drosophila* (Shulman et al., 2000; Tomancak et al., 2000). In neuronal cells, MARK kinases phosphorylate the neuronal microtubule associated protein Tau, resulting in the destabilisation of microtubules (Drewes et al., 1997). Interestingly, the counterpart of mammalian LKB1 in C. *elegans* (Watts et al., 2000), termed PAR4, was originally identified as a member of the maternally expressed *PAR* (partitioning defective) gene family, required for establishing cell polarity during the first cycle of *C. elegans* embryogenesis (Kemphues et al., 1988). Maternal lethal mutations in the gene encoding *C*. *elegans* PAR4, have been shown to affect several aspects of cell polarity (Morton et al., 1992). These lead to phenotypes similar to those observed in *C. elegans* mutated in the PAR1 gene, which encodes the homologue of MARK3 (Guo and Kemphues, 1995). More recently, both the *Drosophila* (Martin and St Johnston, 2003) and *Xenopus* (Ossipova et al., 2003) homologues of human LKB1 were shown to play important roles in regulating cell polarity. Originally, it was suggested that *Drosophila* LKB 1 functioned downstream of PAR1/MARK3, as over-expression of *Drosophila* LKB1 suppressed the polarity phenotype of PAR1/MARK3 mutants (Martin and St Johnston, 2003). However, in HeLa cells that do not express LKB1, MARK3 was found to exist in a dephosphorylated state and reintroduction of LKB 1 promoted phosphorylation of MARK3 at its T-loop (Spicer et al., 2003), signifying that LKB1 was upstream of MARK3. One explanation that would reconcile the apparent discrepancy between the *Drosophila* and mammalian studies would be if over-expression of *Drosophila* LKB1 suppressed the polarity phenotype of PAR1/MARK3 mutants, through the activation of other AMPK-related kinases, which might be able to compensate for the loss of PAR1/MARK3 function.

Much less is known regarding the function of other AMPK-related kinases, i.e. BRSK1, BRSK2, NUAK1, NUAK2, QIK, QSK and SIK. Previous Northern blotting of NUAK1, NUAK2, QIK and SIK performed by other groups (see below) and analysis of EST clones (Table 2), indicated that the AMPK-related kinases with the exception of BRSK1, BRSK2 and MELK are likely to be expressed in many tissues. BRSK1 and BRSK2 EST clones were mainly derived from neuronal tissues and, after immunoblot analysis of a variety of rat tissues, these enzymes were only detected in brain and at low levels in testis (K.Sakamoto, unpublished results). To date, MELK (maternal embryonic leucine zipper kinase) has only been reported to be expressed during mammalian embryogenesis, with the strongest expression detected during maturation of oocytes and preimplantation development (Heyer et al., 1999; Heyer et al., 1997). Although our antibodies raised against BRSK1, BRSK2 and MELK readily immunoprecipitated the recombinant enzymes, we were unable to detect their activity in MEFs or HeLa cells (OG, data not shown). SIK (Salt-inducible kinase) was first cloned from the adrenal glands of rats fed a high salt diet (Wang et al., 1999). SIK mRNA was also induced by membrane depolarization in brain (Feldman et al., 2000) and recent studies have indicated that when SIK is over-expressed in cells, it might play a role in steroidogenesis (Takemori et al., 2003). The mRNA expressing QIK (also termed SIK2), was highest in adipose tissue and, in over-expression studies, QIK was reported to phosphorylate human IRS1 at Ser794 (Horike et al., 2003), the residue equivalent to Ser789 in rat IRS1, shown to be phosphorylated by AMPK (Jakobsen et al., 2001). In other over-expression studies, NUAK1 (ARK5) was shown to suppress apoptosis induced by some stimuli, including nutrient starvation (Suzuki et al., 2003a). Furthermore, it has been claimed that Akt/PKB phosphorylated NUAK1 at a C-terminal site outside of the catalytic domain, leading to a 3-fold activation of the enzyme (Suzuki et al., 2003b). NUAK2 (SNARK) was most highly expressed in the kidney, and its activity reportedly stimulated by glucose starvation of cells (Lefebvre et

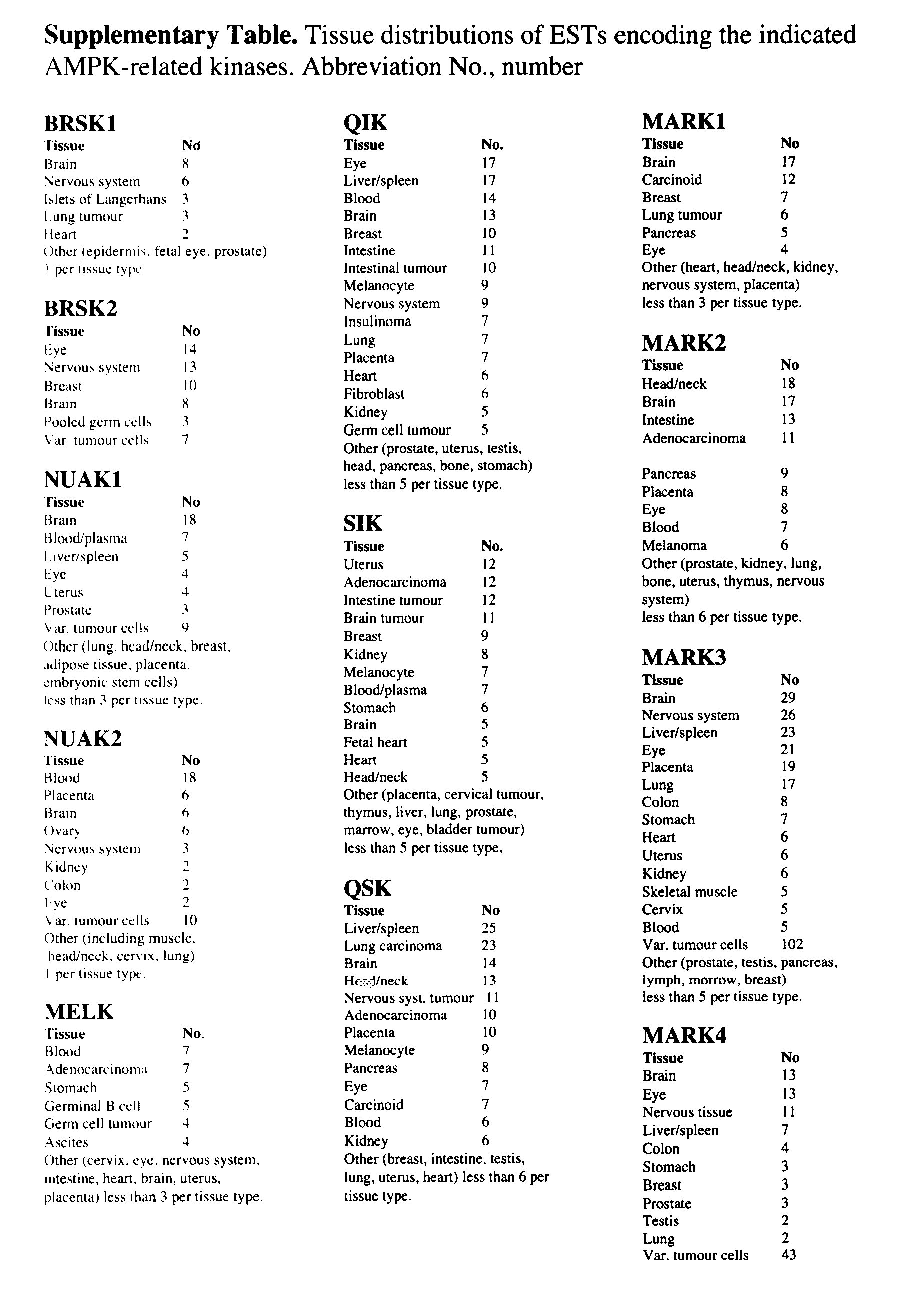

al., 2001; Suzuki et al., 2003b). To our knowledge, no previous studies have addressed the roles of BRSK1, BRSK2 and QSK.

Clearly, further work needs to be carried out to further characterise the mechanism of regulation and function of the AMPK-related kinases. Our studies indicate that, at least in MEFs and HeLa cells that stably express LKB1, the AMPK-related kinases were not activated in response to the drugs phenformin or AICA-riboside, in contrast to AMPK. This suggests that the beneficial anti-diabetic effects of phenformin and metformin may be mediated through the activation of AMPK rather than the AMPK-related kinases. The finding that AMPK-related kinases are not activated by phenformin or AICA riboside, is consistent with our findings that these treatments do not activate LKB1 directly in LKB1^{+/+} MEFs (Fig 28A) or in COS7 cells (Woods et al., 2003a). In future studies it will be important to define the physiological stimuli that do cause activation of the AMPK-related kinases, and establish whether these enzymes, like AMPK, possess regulatory subunits that control their activation.

A significant number of inherited forms of PJS found in certain families do not display mutations in the LKB 1 gene (Buchet-Poyau et al., 2002; Resta et al., 2002), indicating that there is a second causative locus for PJS. It will clearly now be critical to find out whether PJS families that express the normal LKB 1 protein have mutations in other AMPK-related kinases.

In conclusion, we have shown that LKB1 functions as a master upstream protein kinase activating 11 AMPK-related kinases in addition to AMPKα1 and AMPKα2. It is possible that these enzymes mediate some of the physiological effects previously ascribed to LKB1 and that one or more of these kinases may themselves function as tumour suppressors.

**Materials**. Protease-inhibitor cocktail tablets and sequencing grade trypsin were obtained from Roche. N-octyl-glucoside was from Calbiochem, and tissue culture reagents were from Life Technologies. Tetracyclin-free foetal bovine sera (FBS) was from Perbio, and other tissue culture reagents were from Biowhittaker. Precast 4-12% and 10% polyacrylamide Bis-Tris gels were obtained from Invitrogen; P81 phosphocellulose paper was from Whatman; Phenformin from Sigma and AICA riboside from TorontoXX. [γ-³²P]ATP, glutathione-Sepharose, Protein G-Sepharose were purchased from Amersham Biosciences. All peptides were synthesised by Dr Graham Bloomberg at the University of Bristol.

### Antibodies.

The following antibodies were raised in sheep and affinity purified on the appropriate peptide antigen: BRSK1 (MVAGLTLGKGPESPDGDVS, residues 1-20 of human BRSK1), BRSK2 (LSWGAGLKGQKVATSYESSL, residues 655-674 of human BRSK2), NUAK1 (MEGAAAPVAGDRPDLGLGAPG residues 1-21 of human NUAK1), NUAK2 (TDCQEVTATYRQALRVCSKLT, residues 653-673 of human NUAK2), QIK (MVMADGPRHLQRGPVRVGFYD, residues 1-21 of human QIK), SIK (MVIMSEFSADPAGQGQGQQK, residues 1-20 of human SIK used for immunoprecipitation from human cells), SIK (GDCEMEDLMPCSLGTFVLVQ, residues 765-784 of human SIK used for immunoprecipitation from mouse cells), QSK (TDILLSYKHPEVSFSMEQAGV, residues 1349-1369 of human QSK), MARK1 (SGTSIAFKNIASKIANELKL, residues 776-795 of human MARK1), MARK4 (MSSRTVLAPGNDRNSDTHGT, residues 1-20 of human MARK4), MELK (MKDYDELLKYYELHETIGTG, residues 1-20 of human MELK). The specific AMPKα1 antibody employed in Figures 27, 28B and 29 was raised against the peptide (CTSPPDSFLDDHHLTR, residues 344-358 of rat AMPKα1) whilst the antibody recognising both AMPKα1 and AMPKα2 employed in Fig 28A, was raised against the peptide (CDPMKRATIKDIRE residues 252 to 264 or rat AMPKα1). The anti MARK2 and MARK3 peptide antibodies we raised were not specific as they immunoprecipitated other MARK isoforms as well. The anti MARK3 antibody from Upstate Biotech (anti c-TAK #05-680), raised against the human MARK3 protein was found to immunoprecipitate MARK2 as efficiently as MARK3 but did not immunoprecipitate MARK1 and MARK4 (data not shown). The Phosphospecific antibodies recognising AMPK phosphorylated on the T-loop were generated against as described previously against the peptide (KFLRT(P)SCGSPNYA residues 168 to 180 of rat AMPKα1) (Sugden et al., 1999).The LKB1 antibody used for immunoblotting was raised in sheep against the mouse LKB1 (Sapkota et al., 2001) and that for immunoprecipitation was raised in sheep against the human LKB1 protein (Boudeau et al., 2003). Monoclonal antibody recognizing the HA epitope tag was from Roche, monoclonal antibodies recognising the GST and the FLAG epitopes were from Sigma. Anti-myc antibodies were prepared by ammonium sulphate precipitation of medium from Myc1-9E10 hybridoma cells grown in RPMI 1640 medium supplemented with 2 mM glutamine and 15% (v/v) foetal bovine serum. Secondary antibodies coupled to horseradish peroxidase used for immunoblotting were obtained from Pierce.

**General methods and buffers**. Restriction enzyme digests, DNA ligations, and other recombinant DNA procedures were performed using standard protocols. All mutagenesis was performed using the QuikChange site-directed mutagenesis method (Stratagene). DNA constructs used for transfection were purified from *E. coli* DH5α using Qiagen plasmid Mega kit according to the manufacturer's protocol. All DNA constructs were verified by DNA sequencing, which was performed by The Sequencing Service, School of Life Sciences, University of Dundee, Scotland, UK, using DYEnamic ET terminator chemistry (Amersham Biosciences) on Applied Biosystems automated DNA sequencers. Lysis Buffer contained 50 mM Tris/HCl pH 7.5, 1 mM EGTA, 1 mM EDTA, 1% (w/v) Triton-X 100, 1 mM sodium orthovanadate, 50 mM sodium fluoride, 5 mM sodium pyrophosphate, 0.27 M sucrose, 0.1% (v/v) 2-mercaptoethanol and 'complete' proteinase inhibitor cocktail (one tablet/50 ml). Buffer A contained 50 mM Tris/HCl pH 7.5, 0.1 mM EGTA, and 0.1% (v/v) 2-mercaptoethanol. SDS sample Buffer contained 50 mM Tris/HCl pH 6.8, 2% (w/v) SDS, 10% (v/v) glycerol, 0.005% (w/v) bromophenol blue, and 1% (v/v) 2-mercaptoethanol.

### Cloning of AMPK-related kinases.

**NUAK1.** In order to obtain the coding region of human NUAK1 cDNA (NCBI Acc. NM_014840) a PCR reaction was carried out using a brain cDNA library (Clontech) as a template and the sense primer 5'-actgcagccctggagcccaggaagc-3' and the antisense primer 5'-ctagttgagcttgctgcagatctccagcgc-3'. The resulting PCR product covered the coding region of NUAK1 from amino acid residues 31-661. The cDNA of the missing N-terminal 31 amino acids was incorporated into the 5' primer of another PCR reaction, then the HA tag was added by PCR using the sense primer 5'-actagtgccaccatgtacccatacgatgtgccagattacgccgaaggggccgccgcgcctgtggcgggg-3' and antisense primer 5-ctagttgagcttgctgcagatctccagcgc-3'. The resulting PCR product was ligated into pCR2.1-TOPO vector (Invitrogen), and subcloned as a *Spel-Notl* fragment into pEBG2T (Sanchez et al., 1994) and as an *EcoR1-EcoR1* insert into pGEX6P-1 (Amersham) expression vectors.
**NUAK2**. The coding region of human NUAK2 cDNA (NCBI Acc. NP_112214) with an N-terminal HA tag, was amplified by PCR from IMAGE consortium EST clone 6718982 (NCBI Acc. CA487493) with primers 5'-actagtgccaccatgtacccatacgatgtgccagattacgccgagtcgctggttttcgcgcggcgctcc-3' and 5'- tcaggtgagctttgagcagaccctcagtgcctg-3'. The resulting PCR product was ligated into pCR2.1-TOPO vector, sequenced, and subcloned as a *Spel-Spe1* fragment into pEBG2T and as an *EcoR1-EcoR1* insert into pGEX6P-1 expression vectors.
**QIK**. The coding region of human QIK cDNA (NCBI Acc. XM_041314) with an N-terminal HA tag was amplified from IMAGE consortium EST clone 5495545 (NCBI Acc. BM799630) using sense primers 5'-gcgtcgactacccatacgatgtgccagattacgccgtcatggcggatggcccgag-3' or 5'-gcactagttacccatacgatgtgccagattacgccgtcatggcggatggcccgag-3' for subsequent cloning into vectors pGEX6P-3 and pEBG2T respectively, and antisense primer 5'-gagcggccgctaattcaccaggacatacccgttgtg-3'. Amplified PCR products were ligated into pCR2.1 TOPO vector, sequenced, and subcloned as a *Sall-Notl* or *Spel-Notl* insert into pGEX6P-3 or pEBG2T respectively.

**SIK.** The coding region of human SIK cDNA (NCBI Acc. NM_173354) with an N-terminal HA tag was amplified from IMAGE consortium EST clone 4831049 (NCBI Acc. NM_173354) using primers 5'-gcggatcctacccatacgatgtgccagattacgccgttatcatgtcggagttcagcgcgg-3' and 5'-gagcggccgctcactgcaccaggacaaacgtgcc-3'. The PCR product was ligated into pCR2.1 TOPO vector, sequenced, and subcloned as a *BamH1-Not1* insert into pGEX6P-1 and pEBG2T.
**MELK**. The coding region of human MELK cDNA (NCBI Acc. NM_014791) with an N-terminal HA tag was amplified from IMAGE consortium EST clone 4547136 (NCBI Acc. BC014039) using primers 5'-gcggatcctacccatacgatgtgccagattacgccaaagattatgatgaacttctcaaatattatgaattacatg -3' and 5'- gtgcggccgcttataccttgcagctagataggatgtcttcc-3'. The PCR product was ligated into pCR2.1 TOPO vector, sequenced, and subcloned as a *BamH1-Not1* insert into pGEX6P-1 and pEBG2T.
**BRSK1.** Nucleotides 60-1010 of the coding region of human BRSK1 cDNA (NCBI Acc. NM_032430) were amplified from IMAGE consortium clone 6154749 (NCBI Acc. BQ434571) using primers 1. 5'-ccacccccacccaccccagcacgcccaatatgtgggcccctatcggctggagaagacgctgggcaaagg -3' and 2. 5'-cgatgcagcctctcgcggtccctgaagcagc-3', nucleotides 60-106 being added by primer 1. Nucleotides 980-2337 of BRSK1 were amplified from the same EST clone using primers 3. 5'-gctgcttcagggaccgcgagaggctgcatcg-3' and 4. 5'-tcagggcagaggggtcccgttggtggcc-3'. Each PCR product was ligated into pCR2.1 TOPO vector and sequenced. A single nucleotide difference compared with BRSK1 NM_032430 sequence that is present in the EST clone was 'corrected' by site-directed mutagenesis in the pCR2.1 TOPO clone containing the 5' half of BRSK1. The remaining 5' 59 nucleotides of BRSK1 and the HA tag were added to the 5' half of BRSK1 by PCR using sense primers 5. 5'-ggtgggggctctcccgcctaccacctcccccacccccacccccacccaccccagcacgcccaatatg-3' and 6. 5'-ggatcctacccatacgatgtgccagattacgcctcgtccggggccaaggagggaggtgggggctctcccg cctacc-3'. The final PCR product was ligated into pCR2.1 TOPO vector and sequenced. Overlap PCR was then performed using the 5' half and 3' half of BRSK1 as templates and primer 7. 5'- gcggatcctacccatacgatgtgcc-3' and primer 4. and the PCR product ligated into pCR2.1 TOPO vector and sequenced. Full length BRSK1 cDNA with N-terminal HA tag was then subcloned into pGEX6P-1 and pEBG2T as a *BamHl-BamHl* insert.
**BRSK2**. The coding region of HA-tagged human BRSK2 cDNA (NCBI Acc. AF533878) was amplified by PCR from IMAGE consortium EST clone 6144640 (NCBI Acc. BU193218) using primers 5'-ggatccgccaccatgtacccatacgatgtgccagattacgccacatcgacggggaaggacggcggcgcg -3' and 5'-gcggccgctcagaggctactctcgtagctggtggccaccttctggcccttaagccca-3'. The resulting PCR product was cloned into pCR2.1-TOPO vector, sequenced, and subcloned as a *BamHl-Notl* insert into pEBG2T and pGEX6P-1 vectors.
**QSK**. Nucleotides 55-640 of the coding region of human QSK cDNA (sequence obtained from Sugen database www.kinase.com (Manning et al., 2002)) were amplified from IMAGE consortium EST clone 4396995 (NCBI Acc.BF983268) using primers 1. 5'-ggagccgggcccgcgggccgcctgctgcctccgcccgcgccggggtccccagccgcccccgctgccgt gtcccctgcggccggccagccg-3' and 2. 5'-tgaagaggttactgaaaccaaaatctgctattttgatattc-3', nucleotides 55-110 being added by primer 1. The remaining 5' 54 nucleotides and HA tag were subsequently added by PCR using primers 3. 5'-gattacgccgcggcggcggcggcgagcggagctggcggggctgccggggccgggactgggggagcc gggcccgcgggccgcctgctg-3' and 4. 5'-gcggatcctacccatacgatgtgccagattacgccgcggcggcggcggcgagcgg-3'. The final PCR product was ligated into pCR2.1 TOPO vector and sequenced to produce pCR2.1 QSK clone 1. Nucleotides 610-865 of QSK were amplified from IMAGE consortium EST clone 6247938 (NCBI Acc. BQ685213) using primers 5. 5'-atagcagattttggtttcagtaacctcttcactcctgggcagctgctgaagacctggtgtggcagccctcccta tgctgcacctgaactc-3' and 6. 5'- ctgtggacataaaaaatgggatgcggaactttcc-3', nucleotides 610-674 being added by primer 5. The PCR product was ligated into pCR2.1 TOPO vector and sequenced. A single nucleotide deletion at QSK open reading frame position 696, which is present in the EST clone, was corrected by site-directed mutagenesis to produce pCR2.1 QSK clone 2. QSK PCR products from pCR2.1 QSK clones 1 and 2 were input into an overlap PCR using primers 4. and 6. This product (QSK nucleotides 4-865 with N-terminal HA tag) was ligated into pCR2.1 TOPO vector and sequenced to produce pCR2.1 QSK clone 3.
QSK nucleotides 832-4110 were amplified from IMAGE consortium EST clone 360441 (NCBI Acc. AA015726) using primers 7. 5'-ggaaagttccgcatcccattttttatgtccacag-3' and 8. 5'-gagcggccgcttacacgcctgcctgctccatgc-3'. The PCR product was ligated into pCR2.1 TOPO vector and sequenced to produce pCR2.1 QSK clone 4.
QSK PCR products from pCR2.1 clones 3 and 4 were input into an overlap PCR using primers 4. and 8. to generate full length QSK cDNA with N-terminal HA tag. The PCR product was ligated into pCR2.1 TOPO vector and sequenced to produce pCR2.1 QSK clone 5. Full length QSK with N-terminal HA tag was subclaimed from pCR2.1 QSK clone 5 as a *BamH1-BamH1* insert into pGEX6P-1 and pEBG2T vectors. In order to generate the T-loop mutations in QSK, mutagenesis was performed on pCR2.1 QSK clone 3, followed by overlap PCR to generate the full length mutant forms of QSK. The overlap PCR product was ligated into pCR2.1 TOPO, sequenced, and subcloned into pGEX6P-1 and pEBG2T as a *BamH1-BamH1* insert.
**MARK1.** Nucleotides 4-1078 of the coding region of human MARK1 cDNA (NCBI Acc. NM_018650) with an N-terminal HA tag were amplified from a human brain cDNA library using primers 5'-gcgaattctacccatacgatgtgccagattacgcctcggcccggacgccattgc-3' and 5'-catcatacttctgatttattaaggcatcatttatttc-3'. Nucleotides 1042-2388 of MARK1 were amplified from IMAGE consortium EST clone 48109 (NCBI Acc. H11850) using primers 5'- gaaataaatgatgccttaataaatcagaagtatgatg-3' and 5'-gagtcgacttacagcttaagctcatttgctatttttgatgc-3'. Amplified PCR products were input into an overlap PCR to generate the full length HA-tagged MARK1 cDNA. The PCR product was ligated into pCR2.1 TOPO vector, sequenced, and subcloned as an *EcoR1-Sal1* insert into pGEX6P-1. Full length HA-tagged MARK1 was amplified from the pCR2. 1 TOPO MARK1 clone for subsequent cloning into pEBG2T as a *Kpn1-Not1* insert using primers 5'-gcggtacctacccatacgatgtgccagattacgcctcggcccggacgccattgc-3' and 5'-gagcggccgcttacagcttaagctcatttgctatttttgatgc-3'.
**MARK2**. The coding region of human MARK2 cDNA (NCBI Acc. NM_004954) was amplified from IMAGE consortium EST clone 4591688 (NCBI Acc. BG419875) using sense primers 5'-gcgtcgactacccatacgatgtgccagattacgccattcggggccgcaactcagcc-3' or 5'-gcactagttacccatacgatgtgccagattacgccattcggggccgcaactcagcc-3' for subsequent cloning into vectors pGEX6P-3 and pEBG2T respectively, and antisense primer 5'- gagcggccgcttaaagcttcagctcgttggctattttgg-3'. Amplified PCR products were ligated into pCR2.1 TOPO vector, sequenced, and subcloned as a *Sall-Not1* or *Spel-Notl* insert into pGEX6P-3 or pEBG2T vectors respectively.
**MARK3**. The coding region of human MARK3 cDNA (NCBI Acc. NM_002376) with an N-terminal HA tag was amplified from IMAGE EST 5104460 (NCBI Acc. BI223323) using primers 5'-gcggccgcagccaccatgtacccatacgatgtgccagattacgcctccactaggaccccattgccaacggt ga-3' and 5'-gcggccgcttacagctttagctcattggcaattttggaagc-3'. The resulting PCR product was cloned into pCR2.1-TOPO vector, sequenced, and subcloned as a *Not1-Not1* insert into pEBG2T and pGEX6P-2 vectors.
**MARK4**. In order to obtain the full length coding region of human MARK4 cDNA (NCBI Acc.AK075272) two EST clones were used as templates for PCR reactions. The first 228 amino acids with an N-terminal HA tag was amplified from IMAGE consortium EST clone 6301902 (NCBI Acc.BQ709130 ) using primers 1. 5'-agatctgccaccatgtacccatacgatgtgccagattacgcctcttcgcggacggtgctggccccggg-3' and 2. 5'- tgccctgaaacagctccggggcggc-3'. The remaining coding region was amplified from IMAGE consortium EST clone 5503281 (NCBI Acc. BM467107) with primers 3. 5'-gggatcgaagctggacacgttctgc-3' and 4. 5'-gcggccgctcacactccaggggaatcggagcagccgggg-3. The resulting PCR products were used as templates in an overlap PCR reaction with primers 1. and 4. The PCR product was ligated into pCR2.1-TOPO, sequenced, then subcloned further as a *Bgl2-Not1* insert into the *BamH1-Not1* site of pEBG2T and pGEX6P-1 vectors.

**Other DNA constructs**. The DNA constructs encoding wild type GST-LKB 1 or catalytically-inactive GST-LKB1[D194A] in the pEBG-2T vector (Sapkota et al., 2001), FLAG-STRADα, FLAG-STRADβ, *myc*-MO25α, *myc*-MO25β in the pCMV5 vector (Example 1; Boudeau et al., 2003) or the kinase domain of AMPKα1[residues 1-308] in the pGEX vector (Scott et al., 2002) have been described previously.

**Cell culture, stimulation and cell lysis**. The generation of HeLa cells stably expressing wild type or kinase dead LKB1 has been described previously (Sapkota et al., 2002). The cells were cultured in Minimum Essential Medium Eagle supplemented with 10% (v/v) tetracycline-free-FBS, 1 X non-essential amino acid solution, 1X penicillin/streptomycin solution (Invitrogen), 100 µg/ml zeocin and 5 µg/ml blasticidin (Invitrogen). The production and culture of immortalised LKB1^{+/+} and LKB^{-/-} MEF cells from E9.5 embryos, was described previously (Example 2; Hawley et al., 2003). Human kidney embryonic 293 cells were cultured in Dulbecco's modified Eagle's medium containing 10% FBS. Cells cultured on a 10 cm diameter dish in 10% (v/v) serum were left unstimulated, stimulated with 10 mM phenformin or 2mM AICA riboside for 1h and lysed in 1 ml of ice-cold Lysis Buffer after quick rinsing in PBS. MEF cell lysates were snap frozen in liquid nitrogen, thawed prior to use and centrifuged at 4°C for 30 min at 23000 x g to remove cell debris. HeLa cell lysates were centrifuged immediately at 4°C for 15 min at 13 000 rpm. Protein concentrations were determined by the Bradford method with bovine serum albumin as the standard (Bradford, 1976).

**Immunoblotting**. Total cell lysates (10-50 µg) or immunoprecipitated protein (from 1 mg of cell lysate) were heated in SDS Sample Buffer, and subjected to SDS-PAGE and electrotransfer to nitrocellulose membranes. Membranes were then blocked in 50 mM Tris/HCl pH 7.5, 0.15M NaCl (TBS), 0.5 % (by vol) Tween containing 10 % (by mass) skimmed milk, and probed for 16 h at 4°C in TBS, 0.5 % (by vol) Tween, 5 % (by mass) skimmed milk and 1 µg/ml of the indicated antibodies. Detection of proteins was performed using horse radish peroxidase conjugated secondary antibodies and the enhanced chemiluminescence reagent (Amersham Pharmacia Biotech, Little Chalfont, UK).

**Expression and purification of AMPK-related kinases in *E. coli.*** Unless otherwise indicated, pGEX constructs encoding full length wild type or mutant forms of GST-HA tagged AMPK-related kinases, or GST-AMPKα1[1-308], were transformed into *E. coli* BL21 cells. One-litre cultures were grown at 37°C in Luria broth containing 100 µg/ml ampicilin until the absorbance at 600 nm was 0.8. 100 µM isopropyl-β-D-galactoside was added, and the cells were cultured for a further 16h at 26°C. For pGEX constructs encoding the wild type or mutant GST-HA-tagged QSK, induction of protein expression was carried out by culturing the cells until the absorbance at 600 nm was 1, and then 1 mM isopropyl-β-D-galactoside was added, and the cells cultured further for 1h at 30°C. Pelleted cells were resuspended in 35 ml of ice-cold lysis buffer and lysed in one round of freeze/thawing, and the lysates were sonicated to fragment DNA. The lysates were centrifuged for 30 min at 20,000 x g, and the recombinant proteins were affinity purified on glutathione-Sepharose and eluted in Buffer A containing 20 mM glutathione and 0.27 M sucrose.

**Expression and purification of AMPK-related kinases in 293 cells.** Twenty 10 cm diameter dishes of 293 cells were cultured and each dish transfected with 5 µg of the pEBG-2T construct encoding wild type AMPK-related kinase using a modified calcium phosphate method (Alessi et al., 1996). 36 h post-transfection, the cells were lysed in 1 ml of ice-cold Lysis Buffer, the lysates pooled and centrifuged at 4°C for 10 min at 13,000 x g. The GST-fusion proteins were purified by affinity chromatography on glutathione-Sepharose and eluted in Buffer A containing 20 mM glutathione and 0.27 M sucrose.

**Expression and purification of LKB1:STRAD:MO25 complex in 293 cells.** Different combinations of GST-tagged LKB1, FLAG-tagged STRADα or STRADβ, and Myc-tagged MO25α or MO25β were expressed in 293 cells and the complexes purified on glutathione-Sepharose as described previously (Example 1; Boudeau et al., 2003).

**Measurement of activation of AMPK-related kinase**.s AMPKα1 catalytic subunit and AMPK-related kinases was measured following their phosphorylation with LKB1 as follows. 1-2 µg of AMPKα1 catalytic domain or AMPK-related kinase, were incubated with or without 0.1-1 µg of wild type of the indicated LKB1 complex in Buffer A containing 5 mM MgAcetate and 0.1 mM ATP, in a final volume of 20 µl. After incubation at 30°C for the times indicated in the figure legend, AMPKα1 catalytic domain or AMPK-related kinase activities were determined by adding 30 µl of 5 mM magnesium acetate, 0.1 mM [γ-³²P]-ATP (300 cpm/pmol) and 200 µM *AMARA* peptide (AMARAASAAALARRR(Dale et al., 1995)) as substrate After incubation for 5-20 min at 30°C, incorporation of ³²P-phosphate into the peptide substrate was determined by applying the reaction mixture onto P81 phosphocellulose paper and scintillation counting after washing the papers in phosphoric acid as described previously (Alessi et al., 1995). One Unit (U) of activity was defined as that which catalysed the incorporation of 1 nmol of ³²P into the substrate. Time course reactions for the second stage of the assay were performed in order to ensure that the rate of phosphorylation was occurring linearly with time. Kinetic data was analysed according to the Michaelis-Menten relationship by non-linear regression using the computer program GraphPad Prism (GraphPad Software Inc, San Diego, USA).

**Mapping the sites on BRSK2, NUAK2 and MARK3 phosphorylated by the LKB1 complex.** The wild type and mutant AMPK-related kinases (5 µg) were incubated for 30 min with 1-2 µg of wild type LKB1:STRADα:MO25β in Buffer A containing 5 mM magnesium acetate and 100 µM [γ-³²P]-ATP (5000 cpm/pmol) in a total reaction volume of 30 µl. After 30 min, the reactions were terminated by adding SDS to a final concentration of 1% (w/v) and dithiothreitol to 10 mM and heated at 100 °C for 1 min. After cooling, 4-vinylpyridine was added to a concentration of 50 mM, and the samples were left on a shaking platform for 30 min at room temperature to alkylate cysteine residues and then subjected to electrophoresis on a BisTris 4-12% polyacrylamide gel. The gels were stained with Coomasie R250, autoradiographed and the bands corresponding to phosphorylated AMPK-related kinases excised and cut into smaller pieces. There were washed sequentially for 15 minutes on a vibrating platform with 1 ml of the following: water, a 1:1 mixture of water and acetonitrile, 0.1 M ammonium bicarbonate, a 1:1 mixture of 0.2 M ammonium bicarbonate and acetonitrile and finally acetonitrile. The gel pieces were dried by rotary evaporation and incubated in 0.1 ml of 50 mM ammonium bicarbonate, 0.1% (by mass) n-octyl-glucoside containing 1µg of alkylated trypsin. After 16 h, 0.1 ml of acetonitrile was added and the mixture incubated on a shaking platform for 10 min. The supernatant was removed and the gel pieces were further washed for 10 min in 0.3 ml of 50 mM ammonium bicarbonate, and 0.1% (v/v) trifluoroacetic acid. The combined supernatants, containing >90% of the ³²P-radioactivity, were chromatographed on a Vydac 218TP5215 C₁₈ column (Separations Group, Hesperia, CA) equilibrated in 0.1% (by vol) trifluoroacetic acid in water. The column was developed with a linear acetonitrile gradient (diagonal line) at a flow rate of 0.2 ml/min and fractions of 0.1 ml were collected.

**Phosphopeptide sequence analysis**. Isolated phosphopeptides were analysed on an Applied Biosystems 4700 Proteomics Analyser (MALDI-TOF-TOF) using 5mg/ml alpha cyannocinnamic acid as the matrix. Spectra were acquired in both reflectron and linear modes and the sequence of potential phosphopeptides were confirmed by performing MALDI-MS/MS on selected masses. The characteristic loss of phosphoric acid (M-98 Da) from the parent phosphopeptide as well as the neutral loss of dehydroalanine(-69) for phosphoserine or dehydroaminobutyric acid (-83) for phosphothreonine were used to assign the position of the phosphorylation site(s). The site of phosphorylation of all the ³²P-labelled peptides was determined by solid-phase Edman degradation on an Applied Biosystems 494A sequenator of the peptide coupled to Sequelon-AA membrane (Milligen) as described previously (Campbell and Morrice, 2002).

**Identification of T-loop Phosphorylation Site in MELK**. The tryptic digest of GST-MELK that had been expressed and purified from *E.coli* was acidified with 0.25M acetic acid in 30% acetonitrile (v/v) and 2 µl (settled volume) of PHOS-select Iron chelate gel (Sigma) was added. The sample was shaken for 30 min and the resin collected in a microC18 ZipTip (Millipore), washed with 2 x 25 µl 0.25 M acetic acid in 30% acetonitrile (v/v) and eluted with 25 µl 0.4 M NH₄OH. The eluate was analysed by MALDI-TOF-TOF mass spectrometry and T-loop phosphopeptide was identified and sequenced by ms/ms.

**Immunoprecipitation and assay of endogenous AMPK and AMPK-related kinase**
0.1-1 mg of Hela or MEF lysate protein was incubated at 4°C for 1h on a shaking platform with 5 µg of the corresponding antibody, which had been previously conjugated to 5 µl of protein G-Sepharose. The immunoprecipitates were washed twice with 1 ml of Lysis Buffer containing 0.5 M NaCl, and twice with 1 ml of Buffer A. Phosphotransferase activity towards the *AMARA* peptide was then measured in a total assay volume of 50 µl as described above.

**Immunoprecipitation and assay of endogenous LKB1 employing LKBtide substrate.**
0.1-1 mg Hela or MEF cell lysate protein was incubated at 4°C for 1h on a shaking platform with 5 µl of protein G-Sepharose conjugated to 5 µg of human LKB 1 antibody. The immunoprecipitates were washed twice with 1 ml of Lysis Buffer containing 0.5 M NaCl, and twice with 1 ml of Buffer A.

Phosphotransferase activity towards the LKBtide peptide (LSNLYHQGKFLQTFCGSPLYRRR residues 241-260 of human NUAK2 with 3 additional Arg residues added to the C-terminal to enable binding to P81 paper), was then measured in a total assay volume of 50 µl consisting of 50 mM Tris/HCl pH 7.5, 0.1 mM EGTA, 0.1% (by vol) 2-mercaptoethanol, 10 mM magnesium acetate, 0.1 mM [γ³²P]ATP (~200 cpm/pmol) and 200 µM LKB1tide peptide. The assays were carried out at 30°C with continuous shaking, to keep the immunoprecipitates in suspension, and were terminated after 10 min by applying 40 µl of the reaction mixture onto p81 membranes. The p81 membranes were washed in phosphoric acid, and the incorporated radioactivity was measured by scintillation counting as described previously for MAP kinase (Alessi et al., 1995).

### References for Materials & Methods section

Alessi, D.R., Andjelkovic, M., Caudwell, B., Cron, P., Morrice, N., Cohen, P. and Hemmings, B.A. (1996) Mechanism of activation of protein kinase B by insulin and IGF-1. EMBO J, 15, 6541-6551.
Alessi, D.R., Cohen, P., Ashworth, A., Cowley, S., Leevers, S.J. and Marshall, C.J. (1995) Assay and expression of mitogen-activated protein kinase, MAP kinase kinase, and Raf. Methods Enzymol, 255, 279-290.
Boudeau, J., Baas, A.F., Deak, M., Morrice, N.A., Kieloch, A., Schutkowski, M., Prescott, A.R., Clevers, H.C. and Alessi, D.R. (2003) MO25alpha/beta interact with STRADalpha/beta enhancing their ability to bind, activate and localize LKB 1 in the cytoplasm. EMBO J, 22, 5102-5114.
Bradford, M.M. (1976) A rapid and sensitive method for the quantitation of microgram quantities of protein utilizing the principle of protein-dye binding. Anal Biochem, 72, 248-254.
Campbell, D.G. and Morrice, N.A. (2002) Identification of Protein Phosphorylation Sites by a Combination of Mass Spectrometry and Solid Phase Edman Sequencing. J. Biomol. Techn., 13, 121-132.
Dale, S., Wilson, W.A., Edelman, A.M. and Hardie, D.G. (1995) Similar substrate recognition motifs for mammalian AMP-activated protein kinase, higher plant HMG-CoA reductase kinase-A, yeast SNF1, and mammalian calmodulin-dependent protein kinase I. FEBS Lett, 361, 191-195.
Hawley, S.A., Boudeau, J., Reid, J.L., Mustard, K.J., Udd, L., Makela, T.P., Alessi, D.R. and Hardie, D.G. (2003) Complexes between the LKB1 tumor suppressor, STRADalpha/beta and MO25alpha/beta are upstream kinases in the AMP-activated protein kinase cascade. J Biol, 2, 28.
Sapkota, G.P., Deak, M., Kieloch, A., Morrice, N., Goodarzi, A.A., Smythe, C., Shiloh, Y., Lees-Miller, S.P. and Alessi, D.R. (2002) Ionizing radiation induces ataxia telangiectasia mutated kinase (ATM)-mediated phosphorylation of LKB1/STK11 at Thr-366. Biochem J, 368,507-516.
Sapkota, G.P., Kieloch, A., Lizcano, J.M., Lain, S., Arthur, J.S., Williams, M.R., Morrice, N., Deak, M. and Alessi, D.R. (2001) Phosphorylation of the protein kinase mutated in Peutz-Jeghers Cancer Syndrome, LKB1/STK11, at Ser431 by p90RSK and cAMP-dependent protein kinase, but not its farnesylation at Cys433, is essential for LKB1 to suppress cell growth. J Biol Chem, 276, 19469-19482.
Scott, J.W., Norman, D.G., Hawley, S.A., Kontogiannis, L. and Hardie, D.G. (2002) Protein kinase substrate recognition studied using the recombinant catalytic domain of AMP-activated protein kinase and a model substrate. J Mol Biol, 317, 309-323.
Sugden, C., Crawford, R.M., Halford, N.G. and Hardie, D.G. (1999) Regulation of spinach SNF1-related (SnRK1) kinases by protein kinases and phosphatases is associated with phosphorylation of the T loop and is regulated by 5'-AMP. Plant J, 19, 433-439.

### References

Alessi, D.R. (2001) Discovery of PDK1, one of the missing links in insulin signal transduction. Biochem Soc Trans, 29, 1-14.
Baas, A.F., Boudeau, J., Sapkota, G.P., Smit, L., Medema, R., Morrice, N.A., Alessi, D.R. and Clevers, H.C. (2003) Activation of the tumour suppressor kinase LKB 1 by the STE20-like pseudokinase STRAD. EMBO J, 22, 3062-3072.
Bardeesy, N., Sinha, M., Hezel, A.F., Signoretti, S., Hathaway, N.A., Sharpless, N.E., Loda, M., Carrasco, D.R. and DePinho, R.A. (2002) Loss of the Lkb1 tumour suppressor provokes intestinal polyposis but resistance to transformation. Nature, 419, 162-167.
Boudeau, J., Baas, A.F., Deak, M., Morrice, N.A., Kieloch, A., Schutkowski, M., Prescott, A.R., Clevers, H.C. and Alessi, D.R. (2003a) MO25alpha/beta interact with STRADalpha/beta enhancing their ability to bind, activate and localize LKB1 in the cytoplasm. EMBO J, 22, 5102-5114.
Boudeau, J., Deak, M., Lawlor, M.A., Morrice, N.A. and Alessi, D.R. (2003b) Heat-shock protein 90 and Cdc37 interact with LKB1 and regulate its stability. Biochem J, 370, 849-857.
Buchet-Poyau, K., Mehenni, H., Radhakrishna, U. and Antonarakis, S.E. (2002) Search for the second Peutz-Jeghers syndrome locus: exclusion of the STK13, PRKCG, KLK10, and PSCD2 genes on chromosome 19 and the STK11IP gene on chromosome 2. Cytogenet Genome Res, 97, 171-178*.*
Corton, J.M., Gillespie, J.G., Hawley, S.A. and Hardie, D.G. (1995) 5-aminoimidazole-4-carboxamide ribonucleoside. A specific method for activating AMP-activated protein kinase in intact cells? Eur J Biochem, 229, 558-565.
Dale, S., Wilson, W.A., Edelman, A.M. and Hardie, D.G. (1995) Similar substrate recognition motifs for mammalian AMP-activated protein kinase, higher plant HMG-CoA reductase kinase-A, yeast SNF1, and mammalian calmodulin-dependent protein kinase I. FEBS Lett, 361, 191-195.
Drewes, G., Ebneth, A., Preuss, U., Mandelkow, E.M. and Mandelkow, E. (1997) MARK, a novel family of protein kinases that phosphorylate microtubule-associated proteins and trigger microtubule disruption. Cell, 89, 297-308.
Drewes, G. and Nurse, P. (2003) The protein kinase kin1, the fission yeast orthologue of mammalian MARK/PAR-1, localises to new cell ends after mitosis and is important for bipolar growth. FEBS Lett, 554, 45-49.
Feldman, J.D., Vician, L., Crispino, M., Hoe, W., Baudry, M. and Herschman, H.R. (2000) The salt-inducible kinase, SIK, is induced by depolarization in brain. J Neurochem, 74, 2227-2238.
Guo, S. and Kemphues, K.J. (1995) par-1, a gene required for establishing polarity in C. elegans embryos, encodes a putative Ser/Thr kinase that is asymmetrically distributed. Cell, 81, 611-620.
Hardie, D.G., Scott, J.W., Pan, D.A. and Hudson, E.R. (2003) Management of cellular energy by the AMP-activated protein kinase system. FEBS Lett, 546, 113-120.
Hawley, S.A., Boudeau, J., Reid, J.L., Mustard, K.J., Udd, L., Makela, T.P., Alessi, D.R. and Hardie, D.G. (2003) Complexes between the LKB1 tumor suppressor, STRADalpha/beta and MO25alpha/beta are upstream kinases in the AMP-activated protein kinase cascade. J Biol, 2, 28.
Hawley, S.A., Gadalla, A.E., Olsen, G.S. and Hardie, D.G. (2002) The antidiabetic drug metformin activates the AMP-activated protein kinase cascade via an adenine nucleotide-independent mechanism. Diabetes, 51,2420-2425.
Hemminki, A., Markie, D., Tomlinson, I., Avizienyte, E., Roth, S., Loukola, A., Bignell, G., Warren, W., Aminoff, M., Hoglund, P., Jarvinen, H., Kristo, P., Pelin, K., Ridanpaa, M., Salovaara, R., Toro, T., Bodmer, W., Olschwang, S., Olsen, A.S., Stratton, M.R., de la Chapelle, A. and Aaltonen, L.A. (1998) A serine/threonine kinase gene defective in Peutz-Jeghers syndrome. Nature, 391, 184-187.
Heyer, B.S., Kochanowski, H. and Solter, D. (1999) Expression of Melk, a new protein kinase, during early mouse development. Dev Dyn, 215, 344-351.
Heyer, B.S., Warsowe, J., Solter, D., Knowles, B.B. and Ackerman, S.L. (1997) New member of the Snf1/AMPK kinase family, Melk, is expressed in the mouse egg and preimplantation embryo. Mol Reprod Dev, 47, 148-156.
Hong, S.P., Leiper, F.C., Woods, A., Carling, D. and Carlson, M. (2003) Activation of yeast Snf1 and mammalian AMP-activated protein kinase by upstream kinases. Proc Natl Acad Sci U S A, 100, 8839-8843.
Horike, N., Takemori, H., Katoh, Y., Doi, J., Min, L., Asano, T., Sun, X.J., Yamamoto, H., Kasayama, S., Muraoka, M., Nonaka, Y. and Okamoto, M. (2003) Adipose-specific expression, phosphorylation of Ser794 in insulin receptor substrate-1, and activation in diabetic animals of salt-inducible kinase-2. J Biol Chem, 278, 18440-18447.
Jakobsen, S.N., Hardie, D.G., Morrice, N. and Tornqvist, H.E. (2001) 5'-AMP-activated protein kinase phosphorylates IRS-1 on Ser-789 in mouse C2C12 myotubes in response to 5-aminoimidazole-4-carboxamide riboside. J Biol Chem, 276, 46912-46916.
Jenne, D.E., Reimann, H., Nezu, J., Friedel, W., Loff, S., Jeschke, R., Muller, O., Back, W. and Zimmer, M. (1998) Peutz-Jeghers syndrome is caused by mutations in a novel serine threonine kinase. Nat Genet, 18,38-43.
Jishage, K.I., Nezu, J.I., Kawase, Y., Iwata, T., Watanabe, M., Miyoshi, A., Ose, A., Habu, K., Kake, T., Kamada, N., Ueda, O., Kinoshita, M., Jenne, D.E., Shimane, M. and Suzuki, H. (2002) Role of Lkb1, the causative gene of Peutz-Jegher's syndrome, in embryogenesis and polyposis. Proc Natl Acad Sci USA, 99, 8903-8908.
Johnson, L.N., Noble, M.E. and Owen, D.J. (1996) Active and inactive protein kinases: structural basis for regulation. Cell, 85, 149-158.
Kemp, B.E., Mitchelhill, K.I., Stapleton, D., Michell, B.J., Chen, Z.P. and Witters, L.A. (1999) Dealing with energy demand: the AMP-activated protein kinase. Trends Biochem Sci, 24, 22-25.
Kemphues, K.J., Priess, J.R., Morton, D.G. and Cheng, N.S. (1988) Identification of genes required for cytoplasmic localization in early C. elegans embryos. Cell, 52, 311-320.
Lefebvre, D.L., Bai, Y., Shahmolky, N., Sharma, M., Poon, R., Drucker, D.J. and Rosen, C.F. (2001) Identification and characterization of a novel sucrose-non-fermenting protein kinase/AMP-activated protein kinase-related protein kinase, SNARK. Biochem J, 355, 297-305.
Manning, G., Whyte, D.B., Martinez, R., Hunter, T. and Sudarsanam, S. (2002) The protein kinase complement of the human genome. Science, 298, 1912-1934.
Martin, S.G. and St Johnston, D. (2003) A role for Drosophila LKB1 in anterior-posterior axis formation and epithelial polarity. Nature, 421, 379-384.
Milburn, C.C., Boudeau, J., Deak, M., Alessi, D.R. and van aalten, D.M.F. (2003) Crystal structure of MO25a in complex with the C-terminus of the pseudokinase STE20-Related ADaptor (STRAD). Nature Struct. Biol*.,* **In the press**.
Miyoshi, H., Nakau, M., Ishikawa, T.O., Seldin, M.F., Oshima, M. and Taketo, M.M. (2002) Gastrointestinal hamartomatous polyposis in Lkb1 heterozygous knockout mice. Cancer Res, 62, 2261-2266.
Morton, D.G., Roos, J.M. and Kemphues, K.J. (1992) par-4, a gene required for cytoplasmic localization and determination of specific cell types in Caenorhabditis elegans embryogenesis. Genetics, 130, 771-790.
Nath, N., McCartney, R.R. and Schmidt, M.C. (2003) Yeast Pak1 kinase associates with and activates Snf1. Mol Cell Biol, 23, 3909-3917.
Ossipova, O., Bardeesy, N., DePinho, R.A. and Green, J.B. (2003) LKB 1 (XEEK1) regulates Wnt signalling in vertebrate development. Nat Cell Biol, 5, 889-894.
Peng, C.Y., Graves, P.R., Ogg, S., Thoma, R.S., Byrnes, M.J., 3rd, Wu, Z., Stephenson, M.T. and Piwnica-Worms, H. (1998) C-TAK1 protein kinase phosphorylates human Cdc25C on serine 216 and promotes 14-3-3 protein binding. Cell Growth Differ, 9, 197-208.
Resta, N., Stella, A., Susca, F.C., Di Giacomo, M., Forleo, G., Miccolis, I., Rossini, F.P., Genuardi, M., Piepoli, A., Grammatico, P. and Guanti, G. (2002) Two novel mutations and a new STK11/LKB1 gene isoform in Peutz-Jeghers patients. Hum Mutat, 20, 78-79.
Rossi, D.J., Ylikorkala, A., Korsisaari, N., Salovaara, R., Luukko, K., Launonen, V., Henkemeyer, M., Ristimaki, A., Aaltonen, L.A. and Makela, T.P. (2002) Induction of cyclooxygenase-2 in a mouse model of Peutz-Jeghers polyposis. Proc Natl Acad Sci U S A, 99, 12327-12332.
Sapkota, G.P., Deak, M., Kieloch, A., Morrice, N., Goodarzi, A.A., Smythe, C., Shiloh, Y., Lees-Miller, S.P. and Alessi, D.R. (2002) Ionizing radiation induces ataxia telangiectasia mutated kinase (ATM)-mediated phosphorylation of LKB1/STK11 at Thr-366. Biochem J, 368, 507-516.
Shulman, J.M., Benton, R. and St Johnston, D. (2000) The Drosophila homolog of C. elegans PAR-1 organizes the oocyte cytoskeleton and directs oskar mRNA localization to the posterior pole. Cell, 101, 377-388.
Spicer, J., Rayter, S., Young, N., Elliott, R., Ashworth, A. and Smith, D. (2003) Regulation of the Wnt signalling component PAR1A by the Peutz-Jeghers syndrome kinase LKB1. Oncogene, 22, 4752-4756.
Sutherland, C.M., Hawley, S.A., McCartney, R.R., Leech, A., Stark, M.J., Schmidt, M.C. and Hardie, D.G. (2003) Elmlp is one of three upstream kinases for the Saccharomyces cerevisiae SNF1 complex. Curr Biol, 13, 1299-1305.
Suzuki, A., Kusakai, G., Kishimoto, A., Lu, J., Ogura, T. and Esumi, H. (2003a) ARK5 suppresses the cell death induced by nutrient starvation and death receptors via inhibition of caspase 8 activation, but not by chemotherapeutic agents or UV irradiation. Oncogene, 22, 6177-6182.
Suzuki, A., Kusakai, G., Kishimoto, A., Lu, J., Ogura, T., Lavin, M.F. and Esumi, H. (2003b) Identification of a novel protein kinase mediating Akt survival signaling to the ATM protein. J Biol Chem, 278, 48-53.
Takemori, H., Doi, J., Horike, N., Katoh, Y., Min, L., Lin, X.Z., Wang, Z.N., Muraoka, M. and Okamoto, M. (2003) Salt-inducible kinase-mediated regulation of steroidogenesis at the early stage of ACTH-stimulation. J Steroid Biochem Mol Biol, 85, 397-400.
Tiainen, M., Vaahtomeri, K., Ylikorkala, A. and Makela, T.P. (2002) Growth arrest by the LKB 1 tumor suppressor: induction of p21 (WAF1/CIP1). Hum Mol Genet,11, 1497-1504.
Tiainen, M., Ylikorkala, A. and Makela, T.P. (1999) Growth suppression by Lkb1 is mediated by a G(1) cell cycle arrest. Proc Natl Acad Sci US A, 96, 9248-9251.
Timm, T., Li, X.Y., Biernat, J., Jiao, J., Mandelkow, E., Vandekerckhove, J. and Mandelkow, E.M. (2003) MARKK, a Ste20-like kinase, activates the polarity-inducing kinase MARKlPAR-1. Embo J, 22, 5090-5101.
Tomancak, P., Piano, F., Riechmann, V., Gunsalus, K.C., Kemphues, K.J. and Ephrussi, A. (2000) A Drosophila melanogaster homologue of Caenorhabditis elegans par-1 acts at an early step in embryonic-axis formation. Nat Cell Biol, 2, 458-460.
Wang, Z., Takemori, H., Halder, S.K., Nonaka, Y. and Okamoto, M. (1999) Cloning of a novel kinase (SIK) of the SNF1/AMPK family from high salt diet-treated rat adrenal. FEBS Lett, 453, 135-139.
Watts, J.L., Morton, D.G., Bestman, J. and Kemphues, K.J. (2000) The C. elegans par-4 gene encodes a putative serine-threonine kinase required for establishing embryonic asymmetry. Development, 127, 1467-1475.
Woods, A., Johnstone, S.R., Dickerson, K., Leiper, F.C., Fryer, L.G., Neumann, D., Schlattner, U., Wallimann, T., Carlson, M. and Carling, D. (2003a) LKB1 Is the Upstream Kinase in the AMP-Activated Protein Kinase Cascade. Curr Biol, 13, 2004-2008.
Woods, A., Vertommen, D., Neumann, D., Turk, R., Bayliss, J., Schlattner, U., Wallimann, T., Carling, D. and Rider, M.H. (2003b) Identification of phosphorylation sites in AMP-activated protein kinase (AMPK) for upstream AMPK kinases and study of their roles by site-directed mutagenesis. J Biol Chem, 278, 28434-28442.
Zhou, G., Myers, R., Li, Y., Chen, Y., Shen, X., Fenyk-Melody, J., Wu, M., Ventre, J., Doebber, T., Fujii, N., Musi, N., Hirshman, M.F., Goodyear, L.J. and Moller, D.E. (2001) Role of AMP-activated protein kinase in mechanism of metformin action. J Clin Invest, 108, 1167-1174.

## Claims

1. A method for identifying a compound for use in modulating, for example promoting, the activation or phosphorylation of AMPK (AMP-activated protein kinase) or AMPK subfamily member in a cell, the method comprising the steps of (1) determining whether a test compound modulates, for example promotes, the protein kinase activity of LKB1 and (2) selecting a compound which modulates, for example promotes, the protein kinase activity of LKB 1, wherein the LKB1 is in a preparation with STRAD and MO25 wherein the M025 is recombinant and is expressed from a recombinant nucleic acid.

2. The method of claim 1 wherein the LKB 1 or STRAD is recombinant and is expressed from a recombinant nucleic acid.

3. A cell capable of expressing LKB1, STRAD and overexpressed or recombinant MO25 expressed from a recombinant nucleic acid.

4. The cell of claim 3 comprising a recombinant nucleic acid encoding M025.

5. The cell of claim 3 or 4 comprising a recombinant nucleic acid encoding LKB1.

6. The cell of any one of claims 3 to 5 comprising a recombinant nucleic acid encoding STRAD.

7. A cell comprising LKB1, STRAD and overexpressed or recombinant MO25 expressed from a recombinant nucleic acid.

8. A cell according to claim 7 comprising recombinant LKB 1 expressed from a recombinant nucleic acid.

9. A cell according to claim 7 or 8 comprising recombinant STRAD expressed from a recombinant nucleic acid.

10. A cell according to any one of claims 7 to 9 wherein the cell is a cell according to any one of claims 3 to 6.

11. A method for making a preparation comprising LKB1, STRAD and recombinant M025 expressed from a recombinant nucleic acid comprising the step of purifying the preparation from a cell according to any one of claims 7 to 10.

12. The method of claim 11 wherein the preparation comprises recombinant LKB 1 expressed from a recombinant nucleic acid.

13. The method of claim 11 or 12 wherein the preparation comprises recombinant STRAD expressed from a recombinant nucleic acid.

14. The method of any one of claims 11 to 13 wherein the LKB 1:STRAD:MO25 ratios in the preparation are 1:1:1.

15. The method of claim 1 or 2 wherein the LKB 1 is in a preparation obtained by or obtainable by the method of any one of claims 11 to 14 or in a cell as defined in any one of claims 3 to 10.

16. The method of any one of claims 1 to 2, 11 to 15 wherein the preparation comprises a complex comprising the LKB 1, STRAD and MO25.

17. A method for identifying a compound for modulating cellular LKB 1 activity, the method comprising the steps of (1) determining whether a test compound modulates the LKB 1 protein kinase activity of a preparation or complex obtained by the method of any one of claims 11 to 14 or 16 or in a cell as defined in any one of claims 3 to 10 and (2) selecting a compound which modulates the said LKB 1 protein kinase activity.

18. The method of claim 1 or claim 17 wherein the LKB1 protein kinase activity is measured using AMPK or AMPK subfamily member or a fragment thereof as the substrate.

19. A kit of parts comprising LKB 1 or a recombinant polynucleotide encoding LKB1, STRAD or a recombinant polynucleotide encoding STRAD, and a recombinant polynucleotide encoding M025.

20. A kit of parts comprising (1) AMPK or AMPK subfamily member, or recombinant polynucleotide encoding AMPK or AMPK subfamily member or a fragment thereof and (2) a kit of parts as defined in claim 19 or a cell as defined in any one of claims 3 to 10.

21. A method for preparing an LKB1/STRAD/MO25 complex comprising the steps of (1) selecting a cell type in which to overexpress LKB1, comprising the step of determining whether the cell type is one that expresses STRAD and M025 (2) preparing an LKB1/STRAD/MO25 complex by overexpressing LKB 1 in the selected cell type.

22. A method for preparing an LKB1/STRAD/MO25 complex comprising the steps of (1) overexpressing LKB1 in a cell using a method according to claim 21 and (2) preparing said complex from the cell.

23. A method for identifying a genetic difference associated with PJS (Peutz-Jeghers Syndrome) comprising the steps of (1) investigating the sequence of a gene encoding a M025 isoform in at least one patient having PJS (2) identifying any difference between the said patient sequence and equivalent sequence from an individual without PJS.

24. A method for identifying a compound which activates AMPK or AMPK subfamily member by a similar mechanism to metformin or phenformin or AICA riboside in which the effect of a test compound on the activation of AMPK or AMPK subfamily member by a preparation or complex obtained by the method of any one of claims 11 to 14 or 16 or a cell as defined in any one of claims 3 to 10 is compared with the effect of metformin or phenformin or AICA riboside on the activation of AMPK or AMPK subfamily member and a compound with a similar effect is selected.

25. The method of any one of claims 1, 18 or 24 or kit of parts of claim 20, wherein the AMPK subfamily member is or comprises an AMPKα1 or AMPKα2 polypeptide.

26. The method of any one of claims 1, 18 or 24 or kit of parts of claim 20 wherein the AMPK subfamily member is or comprises a NUAK1, NUAK2, BRSK1, BRSK2, SIK, QIK, QSK, MARK1, MARK2, MARK3, MARK4 or MELK polypeptide.

## Patentansprüche

1. Verfahren zum Identifizieren einer Verbindung zur Verwendung beim Ändern, beispielsweise Fördern der Aktivierung oder der Phosphorylierung von AMPK (AMP-aktivierte Proteinkinase) oder einem Mitglied der AMPK-Unterfamilie in einer Zelle, wobei das Verfahren die Schritte umfasst (1) Ermitteln, ob eine Testverbindung die Proteinkinase-Aktivität von LKB1 verändert, beispielsweise fördert, und (2) Auswählen einer Verbindung, welche die Proteinkinase-Aktivität von LKB1 verändert, beispielsweise fördert, wobei sich die LKB1 in einer Zubereitung mit STRAD und MO25 befindet, wobei das MO25 rekombinant ist und von einer rekombinanten Nukleinsäure exprimiert wird.

2. Verfahren nach Anspruch 1, wobei die LKB1 oder die STRAD rekombinant ist und von einer rekombinanten Nukleinsäure exprimiert wird.

3. Zelle, die in der Lage ist, LKB1, STRAD und überexprimiertes oder rekombinantes MO25 zu exprimieren, die von einer rekombinanten Nukleinsäure exprimiert sind.

4. Zelle nach Anspruch 3, die eine rekombinante Nukleinsäure umfasst, welche MO25 kodiert.

5. Zelle nach Anspruch 3 oder 4, die eine rekombinante Nukleinsäure umfasst, die LKB1 kodiert.

6. Zelle nach einem der Ansprüche 3 bis 5, die eine rekombinante Nukleinsäure umfasst, die STRAD kodiert.

7. Eine Zelle, die LKB1, STRAD und überexprimiertes oder rekombinantes MO25 umfasst, die von einer rekombinanten Nukleinsäure exprimiert sind.

8. Zelle nach Anspruch 7, die rekombinante LKB1 umfasst, die von einer rekombinanten Nukleinsäure exprimiert ist.

9. Zelle nach Anspruch 7 oder 8, die rekombinante STRAD umfasst, die von einer rekombinanten Nukleinsäure exprimiert ist.

10. Zelle nach einem der Ansprüche 7 bis 9, wobei die Zelle eine Zelle nach einem der Ansprüche 3 bis 6 ist.

11. Verfahren zur Herstellung einer Zubereitung, die LKB1, STRAD und rekombinantes MO25 umfasst, exprimiert von einer rekombinanten Nukleinsäure, wobei das Verfahren den Schritt der Reinigung der Zubereitung aus einer Zelle nach einem der Ansprüche 7 bis 10 umfasst.

12. Verfahren nach Anspruch 11, wobei die Zubereitung rekombinante LKB1 umfasst, exprimiert von einer rekombinanten Nukleinsäure.

13. Verfahren nach Anspruch 11 oder 12, wobei die Zubereitung rekombinantes STRAD umfasst, exprimiert von einer rekombinanten Nukleinsäure.

14. Verfahren nach einem der Ansprüche 11 bis 13, wobei die Verhältnisse von LKB1:STRAD:MO25 in der Zubereitung 1:1:1 sind.

15. Verfahren nach Anspruch 1 oder 2, wobei die LKB1 in einer Zubereitung vorhanden ist, die durch das Verfahren nach einem der Ansprüche 11 bis 14 erhalten wird oder erhalten werden kann, oder in einer Zelle nach einem der Ansprüche 3 bis 10.

16. Verfahren nach einem der Ansprüche 1, 2 oder 11 bis 15, wobei die Zubereitung einen Komplex umfasst, der LKB1, STRAD und MO25 umfasst.

17. Verfahren zum Identifizieren einer Verbindung zur Veränderung der zellulären LKB1-Aktivität, wobei das Verfahren die Schritte umfasst (1) Ermitteln, ob eine Testverbindung die LKB1-Proteinkinase-Aktivität einer Zubereitung oder eines Komplexes verändert, die bzw. der durch das Verfahren nach einem der Ansprüche 1 bis 14 oder 16 erhalten wurde, oder in einer Zelle nach einem der Ansprüche 3 bis 10 und (2) Auswählen einer Verbindung, welche die besagte LKB1-Proteinkinase-Aktivität verändert.

18. Verfahren nach Anspruch 1 oder 17, wobei die LKB1-Proteinkinase-Aktivität unter Verwendung von AMPK oder eines Mitglieds der AMPK-Unterfamilie oder eines Fragmentes hiervon als Substrat gemessen wird.

19. Teilesatz, der LKB1 oder ein rekombinantes Polynukleotid umfasst, das LKB1, STRAD kodiert, oder ein rekombinantes Polynukleotid, das STRAD kodiert und ein rekombinantes Polynukleotid, das MO25 kodiert.

20. Teilesatz, der Folgendes umfasst (1) AMPK oder ein Mitglied der AMPK-Unterfamilie oder ein rekombinantes Polynukleotid, das AMPK oder ein Mitglied der AMPK-Unterfamilie oder ein Fragment hiervon kodiert, und (2) einen Teilesatz nach Anspruch 19 oder eine Zelle nach einem der Ansprüche 3 bis 10.

21. Verfahren zur Zubereitung eines LKB1/STRAD/MO25-Komplexes, das folgende Schritte umfasst (1) Auswählen eines Zelltyps in dem LKB1 überexprimiert werden soll, einschließlich der Schritte der Ermittelung, ob der Zelltyp ein Zelltyp ist, der STRAD und M025 exprimiert, (2) Zubereiten eines LKB1/STRAD/MO25-Komplexes durch Überexprimieren von LKB1 in dem ausgewählten Zellentyp.

22. Verfahren zum Zubereiten eines LKB1/STRAD/MO25-Komplexes, das die Schritte umfasst (1) Überexprimieren von LKB1 in einer Zelle unter Verwendung eines Verfahrens nach Anspruch 21 und (2) Zubereiten des Komplexes aus der Zelle.

23. Verfahren zur Identifizierung eines genetischen Unterschiedes, der mit PJS (Peutz-Jeghers-Syndrom) verbunden ist, das folgende Schritte umfasst (1) Aufspüren der Sequenz eines Gens, das eine MO25-lsoform kodiert bei wenigstens einem Patienten, der PJS aufweist, (2) Identifizieren irgend eines Unterschieds zwischen der Sequenz dieses Patienten und einer äquivalenten Sequenz von einer Person ohne PJS.

24. Verfahren zum Identifizieren einer Verbindung, die AMPK oder ein Mitglied der AMPK-Unterfamilie durch einen Mechanismus aktiviert, der zu Metformin oder Phenformin oder AICA-Ribosid ähnlich ist, bei dem der Einfluss einer Testverbindung auf die Aktivierung von AMPK oder einem Mitglied der AMPK-Unterfamilie durch eine Zubereitung oder einen Komplex, die bzw. der durch das Verfahren nach einem der Ansprüche 11 bis 14 oder 16 oder aus einer Zelle gewonnen wurde, wie in einem der Ansprüche 3 bis 10 definiert, mit dem Einfluss von Metformin oder Phenformin oder AICA-Ribosid auf die Aktivierung von AMPK oder einem Mitglied der AMPK-Unterfamilie verglichen wird und bei dem eine Verbindung mit einem ähnlichen Effekt ausgewählt wird.

25. Verfahren nach einem der Ansprüche 1, 18 oder 24 oder Teilesatz nach Anspruch 20, bei dem das Mitglied der AMPK-Unterfamilie ein AMPKα1- oder AMPKα2-Polypeptid ist oder umfasst.

26. Verfahren nach einem der Ansprüche 1, 18 oder 24 oder Teilesatz nach Anspruch 20, bei dem das Mitglied der AMPK-Unterfamilie ein NUAK1-, NUAK2-, BRSK1-, BRSK2-, SIK-, QIK-, QSK-, MARK1-, MARK2-, MARK3-, MARK4 oder ein MELK-Polypeptid ist oder umfasst.

## Revendications

1. Procédé d'identification d'un composé destiné à une utilisation de modulation, par exemple de promotion, de l'activation ou de la phosphorylation de l'AMPK (protéine kinase activée par l'AMP) ou d'un membre de la sous-famille de l'AMPK dans une cellule, le procédé comprenant les étapes consistant à (1) déterminer si un composé à tester module, par exemple promeut, l'activité protéine kinase de la LKB1 et (2) sélectionner un composé qui module, par exemple promeut, l'activité protéine kinase de la LKB1, dans lequel la LKB1 est dans une préparation avec la STRAD et la M025, la M025 étant recombinante et étant exprimée à partir d'un acide nucléique recombinant.

2. Procédé selon la revendication 1 dans lequel la LKB1 ou la STRAD est recombinante et est exprimée à partir d'un acide nucléique recombinant.

3. Cellule capable d'exprimer la LKB1, la STRAD et la M025 surexprimée ou recombinante exprimée à partir d'un acide nucléique recombinant.

4. Cellule selon la revendication 3 qui comprend un acide nucléique recombinant codant la M025.

5. Cellule selon la revendication 3 ou 4 qui comprend un acide nucléique recombinant codant la LKB1.

6. Cellule selon l'une quelconque des revendications 3 à 5 qui comprend un acide nucléique recombinant codant la STRAD.

7. Cellule qui comprend la LKB1, la STRAD et la M025 surexprimée ou recombinante exprimée à partir d'un acide nucléique recombinant.

8. Cellule selon la revendication 7 qui comprend la LKB1 recombinante exprimée à partir d'un acide nucléique recombinant.

9. Cellule selon la revendication 7 ou 8 qui comprend la STRAD recombinante exprimée à partir d'un acide nucléique recombinant.

10. Cellule selon l'une quelconque des revendications 7 à 9 dans laquelle la cellule est une cellule selon l'une quelconque des revendications 3 à 6.

11. Procédé de fabrication d'une préparation comprenant la LKB1, la STRAD et la M025 recombinante exprimée à partir d'un acide nucléique recombinant, qui comprend l'étape de purification de la préparation à partir d'une cellule selon l'une quelconque des revendications 7 à 10.

12. Procédé selon la revendication 11 dans lequel la préparation comprend la LKB1 recombinante exprimée à partir d'un acide nucléique recombinant.

13. Procédé selon la revendication 11 ou 12 dans lequel la préparation comprend la STRAD recombinante exprimée à partir d'un acide nucléique recombinant.

14. Procédé selon l'une quelconque des revendications 11 à 13 dans lequel les rapports LKB1 : STRAD : M025 dans la préparation sont 1 : 1 : 1.

15. Procédé selon la revendication 1 ou 2 dans lequel la LKB1 est dans une préparation obtenue par ou susceptible d'être obtenue par le procédé selon l'une quelconque des revendications 11 à 14 ou dans une cellule selon l'une quelconque des revendications 3 à 10.

16. Procédé selon l'une quelconque des revendications 1 ou 2, 11 à 15 dans lequel la préparation comprend un complexe comprenant la LKB1, la STRAD et la M025.

17. Procédé d'identification d'un composé destiné à moduler l'activité cellulaire de la LKB1, le procédé comprenant les étapes consistant à (1) déterminer si un composé à tester module l'activité protéine kinase de la LKB1 d'une préparation ou d'un complexe obtenu par le procédé selon l'une quelconque des revendications 11 à 14 ou 16 ou dans une cellule selon l'une quelconque des revendications 3 à 10 et (2) sélectionner un composé qui module ladite activité protéine kinase de la LKB1.

18. Procédé selon la revendication 1 ou 17 dans lequel l'activité protéine kinase de la LKB1 est mesurée en utilisant l'AMPK ou un membre de la sous-famille de l'AMPK ou un fragment de ceux-ci comme substrat.

19. Kit d'éléments qui comprend la LKB1 ou un polynucléotide recombinant codant la LKB1, la STRAD ou un polynucléotide recombinant codant la STRAD, et un polynucléotide recombinant codant la M025.

20. Kit d'éléments qui comprend (1) l'AMPK ou un membre de la sous-famille de l'AMPK, ou un polynucléotide recombinant codant l'AMPK ou un membre de la sous-famille de l'AMPK ou un fragment de ceux-ci et (2) un kit d'éléments tel que défini dans la revendication 19 ou une cellule telle que définie dans l'une quelconque des revendications 3 à 10.

21. Procédé de préparation d'un complexe LKB1/STRAD/MO25 comprenant les étapes consistant à (1) sélectionner un type de cellules dans lequel on surexprimera la LKB1, qui comprend l'étape consistant à déterminer si le type de cellules est un de ceux qui expriment la STRAD et la M025 et (2) préparer un complexe LKB1/STRAD/M025 en surexprimant la LKB1 dans le type de cellules choisi.

22. Procédé de préparation d'un complexe LKB1/STRAD/MO25 comprenant les étapes consistant à (1) surexprimer la LKB1 dans une cellule en utilisant un procédé selon la revendication 21 et (2) préparer ledit complexe à partir de la cellule.

23. Procédé d'identification d'une différence génétique associée au PJS (Syndrome de Peutz-Jeghers) qui comprend les étapes consistant à rechercher (1) la séquence d'un gène codant pour un isoforme de la M025 dans au moins un patient ayant le PJS et (2) identifier toute différence entre la séquence dudit patient et la séquence équivalente d'un individu sans PJS.

24. Procédé d'identification d'un composé qui active l'AMPK ou un membre de la sous-famille de l'AMPK par un mécanisme similaire à la metformine ou à la phenformine ou au AICA riboside dans lequel l'effet d'un composé à tester sur l'activation de l'AMPK ou d'un membre de la sous-famille de l'AMPK par une préparation ou un complexe obtenu par le procédé selon l'une quelconque des revendications 11 à 14 ou 16 ou une cellule telle que définie dans l'une quelconque des revendications 3 à 10 est comparé avec l'effet de la metformine ou de la phenformine ou du AICA riboside sur l'activation de l'AMPK ou d'un membre de la sous-famille de l'AMPK et un composé avec un effet similaire est choisi.

25. Procédé selon l'une quelconque des revendications 1, 18 ou 24 ou kit d'éléments selon la revendication 20, dans lequel le membre de la sous-famille de l'AMPK est ou comprend un polypeptide AMPKα1 ou AMPKα2.

26. Procédé selon l'une quelconque des revendications 1, 18 ou 24 ou kit d'éléments selon la revendication 20, dans lequel le membre de la sous-famille de l'AMPK est ou comprend un polypeptide NUAK1, NUAK2, BRSK1, BRSK2, SIK, QIK, QSK, MARK1, MARK2, MARK3, MARK4 ou MELK.


### SEQUENCE LISTING

<110> University of Dundee
<120> Methods
<130> DUNY/P30950PC
<160> 158
<170> PatentIn version 3.1
<210> 1
   <211> 550
   <212> PRT
   <213> Homo sapiens
<400> 1
   Met Ala Thr Ala Glu Lys Gln Lys His Asp Gly Arg Val Lys Ile Gly
   1               5                   10                  15
   His Tyr Ile Leu Gly Asp Thr Leu Gly Val Gly Thr Phe Gly Lys Val
               20                  25                  30
   Lys Val Gly Lys His Glu Leu Thr Gly His Lys Val Ala Val Lys Ile
           35                  40                  45
   Leu Asn Arg Gln Lys Ile Arg Ser Leu Asp Val Val Gly Lys Ile Arg
       50                  55                  60
   Arg Glu Ile Gln Asn Leu Lys Leu Phe Arg His Pro His Ile Ile Lys
   65                  70                  75                  80
   Leu Tyr Gln Val Ile Ser Thr Pro Ser Asp Ile Phe Met Val Met Glu
                   85                  90                  95
   Tyr Val Ser Gly Gly Glu Leu Phe Asp Tyr Ile Cys Lys Asn Gly Arg
               100                 105                 110
   Leu Asp Glu Lys Glu Ser Arg Arg Leu Phe Gln Gln Ile Leu Ser Gly
           115                 120                 125
   Val Asp Tyr Cys His Arg His Met Val Val His Arg Asp Leu Lys Pro
       130                 135                 140
   Glu Asn Val Leu Leu Asp Ala His Met Asn Ala Lys Ile Ala Asp Phe
   145                 150                 155                 160
   Gly Leu Ser Asn Met Met Ser Asp Gly Glu Phe Leu Arg Thr Ser Cys
                   165                 170                 175
   Gly Ser Pro Asn Tyr Ala Ala Pro Glu Val Ile Ser Gly Arg Leu Tyr
               180                 185                 190
   Ala Gly Pro Glu Val Asp Ile Trp Ser Ser Gly Val Ile Leu Tyr Ala
   195 200 205
   Leu Leu Cys Gly Thr Leu Pro Phe Asp Asp Asp His Val Pro Thr Leu
   210 215 220
   Phe Lys Lys Ile Cys Asp Gly Ile Phe Tyr Thr Pro Gln Tyr Leu Asn
   225 230 235 240
   Pro Ser Val Ile Ser Leu Leu Lys His Met Leu Gln Val Asp Pro Met
   245 250 255
   Lys Arg Ala Thr Ile Lys Asp Ile Arg Glu His Glu Trp Phe Lys Gln
   260 265 270
   Asp Leu Pro Lys Tyr Leu Phe Pro Glu Asp Pro Ser Tyr Ser Ser Thr
   275 280 285
   Met Ile Asp Asp Glu Ala Leu Lys Glu Val Cys Glu Lys Phe Glu Cys
   290 295 300
   Ser Glu Glu Glu Val Leu Ser Cys Leu Tyr Asn Arg Asn His Gln Asp
   305 310 315 320
   Pro Leu Ala Val Ala Tyr His Leu Ile Ile Asp Asn Arg Arg Ile Met
   325 330 335
   Asn Glu Ala Lys Asp Phe Tyr Leu Ala Thr Ser Pro Pro Asp Ser Phe
   340 345 350
   Leu Asp Asp His His Leu Thr Arg Pro His Pro Glu Arg Val Pro Phe
   355 360 365
   Leu Val Ala Glu Thr Pro Arg Ala Arg His Thr Leu Asp Glu Leu Asn
   370 375 380
   Pro Gln Lys Ser Lys His Gln Gly Val Arg Lys Ala Lys Trp His Leu
   385 390 395 400
   Gly Ile Arg Ser Gln Ser Arg Pro Asn Asp Ile Met Ala Glu Val Cys
   405 410 415
   Arg Ala Ile Lys Gln Leu Asp Tyr Glu Trp Lys Val Val Asn Pro Tyr
   420 425 430
   Tyr Leu Arg Val Arg Arg Lys Asn Pro Val Thr Ser Thr Tyr Ser Lys
   435 440 445
   Met Ser Leu Gln Leu Tyr Gln Val Asp Ser Arg Thr Tyr Leu Leu Asp
   450 455 460
   Phe Arg Ser Ile Asp Asp Glu Ile Thr Glu Ala Lys Ser Gly Thr Ala
   465 470 475 480
   Thr Pro Gln Arg Ser Gly Ser Val Ser Asn Tyr Arg Ser Cys Gln Arg
   485 490 495
   Ser Asp Ser Asp Ala Glu Ala Gln Gly Lys Ser Ser Glu Val Ser Leu
   500 505 510
   Thr Ser Ser Val Thr Ser Leu Asp Ser Ser Pro Val Asp Leu Thr Pro
   515 520 525
   Arg Pro Gly Ser His Thr Ile Glu Phe Phe Glu Met Cys Ala Asn Leu
   530 535 540
   Ile Lys Ile Leu Ala Gln
   545 550
<210> 2
   <211> 550
   <212> PRT
   <213> Homo sapiens
<400> 2
   Met Ala Thr Ala Glu Lys Gln Lys His Asp Gly Arg Val Lys Ile Gly
   1 5 10 15
   His Tyr Ile Leu Gly Asp Thr Leu Gly Val Gly Thr Phe Gly Lys Val
   20 25 30
   Lys Val Gly Lys His Glu Leu Thr Gly His Lys Val Ala Val Lys Ile
   35 40 45
   Leu Asn Arg Gln Lys Ile Arg Ser Leu Asp Val Val Gly Lys Ile Arg
   50 55 60
   Arg Glu Ile Gln Asn Leu Lys Leu Phe Arg His Pro His Ile Ile Lys
   65 70 75 80
   Leu Tyr Gln Val Ile Ser Thr Pro Ser Asp Ile Phe Met Val Met Glu
   85 90 95
   Tyr Val Ser Gly Gly Glu Leu Phe Asp Tyr Ile Cys Lys Asn Gly Arg
   100 105 110
   Leu Asp Glu Lys Glu Ser Arg Arg Leu Phe Gln Gln Ile Leu Ser Gly
   115 120 125
   Val Asp Tyr Cys His Arg His Met Val Val His Arg Asp Leu Lys Pro
   130 135 140
   Glu Asn Val Leu Leu Asp Ala His Met Asn Ala Lys Ile Ala Asp Phe
   145 150 155 160
   Gly Leu Ser Asn Met Met Ser Asp Gly Glu Phe Leu Arg Thr Ser Cys
                   165                 170                 175
   Gly Ser Pro Asn Tyr Ala Ala Pro Glu Val Ile Ser Gly Arg Leu Tyr
               180                 185                 190
   Ala Gly Pro Glu Val Asp Ile Trp Ser Ser Gly Val Ile Leu Tyr Ala
           195                 200                 205
   Leu Leu Cys Gly Thr Leu Pro Phe Asp Asp Asp His Val Pro Thr Leu
       210                 215                 220
   Phe Lys Lys Ile Cys Asp Gly Ile Phe Tyr Thr Pro Gln Tyr Leu Asn
   225                 230                 235                 240
   Pro Ser Val Ile Ser Leu Leu Lys His Met Leu Gln Val Asp Pro Met
                   245                 250                 255
   Lys Arg Ala Ser Ile Lys Asp Ile Arg Glu His Glu Trp Phe Lys Gln
               260                 265                 270
   Asp Leu Pro Lys Tyr Leu Phe Pro Glu Asp Pro Ser Tyr Ser Ser Thr
           275                 280                 285
   Met Ile Asp Asp Glu Ala Leu Lys Glu Val Cys Glu Lys Phe Glu Cys
       290                 295                 300
   Ser Glu Glu Glu Val Leu Ser Cys Leu Tyr Asn Arg Asn His Gln Asp
   305                 310                 315                 320
   Pro Leu Ala Val Ala Tyr His Leu Ile Ile Asp Asn Arg Arg Ile Met
                   325                 330                 335
   Asn Glu Ala Lys Asp Phe Tyr Leu Ala Thr Ser Pro Pro Asp Ser Phe
               340                 345                 350
   Leu Asp Asp His His Leu Thr Arg Pro His Pro Glu Arg Val Pro Phe
           355                 360                 365
   Leu Val Ala Glu Thr Pro Arg Ala Arg His Thr Leu Asp Glu Leu Asn
       370                 375                 380
   Pro Gln Lys Ser Lys His Gln Gly Val Arg Lys Ala Lys Trp His Leu
   385                 390                 395                 400
   Gly Ile Arg Ser Gln Ser Arg Pro Asn Asp Ile Met Ala Glu Val Cys
                   405                 410                 415
   Arg Ala Ile Lys Gln Leu Asp Tyr Glu Trp Lys Val Val Asn Pro Tyr
               420                 425                 430
   Tyr Leu Arg Val Arg Arg Lys Asn Pro Val Thr Ser Thr Tyr Ser Lys
   435 440 445
   Met Ser Leu Gln Leu Tyr Gln Val Asp Ser Arg Thr Tyr Leu Leu Asp
   450 455 460
   Phe Arg Ser Ile Asp Asp Glu Ile Thr Glu Ala Lys Ser Gly Thr Ala
   465 470 475 480
   Thr Pro Gln Arg Ser Gly Ser Val Ser Asn Tyr Arg Ser Cys Gln Arg
   485 490 495
   Ser Asp Ser Asp Ala Glu Ala Gln Gly Lys Ser Ser Glu Val Ser Leu
   500 505 510
   Thr Ser Ser Val Thr Ser Leu Asp Ser Ser Pro Val Asp Leu Thr Pro
   515 520 525
   Arg Pro Gly Ser His Thr Ile Glu Phe Phe Glu Met Cys Ala Asn Leu
   530 535 540
   Ile Lys Ile Leu Ala Gln
   545 550
<210> 3
   <211> 550
   <212> PRT
   <213> Homo sapiens
<400> 3
   Met Ala Thr Ala Glu Lys Gln Lys His Asp Gly Arg Val Lys Ile Gly
   1               5                   10                  15
   His Tyr Ile Leu Gly Asp Thr Leu Gly Val Gly Thr Phe Gly Lys Val
               20                  25                  30
   Lys Val Gly Lys His Glu Leu Thr Gly His Lys Val Ala Val Lys Ile
           35                  40                  45
   Leu Asn Arg Gln Lys Ile Arg Ser Leu Asp Val Val Gly Lys Ile Arg
       50                  55                  60
   Arg Glu Ile Gln Asn Leu Lys Leu Phe Arg His Pro His Ile Ile Lys
   65                  70                  75                  80
   Leu Tyr Gln Val Ile Ser Thr Pro Ser Asp Ile Phe Met Val Met Glu
                   85                  90                  95
   Tyr Val Ser Gly Gly Glu Leu Phe Asp Tyr Ile Cys Lys Asn Gly Arg
               100                 105                 110
   Leu Asp Glu Lys Glu Ser Arg Arg Leu Phe Gln Gln Ile Leu Ser Gly
           115                 120                 125
   Val Asp Tyr Cys His Arg His Met Val Val His Arg Asp Leu Lys Pro
       130                 135                 140
   Glu Asn Val Leu Leu Asp Ala His Met Asn Ala Lys Ile Ala Asp Phe
   145                 150                 155                 160
   Gly Leu Ser Asn Met Met Ser Asp Gly Glu Phe Leu Arg Thr Ser Cys
                   165                 170                 175
   Gly Ser Pro Asn Tyr Ala Ala Pro Glu Val Ile Ser Gly Arg Leu Tyr
               180                 185                 190
   Ala Gly Pro Glu Val Asp Ile Trp Ser Ser Gly Val Ile Leu Tyr Ala
           195                 200                 205
   Leu Leu Cys Gly Thr Leu Pro Phe Asp Asp Asp His Val Pro Thr Leu
       210                 215                 220
   Phe Lys Lys Ile Cys Asp Gly Ile Phe Tyr Thr Pro Gln Tyr Leu Asn
   225                 230                 235                 240
   Pro Ser Val Ile Ser Leu Leu Lys His Met Leu Gln Val Asp Pro Met
                   245                 250                 255
   Lys Arg Ala Ser Ile Lys Asp Ile Arg Glu His Glu Trp Phe Lys Gln
               260                 265                 270
   Asp Leu Pro Lys Tyr Leu Phe Pro Glu Asp Pro Ser Tyr Ser Ser Thr
           275                 280                 285
   Met Ile Asp Asp Glu Ala Leu Lys Glu Val Cys Glu Lys Phe Glu Cys
       290                 295                 300
   Ser Glu Glu Glu Val Leu Ser Cys Leu Tyr Asn Arg Asn His Gln Asp
   305                 310                 315                 320
   Pro Leu Ala Val Ala Tyr His Leu Ile Ile Asp Asn Arg Arg Ile Met
                   325                 330                 335
   Asn Glu Ala Lys Asp Phe Tyr Leu Ala Thr Ser Pro Pro Asp Ser Phe
               340                 345                 350
   Leu Asp Asp His His Leu Thr Arg Pro His Pro Glu Arg Val Pro Phe
           355                 360                 365
   Leu Val Ala Glu Thr Pro Arg Ala Arg His Thr Leu Asp Glu Leu Asn
       370                 375                 380
   Pro Gln Lys Ser Lys His Gln Gly Val Arg Lys Ala Lys Trp His Leu
   385 390 395 400
   Gly Ile Arg Ser Gln Ser Arg Pro Asn Asp Ile Met Ala Glu Val Cys
   405 410 415
   Arg Ala Ile Lys Gln Leu Asp Tyr Glu Trp Lys Val Val Asn Pro Tyr
   420 425 430
   Tyr Leu Arg Val Arg Arg Lys Asn Pro Val Thr Ser Thr Tyr Ser Lys
   435 440 445
   Met Ser Leu Gln Leu Tyr Gln Val Asp Ser Arg Thr Tyr Leu Leu Asp
   450 455 460
   Phe Arg Ser Ile Asp Asp Glu Ile Thr Glu Ala Lys Ser Gly Thr Ala
   465 470 475 480
   Thr Pro Gln Arg Ser Gly Ser Val Ser Asn Tyr Arg Ser Cys Gln Arg
   485 490 495
   Ser Asp Ser Asp Ala Glu Ala Gln Gly Lys Ser Ser Glu Val Ser Leu
   500 505 510
   Thr Ser Ser Val Thr Ser Leu Asp Ser Ser Pro Val Asp Leu Thr Pro
   515 520 525
   Arg Pro Gly Ser His Thr Ile Glu Phe Phe Glu Met Cys Ala Asn Leu
   530 535 540
   Ile Lys Ile Leu Ala Gln
   545 550
<210> 4
   <211> 520
   <212> PRT
   <213> Homo sapiens
<400> 4
   Gly Glu His Gln Leu Thr Gly His Lys Val Ala Val Lys Ile Leu Asn
   1 5 10 15
   Arg Gln Lys Ile Arg Ser Leu Asp Val Val Gly Lys Ile Lys Arg Glu
   20 25 30
   Ile Gln Asn Leu Lys Leu Phe Arg His Pro His Ile Ile Lys Leu Tyr
   35 40 45
   Gln Val Ile Ser Thr Pro Thr Asp Phe Phe Met Val Met Glu Tyr Val
   50 55 60
   Ser Gly Gly Glu Leu Phe Asp Tyr Ile Cys Lys His Gly Arg Val Glu
   65 70 75 80
   Glu Met Glu Ala Arg Arg Leu Phe Gln Gln Ile Leu Ser Ala Val Asp
   85 90 95
   Tyr Cys His Arg His Met Val Val His Arg Asp Leu Lys Pro Glu Asn
   100 105 110
   Val Leu Leu Asp Ala His Met Asn Ala Lys Ile Ala Asp Phe Gly Leu
   115 120 125
   Ser Asn Met Met Ser Asp Gly Glu Phe Leu Arg Thr Ser Cys Gly Ser
   130 135 140
   Pro Asn Tyr Ala Ala Pro Glu Val Ile Ser Gly Arg Leu Tyr Ala Gly
   145 150 155 160
   Pro Glu Val Asp Ile Trp Ser Cys Gly Val Ile Leu Tyr Ala Leu Leu
   165 170 175
   Cys Gly Thr Leu Pro Phe Asp Asp Glu His Val Pro Thr Leu Phe Lys
   180 185 190
   Lys Ile Arg Gly Gly Val Phe Tyr Ile Pro Glu Tyr Leu Asn Arg Ser
   195 200 205
   Val Ala Thr Leu Leu Met His Met Leu Gln Val Asp Pro Leu Lys Arg
   210 215 220
   Ala Thr Ile Lys Asp Ile Arg Glu His Glu Trp Phe Lys Gln Asp Leu
   225 230 235 240
   Pro Ser Tyr Leu Phe Pro Glu Asp Pro Ser Tyr Asp Ala Asn Val Ile
   245 250 255
   Asp Asp Glu Ala Val Lys Glu Val Cys Glu Lys Phe Glu Cys Thr Glu
   260 265 270
   Ser Glu Val Met Asn Ser Leu Tyr Ser Gly Asp Pro Gln Asp Gln Leu
   275 280 285
   Ala Val Ala Tyr His Leu Ile Ile Asp Asn Arg Arg Ile Met Asn Gln
   290 295 300
   Ala Ser Glu Phe Tyr Leu Ala Ser Ser Pro Pro Ser Gly Ser Phe Met
   305 310 315 320
   Asp Asp Ser Ala Met His Ile Pro Pro Gly Leu Lys Pro His Pro Glu
   325 330 335
   Arg Met Pro Pro Leu Ile Ala Asp Ser Pro Lys Ala Arg Cys Pro Leu
               340                 345                 350
   Asp Ala Leu Asn Thr Thr Lys Pro Lys Ser Leu Ala Val Lys Lys Ala
           355                 360                 365
   Lys Trp His Leu Gly Ile Arg Ser Gln Ser Lys Pro Tyr Asp Ile Met
       370                 375                 380
   Ala Glu Val Tyr Arg Ala Met Lys Gln Leu Asp Phe Glu Trp Lys Val
   385                 390                 395                 400
   Val Asn Ala Tyr His Leu Arg Val Arg Arg Lys Asn Pro Val Thr Gly
                   405                 410                 415
   Asn Tyr Val Lys Met Ser Leu Gln Leu Tyr Leu Val Asp Asn Arg Ser
               420                 425                 430
   Tyr Leu Leu Asp Phe Lys Ser Ile Asp Asp Glu Val Val Glu Gln Arg
           435                 440                 445
   Ser Gly Ser Ser Thr Pro Gln Arg Ser Cys Ser Ala Ala Gly Leu His
       450                 455                 460
   Arg Pro Arg Ser Ser Phe Asp Ser Thr Thr Ala Glu Ser His Ser Leu
   465                 470                 475                 480
   Ser Gly Ser Leu Thr Gly Ser Leu Thr Gly Ser Thr Leu Ser Ser Val
                   485                 490                 495
   Ser Pro Arg Leu Gly Ser His Thr Met Asp Phe Phe Glu Met Cys Ala
               500                 505                 510
   Ser Leu Ile Thr Thr Leu Ala Arg
           515                 520
<210> 5
   <211> 552
   <212> PRT
   <213> Homo sapiens
<400> 5
   Met Ala Glu Lys Gln Lys His Asp Gly Arg Val Lys Ile Gly His Tyr
   1               5                   10                  15
   Val Leu Gly Asp Thr Leu Gly Val Gly Thr Phe Gly Lys Val Lys Ile
               20                  25                  30
   Gly Glu His Gln Leu Thr Gly His Lys Val Ala Val Lys Ile Leu Asn
           35                  40                  45
   Arg Gln Lys Ile Arg Ser Leu Asp Val Val Gly Lys Ile Lys Arg Glu
   50 55 60
   Ile Gln Asn Leu Lys Leu Phe Arg His Pro His Ile Ile Lys Leu Tyr
   65 70 75 80
   Gln Val Ile Ser Thr Pro Thr Asp Phe Phe Met Val Met Glu Tyr Val
   85 90 95
   Ser Gly Gly Glu Leu Phe Asp Tyr Ile Cys Lys His Gly Arg Val Glu
   100 105 110
   Glu Met Glu Ala Arg Arg Leu Phe Gln Gln Ile Leu Ser Ala Val Asp
   115 120 125
   Tyr Cys His Arg His Met Val Val His Arg Asp Leu Lys Pro Glu Asn
   130 135 140
   Val Leu Leu Asp Ala His Met Asn Ala Lys Ile Ala Asp Phe Gly Leu
   145 150 155 160
   Ser Asn Met Met Ser Asp Gly Glu Phe Leu Arg Thr Ser Cys Gly Ser
   165 170 175
   Pro Asn Tyr Ala Ala Pro Glu Val Ile Ser Gly Arg Leu Tyr Ala Gly
   180 185 190
   Pro Glu Val Asp Ile Trp Ser Cys Gly Val Ile Leu Tyr Ala Leu Leu
   195 200 205
   Cys Gly Thr Leu Pro Phe Asp Asp Glu His Val Pro Thr Leu Phe Lys
   210 215 220
   Lys Ile Arg Gly Gly Val Phe Tyr Ile Pro Glu Tyr Leu Asn Arg Ser
   225 230 235 240
   Val Ala Thr Leu Leu Met His Met Leu Gln Val Asp Pro Leu Lys Arg
   245 250 255
   Ala Thr Ile Lys Asp Ile Arg Glu His Glu Trp Phe Lys Gln Asp Leu
   260 265 270
   Pro Ser Tyr Leu Phe Pro Glu Asp Pro Ser Tyr Asp Ala Asn Val Ile
   275 280 285
   Asp Asp Glu Ala Val Lys Glu Val Cys Glu Lys Phe Glu Cys Thr Glu
   290 295 300
   Ser Glu Val Met Asn Ser Leu Tyr Ser Gly Asp Pro Gln Asp Gln Leu
   305 310 315 320
   Ala Val Ala Tyr His Leu Ile Ile Asp Asn Arg Arg Ile Met Asn Gln
   325 330 335
   Ala Ser Glu Phe Tyr Leu Ala Ser Ser Pro Pro Ser Gly Ser Phe Met
   340 345 350
   Asp Asp Ser Ala Met His Ile Pro Pro Gly Leu Lys Pro His Pro Glu
   355 360 365
   Arg Met Pro Pro Leu Ile Ala Asp Ser Pro Lys Ala Arg Cys Pro Leu
   370 375 380
   Asp Ala Leu Asn Thr Thr Lys Pro Lys Ser Leu Ala Val Lys Lys Ala
   385 390 395 400
   Lys Trp His Leu Gly Ile Arg Ser Gln Ser Lys Pro Tyr Asp Ile Met
   405 410 415
   Ala Glu Val Tyr Arg Ala Met Lys Gln Leu Asp Phe Glu Trp Lys Val
   420 425 430
   Val Asn Ala Tyr His Leu Arg Val Arg Arg Lys Asn Pro Val Thr Gly
   435 440 445
   Asn Tyr Val Lys Met Ser Leu Gln Leu Tyr Leu Val Asp Asn Arg Ser
   450 455 460
   Tyr Leu Leu Asp Phe Lys Ser Ile Asp Asp Glu Val Val Glu Gln Arg
   465 470 475 480
   Ser Gly Ser Ser Thr Pro Gln Arg Ser Cys Ser Ala Ala Gly Leu His
   485 490 495
   Arg Pro Arg Ser Ser Phe Asp Ser Thr Thr Ala Glu Ser His Ser Leu
   500 505 510
   Ser Gly Ser Leu Thr Gly Ser Leu Thr Gly Ser Thr Leu Ser Ser Val
   515 520 525
   Ser Pro Arg Leu Gly Ser His Thr Met Asp Phe Phe Glu Met Cys Ala
   530 535 540
   Ser Leu Ile Thr Thr Leu Ala Arg
   545 550
<210> 6
   <211> 433
   <212> PRT
   <213> Homo sapiens
<400> 6
<210> 7
   <211> 433
   <212> PRT
   <213> Homo sapiens
<400> 7
<210> 8
   <211> 433
   <212> PRT
   <213> Homo sapiens
<400> 8
<210> 9
   <211> 431
   <212> PRT
   <213> Homo sapiens
<400> 9
<210> 10
   <211> 418
   <212> PRT
   <213> Homo sapiens
<400> 10
   Met Ser Leu Leu Asp Cys Phe Cys Thr Ser Arg Thr Gln Val Glu Ser
   1               5                   10                  15
   Leu Arg Pro Glu Lys Gln Ser Glu Thr Ser Ile His Gln Tyr Leu Val
               20                  25                  30
   Asp Glu Pro Thr Leu Ser Trp Ser Arg Pro Ser Thr Arg Ala Ser Glu
   35 40 45
   Val Leu Cys Ser Thr Asn Val Ser His Tyr Glu Leu Gln Val Glu Ile
   50 55 60
   Gly Arg Gly Phe Asp Asn Leu Thr Ser Val His Leu Ala Arg His Thr
   65 70 75 80
   Pro Thr Gly Thr Leu Val Thr Ile Lys Ile Thr Asn Leu Glu Asn Cys
   85 90 95
   Asn Glu Glu Arg Leu Lys Ala Leu Gln Lys Ala Val Ile Leu Ser His
   100 105 110
   Phe Phe Arg His Pro Asn Ile Thr Thr Tyr Trp Thr Val Phe Thr Val
   115 120 125
   Gly Ser Trp Leu Trp Val Ile Ser Pro Phe Met Ala Tyr Gly Ser Ala
   130 135 140
   Ser Gln Leu Leu Arg Thr Tyr Phe Pro Glu Gly Met Ser Glu Thr Leu
   145 150 155 160
   Ile Arg Asn Ile Leu Phe Gly Ala Val Arg Gly Leu Asn Tyr Leu His
   165 170 175
   Gln Asn Gly Cys Ile His Arg Ser Ile Lys Ala Ser His Ile Leu Ile
   180 185 190
   Ser Gly Asp Gly Leu Val Thr Leu Ser Gly Leu Ser His Leu His Ser
   195 200 205
   Leu Val Lys His Gly Gln Arg His Arg Ala Val Tyr Asp Phe Pro Gln
   210 215 220
   Phe Ser Thr Ser Val Gln Pro Trp Leu Ser Pro Glu Leu Leu Arg Gln
   225 230 235 240
   Asp Leu His Gly Tyr Asn Val Lys Ser Asp Ile Tyr Ser Val Gly Ile
   245 250 255
   Thr Ala Cys Glu Leu Ala Ser Gly Gln Val Pro Phe Gln Asp Met His
   260 265 270
   Arg Thr Gln Met Leu Leu Gln Lys Leu Lys Gly Pro Pro Tyr Ser Pro
   275 280 285
   Leu Asp Ile Ser Ile Phe Pro Gln Ser Glu Ser Arg Met Lys Asn Ser
   290 295 300
   Gln Ser Gly Val Asp Ser Gly Ile Gly Glu Ser Val Leu Val Ser Ser
   305 310 315 320
   Gly Thr His Thr Val Asn Ser Asp Arg Leu His Thr Pro Ser Ser Lys
   325 330 335
   Thr Phe Ser Pro Ala Phe Phe Ser Leu Val Gln Leu Cys Leu Gln Gln
   340 345 350
   Asp Pro Glu Lys Arg Pro Ser Ala Ser Ser Leu Leu Ser His Val Phe
   355 360 365
   Phe Lys Gln Met Lys Glu Glu Ser Gln Asp Ser Ile Leu Ser Leu Leu
   370 375 380
   Pro Pro Ala Tyr Asn Lys Pro Ser Ile Ser Leu Pro Pro Val Leu Pro
   385 390 395 400
   Trp Thr Glu Pro Glu Cys Asp Phe Pro Asp Glu Lys Asp Ser Tyr Trp
   405 410 415
   Glu Phe
<210> 11
   <211> 341
   <212> PRT
   <213> Homo sapiens
<400> 11
   Met Pro Phe Pro Phe Gly Lys Ser His Lys Ser Pro Ala Asp Ile Val
   1 5 10 15
   Lys Asn Leu Lys Glu Ser Met Ala Val Leu Glu Lys Gln Asp Ile Ser
   20 25 30
   Asp Lys Lys Ala Glu Lys Ala Thr Glu Glu Val Ser Lys Asn Leu Val
   35 40 45
   Ala Met Lys Glu Ile Leu Tyr Gly Thr Asn Glu Lys Glu Pro Gln Thr
   50 55 60
   Glu Ala Val Ala Gln Leu Ala Gln Glu Leu Tyr Asn Ser Gly Leu Leu
   65 70 75 80
   Ser Thr Leu Val Ala Asp Leu Gln Leu Ile Asp Phe Glu Gly Lys Lys
   85 90 95
   Asp Val Ala Gln Ile Phe Asn Asn Ile Leu Arg Arg Gln Ile Gly Thr
   100 105 110
   Arg Thr Pro Thr Val Glu Tyr Ile Cys Thr Gln Gln Asn Ile Leu Phe
   115 120 125
   Met Leu Leu Lys Gly Tyr Glu Ser Pro Glu Ile Ala Leu Asn Cys Gly
   130 135 140
   Ile Met Leu Arg Glu Cys Ile Arg His Glu Pro Leu Ala Lys Ile Ile
   145 150 155 160
   Leu Trp Ser Glu Gln Phe Tyr Asp Phe Phe Arg Tyr Val Glu Met Ser
   165 170 175
   Thr Phe Asp Ile Ala Ser Asp Ala Phe Ala Thr Phe Lys Asp Leu Leu
   180 185 190
   Thr Arg His Lys Leu Leu Ser Ala Glu Phe Leu Glu Gln His Tyr Asp
   195 200 205
   Arg Phe Phe Ser Glu Tyr Glu Lys Leu Leu His Ser Glu Asn Tyr Val
   210 215 220
   Thr Lys Arg Gln Ser Leu Lys Leu Leu Gly Glu Leu Leu Leu Asp Arg
   225 230 235 240
   His Asn Phe Thr Ile Met Thr Lys Tyr Ile Ser Lys Pro Glu Asn Leu
   245 250 255
   Lys Leu Met Met Asn Leu Leu Arg Asp Lys Ser Arg Asn Ile Gln Phe
   260 265 270
   Glu Ala Phe His Val Phe Lys Val Phe Val Ala Asn Pro Asn Lys Thr
   275 280 285
   Gln Pro Ile Leu Asp Ile Leu Leu Lys Asn Gln Ala Lys Leu Ile Glu
   290 295 300
   Phe Leu Ser Lys Phe Gln Asn Asp Arg Thr Glu Asp Glu Gln Phe Asn
   305 310 315 320
   Asp Glu Lys Thr Tyr Leu Val Lys Gln Ile Arg Asp Leu Lys Arg Pro
   325 330 335
   Ala Gln Gln Glu Ala
   340
<210> 12
   <211> 337
   <212> PRT
   <213> Homo sapiens
<400> 12
   Met Lys Lys Met Pro Leu Phe Ser Lys Ser His Lys Asn Pro Ala Glu
   1               5                   10                  15
   Ile Val Lys Ile Leu Lys Asp Asn Leu Ala Ile Leu Glu Lys Gln Asp
               20                  25                  30
   Lys Lys Thr Asp Lys Ala Ser Glu Glu Val Ser Lys Ser Leu Gln Ala
           35                  40                  45
   Met Lys Glu Ile Leu Cys Gly Thr Asn Glu Lys Glu Pro Pro Thr Glu
       50                  55                  60
   Ala Val Ala Gln Leu Ala Gln Glu Leu Tyr Ser Ser Gly Leu Leu Val
   65                  70                  75                  80
   Thr Leu Ile Ala Asp Leu Gln Leu Ile Asp Phe Glu Gly Lys Lys Asp
                   85                  90                  95
   Val Thr Gln Ile Phe Asn Asn Ile Leu Arg Arg Gln Ile Gly Thr Arg
               100                 105                 110
   Ser Pro Thr Val Glu Tyr Ile Ser Ala His Pro His Ile Leu Phe Met
           115                 120                 125
   Leu Leu Lys Gly Tyr Glu Ala Pro Gln Ile Ala Leu Arg Cys Gly Ile
       130                 135                 140
   Met Leu Arg Glu Cys Ile Arg His Glu Pro Leu Ala Lys Ile Ile Leu
   145                 150                 155                 160
   Phe Ser Asn Gln Phe Arg Asp Phe Phe Lys Tyr Val Glu Leu Ser Thr
                   165                 170                 175
   Phe Asp Ile Ala Ser Asp Ala Phe Ala Thr Phe Lys Asp Leu Leu Thr
               180                 185                 190
   Arg His Lys Val Leu Val Ala Asp Phe Leu Glu Gln Asn Tyr Asp Thr
           195                 200                 205
   Ile Phe Glu Asp Tyr Glu Lys Leu Leu Gln Ser Glu Asn Tyr Val Thr
       210                 215                 220
   Lys Arg Gln Ser Leu Lys Leu Leu Gly Glu Leu Ile Leu Asp Arg His
   225                 230                 235                 240
   Asn Phe Ala Ile Met Thr Lys Tyr Ile Ser Lys Pro Glu Asn Leu Lys
                   245                 250                 255
   Leu Met Met Asn Leu Leu Arg Asp Lys Ser Pro Asn Ile Gln Phe Glu
               260                 265                 270
   Ala Phe His Val Phe Lys Val Phe Val Ala Ser Pro His Lys Thr Gln
   275 280 285
   Pro Ile Val Glu Ile Leu Leu Lys Asn Gln Pro Lys Leu Ile Glu Phe
   290 295 300
   Leu Ser Ser Phe Gln Lys Glu Arg Thr Asp Asp Glu Gln Phe Ala Asp
   305 310 315 320
   Glu Lys Asn Tyr Leu Ile Lys Gln Ile Arg Asp Leu Lys Lys Thr Ala
   325 330 335
   Pro
<210> 13
   <211> 338
   <212> PRT
   <213> Caenorhabditis elegans
<400> 13
   Met Leu Lys Pro Leu Phe Gly Lys Ala Asp Lys Thr Pro Ala Asp Val
   1               5                   10                  15
   Val Lys Asn Leu Arg Asp Ala Leu Leu Val Ile Asp Arg His Gly Thr
               20                  25                  30
   Asn Thr Ser Glu Arg Lys Val Glu Lys Ala Ile Glu Glu Thr Ala Lys
           35                  40                  45
   Met Leu Ala Leu Ala Lys Thr Phe Ile Tyr Gly Ser Asp Ala Asn Glu
       50                  55                  60
   Pro Asn Asn Glu Gln Val Thr Gln Leu Ala Gln Glu Val Tyr Asn Ala
   65                  70                  75                  80
   Asn Val Leu Pro Met Leu Ile Lys His Leu His Lys Phe Glu Phe Glu
                   85                  90                  95
   Cys Lys Lys Asp Val Ala Ser Val Phe Asn Asn Leu Leu Arg Arg Gln
               100                 105                 110
   Ile Gly Thr Arg Ser Pro Thr Val Glu Tyr Leu Ala Ala Arg Pro Glu
           115                 120                 125
   Ile Leu Ile Thr Leu Leu Leu Gly Tyr Glu Gln Pro Asp Ile Ala Leu
       130                 135                 140
   Thr Cys Gly Ser Met Leu Arg Glu Ala Val Arg His Glu His Leu Ala
   145                 150                 155                 160
   Arg Ile Val Leu Tyr Ser Glu Tyr Phe Gln Arg Phe Phe Val Phe Val
   165 170 175
   Gln Ser Asp Val Phe Asp Ile Ala Thr Asp Ala Phe Ser Thr Phe Lys
   180 185 190
   Asp Leu Met Thr Lys His Lys Asn Met Cys Ala Glu Tyr Leu Asp Asn
   195 200 205
   Asn Tyr Asp Arg Phe Phe Gly Gln Tyr Ser Ala Leu Thr Asn Ser Glu
   210 215 220
   Asn Tyr Val Thr Arg Arg Gln Ser Leu Lys Leu Leu Gly Glu Leu Leu
   225 230 235 240
   Leu Asp Arg His Asn Phe Ser Thr Met Asn Lys Tyr Ile Thr Ser Pro
   245 250 255
   Glu Asn Leu Lys Thr Val Met Glu Leu Leu Arg Asp Lys Arg Arg Asn
   260 265 270
   Ile Gln Tyr Glu Ala Phe His Val Phe Lys Ile Phe Val Ala Asn Pro
   275 280 285
   Asn Lys Pro Arg Pro Ile Thr Asp Ile Leu Thr Arg Asn Arg Asp Lys
   290 295 300
   Leu Val Glu Phe Leu Thr Ala Phe His Asn Asp Arg Thr Asn Asp Glu
   305 310 315 320
   Gln Phe Asn Asp Glu Lys Ala Tyr Leu Ile Lys Gln Ile Gln Glu Leu
   325 330 335
   Arg Val
<210> 14
   <211> 636
   <212> PRT
   <213> Caenorhabditis elegans
<400> 14
   Met Asp Ser Thr Thr Ser Leu Pro Asn Asn Val Leu Leu Lys Lys Ala
   1               5                   10                  15
   Arg Pro Ser Lys Ile Phe Ala Val Thr Ser Ala Asn Ala Leu Asn Val
               20                  25                  30
   Lys Thr Glu Pro Val Ile Phe Val Lys Ser Asp Asp Leu Asn Gln Ala
           35                  40                  45
   Asn Thr Pro Leu Thr Gly Ser Lys Phe Gly Thr His Leu Ala Cys Ile
       50                  55                  60
   Arg Thr Ser Cys Leu His Arg Thr Val Asn Ala Ser Asn Tyr Ser Thr
   65                  70                  75                  80
   Met Ser Asp Gly Gly Leu Tyr Thr Ser Asp Glu Pro Cys Ser Ser Ala
                   85                  90                  95
   Gln Ala Glu Phe Arg Leu Ala Ala His Trp Glu Ser Thr Phe Thr Arg
               100                 105                 110
   Thr Arg Glu Ile His Cys Asp Thr Gly Tyr Ser Ser Gln Ser Pro Pro
           115                 120                 125
   Glu Thr Thr Val Phe Ile Gln Lys Ser Arg Phe Pro Val Ala Glu Lys
       130                 135                 140
   Pro Gly Thr Pro Glu Leu Lys Ser Phe Glu Ser Lys Lys Leu Val Gln
   145                 150                 155                 160
   Lys Lys Ser Gly Asn Ala Ser Thr Pro Thr Arg Lys Leu Ala Ser Glu
                   165                 170                 175
   Glu Lys Lys Ala Lys Asn Thr Ser Met Gly Gln Thr Pro Ser Lys Leu
               180                 185                 190
   Lys Ser Pro Lys Ala Leu Lys Met Val Lys Lys Glu Asn Glu Pro Ala
           195                 200                 205
   Ile Pro Pro Asn His Phe Glu Gly Lys Val Tyr Gly Tyr Leu Val Asp
       210                 215                 220
   Asp Met Ser Ala Ile Gly Ile Gln Pro Ile Leu Asp Lys Tyr Asn Glu
   225                 230                 235                 240
   Asp Pro Glu Lys Phe Phe Lys Arg Phe Asp Ser Lys Pro Trp Phe Arg
                   245                 250                 255
   Arg Lys Val Met Pro Leu Leu Phe Gly Lys Ser His Lys Ser Pro Ala
               260                 265                 270
   Asp Val Val Lys Thr Leu Arg Glu Val Leu Thr Ile Leu Asp Lys Leu
           275                 280                 285
   Pro Pro Pro Lys Leu Asp Lys Asp Gly Asn Ile Gln Ser Asp Lys Lys
       290                 295                 300
   Tyr Asp Lys Ala Leu Asp Glu Val Ser Lys Asn Val Ala Met Ile Lys
   305                 310                 315                 320
   Ser Phe Ile Tyr Gly Asn Asp Ser Ala Glu Pro Ser Ser Glu His Val
                   325                 330                 335
   Val Gln Val Ala Gln Leu Ala Gln Glu Val Tyr Asn Ala Asn Ile Leu
   340 345 350
   Pro Met Leu Ile Lys Met Leu Pro Lys Phe Glu Phe Glu Cys Lys Lys
   355 360 365
   Asp Val Gly Gln Ile Phe Asn Asn Leu Leu Arg Arg Gln Ile Gly Thr
   370 375 380
   Arg Ser Pro Thr Val Glu Tyr Leu Gly Ala Arg Pro Glu Ile Leu Ile
   385 390 395 400
   Gln Leu Val Gln Gly Tyr Ser Val Pro Asp Ile Ala Leu Thr Cys Gly
   405 410 415
   Leu Met Leu Arg Glu Ser Ile Arg His Asp His Leu Ala Lys Ile Ile
   420 425 430
   Leu Tyr Ser Asp Val Phe Tyr Thr Phe Phe Leu Tyr Val Gln Ser Glu
   435 440 445
   Val Phe Asp Ile Ser Ser Asp Ala Phe Ser Thr Phe Lys Glu Leu Thr
   450 455 460
   Thr Arg His Lys Ala Ile Ile Ala Glu Phe Leu Asp Ser Asn Tyr Asp
   465 470 475 480
   Thr Phe Phe Ala Gln Tyr Gln Asn Leu Leu Asn Ser Lys Asn Tyr Val
   485 490 495
   Thr Arg Arg Gln Ser Leu Lys Leu Leu Gly Glu Leu Leu Leu Asp Arg
   500 505 510
   His Asn Phe Asn Thr Met Thr Lys Tyr Ile Ser Asn Pro Asp Asn Leu
   515 520 525
   Arg Leu Met Met Glu Leu Leu Arg Asp Lys Ser Arg Asn Ile Gln Tyr
   530 535 540
   Glu Ala Phe His Val Phe Lys Val Phe Val Ala Asn Pro Asn Lys Pro
   545 550 555 560
   Lys Pro Ile Ser Asp Ile Leu Asn Arg Asn Arg Glu Lys Leu Val Glu
   565 570 575
   Phe Leu Ser Glu Phe His Asn Asp Arg Thr Asp Asp Glu Gln Phe Asn
   580 585 590
   Asp Glu Lys Ala Tyr Leu Ile Lys Gln Ile Gln Glu Met Lys Ser Ser
   595 600 605
   Pro Lys Glu Ala Lys Lys Pro Lys Ser Lys Glu Asp Glu Asn Gln Glu
   610 615 620
   Pro Ala Gly Pro Ser Glu Gly Pro Ser Thr Ser Gln
   625 630 635
<210> 15
   <211> 339
   <212> PRT
   <213> Drosophila melanogaster
<400> 15
   Met Pro Leu Phe Gly Lys Ser Gln Lys Ser Pro Val Glu Leu Val Lys
   1               5                   10                  15
   Ser Leu Lys Glu Ala Ile Asn Ala Leu Glu Ala Gly Asp Arg Lys Val
               20                  25                  30
   Glu Lys Ala Gln Glu Asp Val Ser Lys Asn Leu Val Ser Ile Lys Asn
           35                  40                  45
   Met Leu Tyr Gly Ser Ser Asp Ala Glu Pro Pro Ala Asp Tyr Val Val
       50                  55                  60
   Ala Gln Leu Ser Gln Glu Leu Tyr Asn Ser Asn Leu Leu Leu Leu Leu
   65                  70                  75                  80
   Ile Gln Asn Leu His Arg Ile Asp Phe Glu Gly Lys Lys His Val Ala
                   85                  90                  95
   Leu Ile Phe Asn Asn Val Leu Arg Arg Gln Ile Gly Thr Arg Ser Pro
               100                 105                 110
   Thr Val Glu Tyr Ile Cys Thr Lys Pro Glu Ile Leu Phe Thr Leu Met
           115                 120                 125
   Ala Gly Tyr Glu Asp Ala His Pro Glu Ile Ala Leu Asn Ser Gly Thr
       130                 135                 140
   Met Leu Arg Glu Cys Ala Arg Tyr Glu Ala Leu Ala Lys Ile Met Leu
   145                 150                 155                 160
   His Ser Asp Glu Phe Phe Lys Phe Phe Arg Tyr Val Glu Val Ser Thr
                   165                 170                 175
   Phe Asp Ile Ala Ser Asp Ala Phe Ser Thr Phe Lys Glu Leu Leu Thr
               180                 185                 190
   Arg His Lys Leu Leu Cys Ala Glu Phe Leu Asp Ala Asn Tyr Asp Lys
           195                 200                 205
   Phe Phe Ser Gln His Tyr Gln Arg Leu Leu Asn Ser Glu Asn Tyr Val
   210 215 220
   Thr Arg Arg Gln Ser Leu Lys Leu Leu Gly Glu Leu Leu Leu Asp Arg
   225 230 235 240
   His Asn Phe Thr Val Met Thr Arg Tyr Ile Ser Glu Pro Glu Asn Leu
   245 250 255
   Lys Leu Met Met Asn Met Leu Lys Glu Lys Ser Arg Asn Ile Gln Phe
   260 265 270
   Glu Ala Phe His Val Phe Lys Val Phe Val Ala Asn Pro Asn Lys Pro
   275 280 285
   Lys Pro Ile Leu Asp Ile Leu Leu Arg Asn Gln Thr Lys Leu Val Asp
   290 295 300
   Phe Leu Thr Asn Phe His Thr Asp Arg Ser Glu Asp Glu Gln Phe Asn
   305 310 315 320
   Asp Glu Lys Ala Tyr Leu Ile Lys Gln Ile Lys Glu Leu Lys Pro Leu
   325 330 335
   Pro Glu Ala
<210> 16
   <211> 20
   <212> PRT
   <213> Artificial
<220>
   <223> LKB1 substrate
<400> 16
   Leu Ser Asn Leu Tyr His Gln Gly Lys Phe Leu Gln Thr Phe Cys Gly
   1 5 10 15
   Ser Pro Leu Tyr
   20
<210> 17
   <211> 20
   <212> PRT
   <213> Artificial
<220>
   <223> LKB1 substrate
<400> 17
   Phe Gly Asn Phe Tyr Lys Ser Gly Glu Pro Leu Ser Thr Trp Cys Gly
   1               5                   10                  15
   Ser Pro Pro Tyr
               20
<210> 18
   <211> 20
   <212> PRT
   <213> Artificial
<220>
   <223> LKB1 substrate
<400> 18
   Leu Ser Asn Met Met Ser Asp Gly Glu Phe Leu Arg Thr Ser Cys Gly
   1               5                   10                  15
   Ser Pro Asn Tyr
               20
<210> 19
   <211> 20
   <212> PRT
   <213> Artificial
<220>
   <223> LKB1 substrate
<400> 19
   Met Ala Ser Leu Gln Val Gly Asp Ser Leu Leu Glu Thr Ser Cys Gly
   1               5                   10                  15
   Ser Pro His Tyr
               20
<210> 20
   <211> 20
   <212> PRT
   <213> Artificial
<220>
   <223> LKB1 substrate
<400> 20
   Phe Ser Asn Glu Phe Thr Val Gly Gly Lys Leu Asp Thr Phe Cys Gly
   1               5                   10                  15
   Ser Pro Pro Tyr
               20
<210> 21
   <211> 20
   <212> PRT
   <213> Artificial
<220>
   <223> LKB1 substrate
<400> 21
   Ala Lys Pro Lys Gly Asn Lys Asp Tyr His Leu Gln Thr Cys Cys Gly
   1               5                   10                  15
   Ser Leu Ala Tyr
               20
<210> 22
   <211> 20
   <212> PRT
   <213> Artificial
<220>
   <223> LKB1 substrate
<400> 22
   Leu Ser Asn Leu Tyr His Gln Gly Lys Phe Leu Gln Thr Phe Cys Gly
   1               5                   10                  15
   Ser Pro Leu Tyr
               20
<210> 23
   <211> 23
   <212> PRT
   <213> Artificial
<220>
   <223> LKB1 substrate
<400> 23
   Leu Ser Asn Leu Tyr His Gln Gly Lys Phe Leu Gln Thr Phe Cys Gly
   1 5 10 15
   Ser Pro Leu Tyr Arg Arg Arg
   20
<210> 24
   <211> 19
   <212> PRT
   <213> Artificial
<220>
   <223> LKB1 substrate
<400> 24
   Ser Asn Leu Tyr His Gln Gly Lys Phe Leu Gln Thr Phe Cys Gly Ser
   1 5 10 15
   Pro Leu Tyr
<210> 25
   <211> 22
   <212> PRT
   <213> Artificial
<220>
   <223> LKB1 substrate
<400> 25
   Ser Asn Leu Tyr His Gln Gly Lys Phe Leu Gln Thr Phe Cys Gly Ser
   1 5 10 15
   Pro Leu Tyr Arg Arg Arg
   20
<210> 26
   <211> 20
   <212> PRT
   <213> Artificial
<220>
   <223> LKB1 substrate
<400> 26
   Leu Ser Asn Leu Tyr His Gln Gly Lys Phe Leu Gln Thr Phe Cys Gly
   1               5                   10                  15
   Ser Pro Leu Tyr
               20
<210> 27
   <211> 23
   <212> PRT
   <213> Artificial
<220>
   <223> LKB1 substrate
<400> 27
   Leu Ser Asn Leu Tyr His Gln Gly Lys Phe Leu Gln Thr Phe Cys Gly
   1               5                   10                  15
   Ser Pro Leu Tyr Arg Arg Arg
               20
<210> 28
   <211> 20
   <212> PRT
   <213> Artificial
<220>
   <223> LKB1 substrate
<400> 28
   Phe Gly Asn Phe Tyr Lys Ser Gly Glu Pro Leu Ser Thr Trp Cys Gly
   1               5                   10                  15
   Ser Pro Pro Tyr
               20
<210> 29
   <211> 23
   <212> PRT
   <213> Artificial
<220>
   <223> LKB1 substrate
<400> 29
   Phe Gly Asn Phe Tyr Lys Ser Gly Glu Pro Leu Ser Thr Trp Cys Gly
   1 5 10 15
   Ser Pro Pro Tyr Arg Arg Arg
   20
<210> 30
   <211> 20
   <212> PRT
   <213> Artificial
<220>
   <223> LKB1 substrate
<400> 30
   Leu Ser Asn Met Met Ser Asp Gly Glu Phe Leu Arg Thr Ser Cys Gly
   1 5 10 15
   Ser Pro Asn Tyr
   20
<210> 31
   <211> 23
   <212> PRT
   <213> Artificial
<220>
   <223> LKB1 substrate
<400> 31
   Leu Ser Asn Met Met Ser Asp Gly Glu Phe Leu Arg Thr Ser Cys Gly
   1 5 10 15
   Ser Pro Asn Tyr Arg Arg Arg
   20
<210> 32
   <211> 20
   <212> PRT
   <213> Artificial
<220>
   <223> LKB1 substrate
<400> 32
   Met Ala Ser Leu Gln Val Gly Asp Ser Leu Leu Glu Thr Ser Cys Gly
   1 5 10 15
   Ser Pro His Tyr
   20
<210> 33
   <211> 23
   <212> PRT
   <213> Artificial
<220>
   <223> LKB1 substrate
<400> 33
   Met Ala Ser Leu Gln Val Gly Asp Ser Leu Leu Glu Thr Ser Cys Gly
   1 5 10 15
   Ser Pro His Tyr Arg Arg Arg
   20
<210> 34
   <211> 20
   <212> PRT
   <213> Artificial
<220>
   <223> LKB1 substrate
<400> 34
   Phe Ser Asn Glu Phe Thr Val Gly Gly Lys Leu Asp Thr Phe Cys Gly
   1 5 10 15
   Ser Pro Pro Tyr
   20
<210> 35
   <211> 23
   <212> PRT
   <213> Artificial
<220>
   <223> LKB1 substrate
<400> 35
   Phe Ser Asn Glu Phe Thr Val Gly Gly Lys Leu Asp Thr Phe Cys Gly
   1               5                   10                  15
   Ser Pro Pro Tyr Arg Arg Arg
               20
<210> 36
   <211> 20
   <212> PRT
   <213> Artificial
<220>
   <223> LKB1 substrate
<400> 36
   Ala Lys Pro Lys Gly Asn Lys Asp Tyr His Leu Gln Thr Cys Cys Gly
   1               5                   10                  15
   Ser Leu Ala Tyr
               20
<210> 37
   <211> 23
   <212> PRT
   <213> Artificial
<220>
   <223> LKB1 substrate
<400> 37
   Ala Lys Pro Lys Gly Asn Lys Asp Tyr His Leu Gln Thr Cys Cys Gly
   1               5                   10                  15
   Ser Leu Ala Tyr Arg Arg Arg
               20
<210> 38
   <211> 19
   <212> PRT
   <213> Homo sapiens
<400> 38
   Met Val Ala Gly Leu Thr Leu Gly Lys Gly Pro Glu Ser Pro Asp Gly
   1               5                   10                  15
   Asp Val Ser
<210> 39
   <211> 20
   <212> PRT
   <213> Homo sapiens
<400> 39
   Leu Ser Trp Gly Ala Gly Leu Lys Gly Gln Lys Val Ala Thr Ser Tyr
   1               5                   10                  15
   Glu Ser Ser Leu
               20
<210> 40
   <211> 21
   <212> PRT
   <213> Homo sapiens
<400> 40
   Met Glu Gly Ala Ala Ala Pro Val Ala Gly Asp Arg Pro Asp Leu Gly
   1               5                   10                  15
   Leu Gly Ala Pro Gly
               20
<210> 41
   <211> 21
   <212> PRT
   <213> Homo sapiens
<400> 41
   Thr Asp Cys Gln Glu Val Thr Ala Thr Tyr Arg Gln Ala Leu Arg Val
   1               5                   10                  15
   Cys Ser Lys Leu Thr
               20
<210> 42
   <211> 21
   <212> PRT
   <213> Homo sapiens
<400> 42
   Met Val Met Ala Asp Gly Pro Arg His Leu Gln Arg Gly Pro Val Arg
   1 5 10 15
   Val Gly Phe Tyr Asp
   20
<210> 43
   <211> 20
   <212> PRT
   <213> Homo sapiens
<400> 43
   Met Val Ile Met Ser Glu Phe Ser Ala Asp Pro Ala Gly Gln Gly Gln
   1 5 10 15
   Gly Gln Gln Lys
   20
<210> 44
   <211> 20
   <212> PRT
   <213> Homo sapiens
<400> 44
   Gly Asp Cys Glu Met Glu Asp Leu Met Pro Cys Ser Leu Gly Thr Phe
   1 5 10 15
   Val Leu Val Gln
   20
<210> 45
   <211> 21
   <212> PRT
   <213> Homo sapiens
<400> 45
   Thr Asp Ile Leu Leu Ser Tyr Lys His Pro Glu Val Ser Phe Ser Met
   1 5 10 15
   Glu Gln Ala Gly Val
   20
<210> 46
   <211> 20
   <212> PRT
   <213> Homo sapiens
<400> 46
   Ser Gly Thr Ser Ile Ala Phe Lys Asn Ile Ala Ser Lys Ile Ala Asn
   1               5                   10                  15
   Glu Leu Lys Leu
               20
<210> 47
   <211> 20
   <212> PRT
   <213> Homo sapiens
<400> 47
   Met Ser Ser Arg Thr Val Leu Ala Pro Gly Asn Asp Arg Asn Ser Asp
   1               5                   10                  15
   Thr His Gly Thr
               20
<210> 48
   <211> 20
   <212> PRT
   <213> Homo sapiens
<400> 48
   Met Lys Asp Tyr Asp Glu Leu Leu Lys Tyr Tyr Glu Leu His Glu Thr
   1               5                   10                  15
   Ile Gly Thr Gly
               20
<210> 49
   <211> 16
   <212> PRT
   <213> Homo sapiens
<400> 49
   Cys Thr Ser Pro Pro Asp Ser Phe Leu Asp Asp His His Leu Thr Arg
   1               5                   10                  15
<210> 50
   <211> 14
   <212> PRT
   <213> Homo sapiens
<400> 50
   Cys Asp Pro Met Lys Arg Ala Thr Ile Lys Asp Ile Arg Glu
   1 5 10
<210> 51
   <211> 12
   <212> PRT
   <213> Artificial
<220>
   <223> C-terminal 12 residues STRAD alpha
<400> 51
   Asn Leu Glu Glu Leu Glu Val Asp Asp Trp Glu Phe
   1 5 10
<210> 52
   <211> 12
   <212> PRT
   <213> Artificial
<220>
   <223> C-terminal 12 residues STRAD alpha, last residue mutated to Ala
<400> 52
   Asn Leu Glu Glu Leu Glu Val Asp Asp Trp Glu Ala
   1 5 10
<210> 53
   <211> 12
   <212> PRT
   <213> Artificial
<220>
   <223> c-terminal 12 residues STRAD alpha, third last residue mutated t o Ala
<400> 53
   Asn Leu Glu Glu Leu Glu Val Asp Asp Ala Glu Phe
   1 5 10
<210> 54
   <211> 12
   <212> PRT
   <213> Artificial
<220>
   <223> C-terminal 12 residues STRAD alpha, second last residue mutated t o Ala
<400> 54
   Asn Leu Glu Glu Leu Glu Val Asp Asp Trp Ala Phe
   1 5 10
<210> 55
   <211> 6
   <212> PRT
   <213> Artificial
<220>
   <223> C-terminal 6 residues STRAD alpha
<400> 55
   Val Asp Asp Trp Glu Phe
   1 5
<210> 56
   <211> 547
   <212> PRT
   <213> Homo sapiens
<400> 56
   Met Ala Glu Pro Ser Gly Ser Pro Val His Val Gln Leu Pro Gln Gln
   1               5                   10                  15
   Ala Ala Pro Val Thr Ala Ala Ala Ala Ala Ala Pro Ala Ala Ala Thr
               20                  25                  30
   Ala Ala Pro Ala Pro Ala Ala Pro Ala Ala Pro Ala Pro Ala Pro Ala
           35                  40                  45
   Pro Ala Pro Ala Ala Gln Ala Val Gly Trp Pro Ile Cys Arg Asp Ala
       50                  55                  60
   Tyr Glu Leu Gln Glu Val Ile Gly Ser Gly Ala Thr Ala Val Val Gln
   65                  70                  75                  80
   Ala Ala Leu Cys Lys Pro Arg Gln Glu Arg Val Ala Ile Lys Arg Ile
                   85                  90                  95
   Asn Leu Glu Lys Cys Gln Thr Ser Met Asp Glu Leu Leu Lys Glu Ile
               100                 105                 110
   Gln Ala Met Ser Gln Cys Ser His Pro Asn Val Val Thr Tyr Tyr Thr
           115                 120                 125
   Ser Phe Val Val Lys Asp Glu Leu Trp Leu Val Met Lys Leu Leu Ser
       130                 135                 140
   Gly Gly Ser Met Leu Asp Ile Ile Lys Tyr Ile Val Asn Arg Gly Glu
   145                 150                 155                 160
   His Lys Asn Gly Val Leu Glu Glu Ala Ile Ile Ala Thr Ile Leu Lys
                   165                 170                 175
   Glu Val Leu Glu Gly Leu Asp Tyr Leu His Arg Asn Gly Gln Ile His
               180                 185                 190
   Arg Asp Leu Lys Ala Gly Asn Ile Leu Leu Gly Glu Asp Gly Ser Val
   195 200 205
   Gln Ile Ala Asp Phe Gly Val Ser Ala Phe Leu Ala Thr Gly Gly Asp
   210 215 220
   Val Thr Arg Asn Lys Val Arg Lys Thr Phe Val Gly Thr Pro Cys Trp
   225 230 235 240
   Met Ala Pro Glu Val Met Glu Gln Val Arg Gly Tyr Asp Phe Lys Ala
   245 250 255
   Asp Met Trp Ser Phe Gly Ile Thr Ala Ile Glu Leu Ala Thr Gly Ala
   260 265 270
   Ala Pro Tyr His Lys Tyr Pro Pro Met Lys Val Leu Met Leu Thr Leu
   275 280 285
   Gln Asn Asp Pro Pro Thr Leu Glu Thr Gly Val Glu Asp Lys Glu Met
   290 295 300
   Met Lys Lys Tyr Gly Lys Ser Phe Arg Lys Leu Leu Ser Leu Cys Leu
   305 310 315 320
   Gln Lys Asp Pro Ser Lys Arg Pro Thr Ala Ala Glu Leu Leu Lys Cys
   325 330 335
   Lys Phe Phe Gln Lys Ala Lys Asn Arg Glu Tyr Leu Ile Glu Lys Leu
   340 345 350
   Leu Thr Arg Thr Pro Asp Ile Ala Gln Arg Ala Lys Lys Val Arg Arg
   355 360 365
   Val Pro Gly Ser Ser Gly His Leu His Lys Thr Glu Asp Gly Asp Trp
   370 375 380
   Glu Trp Ser Asp Asp Glu Met Asp Glu Lys Ser Glu Glu Gly Lys Ala
   385 390 395 400
   Ala Phe Ser Gln Glu Lys Ser Arg Arg Val Lys Glu Glu Asn Pro Glu
   405 410 415
   Ile Ala Val Ser Ala Ser Thr Ile Pro Glu Gln Ile Gln Ser Leu Ser
   420 425 430
   Val His Asp Ser Gln Gly Pro Pro Asn Ala Asn Glu Asp Tyr Arg Glu
   435 440 445
   Ala Ser Ser Cys Ala Val Asn Leu Val Leu Arg Leu Arg Asn Ser Arg
   450 455 460
   Lys Glu Leu Asn Asp Ile Arg Phe Glu Phe Thr Pro Gly Arg Asp Thr
   465 470 475 480
   Ala Asp Gly Val Ser Gln Glu Leu Phe Ser Ala Gly Leu Val Asp Gly
   485 490 495
   His Asp Val Val Ile Val Ala Ala Asn Leu Gln Lys Ile Val Asp Asp
   500 505 510
   Pro Lys Ala Leu Lys Thr Leu Thr Phe Lys Leu Ala Ser Gly Cys Asp
   515 520 525
   Gly Ser Glu Ile Pro Asp Glu Val Lys Leu Ile Gly Phe Ala Gln Leu
   530 535 540
   Ser Val Ser
   545
<210> 57
   <211> 527
   <212> PRT
   <213> Homo sapiens
<400> 57
   Met Ser Glu Asp Ser Ser Ala Leu Pro Trp Ser Ile Asn Arg Asp Asp
   1               5                   10                  15
   Tyr Glu Leu Gln Glu Val Ile Gly Ser Gly Ala Thr Ala Val Val Gln
               20                  25                  30
   Ala Ala Tyr Cys Ala Pro Lys Lys Glu Lys Val Ala Ile Lys Arg Ile
           35                  40                  45
   Asn Leu Glu Lys Cys Gln Thr Ser Met Asp Glu Leu Leu Lys Glu Ile
       50                  55                  60
   Gln Ala Met Ser Gln Cys His His Pro Asn Ile Val Ser Tyr Tyr Thr
   65                  70                  75                  80
   Ser Phe Val Val Lys Asp Glu Leu Trp Leu Val Met Lys Leu Leu Ser
                   85                  90                  95
   Gly Gly Ser Val Leu Asp Ile Ile Lys His Ile Val Ala Lys Gly Glu
               100                 105                 110
   His Lys Ser Gly Val Leu Asp Glu Ser Thr Ile Ala Thr Ile Leu Arg
           115                 120                 125
   Glu Val Leu Glu Gly Leu Glu Tyr Leu His Lys Asn Gly Gln Ile His
       130                 135                 140
   Arg Asp Val Lys Ala Gly Asn Ile Leu Leu Gly Glu Asp Gly Ser Val
   145                 150                 155                 160
   Gln Ile Ala Asp Phe Gly Val Ser Ala Phe Leu Ala Thr Gly Gly Asp
                   165                 170                 175
   Ile Thr Arg Asn Lys Val Arg Lys Thr Phe Val Gly Thr Pro Cys Trp
               180                 185                 190
   Met Ala Pro Glu Val Met Glu Gln Val Arg Gly Tyr Asp Phe Lys Ala
           195                 200                 205
   Asp Ile Trp Ser Phe Gly Ile Thr Ala Ile Glu Leu Ala Thr Gly Ala
       210                 215                 220
   Ala Pro Tyr His Lys Tyr Pro Pro Met Lys Val Leu Met Leu Thr Leu
   225                 230                 235                 240
   Gln Asn Asp Pro Pro Ser Leu Glu Thr Gly Val Gln Asp Lys Glu Met
                   245                 250                 255
   Leu Lys Lys Tyr Gly Lys Ser Phe Arg Lys Met Ile Ser Leu Cys Leu
               260                 265                 270
   Gln Lys Asp Pro Glu Lys Arg Pro Thr Ala Ala Glu Leu Leu Arg His
           275                 280                 285
   Lys Phe Phe Gln Lys Ala Lys Asn Lys Glu Phe Leu Gln Glu Lys Thr
       290                 295                 300
   Leu Gln Arg Ala Pro Thr Ile Ser Glu Arg Ala Lys Lys Val Arg Arg
   305                 310                 315                 320
   Val Pro Gly Ser Ser Gly Arg Leu His Lys Thr Glu Asp Gly Gly Trp
                   325                 330                 335
   Glu Trp Ser Asp Asp Glu Phe Asp Glu Glu Ser Glu Glu Gly Lys Ala
               340                 345                 350
   Ala Ile Ser Gln Leu Arg Ser Pro Arg Val Lys Glu Ser Ile Ser Asn
           355                 360                 365
   Ser Glu Leu Phe Pro Thr Thr Asp Pro Val Gly Thr Leu Leu Gln Val
       370                 375                 380
   Pro Glu Gln Ile Ser Ala His Leu Pro Gln Pro Ala Gly Gln Ile Ala
   385                 390                 395                 400
   Thr Gln Pro Thr Gln Val Ser Leu Pro Pro Thr Ala Glu Pro Ala Lys
                   405                 410                 415
   Thr Ala Gln Ala Leu Ser Ser Gly Ser Gly Ser Gln Glu Thr Lys Ile
               420                 425                 430
   Pro Ile Ser Leu Val Leu Arg Leu Arg Asn Ser Lys Lys Glu Leu Asn
   435 440 445
   Asp Ile Arg Phe Glu Phe Thr Pro Gly Arg Asp Thr Ala Glu Gly Val
   450 455 460
   Ser Gln Glu Leu Ile Ser Ala Gly Leu Val Asp Gly Arg Asp Leu Val
   465 470 475 480
   Ile Val Ala Ala Asn Leu Gln Lys Ile Val Glu Glu Pro Gln Ser Asn
   485 490 495
   Arg Ser Val Thr Phe Lys Leu Ala Ser Gly Val Glu Gly Ser Asp Ile
   500 505 510
   Pro Asp Asp Gly Lys Leu Ile Gly Phe Ala Gln Leu Ser Ile Ser
   515 520 525
<210> 58
   <211> 560
   <212> PRT
   <213> Saccharomyces cerevisiae
<400> 58
   Met Val Leu Leu Lys Glu Pro Val Gln Pro Leu Pro Arg Ser Ser Leu
   1 5 10 15
   Leu Tyr Asn Asn Ala Ser Asn Ser Ser Ser Arg Ile Lys Glu Thr Arg
   20 25 30
   Lys Val Lys Leu Leu Tyr Asn Pro Leu Thr Lys Arg Gln Ile Leu Asn
   35 40 45
   Asn Phe Glu Ile Leu Ala Thr Leu Gly Asn Gly Gln Tyr Gly Lys Val
   50 55 60
   Lys Leu Ala Arg Asp Leu Gly Thr Gly Ala Leu Val Ala Ile Lys Ile
   65 70 75 80
   Leu Asn Arg Phe Glu Lys Arg Ser Gly Tyr Ser Leu Gln Leu Lys Val
   85 90 95
   Glu Asn Pro Arg Val Asn Gln Glu Ile Glu Val Met Lys Arg Cys His
   100 105 110
   His Glu Asn Val Val Glu Leu Tyr Glu Ile Leu Asn Asp Pro Glu Ser
   115 120 125
   Thr Lys Val Tyr Leu Val Leu Glu Tyr Cys Ser Arg Gly Pro Val Lys
   130 135 140
   Trp Cys Pro Glu Asn Lys Met Glu Ile Lys Ala Val Gly Pro Ser Ile
   145                 150                 155                 160
   Leu Thr Phe Gln Gln Ser Arg Lys Val Val Leu Asp Val Val Ser Gly
                   165                 170                 175
   Leu Glu Tyr Leu His Ser Gln Gly Ile Thr His Arg Asp Ile Lys Pro
               180                 185                 190
   Ser Asn Leu Leu Ile Ser Ser Asn Gly Thr Val Lys Ile Ser Asp Phe
           195                 200                 205
   Gly Val Ala Met Ser Thr Ala Thr Gly Ser Thr Asn Ile Gln Ser Ser
       210                 215                 220
   His Glu Gln Leu Leu Lys Ser Arg Ala Leu Gly Thr Pro Ala Phe Phe
   225                 230                 235                 240
   Ala Pro Glu Leu Cys Ser Thr Glu Lys Glu Tyr Ser Cys Ser Ser Ala
                   245                 250                 255
   Ile Asp Ile Trp Ser Leu Gly Val Thr Ile Tyr Cys Leu Leu Phe Gly
               260                 265                 270
   Lys Leu Pro Phe Asn Ala Asn Ser Gly Leu Glu Leu Phe Asp Ser Ile
           275                 280                 285
   Ile Asn Lys Pro Leu Glu Phe Pro Ser Tyr Glu Glu Met Leu Asn Gly
       290                 295                 300
   Ala Thr Ser Gly Ile Thr Met Glu Glu Tyr Thr Asp Ala Lys Asp Leu
   305                 310                 315                 320
   Leu Lys Lys Leu Leu Gln Lys Asp Pro Asp Lys Arg Ile Lys Leu Ala
                   325                 330                 335
   Asp Ile Lys Val His Pro Phe Met Cys His Tyr Gly Lys Ser Asp Ala
               340                 345                 350
   Ala Ser Val Leu Thr Asn Leu Glu Thr Phe His Glu Leu Lys Val Ser
           355                 360                 365
   Pro Pro Ser Ser Cys Lys Arg Val Glu Leu Val Ser Leu Pro Val Asn
       370                 375                 380
   Ser Ser Phe Ala Ser Leu Asp Ser Val Tyr Met Glu Asn Phe Asp His
   385                 390                 395                 400
   Asn Asn Leu Arg Thr Gly Ala Asp Arg Asn Ser Thr Tyr Ser Pro Ser
                   405                 410                 415
   Ile Tyr Asp Ala Asn Thr Leu Ser Pro Ser Ala Tyr His Asn Ile Gly
   420 425 430
   Ser Arg Glu Ser Ser Tyr Ser Ser Phe Ser Ser Phe Thr Ser Ser Thr
   435 440 445
   Ala Phe Ala Ser Gln Ile Ser Ile Gln Asp Ala Pro Ala Ile Gly Asp
   450 455 460
   Gln Gln Cys Leu Ile Gly Glu Ser Gly Ser Ser Leu Arg Val Asn Ser
   465 470 475 480
   Cys Glu Phe Pro Gln Tyr Thr Thr Met Ser Pro Val Gly Glu Tyr Pro
   485 490 495
   Phe Glu Ser Thr Glu Ala Ser Leu Ser Ser Thr Leu Thr Pro Val Gly
   500 505 510
   Asn Val Pro Gln Arg Ile Lys Ala His Leu Val Glu Gly Lys Ser Asn
   515 520 525
   Ser Lys Asp Asp Leu Arg Ile Glu Ala Asp Ala Ser Leu Val Phe Glu
   530 535 540
   Ala Ser Asp Ala Gln Arg Thr Arg Arg Arg Met Ser Leu Tyr Lys Leu
   545 550 555 560
<210> 59
   <211> 1142
   <212> PRT
   <213> Saccharomyces cerevisiae
<400> 59
   Met Asp Arg Ser Asp Lys Lys Val Asn Val Glu Glu Val Asn Val Pro
   1 5 10 15
   Ser Asn Leu Gln Ile Glu Leu Glu Lys Ser Gly Thr Ser Ser Ser Val
   20 25 30
   Ser Leu Arg Ser Pro Thr Lys Ser Ser Ala Thr Asn Leu Ala Gly Met
   35 40 45
   Ala Glu Gly Ala Arg Asp Asn Ala Ser Ile Ala Ser Ser Ser Val Asp
   50 55 60
   Ser Leu Asn Met Leu Leu Glu Arg Gln Arg Val Arg Gln Leu Asn His
   65 70 75 80
   Pro Gln His Gln Gln His Ile Ser Ser Ser Leu Ala Lys Thr Pro Thr
   85 90 95
   Thr Thr Ser Ser Phe Cys Ser Ser Gly Ser Ser Lys Asn Lys Val Lys
   100 105 110
   Glu Thr Asn Arg Ile Ser Leu Thr Tyr Asp Pro Val Ser Lys Arg Lys
   115 120 125
   Val Leu Asn Thr Tyr Glu Ile Ile Lys Glu Leu Gly His Gly Gln His
   130 135 140
   Gly Lys Val Lys Leu Ala Arg Asp Ile Leu Ser Lys Gln Leu Val Ala
   145 150 155 160
   Ile Lys Ile Val Asp Arg His Glu Lys Lys Gln Arg Lys Phe Phe Thr
   165 170 175
   Phe Ile Lys Ser Ser Lys Ile Ser Glu Asn Asp Lys Ile Lys Arg Glu
   180 185 190
   Ile Ala Ile Met Lys Lys Cys His His Lys His Val Val Gln Leu Ile
   195 200 205
   Glu Val Leu Asp Asp Leu Lys Ser Arg Lys Ile Tyr Leu Val Leu Glu
   210 215 220
   Tyr Cys Ser Arg Gly Glu Val Lys Trp Cys Pro Pro Asp Cys Met Glu
   225 230 235 240
   Ser Asp Ala Lys Gly Pro Ser Leu Leu Ser Phe Gln Glu Thr Arg Glu
   245 250 255
   Ile Leu Arg Gly Val Val Leu Gly Leu Glu Tyr Leu His Tyr Gln Gly
   260 265 270
   Ile Ile His Arg Asp Ile Lys Pro Ala Asn Leu Leu Ile Ser Gly Asp
   275 280 285
   Gly Thr Val Lys Ile Ser Asp Phe Gly Val Ser Leu Ala Ala Ser Ser
   290 295 300
   Thr Asn Ser Ser Asp Ser Ser Glu Ser Leu Asp Glu Leu Glu Leu Ala
   305 310 315 320
   Lys Thr Val Gly Thr Pro Ala Phe Phe Ala Pro Glu Met Cys Leu Gly
   325 330 335
   Glu Asp Ala Phe Thr Arg Tyr Asn Leu Thr Lys Glu Asn Leu Phe Arg
   340 345 350
   Gly Ser Cys Ile Ser Phe Met Ile Asp Ile Trp Ala Val Gly Val Thr
   355 360 365
   Leu Tyr Cys Leu Leu Phe Gly Met Leu Pro Phe Phe Ser Asp Phe Glu
   370 375 380
   Leu Lys Leu Phe Glu Lys Ile Val Asn Asp Pro Leu Lys Phe Pro Thr
   385 390 395 400
   Phe Lys Glu Ile Gln Ser Asn Lys Val Ser Lys Val Ser Cys Glu Glu
   405 410 415
   Glu Tyr Glu Met Ala Lys Asp Leu Leu Leu Lys Leu Leu Glu Lys Asn
   420 425 430
   Pro Gln Lys Arg Met Thr Ile Pro Ala Ile Lys Lys His Pro Phe Val
   435 440 445
   Ser Trp Asp Phe Asp His Val Pro Glu Asn Asp Glu Lys Leu Leu Ser
   450 455 460
   Ser Val Leu Glu Gln Lys Leu Arg Phe Gln Cys Asn Gln Thr Asp Gln
   465 470 475 480
   Phe Glu Pro Ile Ser Ile Ser Lys His Glu Leu Lys Asn Ala Val Ser
   485 490 495
   Gly Val Gly Lys Lys Ile Lys Glu Ser Val Leu Lys Ser Ile Pro Leu
   500 505 510
   Lys Asp Pro Ser Asp Leu Ser Asn Lys Asn Tyr Leu His Pro Thr Glu
   515 520 525
   Thr Thr Arg Gly Arg Gly Asp Ala Asn Val Ile Val Ser Glu Gly Ser
   530 535 540
   Val Leu Ser Asn Ile Lys Glu Leu Ser Ala Asn Asp Gly Cys Leu Asn
   545 550 555 560
   Thr Asp Ser Asp Thr Asn Ile Asn Ile Asn Asp Asp Asp His Tyr Ser
   565 570 575
   Gly Asp Asp Asn Asp Gly His Leu Thr Lys Arg Glu Leu Glu Arg Glu
   580 585 590
   Leu Asn Lys Phe Asp Asp Lys His Glu Ala Gly Asn Met Val Asn Leu
   595 600 605
   Pro Ile Asn Ser Ser Phe Ala Ser Leu Asp Ser Phe Tyr Ile Asp Asn
   610 615 620
   Phe Ala Met Ala Arg Met Gly Met Ser Ser Pro Glu Ala Gly Asp Ser
   625 630 635 640
   Val Ser Ser Val Pro Asn Leu Pro Ser Ala Pro Ser Ser Thr Arg Leu
                   645                 650                 655
   Gly Arg Ser Pro Val Phe Ser Gly Val Thr Asn Gln Pro Ser Pro Ile
               660                 665                 670
   Arg Pro Val Leu Pro Gln Gln Lys Ser Ser Phe Cys Ala Thr Gly Arg
           675                 680                 685
   Tyr Asp Lys Ser His Asn Ser Leu Leu Arg Asn Ser Ser Ser His Leu
       690                 695                 700
   Thr Ser Tyr Asn Ser Gly Arg Pro Ser Ser Arg Thr Gly Arg Met Asn
   705                 710                 715                 720
   Ser Arg Asn Gln Asn Leu Pro Lys Ile Pro Asn Ser Leu Ser Lys Ile
                   725                 730                 735
   Ser Thr Thr Lys Leu Thr Glu Leu Arg Val Pro Lys Asp Ser Glu Ile
               740                 745                 750
   Pro Ser Pro Ala Lys Asn Pro Asn Ala Asp Arg Leu Arg Arg Phe Pro
           755                 760                 765
   Val Lys Lys Asn Thr Lys Thr Pro Ala Ile Lys Asp Pro Pro Arg Ile
       770                 775                 780
   Asn Ile Asn Ser Ser Asp Lys Ser Gly Ser Lys Asn Ser Pro Ile Lys
   785                 790                 795                 800
   Ser Leu Tyr Gln Arg Met Lys Gln Ser Lys Asp Asn Ser Lys Thr Phe
                   805                 810                 815
   Glu Val Arg Arg Gly Asn Phe Phe Ser His Phe Asn Gly Asp Asp Asp
               820                 825                 830
   Asp Ser Ser Ser Gln Ser Ser Val Thr Ser Ser Gly Ser Glu Ser Asp
           835                 840                 845
   Ser Glu Leu Ser Ser Thr Ser Ser Ser Cys Thr Ser Gly Thr Gln Ser
       850                 855                 860
   Arg Asn Ser Ser Asn Asn Asn Ala Tyr Ser Glu Thr Glu Ser Leu Pro
   865                 870                 875                 880
   Phe Glu Phe Gly Val Asp Ser Glu Asp Gly Ser Gly Val Leu Leu Arg
                   885                 890                 895
   Asp Leu Pro Asn Glu Asp Gln Ile Arg Pro Phe Leu Asp Ile Gln Pro
               900                 905                 910
   Cys Arg Arg Met Lys Val Lys Ser Ser Leu Asn Leu Glu Pro Pro Ser
   915 920 925
   Val Ser Ser Ser Ser Ser Ser Ser Ser Asp Glu Asp Glu Leu Ile Leu
   930 935 940
   Asn Val Gly Thr Ala Gly His Arg Arg Arg His Asn Ser Ser Lys Leu
   945 950 955 960
   Ser Glu Leu Ser Asn Ser Pro Gln Lys Gly Ser Asn Asn Phe Met Tyr
   965 970 975
   Ser Asn Gly Ser Val His Asp Ser Glu Thr Thr Ile Thr Pro Gln Asn
   980 985 990
   Met Asp Asp Leu Thr Leu His Gln Ala Leu Ser Arg Ser Gln Pro Ile
   995 1000 1005
   Ser Lys Pro Gly Pro Leu Val Leu Pro Lys Arg Leu Asp Gln Lys
   1010 1015 1020
   Lys Ala Thr Thr Glu Thr Ser Asn Leu Thr Asp Ile Val Glu Phe
   1025 1030 1035
   Asn Gly Asn Asn Asp His Arg Lys Asp Lys Asn Phe Asp Lys Val
   1040 1045 1050
   Leu Tyr Ser Arg Asp Leu Leu Lys Asp Ala Leu Ser Ser Thr Asn
   1055 1060 1065
   Ala Gly Arg Arg Arg Ser Ile Pro Ser Asn Lys Ile Arg Gly Arg
   1070 1075 1080
   Lys Asp Ala Ser Ile Thr Met Ser Thr Asn Val Gly Asn Asp Glu
   1085 1090 1095
   His Ala Arg Asn Thr Ser Cys His Gly Asp Lys Gly Gln Glu Asn
   1100 1105 1110
   Gly Ala Ile Lys Gln Arg Thr His Glu Arg Ser Arg Ser Leu Thr
   1115 1120 1125
   Val Ala Glu Leu Asn Glu Glu Lys Arg Arg Ser Ala Leu Pro
   1130 1135 1140
<210> 60
   <211> 640
   <212> PRT
   <213> Saccharomyces cerevisiae
<400> 60
   Met Ser Pro Arg Gln Leu Ile Pro Thr Leu Ile Pro Glu Trp Ala Pro
   1 5 10 15
   Leu Ser Gln Gln Ser Cys Ile Arg Glu Asp Glu Leu Asp Ser Pro Pro
   20 25 30
   Ile Thr Pro Thr Ser Gln Thr Ser Ser Phe Gly Ser Ser Phe Ser Gln
   35 40 45
   Gln Lys Pro Thr Tyr Ser Thr Ile Ile Gly Glu Asn Ile His Thr Ile
   50 55 60
   Leu Asp Glu Ile Arg Pro Tyr Val Lys Lys Ile Thr Val Ser Asp Gln
   65 70 75 80
   Asp Lys Lys Thr Ile Asn Gln Tyr Thr Leu Gly Val Ser Ala Gly Ser
   85 90 95
   Gly Gln Phe Gly Tyr Val Arg Lys Ala Tyr Ser Ser Thr Leu Gly Lys
   100 105 110
   Val Val Ala Val Lys Ile Ile Pro Lys Lys Pro Trp Asn Ala Gln Gln
   115 120 125
   Tyr Ser Val Asn Gln Val Met Arg Gln Ile Gln Leu Trp Lys Ser Lys
   130 135 140
   Gly Lys Ile Thr Thr Asn Met Ser Gly Asn Glu Ala Met Arg Leu Met
   145 150 155 160
   Asn Ile Glu Lys Cys Arg Trp Glu Ile Phe Ala Ala Ser Arg Leu Arg
   165 170 175
   Asn Asn Val His Ile Val Arg Leu Ile Glu Cys Leu Asp Ser Pro Phe
   180 185 190
   Ser Glu Ser Ile Trp Ile Val Thr Asn Trp Cys Ser Leu Gly Glu Leu
   195 200 205
   Gln Trp Lys Arg Asp Asp Asp Glu Asp Ile Leu Pro Gln Trp Lys Lys
   210 215 220
   Ile Val Ile Ser Asn Cys Ser Val Ser Thr Phe Ala Lys Lys Ile Leu
   225 230 235 240
   Glu Asp Met Thr Lys Gly Leu Glu Tyr Leu His Ser Gln Gly Cys Ile
   245 250 255
   His Arg Asp Ile Lys Pro Ser Asn Ile Leu Leu Asp Glu Glu Glu Lys
   260 265 270
   Val Ala Lys Leu Ser Asp Phe Gly Ser Cys Ile Phe Thr Pro Gln Ser
   275 280 285
   Leu Pro Phe Ser Asp Ala Asn Phe Glu Asp Cys Phe Gln Arg Glu Leu
   290 295 300
   Asn Lys Ile Val Gly Thr Pro Ala Phe Ile Ala Pro Glu Leu Cys His
   305 310 315 320
   Leu Gly Asn Ser Lys Arg Asp Phe Val Thr Asp Gly Phe Lys Leu Asp
   325 330 335
   Ile Trp Ser Leu Gly Val Thr Leu Tyr Cys Leu Leu Tyr Asn Glu Leu
   340 345 350
   Pro Phe Phe Gly Glu Asn Glu Phe Glu Thr Tyr His Lys Ile Ile Glu
   355 360 365
   Val Ser Leu Ser Ser Lys Ile Asn Gly Asn Thr Leu Asn Asp Leu Val
   370 375 380
   Ile Lys Arg Leu Leu Glu Lys Asp Val Thr Leu Arg Ile Ser Ile Gln
   385 390 395 400
   Asp Leu Val Lys Val Leu Ser Arg Asp Gln Pro Ile Asp Ser Arg Asn
   405 410 415
   His Ser Gln Ile Ser Ser Ser Ser Val Asn Pro Val Arg Asn Glu Gly
   420 425 430
   Pro Val Arg Arg Phe Phe Gly Arg Leu Leu Thr Lys Lys Gly Lys Lys
   435 440 445
   Lys Thr Ser Gly Lys Gly Lys Asp Lys Val Leu Val Ser Ala Thr Ser
   450 455 460
   Lys Val Thr Pro Ser Ile His Ile Asp Glu Glu Pro Asp Lys Glu Cys
   465 470 475 480
   Phe Ser Thr Thr Val Leu Arg Ser Ser Pro Asp Ser Ser Asp Tyr Cys
   485 490 495
   Ser Ser Leu Gly Glu Glu Ala Ile Gln Val Thr Asp Phe Leu Asp Thr
   500 505 510
   Phe Cys Arg Ser Asn Glu Ser Leu Pro Asn Leu Thr Val Asn Asn Asp
   515 520 525
   Lys Gln Asn Ser Asp Met Lys Thr Asp Arg Ser Glu Ser Ser Ser His
   530 535 540
   Ser Ser Leu Lys Ile Pro Thr Pro Ile Lys Ala Met Ile Arg Leu Lys
   545 550 555 560
   Ser Ser Pro Lys Glu Asn Gly Asn Arg Thr His Ile Asn Cys Ser Gln
   565 570 575
   Asp Lys Pro Ser Ser Pro Leu Met Asp Arg Thr Val Gly Lys Arg Thr
   580 585 590
   Val Asn Asn Ser Gly Ala Arg Lys Leu Ala His Ser Ser Asn Ile Leu
   595 600 605
   Asn Phe Lys Ala Tyr Ile Asn Ser Glu Asp Ser Asp Ile Arg Glu Thr
   610 615 620
   Val Glu Asp Val Lys Thr Tyr Leu Asn Phe Ala Asp Asn Gly Gln Ile
   625 630 635 640
<210> 61
   <211> 545
   <212> PRT
   <213> Homo sapiens
<400> 61
   Met Ser Ser Cys Val Ser Ser Gln Pro Ser Ser Asn Arg Ala Ala Pro
   1               5                   10                  15
   Gln Asp Glu Leu Gly Gly Arg Gly Ser Ser Ser Ser Glu Ser Gln Lys
               20                  25                  30
   Pro Cys Glu Ala Leu Arg Gly Leu Ser Ser Leu Ser Ile His Leu Gly
           35                  40                  45
   Met Glu Ser Phe Ile Val Val Thr Glu Cys Glu Pro Gly Cys Ala Val
       50                  55                  60
   Asp Leu Gly Leu Ala Arg Asp Arg Pro Leu Glu Ala Asp Gly Gln Glu
   65                  70                  75                  80
   Val Pro Leu Asp Thr Ser Gly Ser Gln Ala Arg Pro His Leu Ser Gly
                   85                  90                  95
   Arg Lys Leu Ser Leu Gln Glu Arg Ser Gln Gly Gly Leu Ala Ala Gly
               100                 105                 110
   Gly Ser Leu Asp Met Asn Gly Arg Cys Ile Cys Pro Ser Leu Pro Tyr
           115                 120                 125
   Ser Pro Val Ser Ser Pro Gln Ser Ser Pro Arg Leu Pro Arg Arg Pro
       130                 135                 140
   Thr Val Glu Ser His His Val Ser Ile Thr Gly Met Gln Asp Cys Val
   145                 150                 155                 160
   Gln Leu Asn Gln Tyr Thr Leu Lys Asp Glu Ile Gly Lys Gly Ser Tyr
                   165                 170                 175
   Gly Val Val Lys Leu Ala Tyr Asn Glu Asn Asp Asn Thr Tyr Tyr Ala
               180                 185                 190
   Met Lys Val Leu Ser Lys Lys Lys Leu Ile Arg Gln Ala Gly Phe Pro
           195                 200                 205
   Arg Arg Pro Pro Pro Arg Gly Thr Arg Pro Ala Pro Gly Gly Cys Ile
       210                 215                 220
   Gln Pro Arg Gly Pro Ile Glu Gln Val Tyr Gln Glu Ile Ala Ile Leu
   225                 230                 235                 240
   Lys Lys Leu Asp His Pro Asn Val Val Lys Leu Val Glu Val Leu Asp
                   245                 250                 255
   Asp Pro Asn Glu Asp His Leu Tyr Met Val Phe Glu Leu Val Asn Gln
               260                 265                 270
   Gly Pro Val Met Glu Val Pro Thr Leu Lys Pro Leu Ser Glu Asp Gln
           275                 280                 285
   Ala Arg Phe Tyr Phe Gln Asp Leu Ile Lys Gly Ile Glu Tyr Leu His
       290                 295                 300
   Tyr Gln Lys Ile Ile His Arg Asp Ile Lys Pro Ser Asn Leu Leu Val
   305                 310                 315                 320
   Gly Glu Asp Gly His Ile Lys Ile Ala Asp Phe Gly Val Ser Asn Glu
                   325                 330                 335
   Phe Lys Gly Ser Asp Ala Leu Leu Ser Asn Thr Val Gly Thr Pro Ala
               340                 345                 350
   Phe Met Ala Pro Glu Ser Leu Ser Glu Thr Arg Lys Ile Phe Ser Gly
           355                 360                 365
   Lys Ala Leu Asp Val Trp Ala Met Gly Val Thr Leu Tyr Cys Phe Val
       370                 375                 380
   Phe Gly Gln Cys Pro Phe Met Asp Glu Arg Ile Met Cys Leu His Ser
   385                 390                 395                 400
   Lys Ile Lys Ser Gln Ala Leu Glu Phe Pro Asp Gln Pro Asp Ile Ala
                   405                 410                 415
   Glu Asp Leu Lys Asp Leu Ile Thr Arg Met Leu Asp Lys Asn Pro Glu
               420                 425                 430
   Ser Arg Ile Val Val Pro Glu Ile Lys Ile Leu Val Lys Thr Met Ile
   435 440 445
   Arg Lys Arg Ser Phe Gly Asn Pro Phe Glu Gly Ser Arg Arg Glu Glu
   450 455 460
   Arg Ser Leu Ser Ala Pro Gly Asn Leu Leu Thr Lys Lys Pro Thr Arg
   465 470 475 480
   Glu Cys Glu Ser Leu Ser Glu Leu Lys Glu Ala Arg Gln Arg Arg Gln
   485 490 495
   Pro Pro Gly His Arg Pro Ala Pro Arg Gly Gly Gly Gly Ser Ala Leu
   500 505 510
   Val Arg Gly Ser Pro Cys Val Glu Ser Cys Trp Ala Pro Ala Pro Gly
   515 520 525
   Ser Pro Ala Arg Met His Pro Leu Arg Pro Glu Glu Ala Met Glu Pro
   530 535 540
   Glu
   545
<210> 62
   <211> 243
   <212> PRT
   <213> Artificial
<220>
   <223> Consensus for figure 12
<400> 62
   Pro Ser Ser Ser Ser Ser Ser Arg Ile Lys Thr Val Leu Tyr Pro Leu
   1 5 10 15
   Thr Lys Arg Gln Ile Leu Asn Asn Tyr Ile Leu Gly Gly Gln Tyr Gly
   20 25 30
   Lys Val Lys Leu Ala Asp Thr Leu Val Ala Ile Lys Ile Leu Lys Lys
   35 40 45
   Lys Lys Tyr Lys Asp Arg Val Lys Glu Ile Val Met Lys Arg Leu His
   50 55 60
   His Asn Val Val Leu Ile Glu Val Leu Asp Asp Pro Ser Lys Val Tyr
   65 70 75 80
   Leu Val Leu Glu Tyr Cys Ser Gly Val Trp Cys Met Glu Ile Val Pro
   85 90 95
   Ile Leu Ser Gln Ala Arg Val Val Asp Val Val Gly Leu Glu Tyr Leu
   100 105 110
   His Ser Gln Gly Ile Ile His Arg Asp Ile Lys Pro Ser Asn Ile Leu
   115 120 125
   Ile Ser Asp Gly Thr Val Lys Ile Ser Asp Phe Gly Val Thr Ser Asp
   130 135 140
   Ser Leu Arg Val Gly Thr Pro Ala Phe Ala Pro Glu Leu Cys Tyr Phe
   145 150 155 160
   Ile Asp Ile Trp Ser Leu Gly Val Thr Leu Tyr Cys Leu Leu Phe Gly
   165 170 175
   Leu Pro Phe Ala Asp Leu Leu Phe Asp Lys Ile Ile Leu Phe Pro Glu
   180 185 190
   Met Glu Glu Leu Lys Asp Leu Leu Lys Lys Leu Leu Glu Asn Lys Asn
   195 200 205
   Pro Lys Arg Ile Leu Ile Lys His Pro Phe Val Asp His Pro Asp Val
   210 215 220
   Leu Thr Glu Leu Lys Pro Leu Arg Val Glu Pro Val Ser Leu Lys Ser
   225 230 235 240
   Ser Leu Gly
<210> 63
   <211> 25
   <212> PRT
   <213> Homo sapiens
<400> 63
   Asp Phe Gly Phe Ala Lys Arg Val Lys Gly Arg Thr Trp Thr Leu Cys
   1 5 10 15
   Gly Thr Pro Glu Tyr Leu Ala Pro Glu
   20 25
<210> 64
   <211> 28
   <212> PRT
   <213> Homo sapiens
<400> 64
   Asp Phe Gly Met Cys Lys Glu His Met Met Asp Gly Val Thr Thr Arg
   1 5 10 15
   Thr Phe Cys Gly Thr Pro Asp Tyr Ile Ala Pro Glu
   20 25
<210> 65
   <211> 27
   <212> PRT
   <213> Homo sapiens
<400> 65
   Asp Phe Gly Leu Ser Asn Leu Tyr Gln Lys Asp Lys Phe Leu Gln Thr
   1 5 10 15
   Phe Cys Gly Ser Pro Leu Tyr Ala Ser Pro Glu
   20 25
<210> 66
   <211> 26
   <212> PRT
   <213> Homo sapiens
<400> 66
   Asp Phe Gly Leu Ser Asn Tyr His Gln Gly Lys Phe Leu Gln Thr Phe
   1 5 10 15
   Cys Gly Ser Pro Leu Tyr Ala Ser Pro Glu
   20 25
<210> 67
   <211> 27
   <212> PRT
   <213> Homo sapiens
<400> 67
   Asp Phe Gly Met Ala Ser Leu Gln Val Gly Asp Ser Leu Leu Glu Thr
   1               5                   10                  15
   Ser Cys Gly Ser Pro His Tyr Ala Cys Pro Glu
               20                  25
<210> 68
   <211> 27
   <212> PRT
   <213> Homo sapiens
<400> 68
   Asp Phe Gly Met Ala Ser Leu Gln Val Gly Asp Ser Leu Leu Glu Thr
   1               5                   10                  15
   Ser Cys Gly Ser Pro His Tyr Ala Cys Pro Glu
               20                  25
<210> 69
   <211> 27
   <212> PRT
   <213> Homo sapiens
<400> 69
   Asp Phe Gly Phe Gly Asn Phe Tyr Lys Ser Gly Glu Pro Leu Ser Thr
   1               5                   10                  15
   Trp Cys Gly Ser Pro Pro Tyr Ala Ala Pro Glu
               20                  25
<210> 70
   <211> 27
   <212> PRT
   <213> Homo sapiens
<400> 70
   Asp Phe Gly Phe Gly Asn Phe Phe Lys Ser Gly Glu Leu Leu Ala Thr
   1               5                   10                  15
   Trp Cys Gly Ser Pro Pro Tyr Ala Ala Pro Glu
               20                  25
<210> 71
   <211> 27
   <212> PRT
   <213> Arabidopsis thaliana
<400> 71
   Asp Phe Gly Leu Ser Asn Ile Met Arg Asp Gly His Phe Leu Lys Thr
   1 5 10 15
   Ser Cys Gly Ser Pro Asn Tyr Ala Ala Pro Glu
   20 25
<210> 72
   <211> 27
   <212> PRT
   <213> Arabidopsis thaliana
<400> 72
   Asp Phe Gly Leu Ser Asn Val Met Arg Asp Gly His Phe Leu Lys Thr
   1 5 10 15
   Ser Cys Gly Ser Pro Asn Tyr Ala Ala Pro Glu
   20 25
<210> 73
   <211> 27
   <212> PRT
   <213> Homo sapiens
<400> 73
   Asp Phe Gly Leu Ser Asn Met Met Ser Asp Gly Glu Phe Leu Arg Thr
   1 5 10 15
   Ser Cys Gly Ser Pro Asn Tyr Ala Ala Pro Glu
   20 25
<210> 74
   <211> 27
   <212> PRT
   <213> Homo sapiens
<400> 74
   Asp Phe Gly Leu Ser Asn Met Met Ser Asp Gly Glu Phe Leu Arg Thr
   1 5 10 15
   Ser Cys Gly Ser Pro Asn Tyr Ala Ala Pro Glu
   20 25
<210> 75
   <211> 27
   <212> PRT
   <213> saccharomyces cerevisiae
<400> 75
   Asp Phe Gly Leu Ser Asn Ile Met Thr Asp Gly Asn Phe Leu Lys Thr
   1 5 10 15
   Ser Cys Gly Ser Pro Asn Tyr Ala Ala Pro Glu
   20 25
<210> 76
   <211> 27
   <212> PRT
   <213> Homo sapiens
<400> 76
   Asp Phe Gly Phe Ser Asn Leu Phe Thr Pro Gly Gln Leu Leu Lys Thr
   1 5 10 15
   Trp Cys Gly Ser Pro Pro Tyr Ala Ala Pro Glu
   20 25
<210> 77
   <211> 29
   <212> PRT
   <213> Homo sapiens
<400> 77
   Asp Phe Gly Leu Cys Ala Lys Pro Lys Gly Asn Lys Asp Tyr His Leu
   1 5 10 15
   Gln Thr Cys Cys Gly Ser Leu Ala Tyr Ala Ala Pro Glu
   20 25
<210> 78
   <211> 21
   <212> PRT
   <213> Artificial
<220>
   <223> consensus from figure 19
<400> 78
   Asp Phe Gly Leu Ser Asn Leu Gly Phe Leu Thr Ser Cys Gly Ser Pro
   1 5 10 15
   Tyr Ala Ala Pro Glu
   20
<210> 79
   <211> 27
   <212> PRT
   <213> Homo sapiens
<400> 79
   Asp Phe Gly Phe Ser Asn Glu Phe Thr Val Gly Asn Lys Leu Asp Thr
   1 5 10 15
   Phe Cys Gly Ser Pro Pro Tyr Ala Ala Pro Glu
   20 25
<210> 80
   <211> 27
   <212> PRT
   <213> Homo sapiens
<400> 80
   Asp Phe Gly Phe Ser Asn Glu Phe Thr Val Gly Asn Lys Leu Asp Thr
   1 5 10 15
   Phe Cys Gly Ser Pro Pro Tyr Ala Ala Pro Glu
   20 25
<210> 81
   <211> 27
   <212> PRT
   <213> Homo sapiens
<400> 81
   Asp Phe Gly Phe Ser Asn Glu Phe Thr Val Gly Gly Lys Leu Asp Thr
   1 5 10 15
   Phe Cys Gly Ser Pro Pro Tyr Ala Ala Pro Glu
   20 25
<210> 82
   <211> 27
   <212> PRT
   <213> Homo sapiens
<400> 82
   Asp Phe Gly Phe Ser Asn Glu Phe Thr Leu Gly Ser Lys Leu Asp Thr
   1 5 10 15
   Phe Cys Gly Ser Pro Pro Tyr Ala Ala Pro Glu
   20 25
<210> 83
   <211> 23
   <212> PRT
   <213> Homo sapiens
<400> 83
   Leu Ser Asn Leu Tyr His Gln Gly Lys Phe Leu Gln Thr Phe Cys Gly
   1 5 10 15
   Ser Pro Leu Tyr Arg Arg Arg
   20
<210> 84
   <211> 23
   <212> PRT
   <213> Homo sapiens
<400> 84
   Phe Gly Asn Phe Tyr Lys Ser Gly Glu Pro Leu Ser Thr Trp Cys Gly
   1 5 10 15
   Ser Pro Pro Tyr Arg Arg Arg
   20
<210> 85
   <211> 23
   <212> PRT
   <213> Homo sapiens
<400> 85
   Leu Ser Asn Met Met Ser Asp Gly Glu Phe Leu Arg Thr Ser Cys Gly
   1 5 10 15
   Ser Pro Asn Tyr Arg Arg Arg
   20
<210> 86
   <211> 23
   <212> PRT
   <213> Homo sapiens
<400> 86
   Met Ala Ser Leu Gln Val Gly Asp Ser Leu Leu Glu Thr Ser Cys Gly
   1 5 10 15
   Ser Pro His Tyr Arg Arg Arg
   20
<210> 87
   <211> 23
   <212> PRT
   <213> Homo sapiens
<400> 87
   Phe Ser Asn Glu Phe Thr Val Gly Gly Lys Leu Asp Thr Phe Cys Gly
   1 5 10 15
   Ser Pro Pro Tyr Arg Arg Arg
   20
<210> 88
   <211> 23
   <212> PRT
   <213> Homo sapiens
<400> 88
   Ala Lys Pro Lys Gly Asn Lys Asp Tyr His Leu Gln Thr Cys Cys Gly
   1 5 10 15
   Ser Leu Ala Tyr Arg Arg Arg
   20
<210> 89
   <211> 32
   <212> PRT
   <213> Homo sapiens
<400> 89
   Ile Ala Ala Phe Gly Ala Ser Leu Gln Val Gly Asp Ser Leu Leu Glu
   1               5                   10                  15
   Thr Ser Cys Gly Ser Pro His Tyr Ala Cys Pro Glu Arg Val Ile Arg
               20                  25                  30
<210> 90
   <211> 25
   <212> PRT
   <213> Homo sapiens
<400> 90
   Ser Leu Gln Val Gly Asp Ser Leu Leu Glu Thr Ser Cys Gly Ser Pro
   1               5                   10                  15
   His Tyr Ala Cys Pro Glu Val Ile Arg
               20                  25
<210> 91
   <211> 20
   <212> PRT
   <213> Homo sapiens
<400> 91
   Phe Leu Gln Thr Phe Cys Gly Ser Pro Leu Tyr Ala Ser Pro Glu Ile
   1               5                   10                  15
   Val Asn Gly Lys
               20
<210> 92
   <211> 19
   <212> PRT
   <213> Homo sapiens
<400> 92
   Leu Asp Thr Phe Cys Gly Ser Pro Pro Tyr Ala Ala Pro Glu Leu Phe
   1               5                   10                  15
   Gln Gly Lys
<210> 93
   <211> 25
   <212> PRT
   <213> Homo sapiens
<400> 93
   Gly Asn Lys Asp Tyr His Leu Gln Thr Cys Cys Gly Ser Leu Ala Tyr
   1 5 10 15
   Ala Ala Pro Glu Leu Ile Gln Cys Lys
   20 25
<210> 94
   <211> 329
   <212> PRT
   <213> Schizosaccharomyces pombe
<400> 94
   Met Ser Phe Leu Phe Asn Lys Arg Pro Lys Ser Thr Gln Asp Val Val
   1 5 10 15
   Arg Cys Leu Cys Asp Asn Leu Pro Lys Leu Glu Ile Asn Asn Asp Lys
   20 25 30
   Lys Lys Ser Phe Glu Glu Val Ser Lys Cys Leu Gln Asn Leu Arg Val
   35 40 45
   Ser Leu Cys Gly Thr Ala Glu Val Glu Pro Asp Ala Asp Leu Val Ser
   50 55 60
   Asp Leu Ser Phe Gln Ile Tyr Gln Ser Asn Leu Pro Phe Leu Leu Val
   65 70 75 80
   Arg Tyr Leu Pro Lys Leu Glu Phe Glu Ser Lys Lys Asp Thr Gly Leu
   85 90 95
   Ile Phe Ser Ala Leu Leu Arg Arg His Val Ala Ser Arg Tyr Pro Thr
   100 105 110
   Val Asp Tyr Met Leu Ala His Pro Gln Ile Phe Pro Val Leu Val Ser
   115 120 125
   Tyr Tyr Arg Tyr Gln Glu Val Ala Phe Thr Ala Gly Ser Ile Leu Arg
   130 135 140
   Glu Cys Ser Arg His Glu Ala Leu Asn Glu Val Leu Leu Asn Ser Arg
   145 150 155 160
   Asp Phe Trp Thr Phe Phe Ser Leu Ile Gln Ala Ser Ser Phe Asp Met
   165 170 175
   Ala Ser Asp Ala Phe Ser Thr Phe Lys Ser Ile Leu Leu Asn His Lys
   180 185 190
   Ser Gln Val Ala Glu Phe Ile Ser Tyr His Phe Asp Glu Phe Phe Lys
   195 200 205
   Gln Tyr Thr Val Leu Leu Lys Ser Glu Asn Tyr Val Thr Lys Arg Gln
   210 215 220
   Ser Leu Lys Leu Leu Gly Glu Ile Leu Leu Asn Arg Ala Asn Arg Ser
   225 230 235 240
   Val Met Thr Arg Tyr Ile Ser Ser Ala Glu Asn Leu Lys Leu Met Met
   245 250 255
   Ile Leu Leu Arg Asp Lys Ser Lys Asn Ile Gln Phe Glu Ala Phe His
   260 265 270
   Val Phe Lys Leu Phe Val Ala Asn Pro Glu Lys Ser Glu Glu Val Ile
   275 280 285
   Glu Ile Leu Arg Arg Asn Lys Ser Lys Leu Ile Ser Tyr Leu Ser Ala
   290 295 300
   Phe His Thr Asp Arg Lys Asn Asp Glu Gln Phe Asn Asp Glu Arg Ala
   305 310 315 320
   Phe Val Ile Lys Gln Ile Glu Arg Leu
   325
<210> 95
   <211> 399
   <212> PRT
   <213> Saccharomyces cerevisiae
<400> 95
   Met Phe Lys Lys Tyr Lys Asn Gln Asp Leu Asp Met Ala Phe Trp Trp
   1               5                   10                  15
   Lys Lys Asn Pro Lys Thr Pro Ser Asp Tyr Ala Arg Leu Ile Ile Glu
               20                  25                  30
   Gln Leu Asn Lys Phe Ser Ser Pro Ser Leu Thr Gln Asp Asn Lys Arg
   35 40 45
   Lys Val Gln Glu Glu Cys Thr Lys Tyr Leu Ile Gly Thr Lys His Phe
   50 55 60
   Ile Val Gly Asp Thr Asp Pro His Pro Thr Pro Glu Ala Ile Asp Glu
   65 70 75 80
   Leu Tyr Thr Ala Met His Arg Ala Asp Val Phe Tyr Glu Leu Leu Leu
   85 90 95
   His Phe Val Asp Leu Glu Phe Glu Ala Arg Arg Glu Cys Met Leu Ile
   100 105 110
   Phe Ser Ile Cys Leu Gly Tyr Ser Lys Asp Asn Lys Phe Val Thr Val
   115 120 125
   Asp Tyr Leu Val Ser Gln Pro Lys Thr Ile Ser Leu Met Leu Arg Thr
   130 135 140
   Ala Glu Val Ala Leu Gln Gln Lys Gly Cys Gln Asp Ile Phe Leu Thr
   145 150 155 160
   Val Gly Asn Met Ile Ile Glu Cys Ile Lys Tyr Glu Gln Leu Cys Arg
   165 170 175
   Ile Ile Leu Lys Asp Pro Gln Leu Trp Lys Phe Phe Glu Phe Ala Lys
   180 185 190
   Leu Gly Asn Phe Glu Ile Ser Thr Glu Ser Leu Gln Ile Leu Ser Ala
   195 200 205
   Ala Phe Thr Ala His Pro Lys Leu Val Ser Lys Glu Phe Phe Ser Asn
   210 215 220
   Glu Ile Asn Ile Ile Arg Phe Ile Lys Cys Ile Asn Lys Leu Met Ala
   225 230 235 240
   His Gly Ser Tyr Val Thr Lys Arg Gln Ser Thr Lys Leu Leu Ala Ser
   245 250 255
   Leu Ile Val Ile Arg Ser Asn Asn Ala Leu Met Asn Ile Tyr Ile Asn
   260 265 270
   Ser Pro Glu Asn Leu Lys Leu Ile Met Thr Leu Met Thr Asp Lys Ser
   275 280 285
   Lys Asn Leu Gln Leu Glu Ala Phe Asn Val Phe Lys Val Met Val Ala
   290 295 300
   Asn Pro Arg Lys Ser Lys Pro Val Phe Asp Ile Leu Val Lys Asn Arg
   305 310 315 320
   Asp Lys Leu Leu Thr Tyr Phe Lys Thr Phe Gly Leu Asp Ser Gln Asp
   325 330 335
   Ser Thr Phe Leu Asp Glu Arg Glu Phe Ile Val Gln Glu Ile Asp Ser
   340 345 350
   Leu Pro Arg Ile Ile Ser Ser Thr Thr Glu Val Ser Asn Asn Asn Ala
   355 360 365
   Ser Ser Ser Asn Val Ala Ser Ile Thr Ser Pro Ser Ser Val Met Asn
   370 375 380
   Asn Gln Ser Ser Ile Leu Thr His Ser Thr Ser Pro Asp Ser Arg
   385 390 395
<210> 96
   <211> 343
   <212> PRT
   <213> Arabidopsis thaliana
<400> 96
   Met Arg Gly Leu Phe Lys Ser Lys Pro Arg Thr Pro Ala Asp Ile Val
   1 5 10 15
   Arg Gln Thr Arg Asp Leu Leu Leu Tyr Ala Asp Arg Ser Asn Ser Phe
   20 25 30
   Pro Asp Leu Arg Glu Ser Lys Arg Glu Glu Lys Met Val Glu Leu Ser
   35 40 45
   Lys Ser Ile Arg Asp Leu Lys Leu Ile Leu Tyr Gly Asn Ser Glu Ala
   50 55 60
   Glu Pro Val Ala Glu Ala Cys Ala Gln Leu Thr Gln Glu Phe Phe Lys
   65 70 75 80
   Ala Asp Thr Leu Arg Arg Leu Leu Thr Ser Leu Pro Asn Leu Asn Leu
   85 90 95
   Glu Ala Arg Lys Asp Ala Thr Gln Val Val Ala Asn Leu Gln Arg Gln
   100 105 110
   Gln Val Asn Ser Arg Leu Ile Ala Ala Asp Tyr Leu Glu Ser Asn Ile
   115 120 125
   Asp Leu Met Asp Phe Leu Val Asp Gly Phe Glu Asn Thr Asp Met Ala
   130 135 140
   Leu His Tyr Gly Thr Met Phe Arg Glu Cys Ile Arg His Gln Ile Val
   145 150 155 160
   Ala Lys Tyr Val Leu Asp Ser Glu His Val Lys Lys Phe Phe Tyr Tyr
   165 170 175
   Ile Gln Leu Pro Asn Phe Asp Ile Ala Ala Asp Ala Ala Ala Thr Phe
   180 185 190
   Lys Glu Leu Leu Thr Arg His Lys Ser Thr Val Ala Glu Phe Leu Ile
   195 200 205
   Lys Asn Glu Asp Trp Phe Phe Ala Asp Tyr Asn Ser Lys Leu Leu Glu
   210 215 220
   Ser Thr Asn Tyr Ile Thr Arg Arg Gln Ala Ile Lys Leu Leu Gly Asp
   225 230 235 240
   Ile Leu Leu Asp Arg Ser Asn Ser Ala Val Met Thr Lys Tyr Val Ser
   245 250 255
   Ser Met Asp Asn Leu Arg Ile Leu Met Asn Leu Leu Arg Glu Ser Ser
   260 265 270
   Lys Thr Ile Gln Ile Glu Ala Phe His Val Phe Lys Leu Phe Val Ala
   275 280 285
   Asn Gln Asn Lys Pro Ser Asp Ile Ala Asn Ile Leu Val Ala Asn Arg
   290 295 300
   Asn Lys Leu Leu Arg Leu Leu Ala Asp Ile Lys Pro Asp Lys Glu Asp
   305 310 315 320
   Glu Arg Phe Asp Ala Asp Lys Ala Gln Val Val Arg Glu Ile Ala Asn
   325 330 335
   Leu Lys Leu Arg Glu Leu Ala
   340
<210> 97
   <211> 8
   <212> PRT
   <213> Artificial
<220>
   <223> FLAG peptide
<400> 97
   Asp Tyr Lys Asp Asp Asp Asp Lys
   1 5
<210> 98
   <211> 15
   <212> PRT
   <213> Artificial
<220>
   <223> Bovine MBP fragment
<400> 98
   Gly His His Ala Ala Arg Thr Thr His Tyr Gly Ser Leu Pro Gln
   1 5 10 15
<210> 99
   <211> 73
   <212> DNA
   <213> Artificial
<220>
   <223> PCR primer
<400> 99
   ggatccgcca ccatggagca gaagctgatc tctgaagagg acttgccgtt cccgtttggg 60
   aagtctcaca aat 73
<210> 100
   <211> 34
   <212> DNA
   <213> Artificial
<220>
   <223> PCR primer
<400> 100
   ggatccttaa gcttcttgct gagctggtct cttc 34
<210> 101
   <211> 78
   <212> DNA
   <213> Artificial
<220>
   <223> PCR primer
<400> 101
   caccggatcc gccaccatgg agcagaagct gatctctgaa gaggacttgc ctttgtttag 60
   taaatcacac aaaaatcc 78
<210> 102
   <211> 34
   <212> DNA
   <213> Artificial
<220>
   <223> PCR primer
<400> 102
   ggatcctcaa ggggccgttt tcttcaagtc tcgg 34
<210> 103
   <211> 69
   <212> DNA
   <213> Artificial
<220>
   <223> PCR primer
<400> 103
   ggatccgcca ccatggacta caaggacgac gatgacaagt catttcttgt aagtaaacca 60
   gagcgaatc 69
<210> 104
   <211> 35
   <212> DNA
   <213> Artificial
<220>
   <223> PCR prime
<400> 104
   ggatcctcag aactcccaat cgtccacctc cagct 35
<210> 105
   <211> 68
   <212> DNA
   <213> Artificial
<220>
   <223> PCR prime
<400> 105
   ggatccgcca ccatggacta caaggacgac gatgacaagt ctcttttgga ttgcttctgc 60
   acttcaag 68
<210> 106
   <211> 36
   <212> DNA
   <213> Artificial
<220>
   <223> PCR primer
<400> 106
   ggatccctag aattcccagt atgagtcttt ttcatc 36
<210> 107
   <211> 66
   <212> DNA
   <213> Artificial
<220>
   <223> PCR primer
<400> 107
   actagtgcca ccatggacta caaggacgac gatgacaaga agctcatcgg caagtacctg 60
   atgggg 66
<210> 108
   <211> 36
   <212> DNA
   <213> Artificial
<220>
   <223> PCR primer
<400> 108
   actagttcag tcctccaggt agggcactac agtcat 36
<210> 109
   <211> 14
   <212> PRT
   <213> Homo sapiens
<400> 109
   Cys Asp Pro Met Lys Arg Ala Thr Ile Lys Asp Ile Arg Glu
   1 5 10
<210> 110
   <211> 22
   <212> PRT
   <213> Homo sapiens
<400> 110
   Leu Ser Asn Met Met Ser Asp Gly Glu Phe Leu Arg Thr Ser Cys Gly
   1 5 10 15
   Ser Pro Asn Arg Arg Arg
   20
<210> 111
   <211> 13
   <212> PRT
   <213> Rattus rattus
<400> 111
   Lys Phe Leu Arg Thr Ser Cys Gly Ser Pro Asn Tyr Ala
   1               5                   10
<210> 112
   <211> 25
   <212> DNA
   <213> Homo sapiens
<400> 112
   actgcagccc tggagcccag gaagc 25
<210> 113
   <211> 30
   <212> DNA
   <213> Homo sapiens
<400> 113
   ctagttgagc ttgctgcaga tctccagcgc 30
<210> 114
   <211> 69
   <212> DNA
   <213> Homo sapiens
<400> 114
   actagtgcca ccatgtaccc atacgatgtg ccagattacg ccgaaggggc cgccgcgcct 60
   gtggcgggg 69
<210> 115
   <211> 30
   <212> DNA
   <213> Homo sapiens
<400> 115
   ctagttgagc ttgctgcaga tctccagcgc 30
<210> 116
   <211> 69
   <212> DNA
   <213> Homo sapiens
<400> 116
   actagtgcca ccatgtaccc atacgatgtg ccagattacg ccgagtcgct ggttttcgcg 60
   cggcgctcc 69
<210> 117
   <211> 33
   <212> DNA
   <213> Homo sapiens
<400> 117
   tcaggtgagc tttgagcaga ccctcagtgc ctg 33
<210> 118
   <211> 55
   <212> DNA
   <213> Homo sapiens
<400> 118
   gcgtcgacta cccatacgat gtgccagatt acgccgtcat ggcggatggc ccgag 55
<210> 119
   <211> 55
   <212> DNA
   <213> Homo sapiens
<400> 119
   gcactagtta cccatacgat gtgccagatt acgccgtcat ggcggatggc ccgag 55
<210> 120
   <211> 36
   <212> DNA
   <213> Homo sapiens
<400> 120
   gagcggccgc taattcacca ggacataccc gttgtg 36
<210> 121
   <211> 60
   <212> DNA
   <213> Homo sapiens
<400> 121
   gcggatccta cccatacgat gtgccagatt acgccgttat catgtcggag ttcagcgcgg 60
<210> 122
   <211> 34
   <212> DNA
   <213> Homo sapiens
<400> 122
   gagcggccgc tcactgcacc aggacaaacg tgcc 34
<210> 123
   <211> 75
   <212> DNA
   <213> Homo sapiens
<400> 123
   gcggatccta cccatacgat gtgccagatt acgccaaaga ttatgatgaa cttctcaaat 60
   attatgaatt acatg 75
<210> 124
   <211> 41
   <212> DNA
   <213> Homo sapiens
<400> 124
   gtgcggccgc ttataccttg cagctagata ggatgtcttc c 41
<210> 125
   <211> 69
   <212> DNA
   <213> Homo sapiens
<400> 125
   ccacccccac ccaccccagc acgcccaata tgtgggcccc tatcggctgg agaagacgct 60
   gggcaaagg 69
<210> 126
   <211> 31
   <212> DNA
   <213> Homo sapiens
<400> 126
   cgatgcagcc tctcgcggtc cctgaagcag c 31
<210> 127
   <211> 31
   <212> DNA
   <213> Homo sapiens
<400> 127
   gctgcttcag ggaccgcgag aggctgcatc g 31
<210> 128
   <211> 28
   <212> DNA
   <213> Homo sapiens
<400> 128
   tcagggcaga ggggtcccgt tggtggcc 28
<210> 129
   <211> 67
   <212> DNA
   <213> Homo sapiens
<400> 129
   ggtgggggct ctcccgccta ccacctcccc cacccccacc cccacccacc ccagcacgcc 60
   caatatg 67
<210> 130
   <211> 76
   <212> DNA
   <213> Homo sapiens
<400> 130
   ggatcctacc catacgatgt gccagattac gcctcgtccg gggccaagga gggaggtggg 60
   ggctctcccg cctacc 76
<210> 131
   <211> 25
   <212> DNA
   <213> Homo sapiens
<400> 131
   gcggatccta cccatacgat gtgcc 25
<210> 132
   <211> 69
   <212> DNA
   <213> Homo sapiens
<400> 132
   ggatccgcca ccatgtaccc atacgatgtg ccagattacg ccacatcgac ggggaaggac 60
   ggcggcgcg 69
<210> 133
   <211> 57
   <212> DNA
   <213> Homo sapiens
<400> 133
   gcggccgctc agaggctact ctcgtagctg gtggccacct tctggccctt aagccca 57
<210> 134
   <211> 90
   <212> DNA
   <213> Homo sapiens
<400> 134
   ggagccgggc ccgcgggccg cctgctgcct ccgcccgcgc cggggtcccc agccgccccc 60
   gctgccgtgt cccctgcggc cggccagccg 90
<210> 135
   <211> 41
   <212> DNA
   <213> Homo sapiens
<400> 135
   tgaagaggtt actgaaacca aaatctgcta ttttgatatt c 41
<210> 136
   <211> 87
   <212> DNA
   <213> Homo sapiens
<400> 136
   gattacgccg cggcggcggc ggcgagcgga gctggcgggg ctgccggggc cgggactggg 60
   ggagccgggc ccgcgggccg cctgctg 87
<210> 137
   <211> 55
   <212> DNA
   <213> Homo sapiens
<400> 137
   gcggatccta cccatacgat gtgccagatt acgccgcggc ggcggcggcg agcgg 55
<210> 138
   <211> 90
   <212> DNA
   <213> Homo sapiens
<400> 138
   atagcagatt ttggtttcag taacctcttc actcctgggc agctgctgaa gacctggtgt 60
   ggcagccctc cctatgctgc acctgaactc 90
<210> 139
   <211> 34
   <212> DNA
   <213> Homo sapiens
<400> 139
   ctgtggacat aaaaaatggg atgcggaact ttcc 34
<210> 140
   <211> 34
   <212> DNA
   <213> Homo sapiens
<400> 140
   ggaaagttcc gcatcccatt ttttatgtcc acag 34
<210> 141
   <211> 33
   <212> DNA
   <213> Homo sapiens
<400> 141
   gagcggccgc ttacacgcct gcctgctcca tgc 33
<210> 142
   <211> 54
   <212> DNA
   <213> Homo sapiens
<400> 142
   gcgaattcta cccatacgat gtgccagatt acgcctcggc ccggacgcca ttgc 54
<210> 143
   <211> 37
   <212> DNA
   <213> Homo sapiens
<400> 143
   catcatactt ctgatttatt aaggcatcat ttatttc 37
<210> 144
   <211> 37
   <212> DNA
   <213> Homo sapiens
<400> 144
   gaaataaatg atgccttaat aaatcagaag tatgatg 37
<210> 145
   <211> 41
   <212> DNA
   <213> Homo sapiens
<400> 145
   gagtcgactt acagcttaag ctcatttgct atttttgatg c 41
<210> 146
   <211> 54
   <212> DNA
   <213> Homo sapiens
<400> 146
   gcggtaccta cccatacgat gtgccagatt acgcctcggc ccggacgcca ttgc 54
<210> 147
   <211> 43
   <212> DNA
   <213> Homo sapiens
<400> 147
   gagcggccgc ttacagctta agctcatttg ctatttttga tgc 43
<210> 148
   <211> 56
   <212> DNA
   <213> Homo sapiens
<400> 148
   gcgtcgacta cccatacgat gtgccagatt acgccattcg gggccgcaac tcagcc 56
<210> 149
   <211> 56
   <212> DNA
   <213> Homo sapiens
<400> 149
   gcactagtta cccatacgat gtgccagatt acgccattcg gggccgcaac tcagcc 56
<210> 150
   <211> 39
   <212> DNA
   <213> Homo sapiens
<400> 150
   gagcggccgc ttaaagcttc agctcgttgg ctattttgg 39
<210> 151
   <211> 73
   <212> DNA
   <213> Homo sapiens
<400> 151
   gcggccgcag ccaccatgta cccatacgat gtgccagatt acgcctccac taggacccca 60
   ttgccaacgg tga 73
<210> 152
   <211> 41
   <212> DNA
   <213> Homo sapiens
<400> 152
   gcggccgctt acagctttag ctcattggca attttggaag c 41
<210> 153
   <211> 68
   <212> DNA
   <213> Homo sapiens
<400> 153
   agatctgcca ccatgtaccc atacgatgtg ccagattacg cctcttcgcg gacggtgctg 60
   gccccggg 68
<210> 154
   <211> 25
   <212> DNA
   <213> Homo sapiens
<400> 154
   tgccctgaaa cagctccggg gcggc 25
<210> 155
   <211> 25
   <212> DNA
   <213> Homo sapiens
<400> 155
   gggatcgaag ctggacacgt tctgc 25
<210> 156
   <211> 39
   <212> DNA
   <213> Homo sapiens
<400> 156
   gcggccgctc acactccagg ggaatcggag cagccgggg 39
<210> 157
   <211> 15
   <212> PRT
   <213> Artificial
<220>
   <223> Artificial AMPK kinase substrate
<400> 157
   Ala Met Ala Arg Ala Ala Ser Ala Ala Ala Leu Ala Arg Arg Arg
   1               5                   10                  15
<210> 158
   <211> 23
   <212> PRT
   <213> Homo sapiens
<400> 158
   Leu Ser Asn Leu Tyr His Gln Gly Lys Phe Leu Gln Thr Phe Cys Gly
   1               5                   10                  15
   Ser Pro Leu Tyr Arg Arg Arg
               20